(19) Europäisches Patentamt
European Patent Office
Office européen des brevets

(11) EP 4 079 329 A1

(12) EUROPEAN PATENT APPLICATION
published in accordance with Art. 153(4) EPC

(43) Date of publication:
26.10.2022 Bulletin 2022/43

(21) Application number: 20901000.8

(22) Date of filing: 18.12.2020

(51) International Patent Classification (IPC):
*A61K 48/00* (2006.01) *A61P 21/00* (2006.01)
*A61P 43/00* (2006.01) *C12N 15/113* (2010.01)
*A61K 31/7088* (2006.01) *A61K 31/712* (2006.01)
*A61K 31/7125* (2006.01)

(52) Cooperative Patent Classification (CPC):
A61K 31/7088; A61K 31/712; A61K 31/7125;
A61K 48/00; A61P 21/00; A61P 43/00;
C12N 15/113

(86) International application number:
PCT/JP2020/047340

(87) International publication number:
WO 2021/125311 (24.06.2021 Gazette 2021/25)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 19.12.2019 JP 2019229763

(71) Applicants:
- Nippon Shinyaku Co., Ltd.
  Kyoto-shi
  Kyoto 601-8550 (JP)
- NATIONAL CENTER OF NEUROLOGY AND PSYCHIATRY
  Kodaira-shi
  Tokyo 187-8551 (JP)

(72) Inventors:
- WATANABE Naoki
  Tsukuba-shi, Ibaraki 305-0003 (JP)
- TONE Yuichiro
  Tsukuba-shi, Ibaraki 305-0003 (JP)
- TAKEDA Shin'ichi
  Kodaira-shi, Tokyo 187-8551 (JP)
- AOKI Yoshitsugu
  Kodaira-shi, Tokyo 187-8551 (JP)

(74) Representative: Hoffmann Eitle
Patent- und Rechtsanwälte PartmbB
Arabellastraße 30
81925 München (DE)

# (54) ANTISENSE NUCLEIC ACID ENABLING EXON SKIPPING

(57) The present specification provides an antisense oligomer capable of causing simultaneous skipping of a plurality of exons in pre-mRNA of interest, and a pharmaceutical composition comprising the oligomer. The present specification also provides an antisense oligomer or a pharmaceutically acceptable salt thereof, or hydrate thereof which causes simultaneous skipping of two or more numerically consecutive exons from pre-mRNA of interest, the antisense oligomer comprising a base sequence complementary to a base sequence of a region including the vicinity of a donor of any intron in the pre-mRNA of interest, or a region including the vicinity of an acceptor of any intron in the pre-mRNA of interest, or a partial base sequence thereof.



Processed by Luminess, 75001 PARIS (FR)

## Description

### Technical Field

The present invention relates to an antisense oligomer capable of causing simultaneous skipping of a plurality of exons in a target gene, and a pharmaceutical composition comprising the oligomer.

### Background Art

In recent years, exon skipping therapy has received attention which involves causing exon skipping of a gene having a mutation that causes a disease so that a protein having partial functions arises, thereby treating the disease. Examples of the disease that may be treated by such exon skipping therapy include Duchenne muscular dystrophy (DMD).

DMD is the most frequent form of hereditary progressive muscular disease that affects one in about 3,500 newborn boys. Although DMD patients exhibit motor functions rarely different from healthy humans in their infancy and childhood, muscle weakness is observed in children from around 4 to 5 years old. Then, muscle weakness in DMD patients progresses with age to the loss of ambulation by about 12 years old and death due to cardiac or respiratory insufficiency in their twenties. Therefore, it has been strongly desired to develop an effective therapeutic agent.

DMD is known to be caused by a mutation in the dystrophin gene. The dystrophin gene is located on X chromosome and is a huge gene consisting of 2.2 million DNA base pairs. DNA is transcribed into pre-mRNA, and introns are removed by splicing to form mRNA of 13,993 bases in which 79 exons are joined together. This mRNA is translated into 3,685 amino acids to produce the dystrophin protein. The dystrophin protein is associated with the maintenance of membrane stability in muscle cells and necessary to make muscle cells less fragile. Patients with DMD have a mutation in the dystrophin gene and hence, the functional dystrophin protein is rarely expressed in muscle cells of the patients. Therefore, the structure of muscle cells cannot be maintained at the time of muscle contraction in the body of the patients with DMD, leading to a large influx of calcium ions into muscle cells. Consequently, muscle cell necrosis and fibrosis progress so that muscle cells become progressively more difficult to regenerate.

Becker muscular dystrophy (BMD) is also caused by a mutation in the dystrophin gene. The symptoms involve muscle weakness but are typically mild and slow in the progress of muscle weakness, when compared to DMD. In many cases, its onset is in adulthood. Differences in clinical symptoms between DMD and BMD are considered to reside in whether the reading frame for amino acids on the translation of dystrophin mRNA into the dystrophin protein is disrupted by the mutation or not (Non Patent Literature 1). More specifically, in DMD, the presence of mutation shifts the amino acid reading frame so that the expression of functional dystrophin protein is abolished, whereas in BMD the dystrophin protein that is capable of functioning, though imperfectly, is produced because the amino acid reading frame is preserved, while part of the exons are deleted by the mutation.

Exon skipping is expected to serve as a method for treating DMD. This method involves modifying splicing to restore the amino acid reading frame of dystrophin mRNA and induce expression of the dystrophin protein having the function partially restored (Non Patent Literature 2). The amino acid sequence part to be translated from an exon, which is a target for exon skipping, will be lost. For this reason, the dystrophin protein expressed by this treatment becomes shorter than normal one but since the amino acid reading frame is maintained, the function to stabilize muscle cells is partially retained. Consequently, it is expected that exon skipping will lead DMD to the similar symptoms to that of BMD which is milder. The exon skipping approach has passed the animal tests using mice or dogs and now is currently assessed in clinical trials on human DMD patients.

The skipping of an exon can be induced by binding of antisense nucleic acids targeting site(s) surrounding either 5' or 3' splice site or both sites, or exon-internal sites. An exon will only be included in the mRNA when both splice sites thereof are recognized by the spliceosome complex. Thus, exon skipping can be induced by targeting the sites surrounding the splice sites with antisense nucleic acids. Furthermore, the binding of an SR protein rich in serine and arginine to an exonic splicing enhancer (ESE) is considered necessary for an exon to be recognized by the splicing mechanism. Accordingly, exon skipping can also be induced by targeting ESE.

Since a mutation of the dystrophin gene may vary depending on DMD patients, antisense nucleic acids need to be designed based on the site or type of respective genetic mutation. There is a plurality of reports on an antisense nucleic acid that induces exon skipping targeting one sequence of consecutive bases for a single exon in the dystrophin gene (Patent Literatures 1 to 6 and Non Patent Literatures 1 and 2). It has also been reported that when two types of antisense nucleic acids that target the same exon in the dystrophin gene are mixed and allowed to act (dual targeting), skipping activity may be enhanced as compared to use of each antisense nucleic acid alone (Patent Literature 7).

A method called multi-exon skipping has received attention which involves causing skipping of a plurality of exons (exon group), not one exon as described above. This method enables a wide range of mutations in the dystrophin gene to be treated by exon skipping. For example, exons 45 to 55 in the dystrophin gene are known as hot spots of genetic mutation, and it has been reported that skipping of these 11 exons enables about 60% of DMD patients having

a deletion mutation to be treated (Non Patent Literature 3). Most of patients congenitally lacking exons 45 to 55 are known to manifest no or mild symptoms, though developing BMD (Non Patent Literature 4). Thus, it is expected that drugs capable of inducing exon 45 to 55 skipping are promising as therapeutic agents for DMD.

For example, a method using antisense nucleic acids respectively targeting all exons in a region which is the target of exon skipping (Non Patent Literatures 5, 7, 8, and 10), and a method using antisense nucleic acids respectively targeting two different exons on the 3' side and 5' side of a region which is the target of exon skipping (Non Patent Literatures 6 and 9 and Patent Literature 8) have been reported as methods for inducing multi-exon skipping.

However, there is still no report stating that an antisense nucleic acid targeting a region including the vicinity of a donor of an intron or the vicinity of an acceptor induces multi-exon skipping.

Citation List

Patent Literature

Patent Literature 1: International Publication WO2004/048570
Patent Literature 2: International Publication WO2009/139630
Patent Literature 3: International Publication WO2010/048586
Patent Literature 4: U.S. Patent Publication Nos. 2010/0168212
Patent Literature 5: International Publication WO2011/057350
Patent Literature 6: International Publication WO2006/000057
Patent Literature 7: International Publication WO2007/135105
Patent Literature 8: International Publication WO2004/083446


Non Patent Literature

Non Patent Literature 1: Annemieke Aartsma-Rus et al., (2002) Neuromuscular Disorders 12: S71-S77
Non Patent Literature 2: Wilton S. D., e t al., Molecular Therapy 2007: 15: p. 1288-96
Non Patent Literature 3: Christophe Beroud et al., Human Mutation, 28(2), 2007, 196-202
Non Patent Literature 4: Yusuke Echigoya et al., Molecular Therapy-Nucleic Acids, 4(2), 2015, e225
Non Patent Literature 5: Yoshitsugu Aoki et al., PNAS, 109(34), 2012, 13763-13768
Non Patent Literature 6: Laura van Vliet et al., BMC Medical Genetics, 9, 105, 2008
Non Patent Literature 7: Joshua Lee et al., PLoS ONE, 13(5), e0197084, 2018
Non Patent Literature 8: Joshua Lee et al., Methods in Molecular Biology, 1828, 141-150, 2018
Non Patent Literature 9: Annemieke Aartsma-Rus et al, Am. J. Hum. Genet. 74(1), 83-92, 2004
Non Patent Literature 10: Yusuke Echigoya et al., Molecular Therapy, 27(11), 1-13, 2019


Summary of Invention

Technical Problem

Under the foregoing circumstances, medicaments for treating patients having various mutations by causing simultaneous skipping of a plurality of exons (exon group) in pre-mRNA of interest have been desired.

Solution to Problem

As a result of detailed studies of the technical contents of the above documents and the structure of the dystrophin gene, the present inventors have found that an antisense nucleic acid targeting a specific region in human dystrophin pre-mRNA is capable of causing simultaneous skipping of a plurality of exons among exons 45 to 55. Based on this finding, the present inventors have accomplished the present invention.

Specifically, the present invention is as follows.

[1] An antisense oligomer or a pharmaceutically acceptable salt thereof, or hydrate thereof which causes simultaneous skipping of any two or more numerically consecutive exons selected from the group consisting of the 45th exon to the 55th exon in human dystrophin pre-mRNA,

the antisense oligomer comprising a base sequence complementary to a base sequence of at least one region selected from the group consisting of regions R1 to R24 represented by

region Rn (wherein n is an odd number of 1 to 23) which consists of a base sequence of NX bases in the upstream direction from the 3' end of the NAth exon and a base sequence of NY bases in the downstream direction from the 5' end of the NBth intron in the human dystrophin pre-mRNA, and

region Rn (wherein n is an even number of 2 to 24) which consists of a base sequence of NX bases in the upstream direction from the 3' end of the NAth intron and a base sequence of NY bases in the downstream direction from the 5' end of the NBth exon in the human dystrophin pre-mRNA,

or a partial base sequence thereof, wherein

when n is 1, NA = 44, NB = 44, NX = 20, and NY = 400,
when n is 2, NA = 44, NB = 45, NX = 600, and NY = 50,
when n is 3, NA = 45, NB = 45, NX = 20, and NY = 400,
when n is 4, NA = 45, NB = 46, NX = 400, and NY = 50,
when n is 5, NA = 46, NB = 46, NX = 20, and NY = 400,
when n is 6, NA = 46, NB = 47, NX = 400, and NY = 50,
when n is 7, NA = 47, NB = 47, NX = 20, and NY = 400,
when n is 8, NA = 47, NB = 48, NX = 400, and NY = 50,
when n is 9, NA = 48, NB = 48, NX = 20, and NY = 400,
when n is 10, NA = 48, NB = 49, NX = 400, and NY = 50,
when n is 11, NA = 49, NB = 49, NX = 20, and NY = 400,
when n is 12, NA = 49, NB = 50, NX = 400, and NY = 50,
when n is 13, NA = 50, NB = 50, NX = 20, and NY = 400,
when n is 14, NA = 50, NB = 51, NX = 400, and NY = 50,
when n is 15, NA = 51, NB = 51, NX = 20, and NY = 400,
when n is 16, NA = 51, NB = 52, NX = 400, and NY = 50,
when n is 17, NA = 52, NB = 52, NX = 20, and NY = 400,
when n is 18, NA = 52, NB = 53, NX = 400, and NY = 50,
when n is 19, NA = 53, NB = 53, NX = 20, and NY = 400,
when n is 20, NA = 53, NB = 54, NX = 400, and NY = 50,
when n is 21, NA = 54, NB = 54, NX = 20, and NY = 400,
when n is 22, NA = 54, NB = 55, NX = 400, and NY = 50,
when n is 23, NA = 55, NB = 55, NX = 20, and NY = 400, or
when n is 24, NA = 55, NB = 56, NX = 400, and NY = 50.

[2] The antisense oligomer or the pharmaceutically acceptable salt thereof, or hydrate thereof according to [1], wherein

the region R1 is a region that consists of a base sequence of 20 bases in the upstream direction from the 3' end of the 44th exon and a base sequence of 400 bases in the downstream direction from the 5' end of the 44th intron in the human dystrophin pre-mRNA,

the region R2 is a region that consists of a base sequence of 600 bases in the upstream direction from the 3' end of the 44th intron and a base sequence of 50 bases in the downstream direction from the 5' end of the 45th exon in the human dystrophin pre-mRNA,

the region R3 is a region that consists of a base sequence of 20 bases in the upstream direction from the 3' end of the 45th exon and a base sequence of 400 bases in the downstream direction from the 5' end of the 45th intron in the human dystrophin pre-mRNA,

the region R4 is a region that consists of a base sequence of 400 bases in the upstream direction from the 3' end of the 45th intron and a base sequence of 50 bases in the downstream direction from the 5' end of the 46th exon in the human dystrophin pre-mRNA,

the region R5 is a region that consists of a base sequence of 20 bases in the upstream direction from the 3' end of the 46th exon and a base sequence of 400 bases in the downstream direction from the 5' end of the 46th intron in the human dystrophin pre-mRNA,

the region R6 is a region that consists of a base sequence of 400 bases in the upstream direction from the 3' end of the 46th intron and a base sequence of 50 bases in the downstream direction from the 5' end of the 47th exon in the human dystrophin pre-mRNA,

the region R7 is a region that consists of a base sequence of 20 bases in the upstream direction from the 3' end of the 47th exon and a base sequence of 400 bases in the downstream direction from the 5' end of the 47th intron in the human dystrophin pre-mRNA,

the region R8 is a region that consists of a base sequence of 400 bases in the upstream direction from the 3'

end of the 47th intron and a base sequence of 50 bases in the downstream direction from the 5' end of the 48th exon in the human dystrophin pre-mRNA,

the region R9 is a region that consists of a base sequence of 20 bases in the upstream direction from the 3' end of the 48th exon and a base sequence of 400 bases in the downstream direction from the 5' end of the 48th intron in the human dystrophin pre-mRNA,

the region R10 is a region that consists of a base sequence of 400 bases in the upstream direction from the 3' end of the 48th intron and a base sequence of 50 bases in the downstream direction from the 5' end of the 49th exon in the human dystrophin pre-mRNA,

the region R11 is a region that consists of a base sequence of 20 bases in the upstream direction from the 3' end of the 49th exon and a base sequence of 400 bases in the downstream direction from the 5' end of the 49th intron in the human dystrophin pre-mRNA,

the region R12 is a region that consists of a base sequence of 400 bases in the upstream direction from the 3' end of the 49th intron and a base sequence of 50 bases in the downstream direction from the 5' end of the 50th exon in the human dystrophin pre-mRNA,

the region R13 is a region that consists of a base sequence of 20 bases in the upstream direction from the 3' end of the 50th exon and a base sequence of 400 bases in the downstream direction from the 5' end of the 50th intron in the human dystrophin pre-mRNA,

the region R14 is a region that consists of a base sequence of 400 bases in the upstream direction from the 3' end of the 50th intron and a base sequence of 50 bases in the downstream direction from the 5' end of the 51st exon in the human dystrophin pre-mRNA,

the region R15 is a region that consists of a base sequence of 20 bases in the upstream direction from the 3' end of the 51st exon and a base sequence of 400 bases in the downstream direction from the 5' end of the 51st intron in the human dystrophin pre-mRNA,

the region R16 is a region that consists of a base sequence of 400 bases in the upstream direction from the 3' end of the 51st intron and a base sequence of 50 bases in the downstream direction from the 5' end of the 52nd exon in the human dystrophin pre-mRNA,

the region R17 is a region that consists of a base sequence of 20 bases in the upstream direction from the 3' end of the 52nd exon and a base sequence of 400 bases in the downstream direction from the 5' end of the 52nd intron in the human dystrophin pre-mRNA,

the region R18 is a region that consists of a base sequence of 400 bases in the upstream direction from the 3' end of the 52nd intron and a base sequence of 50 bases in the downstream direction from the 5' end of the 53rd exon in the human dystrophin pre-mRNA,

the region R19 is a region that consists of a base sequence of 20 bases in the upstream direction from the 3' end of the 53rd exon and a base sequence of 400 bases in the downstream direction from the 5' end of the 53rd intron in the human dystrophin pre-mRNA,

the region R20 is a region that consists of a base sequence of 400 bases in the upstream direction from the 3' end of the 53rd intron and a base sequence of 50 bases in the downstream direction from the 5' end of the 54th exon in the human dystrophin pre-mRNA,

the region R21 is a region that consists of a base sequence of 20 bases in the upstream direction from the 3' end of the 54th exon and a base sequence of 400 bases in the downstream direction from the 5' end of the 54th intron in the human dystrophin pre-mRNA,

the region R22 is a region that consists of a base sequence of 400 bases in the upstream direction from the 3' end of the 54th intron and a base sequence of 50 bases in the downstream direction from the 5' end of the 55th exon in the human dystrophin pre-mRNA,

the region R23 is a region that consists of a base sequence of 20 bases in the upstream direction from the 3' end of the 55th exon and a base sequence of 400 bases in the downstream direction from the 5' end of the 55th intron in the human dystrophin pre-mRNA, or

the region R24 is a region that consists of a base sequence of 400 bases in the upstream direction from the 3' end of the 55th intron and a base sequence of 50 bases in the downstream direction from the 5' end of the 56th exon in the human dystrophin pre-mRNA.

[3] The antisense oligomer or the pharmaceutically acceptable salt thereof, or hydrate thereof according to [1] or [2], wherein

the antisense oligomer comprises a base sequence complementary to

(a) any one base sequence selected from the group consisting of SEQ ID NOs: 233 to 256, 341 to 369, and 385 to 389,

(b) a base sequence that hybridizes under stringent conditions to a base sequence complementary to any one

base sequence selected from the group consisting of SEQ ID NOs: 233 to 256, 341 to 369, and 385 to 389,
(c) a base sequence that has at least 85% identity with any one base sequence selected from the group consisting of SEQ ID NOs: 233 to 256, 341 to 369, and 385 to 389, and has a length within ±15% of the length of the any one base sequence selected, or
(d) a partial base sequence of any one base sequence selected from the group consisting of the base sequences (a), (b), and (c).

[4] The antisense oligomer or the pharmaceutically acceptable salt thereof, or hydrate thereof according to any one of [1] to [3], wherein

the antisense oligomer is an antisense oligomer comprising two or more unit oligomers linked to each other, wherein
each of the unit oligomers comprises a base sequence complementary to a base sequence of any one region selected from the group consisting of the regions R1 to R24, or a partial base sequence thereof, and the respective base sequences of the unit oligomers are neither consecutive nor overlapped with each other.

[5] The antisense oligomer or the pharmaceutically acceptable salt thereof, or hydrate thereof according to any one of [1] to [3], wherein

the antisense oligomer is an antisense oligomer comprising two or more unit oligomers linked to each other, wherein
each of the unit oligomers comprises a base sequence complementary to

(a) any one base sequence selected from the group consisting of SEQ ID NOs: 233 to 256, 341 to 369, and 385 to 389,
(b) a base sequence that hybridizes under stringent conditions to a base sequence complementary to any one base sequence selected from the group consisting of SEQ ID NOs: 233 to 256, 341 to 369, and 385 to 389,
(c) a base sequence that has at least 85% identity with any one base sequence selected from the group consisting of SEQ ID NOs: 233 to 256, 341 to 369, and 385 to 389, and has a length within ±15% of the length of the any one base sequence selected, or
(d) a partial base sequence of any one base sequence selected from the group consisting of the base sequences (a), (b), and (c),

and the respective base sequences of the unit oligomers are neither consecutive nor overlapped with each other.

[6] The antisense oligomer or the pharmaceutically acceptable salt thereof, or hydrate thereof according to [4] or [5], wherein each of the unit oligomers comprises a base sequence complementary to a consecutive base sequence of 5- to 20-base length in the region.
[7] The antisense oligomer or the pharmaceutically acceptable salt thereof, or hydrate thereof according to any one of [1] to [3], wherein the antisense oligomer consists of (1) any one base sequence selected from the group consisting of SEQ ID NOs: 1 to 111, 113 to 177, 179, 180, 182, 183, 185 to 193, 195 to 198, 200 to 223, and 225 to 232, or (2) a base sequence that has at least 85% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±15% of the length of the any one base sequence selected.
[8] The antisense oligomer or the pharmaceutically acceptable salt thereof, or hydrate thereof according to any one of [1] to [3] and [7], wherein the antisense oligomer consists of any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232.
[9] The antisense oligomer or the pharmaceutically acceptable salt thereof, or hydrate thereof according to any one of [1] to [3], [7] and [8], wherein the antisense oligomer consists of any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228.
[10] The antisense oligomer or the pharmaceutically acceptable salt thereof, or hydrate thereof according to any one of [1] to [9], wherein the antisense oligomer is an oligonucleotide.
[11] The antisense oligomer or the pharmaceutically acceptable salt thereof, or hydrate thereof according to [10],

wherein the sugar moiety and/or the phosphate bond moiety of at least one nucleotide constituting the oligonucleotide is modified.

[12] The antisense oligomer or the pharmaceutically acceptable salt thereof, or hydrate thereof according to [10] or [11], wherein the sugar moiety of at least one nucleotide constituting the oligonucleotide is a ribose in which the 2'-OH group is replaced by any one selected from the group consisting of -OR, -R, -R'OR, -SH, -SR,-$NH_2$, -NHR, -$NR_2$, -$N_3$, -CN, -F, -Cl, -Br, and -I (wherein R is an alkyl or an aryl and R' is an alkylene).

[13] The antisense oligomer or the pharmaceutically acceptable salt thereof, or hydrate thereof according to any one of [10] to [12], wherein the phosphate bond moiety of at least one nucleotide constituting the oligonucleotide is any one selected from the group consisting of a phosphorothioate bond, a phosphorodithioate bond, an alkylphosphonate bond, a phosphoramidate bond and a boranophosphate bond.

[14] The antisense oligomer or the pharmaceutically acceptable salt thereof, or hydrate thereof according to any one of [1] to [9], wherein the antisense oligomer is a morpholino oligomer.

[15] The antisense oligomer or the pharmaceutically acceptable salt thereof, or hydrate thereof according to [14], wherein the antisense oligomer is a phosphorodiamidate morpholino oligomer.

[16] The antisense oligomer or the pharmaceutically acceptable salt thereof, or hydrate thereof according to [14] or [15], wherein the 5' end is any one of chemical formulae (1) to (3) below:

[Formula 1]

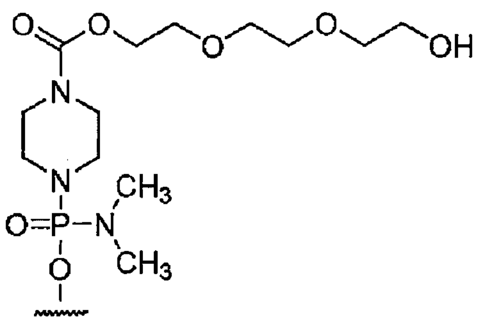

(1)

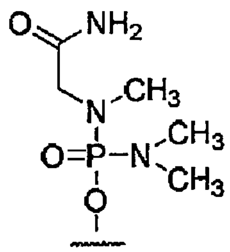

(2)

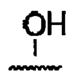

(3)

[17] A suppressor antisense oligomer or a pharmaceutically acceptable salt thereof, or hydrate thereof which suppresses single skipping of any one exon selected from the group consisting of the 45th exon to the 55th exon in human dystrophin pre-mRNA,
the suppressor antisense oligomer comprising a base sequence complementary to

(a) any one base sequence of a base sequence selected from the group consisting of SEQ ID NOs: 370 to 384,
(b) a base sequence that has at least 85% identity with any one base sequence of a base sequence selected from the group consisting of SEQ ID NOs: 370 to 384, and has a length within ±15% of the length of the any one base sequence selected, or
(c) a partial base sequence of the base sequence (a) or (b) .

[18] The suppressor antisense oligomer or the pharmaceutically acceptable salt thereof, or hydrate thereof according to [17], wherein the suppressor antisense oligomer consists of (1) any one base sequence selected from the group consisting of SEQ ID NOs: 257 to 275, or (2) a base sequence that has at least 85% identity with any one base sequence selected from the group consisting of SEQ ID NOs: 257 to 275, and has a length within ±15% of the length of the any one base sequence selected.

[19] The suppressor antisense oligomer or the pharmaceutically acceptable salt thereof, or hydrate thereof according to [17] or [18], wherein the suppressor antisense oligomer consists of any one base sequence selected from the group consisting of SEQ ID NOs: 260, 261, and 263.

[20] The suppressor antisense oligomer or the pharmaceutically acceptable salt thereof, or hydrate thereof according to any one of [17] to [19], wherein the suppressor antisense oligomer is an oligonucleotide.

[21] The suppressor antisense oligomer or the pharmaceutically acceptable salt thereof, or hydrate thereof according to [20], wherein the sugar moiety and/or the phosphate bond moiety of at least one nucleotide constituting the oligonucleotide is modified.

[22] The suppressor antisense oligomer or the pharmaceutically acceptable salt thereof, or hydrate thereof according

to [20] or [21], wherein the sugar moiety of at least one nucleotide constituting the oligonucleotide is a ribose in which the 2'-OH group is replaced by any one selected from the group consisting of -OR, -R, -R'OR, -SH, -SR, $-NH_2$, -NHR, $-NR_2$, $-N_3$, -CN, -F, -Cl, -Br, and -I (wherein R is an alkyl or an aryl and R' is an alkylene).
[23] The suppressor antisense oligomer or the pharmaceutically acceptable salt thereof, or hydrate thereof according to any one of [20] to [22], wherein the phosphate bond moiety of at least one nucleotide constituting the oligonucleotide is any one selected from the group consisting of a phosphorothioate bond, a phosphorodithioate bond, an alkylphosphonate bond, a phosphoramidate bond and a boranophosphate bond.
[24] The suppressor antisense oligomer or the pharmaceutically acceptable salt thereof, or hydrate thereof according to any one of [17] to [19], wherein the suppressor antisense oligomer is a morpholino oligomer.
[25] The suppressor antisense oligomer or the pharmaceutically acceptable salt thereof, or hydrate thereof according to [24], wherein the suppressor antisense oligomer is a phosphorodiamidate morpholino oligomer.
[26] The suppressor antisense oligomer or the pharmaceutically acceptable salt thereof, or hydrate thereof according to [24] or [25], wherein the 5' end is any one of chemical formulae (1) to (3) below:

[Formula 2]

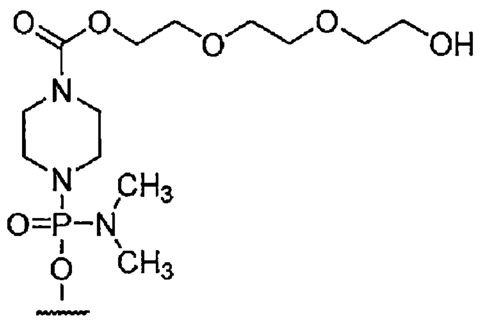

(1)

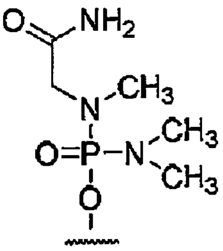

(2)

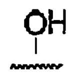

(3)

[27] A pharmaceutical composition comprising the antisense oligomer or the pharmaceutically acceptable salt thereof, or hydrate thereof according to any one of [1] to [16].
[28] The pharmaceutical composition according to [27], further comprising the suppressor antisense oligomer or the pharmaceutically acceptable salt thereof, or hydrate thereof according to any one of [17] to [26].
[29] A pharmaceutical composition comprising the antisense oligomer or the pharmaceutically acceptable salt thereof, or hydrate thereof according to any one of [1] to [16], and the suppressor antisense oligomer or the pharmaceutically acceptable salt thereof, or hydrate thereof according to any one of [17] to [26].
[30] The pharmaceutical composition according to [28] or [29], wherein

(1) the antisense oligomer is an oligomer consisting of SEQ ID NO: 75, and the suppressor antisense oligomer is an oligomer consisting of SEQ ID NO: 260,
(2) the antisense oligomer is an oligomer consisting of SEQ ID NO: 75, and the suppressor antisense oligomer is an oligomer consisting of SEQ ID NO: 261, or
(3) the antisense oligomer is an oligomer consisting of SEQ ID NO: 75, and the suppressor antisense oligomer is an oligomer consisting of SEQ ID NO: 263.

[31] The pharmaceutical composition according to any one of [27] to [30], further comprising a pharmaceutically acceptable carrier.
[32] The pharmaceutical composition according to any one of [27] to [31] for treatment of muscular dystrophy.
[33] The pharmaceutical composition according to any one of [27] to [32] for being administered to a human patient.
[34] A method for treatment of muscular dystrophy, which comprises administering to a patient with muscular dystrophy the antisense oligomer or the pharmaceutically acceptable salt thereof, or hydrate thereof according to any one of [1] to [16], or the pharmaceutical composition according to any one of [27] to [33].
[35] The method for treatment according to [34], wherein the patient with muscular dystrophy is a patient with a mutation that is amenable to exon 45 to 55 skipping in the dystrophin gene.
[36] The method for treatment according to [34] or [35], wherein the patient is a human.
[37] Use of the antisense oligomer or the pharmaceutically acceptable salt thereof, or hydrate thereof according to

any one of [1] to [16], or the pharmaceutical composition according to any one of [27] to [33] in manufacturing of a medicament for the treatment of muscular dystrophy.

[38] The antisense oligomer or the pharmaceutically acceptable salt thereof, or hydrate thereof according to any one of [1] to [16], or the pharmaceutical composition according to any one of [27] to [33] for use in the treatment of muscular dystrophy.

[39] The antisense oligomer or the pharmaceutically acceptable salt thereof, or hydrate thereof, or the pharmaceutical composition according to [38], wherein the treatment involves performing skipping of any two or more numerically consecutive exons selected from the group consisting of the 45th exon to the 55th exon in human dystrophin pre-mRNA.

[40] The antisense oligomer or the pharmaceutically acceptable salt thereof, or hydrate thereof, or the pharmaceutical composition according to [38] or [39], wherein in the treatment, a patient with muscular dystrophy is a human.

[41] A method for enhancing the efficiency of skipping of two or more numerically consecutive exons, which comprises inhibiting a splicing silencer sequence, a splice site sequence, or a branch site sequence of pre-mRNA of interest when the two or more numerically consecutive exons are skipped from the pre-mRNA of interest.

[42] The method according to [41], wherein the splicing silencer sequence is a recognition sequence of heterogeneous nuclear ribonucleoprotein A1 (hnRNPA1).

[43] The method according to [41] or [42], wherein the pre-mRNA of interest is human dystrophin pre-mRNA.

[44] The method according to any one of [41] to [43], wherein the two or more numerically consecutive exons are selected from the group consisting of the 45th exon to the 55th exon in human dystrophin pre-mRNA.

[45] The method according to [44], wherein the skipping of the two or more numerically consecutive exons of the pre-mRNA of interest is performed using the antisense oligomer or the pharmaceutically acceptable salt thereof, or hydrate thereof according to any one of [1] to [16].

[46] The method according to [44] or [45], wherein specific inhibition of the splicing silencer sequence, the splice site sequence, or the branch site sequence is performed using the suppressor antisense oligomer or the pharmaceutically acceptable salt thereof, or hydrate thereof according to any one of [17] to [26].

The present invention may also include the following embodiments.

[47] An antisense oligomer or a pharmaceutically acceptable salt thereof, or hydrate thereof which causes simultaneous skipping of two or more numerically consecutive exons from pre-mRNA of interest, the antisense oligomer comprising a base sequence complementary to a base sequence of any one region selected from the group consisting of

(1) a region that is constituted by a base sequence of 20 bases in the upstream direction from the 3' end of any exon and a base sequence of 400 bases in the downstream direction from the 5' end of an intron which is adjacent to the 3' end of the exon in the pre-mRNA of interest, or
(2) a region that is constituted by a base sequence of 400 bases or 600 bases in the upstream direction from the 3' end of any intron and a base sequence of 50 bases in the downstream direction from the 5' end of an exon which is adjacent to the 3' end of the intron in the pre-mRNA of interest,

or a partial base sequence thereof.

[48] The antisense oligomer or the pharmaceutically acceptable salt thereof, or hydrate thereof according to [47], wherein the pre-mRNA of interest is human dystrophin pre-mRNA.

Advantageous Effects of Invention

The present invention provides an antisense oligomer that causes simultaneous skipping of a plurality of exons in a target. Another embodiment of the present invention provides a pharmaceutical composition for treating patients having various mutations by causing simultaneous skipping of a plurality of exons in pre-mRNA of interest. An alternative embodiment of the present invention provides a suppressor antisense oligomer that suppresses single skipping of an exon in pre-mRNA of interest or a pharmaceutical composition comprising the oligomer. An alternative embodiment of the present invention enables simultaneous skipping of exons 45 to 55 in human dystrophin pre-mRNA to be caused with high efficiency.

Brief Description of Drawings

[Figure 1] Figure 1 is a diagram showing results of studying exon 45 to 55 skipping in human dystrophin pre-mRNA in RD cells (human rhabdomyosarcoma cells) by RT-PCR.

[Figure 2] Figure 2 is a diagram showing results of studying exon 45 to 55 skipping in human dystrophin pre-mRNA in RD cells by RT-PCR.

[Figure 3] Figure 3 is a diagram showing results of studying exon 45 to 55 skipping in human dystrophin pre-mRNA in RD cells by RT-PCR.

[Figure 4] Figure 4 is a diagram showing results of studying exon 45 to 55 skipping in human dystrophin pre-mRNA in RD cells by RT-PCR.

[Figure 5] Figure 5 is a diagram showing results of studying exon 45 to 55 skipping in human dystrophin pre-mRNA in RD cells by RT-PCR.

[Figure 6] Figure 6 is a diagram showing results of studying exon 45 to 55 skipping in human dystrophin pre-mRNA in RD cells by RT-PCR.

[Figure 7] Figure 7 is a diagram showing results of studying exon 45 to 55 skipping in human dystrophin pre-mRNA in RD cells by RT-PCR.

[Figure 8] Figure 8 is a diagram showing results of studying exon 45 to 55 skipping in human dystrophin pre-mRNA in RD cells by RT-PCR.

[Figure 9] Figure 9 is a diagram showing results of studying exon 45 to 55 skipping in human dystrophin pre-mRNA in RD cells by RT-PCR.

[Figure 10] Figure 10 is a diagram showing results of studying exon 45 to 55 skipping in human dystrophin pre-mRNA in RD cells by RT-PCR.

[Figure 11] Figure 11 is a diagram showing results of studying exon 45 to 55 skipping in human dystrophin pre-mRNA in RD cells by RT-PCR.

[Figure 12] Figure 12 is a diagram showing results of studying exon 45 to 55 skipping in human dystrophin pre-mRNA in RD cells by RT-PCR.

[Figure 13] Figure 13 is a diagram showing results of studying exon 45 to 55 skipping in human dystrophin pre-mRNA in RD cells by RT-PCR.

[Figure 14] Figure 14 is a diagram showing results of studying exon 45 to 55 skipping in human dystrophin pre-mRNA in RD cells by RT-PCR.

[Figure 15] Figure 15 is a diagram showing results of studying exon 45 to 55 skipping in human dystrophin pre-mRNA in RD cells by RT-PCR.

[Figure 16] Figure 16 is a diagram showing results of studying exon 45 to 55 skipping in human dystrophin pre-mRNA in RD cells by RT-PCR.

[Figure 17] Figure 17 is a diagram showing results of studying exon 45 to 55 multi-skipping and exon 45 single skipping in human dystrophin pre-mRNA in RD cells by RT-PCR.

[Figure 18] Figure 18 is a diagram showing results of studying exon 45 to 55 multi-skipping and exon 45 single skipping in human dystrophin pre-mRNA in RD cells by RT-PCR.

[Figure 19] Figure 19 is a diagram showing results of studying multi-skipping of exons selected from exons 45 to 49 and exon 45 single skipping in human dystrophin pre-mRNA in RD cells by RT-PCR.

[Figure 20] Figure 20 is a diagram showing results of studying multi-skipping of exons selected from exons 45 to 49 in human dystrophin pre-mRNA in RD cells by RT-PCR, and calculating the total amounts of the respective skips of exons 45 and 46, exons 45 to 47, exons 45 to 48, and exons 45 to 49 (total multi-skipping products). Each of these skips is considered to have therapeutic effects.

[Figure 21] Figure 21 is a diagram showing results of studying multi-skipping of exons selected from exons 45 to 52 and exon 45 single skipping in human dystrophin pre-mRNA in RD cells by RT-PCR.

[Figure 22] Figure 22 is a diagram showing results of studying multi-skipping of exons selected from exons 45 to 52 in human dystrophin pre-mRNA in RD cells by RT-PCR, and calculating the total amounts of the respective skips of exons 45 to 47, exons 45 to 48, exons 45 to 49, exons 45 to 50, exons 45 to 51, and exons 45 to 52 (left) or the total amounts of the respective skips of exons 45 to 47, exons 45 to 48, exons 45 to 49, and exons 45 to 51 (right). Each of the skips of exons 45 to 47, exons 45 to 48, exons 45 to 49, and exons 45 to 51 has therapeutic effects.

## Description of Embodiments

Hereinafter, the present invention is described in detail. The embodiments described below are intended to be presented by way of example merely to describe the invention but not limited only to the following embodiments. The present invention may be implemented in various ways without departing from the gist of the invention.

### 1. Antisense oligomer

The present invention provides an antisense oligomer or a pharmaceutically acceptable salt thereof, or hydrate thereof which causes simultaneous skipping of two or more numerically consecutive exons from pre-mRNA of interest,

the antisense oligomer comprising a base sequence complementary to a base sequence of any one region selected from the group consisting of

(1) a region that is constituted by a base sequence of 20 bases in the upstream direction from the 3' end of any exon and a base sequence of 400 bases in the downstream direction from the 5' end of an intron which is adjacent to the 3' end of the exon in the pre-mRNA of interest, or
(2) a region that is constituted by a base sequence of 400 bases or 600 bases in the upstream direction from the 3' end of any intron and a base sequence of 50 bases in the downstream direction from the 5' end of an exon which is adjacent to the 3' end of the intron in the pre-mRNA of interest,

or a partial base sequence thereof.

As used herein, the term "cause simultaneous skipping" of two or more numerically consecutive exons includes not only removal of the respective exons from pre-mRNA at completely the same timings but also sequential removal of the respective exons within a period from pre-mRNA to mature mRNA. Specifically, the term "cause simultaneous skipping" of two or more numerically consecutive exons refers to removal of a plurality of (two or more) numerically consecutive exons from pre-mRNA.

As used herein, the term "two or more numerically consecutive exons" means a plurality of exons that increase one by one in exon number among exons (the total number of exons is referred to as Texon) included in pre-mRNA of interest. The exon number means a number assigned to exons in order from the 5' end to the 3' end with an exon at the most upstream position of pre-mRNA defined as the first exon, followed by the second, the third, .... In the case of skipping of two or more numerically consecutive exons in a certain gene, its exon numbers $a_1, \ldots, a_j$ can be represented by the sequence $\{a_j\}$. The general term $a_j$ in the sequence $\{a_j\}$ is represented by the expression below:

[Expression 1]

$$a_j = m + (j - 1)$$

wherein m is a given natural number that satisfies $1 \leq m \leq (\text{Texon} - 1)$, and j is a natural number that satisfies $2 \leq (m + j) \leq \text{Texon} + 1$.

When the pre-mRNA of interest is, for example, human dystrophin pre-mRNA, Texon is 79.

In a certain embodiment, j is a given natural number selected from 1 to 11. In another embodiment, j is 11, j is 10, j is 9, j is 8, j is 7, j is 6, j is 5, j is 4, j is 3, j is 2, or j is 1.

As used herein, the term "gene" is intended to mean a genomic gene and also include cDNA, pre-mRNA and mRNA. Preferably, the gene is pre-mRNA. As used herein, the term "pre-mRNA" is an RNA molecule comprising an exon and an intron transcribed from a target gene on the genome and is a mRNA precursor.

In a certain embodiment, the present invention provides an antisense oligomer or a pharmaceutically acceptable salt thereof, or hydrate thereof which causes simultaneous skipping of any two or more numerically consecutive exons selected from the group consisting of the 45th exon to the 55th exon in human dystrophin pre-mRNA (hereinafter, the antisense oligomer and the pharmaceutically acceptable salt thereof and the hydrate of the antisense oligomer or the salt are also collectively referred to as the "antisense oligomer of the present invention"; the antisense oligomer of the present invention may refer to any of the antisense oligomer or the pharmaceutically acceptable salt thereof, or hydrate thereof).

The human dystrophin pre-mRNA is an RNA molecule comprising an exon and an intron transcribed from the human dystrophin gene on the genome and is a mRNA precursor. Those skilled in the art can obtain information on the base sequence of the human dystrophin pre-mRNA by analogy from the genomic sequence of the human dystrophin gene (GenBank Accession Nos. NG_012232.1).

In the human genome, the human dystrophin gene locates at locus Xp21.2. The human dystrophin gene has a size of about 3.0 Mbp and is the largest gene among known human genes. However, the coding regions of the human dystrophin gene are only about 14 kb, distributed as 79 exons throughout the human dystrophin gene (Roberts, RG, et al., Genomics, 16: 536-538 (1993)). The pre-mRNA, which is the transcript of the human dystrophin gene, undergoes splicing to form mature mRNA of about 14 kb. The base sequence of mature mRNA of human wild-type dystrophin gene is known (GenBank Accession Nos. NM_004006).

The antisense oligomer of the present invention according to the foregoing embodiment targets at least any one region selected from the group consisting of regions R1 to R24 represented by

region Rn (wherein n is an odd number of 1 to 23) which consists of a base sequence of NX bases in the upstream direction from the 3' end of the NAth exon and a base sequence of NY bases in the downstream direction from the

5’ end of the NBth intron in the human dystrophin pre-mRNA, and
region Rn (wherein n is an even number of 2 to 24) which consists of a base sequence of NX bases in the upstream direction from the 3’ end of the NAth intron and a base sequence of NY bases in the downstream direction from the 5’ end of the NBth exon in the human dystrophin pre-mRNA.

Hereinafter, the regions R1 to R24 are each or collectively referred to as a "target region of the antisense oligomer of the present invention" or interchangeably the "target region of the present invention".
As used herein, the term "targeting" means that an intended base sequence is a base sequence complementary to the base sequence of a target region or a partial base sequence of the target sequence.
The antisense oligomer of the present invention according to the foregoing embodiment comprises a base sequence complementary to a base sequence of at least any one region selected from the group consisting of the target regions R1 to R24 of the present invention, or a partial base sequence thereof.
Examples of the target region Rn of the present invention according to the foregoing embodiment include regions wherein

when n is 1, NA = 44, NB = 44, NX = 20, and NY = 400,
when n is 2, NA = 44, NB = 45, NX = 600, and NY = 50,
when n is 3, NA = 45, NB = 45, NX = 20, and NY = 400,
when n is 4, NA = 45, NB = 46, NX = 400, and NY = 50,
when n is 5, NA = 46, NB = 46, NX = 20, and NY = 400,
when n is 6, NA = 46, NB = 47, NX = 400, and NY = 50,
when n is 7, NA = 47, NB = 47, NX = 20, and NY = 400,
when n is 8, NA = 47, NB = 48, NX = 400, and NY = 50,
when n is 9, NA = 48, NB = 48, NX = 20, and NY = 400,
when n is 10, NA = 48, NB = 49, NX = 400, and NY = 50,
when n is 11, NA = 49, NB = 49, NX = 20, and NY = 400,
when n is 12, NA = 49, NB = 50, NX = 400, and NY = 50,
when n is 13, NA = 50, NB = 50, NX = 20, and NY = 400,
when n is 14, NA = 50, NB = 51, NX = 400, and NY = 50,
when n is 15, NA = 51, NB = 51, NX = 20, and NY = 400,
when n is 16, NA = 51, NB = 52, NX = 400, and NY = 50,
when n is 17, NA = 52, NB = 52, NX = 20, and NY = 400,
when n is 18, NA = 52, NB = 53, NX = 400, and NY = 50,
when n is 19, NA = 53, NB = 53, NX = 20, and NY = 400,
when n is 20, NA = 53, NB = 54, NX = 400, and NY = 50,
when n is 21, NA = 54, NB = 54, NX = 20, and NY = 400,
when n is 22, NA = 54, NB = 55, NX = 400, and NY = 50,
when n is 23, NA = 55, NB = 55, NX = 20, and NY = 400, or
when n is 24, NA = 55, NB = 56, NX = 400, and NY = 50.

In another embodiment, examples of the target region Rn of the present invention include, but not limited to, regions wherein

when n is 1, NA = 44, NB = 44, NX = 20, and NY = 400,
when n is 2, NA = 44, NB = 45, NX = 600, and NY = 19,
when n is 3, NA = 45, NB = 45, NX = 20, and NY = 400,
when n is 4, NA = 45, NB = 46, NX = 400, and NY = 50,
when n is 5, NA = 46, NB = 46, NX = 20, and NY = 400,
when n is 6, NA = 46, NB = 47, NX = 400, and NY = 37,
when n is 7, NA = 47, NB = 47, NX = 20, and NY = 400,
when n is 8, NA = 47, NB = 48, NX = 400, and NY = 19,
when n is 9, NA = 48, NB = 48, NX = 20, and NY = 400,
when n is 10, NA = 48, NB = 49, NX = 400, and NY = 42,
when n is 11, NA = 49, NB = 49, NX = 20, and NY = 400,
when n is 12, NA = 49, NB = 50, NX = 400, and NY = 44,
when n is 13, NA = 50, NB = 50, NX = 20, and NY = 400,
when n is 14, NA = 50, NB = 51, NX = 400, and NY = 25,
when n is 15, NA = 51, NB = 51, NX = 20, and NY = 400,

when n is 16, NA = 51, NB = 52, NX = 400, and NY = 24,
when n is 17, NA = 52, NB = 52, NX = 20, and NY = 400,
when n is 18, NA = 52, NB = 53, NX = 400, and NY = 34,
when n is 19, NA = 53, NB = 53, NX = 20, and NY = 400,
when n is 20, NA = 53, NB = 54, NX = 400, and NY = 43,
when n is 21, NA = 54, NB = 54, NX = 20, and NY = 400,
when n is 22, NA = 54, NB = 55, NX = 400, and NY = 25,
when n is 23, NA = 55, NB = 55, NX = 20, and NY = 400, or
when n is 24, NA = 55, NB = 56, NX = 400, and NY = 50.

In another embodiment, R1 to R24 which are examples of the target region Rn of the present invention are, but not limited to, as follows:

for example, in a certain embodiment,
the region R1 is a region that consists of a base sequence of 20 bases in the upstream direction from the 3' end of the 44th exon and a base sequence of 400 bases in the downstream direction from the 5' end of the 44th intron in the human dystrophin pre-mRNA,
the region R2 is a region that consists of a base sequence of 600 bases in the upstream direction from the 3' end of the 44th intron and a base sequence of 50 bases in the downstream direction from the 5' end of the 45th exon in the human dystrophin pre-mRNA,
the region R3 is a region that consists of a base sequence of 20 bases in the upstream direction from the 3' end of the 45th exon and a base sequence of 400 bases in the downstream direction from the 5' end of the 45th intron in the human dystrophin pre-mRNA,
the region R4 is a region that consists of a base sequence of 400 bases in the upstream direction from the 3' end of the 45th intron and a base sequence of 50 bases in the downstream direction from the 5' end of the 46th exon in the human dystrophin pre-mRNA,
the region R5 is a region that consists of a base sequence of 20 bases in the upstream direction from the 3' end of the 46th exon and a base sequence of 400 bases in the downstream direction from the 5' end of the 46th intron in the human dystrophin pre-mRNA,
the region R6 is a region that consists of a base sequence of 400 bases in the upstream direction from the 3' end of the 46th intron and a base sequence of 50 bases in the downstream direction from the 5' end of the 47th exon in the human dystrophin pre-mRNA,
the region R7 is a region that consists of a base sequence of 20 bases in the upstream direction from the 3' end of the 47th exon and a base sequence of 400 bases in the downstream direction from the 5' end of the 47th intron in the human dystrophin pre-mRNA,
the region R8 is a region that consists of a base sequence of 400 bases in the upstream direction from the 3' end of the 47th intron and a base sequence of 50 bases in the downstream direction from the 5' end of the 48th exon in the human dystrophin pre-mRNA,
the region R9 is a region that consists of a base sequence of 20 bases in the upstream direction from the 3' end of the 48th exon and a base sequence of 400 bases in the downstream direction from the 5' end of the 48th intron in the human dystrophin pre-mRNA,
the region R10 is a region that consists of a base sequence of 400 bases in the upstream direction from the 3' end of the 48th intron and a base sequence of 50 bases in the downstream direction from the 5' end of the 49th exon in the human dystrophin pre-mRNA,
the region R11 is a region that consists of a base sequence of 20 bases in the upstream direction from the 3' end of the 49th exon and a base sequence of 400 bases in the downstream direction from the 5' end of the 49th intron in the human dystrophin pre-mRNA,
the region R12 is a region that consists of a base sequence of 400 bases in the upstream direction from the 3' end of the 49th intron and a base sequence of 50 bases in the downstream direction from the 5' end of the 50th exon in the human dystrophin pre-mRNA,
the region R13 is a region that consists of a base sequence of 20 bases in the upstream direction from the 3' end of the 50th exon and a base sequence of 400 bases in the downstream direction from the 5' end of the 50th intron in the human dystrophin pre-mRNA,
the region R14 is a region that consists of a base sequence of 400 bases in the upstream direction from the 3' end of the 50th intron and a base sequence of 50 bases in the downstream direction from the 5' end of the 51st exon in the human dystrophin pre-mRNA,
the region R15 is a region that consists of a base sequence of 20 bases in the upstream direction from the 3' end of the 51st exon and a base sequence of 400 bases in the downstream direction from the 5' end of the 51st intron

in the human dystrophin pre-mRNA,
the region R16 is a region that consists of a base sequence of 400 bases in the upstream direction from the 3' end of the 51st intron and a base sequence of 50 bases in the downstream direction from the 5' end of the 52nd exon in the human dystrophin pre-mRNA,
the region R17 is a region that consists of a base sequence of 20 bases in the upstream direction from the 3' end of the 52nd exon and a base sequence of 400 bases in the downstream direction from the 5' end of the 52nd intron in the human dystrophin pre-mRNA,
the region R18 is a region that consists of a base sequence of 400 bases in the upstream direction from the 3' end of the 52nd intron and a base sequence of 50 bases in the downstream direction from the 5' end of the 53rd exon in the human dystrophin pre-mRNA,
the region R19 is a region that consists of a base sequence of 20 bases in the upstream direction from the 3' end of the 53rd exon and a base sequence of 400 bases in the downstream direction from the 5' end of the 53rd intron in the human dystrophin pre-mRNA,
the region R20 is a region that consists of a base sequence of 400 bases in the upstream direction from the 3' end of the 53rd intron and a base sequence of 50 bases in the downstream direction from the 5' end of the 54th exon in the human dystrophin pre-mRNA,
the region R21 is a region that consists of a base sequence of 20 bases in the upstream direction from the 3' end of the 54th exon and a base sequence of 400 bases in the downstream direction from the 5' end of the 54th intron in the human dystrophin pre-mRNA,
the region R22 is a region that consists of a base sequence of 400 bases in the upstream direction from the 3' end of the 54th intron and a base sequence of 50 bases in the downstream direction from the 5' end of the 55th exon in the human dystrophin pre-mRNA,
the region R23 is a region that consists of a base sequence of 20 bases in the upstream direction from the 3' end of the 55th exon and a base sequence of 400 bases in the downstream direction from the 5' end of the 55th intron in the human dystrophin pre-mRNA, or
the region R24 is a region that consists of a base sequence of 400 bases in the upstream direction from the 3' end of the 55th intron and a base sequence of 50 bases in the downstream direction from the 5' end of the 56th exon in the human dystrophin pre-mRNA.

In an alternative embodiment, R1 to R24 which are examples of the target region Rn of the present invention are, but not limited to, as follows:

for example, in a certain embodiment,
the region R1 is a region that consists of a base sequence of 20 bases in the upstream direction from the 3' end of the 44th exon and a base sequence of 400 bases in the downstream direction from the 5' end of the 44th intron in the human dystrophin pre-mRNA,
the region R2 is a region that consists of a base sequence of 600 bases in the upstream direction from the 3' end of the 44th intron and a base sequence of 19 bases in the downstream direction from the 5' end of the 45th exon in the human dystrophin pre-mRNA,
the region R3 is a region that consists of a base sequence of 20 bases in the upstream direction from the 3' end of the 45th exon and a base sequence of 400 bases in the downstream direction from the 5' end of the 45th intron in the human dystrophin pre-mRNA,
the region R4 is a region that consists of a base sequence of 400 bases in the upstream direction from the 3' end of the 45th intron and a base sequence of 50 bases in the downstream direction from the 5' end of the 46th exon in the human dystrophin pre-mRNA,
the region R5 is a region that consists of a base sequence of 20 bases in the upstream direction from the 3' end of the 46th exon and a base sequence of 400 bases in the downstream direction from the 5' end of the 46th intron in the human dystrophin pre-mRNA,
the region R6 is a region that consists of a base sequence of 400 bases in the upstream direction from the 3' end of the 46th intron and a base sequence of 37 bases in the downstream direction from the 5' end of the 47th exon in the human dystrophin pre-mRNA,
the region R7 is a region that consists of a base sequence of 20 bases in the upstream direction from the 3' end of the 47th exon and a base sequence of 400 bases in the downstream direction from the 5' end of the 47th intron in the human dystrophin pre-mRNA,
the region R8 is a region that consists of a base sequence of 400 bases in the upstream direction from the 3' end of the 47th intron and a base sequence of 19 bases in the downstream direction from the 5' end of the 48th exon in the human dystrophin pre-mRNA,
the region R9 is a region that consists of a base sequence of 20 bases in the upstream direction from the 3' end of

the 48th exon and a base sequence of 400 bases in the downstream direction from the 5' end of the 48th intron in the human dystrophin pre-mRNA,
the region R10 is a region that consists of a base sequence of 400 bases in the upstream direction from the 3' end of the 48th intron and a base sequence of 42 bases in the downstream direction from the 5' end of the 49th exon in the human dystrophin pre-mRNA,
the region R11 is a region that consists of a base sequence of 20 bases in the upstream direction from the 3' end of the 49th exon and a base sequence of 400 bases in the downstream direction from the 5' end of the 49th intron in the human dystrophin pre-mRNA,
the region R12 is a region that consists of a base sequence of 400 bases in the upstream direction from the 3' end of the 49th intron and a base sequence of 44 bases in the downstream direction from the 5' end of the 50th exon in the human dystrophin pre-mRNA,
the region R13 is a region that consists of a base sequence of 20 bases in the upstream direction from the 3' end of the 50th exon and a base sequence of 400 bases in the downstream direction from the 5' end of the 50th intron in the human dystrophin pre-mRNA,
the region R14 is a region that consists of a base sequence of 400 bases in the upstream direction from the 3' end of the 50th intron and a base sequence of 25 bases in the downstream direction from the 5' end of the 51st exon in the human dystrophin pre-mRNA,
the region R15 is a region that consists of a base sequence of 20 bases in the upstream direction from the 3' end of the 51st exon and a base sequence of 400 bases in the downstream direction from the 5' end of the 51st intron in the human dystrophin pre-mRNA,
the region R16 is a region that consists of a base sequence of 400 bases in the upstream direction from the 3' end of the 51st intron and a base sequence of 24 bases in the downstream direction from the 5' end of the 52nd exon in the human dystrophin pre-mRNA,
the region R17 is a region that consists of a base sequence of 20 bases in the upstream direction from the 3' end of the 52nd exon and a base sequence of 400 bases in the downstream direction from the 5' end of the 52nd intron in the human dystrophin pre-mRNA,
the region R18 is a region that consists of a base sequence of 400 bases in the upstream direction from the 3' end of the 52nd intron and a base sequence of 34 bases in the downstream direction from the 5' end of the 53rd exon in the human dystrophin pre-mRNA,
the region R19 is a region that consists of a base sequence of 20 bases in the upstream direction from the 3' end of the 53rd exon and a base sequence of 400 bases in the downstream direction from the 5' end of the 53rd intron in the human dystrophin pre-mRNA,
the region R20 is a region that consists of a base sequence of 400 bases in the upstream direction from the 3' end of the 53rd intron and a base sequence of 43 bases in the downstream direction from the 5' end of the 54th exon in the human dystrophin pre-mRNA,
the region R21 is a region that consists of a base sequence of 20 bases in the upstream direction from the 3' end of the 54th exon and a base sequence of 400 bases in the downstream direction from the 5' end of the 54th intron in the human dystrophin pre-mRNA,
the region R22 is a region that consists of a base sequence of 400 bases in the upstream direction from the 3' end of the 54th intron and a base sequence of 25 bases in the downstream direction from the 5' end of the 55th exon in the human dystrophin pre-mRNA,
the region R23 is a region that consists of a base sequence of 20 bases in the upstream direction from the 3' end of the 55th exon and a base sequence of 400 bases in the downstream direction from the 5' end of the 55th intron in the human dystrophin pre-mRNA, or
the region R24 is a region that consists of a base sequence of 400 bases in the upstream direction from the 3' end of the 55th intron and a base sequence of 50 bases in the downstream direction from the 5' end of the 56th exon in the human dystrophin pre-mRNA.

In an alternative embodiment, R1 to R24 which are examples of the target region Rn of the present invention are, but not limited to, as follows:

(A1) Region R1
The region R1 is a region indicated by the range of -20 bases to +400 bases when the boundary between the 3' end of exon 44 and the 5' end of intron 44 is defined as basing point 0, a base sequence region on the 5' side (upstream) from the basing point is indicated by "+", and a base sequence region on the 3' side (downstream) therefrom is indicated by "-" (minus). In this respect, the region indicated by the range of -20 bases to -1 base belongs to exon 44, and the region indicated by the range of +1 base to +400 bases belongs to intron 44.
(A2) Region R2

The region R2 is a region indicated by the range of -600 bases to +50 bases when the boundary between the 3' end of intron 44 and the 5' end of exon 45 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -600 bases to -1 base belongs to intron 44, and the region indicated by the range of +1 base to +50 bases belongs to exon 45.

(A3) Region R3

The region R3 is a region indicated by the range of -20 bases to +400 bases when the boundary between the 3' end of exon 45 and the 5' end of intron 45 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 45, and the region indicated by the range of +1 base to +400 bases belongs to intron 45.

(A4) Region R4

The region R4 is a region indicated by the range of -400 bases to +50 bases when the boundary between the 3' end of intron 45 and the 5' end of exon 46 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 45, and the region indicated by the range of +1 base to +50 bases belongs to exon 46.

(A5) Region R5

The region R5 is a region indicated by the range of -20 bases to +400 bases when the boundary between the 3' end of exon 46 and the 5' end of intron 46 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 46, and the region indicated by the range of +1 base to +400 bases belongs to intron 46.

(A6) Region R6

The region R6 is a region indicated by the range of -400 bases to +50 bases when the boundary between the 3' end of intron 46 and the 5' end of exon 47 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 46, and the region indicated by the range of +1 base to +50 bases belongs to exon 47.

(A7) Region R7

The region R7 is a region indicated by the range of -20 bases to +400 bases when the boundary between the 3' end of exon 47 and the 5' end of intron 47 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 47, and the region indicated by the range of +1 base to +400 bases belongs to intron 47.

(A8) Region R8

The region R8 is a region indicated by the range of -400 bases to +50 bases when the boundary between the 3' end of intron 47 and the 5' end of exon 48 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 47, and the region indicated by the range of +1 base to +50 bases belongs to exon 48.

(A9) Region R9

The region R9 is a region indicated by the range of -20 bases to +400 bases when the boundary between the 3' end of exon 48 and the 5' end of intron 48 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 48, and the region indicated by the range of +1 base to +400 bases belongs to intron 48.

(A10) Region R10

The region R10 is a region indicated by the range of -400 bases to +50 bases when the boundary between the 3' end of intron 48 and the 5' end of exon 49 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 48, and the region indicated by the range of +1 base to +50 bases belongs to exon 49.

(A11) Region R11

The region R11 is a region indicated by the range of -20 bases to +400 bases when the boundary between the 3' end of exon 49 and the 5' end of intron 49 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to

exon 49, and the region indicated by the range of +1 base to +400 bases belongs to intron 49.

(A12) Region R12

The region R12 is a region indicated by the range of -400 bases to +50 bases when the boundary between the 3' end of intron 49 and the 5' end of exon 50 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 49, and the region indicated by the range of +1 base to +50 bases belongs to exon 50.

(A13) Region R13

The region R13 is a region indicated by the range of -20 bases to +400 bases when the boundary between the 3' end of exon 50 and the 5' end of intron 50 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 50, and the region indicated by the range of +1 base to +400 bases belongs to intron 50.

(A14) Region R14

The region R14 is a region indicated by the range of -400 bases to +50 bases when the boundary between the 3' end of intron 50 and the 5' end of exon 51 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 50, and the region indicated by the range of +1 base to +50 bases belongs to exon 51.

(A15) Region R15

The region R15 is a region indicated by the range of -20 bases to +400 bases when the boundary between the 3' end of exon 51 and the 5' end of intron 51 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 51, and the region indicated by the range of +1 base to +400 bases belongs to intron 51.

(A16) Region R16

The region R16 is a region indicated by the range of -400 bases to +50 bases when the boundary between the 3' end of intron 51 and the 5' end of exon 52 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 51, and the region indicated by the range of +1 base to +50 bases belongs to exon 52.

(A17) Region R17

The region R17 is a region indicated by the range of -20 bases to +400 bases when the boundary between the 3' end of exon 52 and the 5' end of intron 52 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 52, and the region indicated by the range of +1 base to +400 bases belongs to intron 52.

(A18) Region R18

The region R18 is a region indicated by the range of -400 bases to +50 bases when the boundary between the 3' end of intron 52 and the 5' end of exon 53 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 52, and the region indicated by the range of +1 base to +50 bases belongs to exon 53.

(A19) Region R19

The region R19 is a region indicated by the range of -20 bases to +400 bases when the boundary between the 3' end of exon 53 and the 5' end of intron 53 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 53, and the region indicated by the range of +1 base to +400 bases belongs to intron 53.

(A20) Region R20

The region R20 is a region indicated by the range of -400 bases to +50 bases when the boundary between the 3' end of intron 53 and the 5' end of exon 54 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 53, and the region indicated by the range of +1 base to +50 bases belongs to exon 54.

(A21) Region R21

The region R21 is a region indicated by the range of -20 bases to +400 bases when the boundary between the 3' end of exon 54 and the 5' end of intron 54 is defined as basing point 0, a base sequence region on the 5' side

(upstream) from the basing point is indicated by "-" (minus), and a base sequence region on the 3' side (downstream) therefrom is indicated by "+". In this respect, the region indicated by the range of -20 bases to -1 base belongs to exon 54, and the region indicated by the range of +1 base to +400 bases belongs to intron 54.

(A22) Region R22

The region R22 is a region indicated by the range of -400 bases to +50 bases when the boundary between the 3' end of intron 54 and the 5' end of exon 55 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 54, and the region indicated by the range of +1 base to +50 bases belongs to exon 55.

(A23) Region R23

The region R23 is a region indicated by the range of -20 bases to +400 bases when the boundary between the 3' end of exon 55 and the 5' end of intron 55 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -20 bases to -1 base belongs to exon 55, and the region indicated by the range of +1 base to +400 bases belongs to intron 55.

(A24) Region R24

The region R24 is a region indicated by the range of -400 bases to +50 bases when the boundary between the 3' end of intron 55 and the 5' end of exon 56 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 55, and the region indicated by the range of +1 base to +50 bases belongs to exon 56.

In an alternative aspect, R2, R6, R8, R10, R12, R14, R16, R18, R20, and R22 which are examples of the target region Rn of the present invention are, but not limited to, as follows:

(A2) Region R2

The region R2 is a region indicated by the range of -600 bases to +19 bases when the boundary between the 3' end of intron 44 and the 5' end of exon 45 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -600 bases to -1 base belongs to intron 44, and the region indicated by the range of +1 base to +19 bases belongs to exon 45.

(A6) Region R6

The region R6 is a region indicated by the range of -400 bases to +37 bases when the boundary between the 3' end of intron 46 and the 5' end of exon 47 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 46, and the region indicated by the range of +1 base to +37 bases belongs to exon 47.

(A8) Region R8

The region R8 is a region indicated by the range of -400 bases to +19 bases when the boundary between the 3' end of intron 47 and the 5' end of exon 48 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 47, and the region indicated by the range of +1 base to +19 bases belongs to exon 48.

(A10) Region R10

The region R10 is a region indicated by the range of -400 bases to +42 bases when the boundary between the 3' end of intron 48 and the 5' end of exon 49 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 48, and the region indicated by the range of +1 base to +42 bases belongs to exon 49.

(A12) Region R12

The region R12 is a region indicated by the range of -400 bases to +44 bases when the boundary between the 3' end of intron 49 and the 5' end of exon 50 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 49, and the region indicated by the range of +1 base to +44 bases belongs to exon 50.

(A14) Region R14

The region R14 is a region indicated by the range of -400 bases to +25 bases when the boundary between the 3' end of intron 50 and the 5' end of exon 51 is defined as basing point 0, a base sequence region on the 5' side

(upstream) from the basing point is indicated by "-" (minus), and a base sequence region on the 3' side (downstream) therefrom is indicated by "+". In this respect, the region indicated by the range of -400 bases to -1 base belongs to intron 50, and the region indicated by the range of +1 base to +25 bases belongs to exon 51.

(A16) Region R16

The region R16 is a region indicated by the range of -400 bases to +24 bases when the boundary between the 3' end of intron 51 and the 5' end of exon 52 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 51, and the region indicated by the range of +1 base to +24 bases belongs to exon 52.

(A18) Region R18

The region R18 is a region indicated by the range of -400 bases to +34 bases when the boundary between the 3' end of intron 52 and the 5' end of exon 53 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 52, and the region indicated by the range of +1 base to +34 bases belongs to exon 53.

(A20) Region R20

The region R20 is a region indicated by the range of -400 bases to +43 bases when the boundary between the 3' end of intron 53 and the 5' end of exon 54 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 53, and the region indicated by the range of +1 base to +43 bases belongs to exon 54.

(A22) Region R22

The region R22 is a region indicated by the range of -400 bases to +25 bases when the boundary between the 3' end of intron 54 and the 5' end of exon 55 is defined as basing point 0, a base sequence region on the 5' side (upstream) from the basing point is indicated by "-" (minus), and a base sequence region on the 3' side (downstream) therefrom is indicated by "+". In this respect, the region indicated by the range of -400 bases to -1 base belongs to intron 54, and the region indicated by the range of +1 base to +25 bases belongs to exon 55.

Specific examples of the base sequences of the regions R1 to R24 will be given below. As used herein, thymine "T" and uracil "U" are interchangeable with each other. It does not essentially affect the exon skipping activity of the antisense oligomer of the present invention whether it is "T" or "U". Therefore, as used herein, identical base sequences, even when "T" are replaced with "U", are also included and represented by the same SEQ ID NO. In the tables below, "U" may be described as "T" even in the base sequence of pre-mRNA. Those skilled in the art can understand an RNA sequence by appropriately replacing "T" with "U".

[Table 1]

Table 1

| Region | Base sequence of target region | SEQ ID NO |
|---|---|---|
| R1 | Region in vicinity of donor of intron 44 (in range of -20 to +400 bases from 5' end of intron 44 as basing point)<br>AATACAAATGGTATCTTAAGGTAAGTCTTTGATTTGTTTTTTCGAAATTGTATTTATCTTC<br>AGCACATCTGGACTCTTTAACTTCTTAAAGATCAGGTTCTGAAGGGTGATGGAAATTACTT<br>TTGACTGTTGTTGTCATCATTATATTACTAGAAAGAAAATTATCATAATGATAATATTAGA<br>GCACGGTGCTATGGACTTTTTGTGTCAGGATGAGAGAGTTTGCCTGGACGGAGCTGGTTTA<br>TCTGATAAACTGCAAAATATAATTGAATCTGTGACAGAGGGAAGCATCGTAACAGCAAGGT<br>GTTTTGTGGCTTTGGGGCAGTGTGTATTTCGGCTTTATGTTGGAACCTTTCCAGAAGGAGA<br>ACTTGTGGCATACTTAGCTAAAATGAAGTTGCTAGAAATATCCATCATGATAAA | SEQ No. 233 |

(continued)

| Region | Base sequence of target region | SEQ ID NO |
|---|---|---|
| R2 | Region in vicinity of acceptor of intron 44 (in range of -600 to +50 bases from 3' end of intron 44 as basing point)<br>TCTTGATGGGATGCTCCTGAAAGCAATTAATTCTCAGTTTTTGTGGCTTCTAATGCAAAA<br>TACATTGACGCAGACAGAATTTGAAATGAATTTTCTTCTAATATAGCAATTAATTTTATTT<br>AAATATCTCTAGAGTTTTTTTTTAATACTGTGACTAACCTATGTTTGTTCTTTTTCACCTC<br>TCGTATCCACGATCACTAAGAAACCCAAATACTTTGTTCATGTTTAAATTTTACAACATTT<br>CATAGACTATTAAACATGGAACATCCTTGTGGGACAAGAAATCGAATTTGCTCTTGAAAA<br>GGTTTCCAACTAATTGATTTGTAGGACATTATAACATCCTCTAGCTGACAAGCTTACAAAA<br>ATAAAAACTGGAGCTAACCGAGAGGGTGCTTTTTTCCCTGACACATAAAAGGTGTCTTTCT<br>GTCTTGTATCCTTTGGATATGGGCATGTCAGTTTCATAGGGAAATTTTCACATGGAGCTTT<br>TGTATTTCTTTCTTTGCCAGTACAACTGCATGTGGTAGCACACTGTTTAATCTTTTCTCAA<br>ATAAAAAGACATGGGGCTTCATTTTTGTTTTGCCTTTTTGGTATCTTACAGGAACTCCAGG<br>ATGGCATTGGGCAGCGGCAAACTGTTGTCAGAACATTGAA | SEQ No. 234 |
| R3 | Region in vicinity of donor of intron 45 (in range of -20 to +400 bases from 5' end of intron 45 as basing point)<br>CTGTCAGACAGAAAAAAGAGGTAGGGCGACAGATCTAATAGGAATGAAAACATTTTAGCAG<br>ACTTTTTAAGCTTTCTTTAGAAGAATATTTCATGAGAGATTATAAGCAGGGTGAAAGGCAC<br>TAACATTAAAGAACCTATCAACCATTAATCAACAGCAGTAAAGAAATTTTTTATTTCTTTT<br>TTTCATATACTAAAATATATACTTGTGGCTAGTTAGTGGTTTTCTGCTATTTTAAACTTGA<br>AGTTTGCTTTAAAAATCACCCATGATTGCTTAAAGGTGAATATCTTCAATATATTTTAACT<br>TCAACAAGCTGAATCTCAGTTGTTTTTCAAGAAGATTTTAGAAAGCAATTATAAATGATTG<br>TTTTGTAGGAAAGACAGATCTTTGCTTAGTTTTAAAAATAGCTATGAATATGAC | SEQ No. 235 |
| R4 | Region in vicinity of acceptor of intron 45 (in range of -400 to +50 bases from 3' end of intron 45 as basing point)<br>ATTACAGGCGCCTGCCACCAAACCTGGCAAATTTTTGTATTTTTAGTGTAGACGGGGTTTC<br>ACCATATTTGCCAGGCTGGTCGCAAACTCCTGACCTCAAGTGATCCGCCCACATCGGCCTC<br>CCTAAGCGCTAGGGTTACAGGCATGAGCCACTGCGCCTGGCCAGGAATTTTTGAATCAGAA<br>TTTTTCTTGTTCGATTTTAATCTCTTATCATTTAGAGATTCTTGAAATATTGAAATTACTT<br>TGTTCAAAGTGAATGAATTTTCTTAAATTATGTATGGTTAACATCTTTTAAATTGCTTATT<br>TTTAAATTGCCATGTTTGTGTCCCAGTTTGCATTAACAAATAGTTTGAGAACTATGTTGGA<br>AAAAAAAATAACAATTTTATTCTTCTTTCTCCAGGCTAGAAGAACAAAAGAATATCTTGTC<br>AGAATTTCAAAGAGATTTAAATG | SEQ No. 236 |
| R5 | Region in vicinity of donor of intron 46 (in range of -20 to +400 bases from 5' end of intron 46 as basing point)<br>AAAAGCTTGAGCAAGTCAAGGTAATTTTATTTTCTCAAATCCCCCAGGGCCTGCTTGCATA<br>AAGAAGTATATGAATCTATTTTTTAATTCAATCATTGGTTTTCTGCCCATTAGGTTATTCA<br>TAGTTCCTTGCTAAAGTGTTTTTCTCACAACTTTATTTCTTCTTAACCCTGCAGTTCTGAA<br>CCAGTGCACATAAGAACATATGTATATATGTGTGTGTGTGTATTTATATATACACACACAC | SEQ No. 237 |
| | ATATTGCATCTATACATCTACACATATAGATGTATAGATTCAATATGTCTAAATGTATATA<br>ATTCACAGTTTTTATCTTTGATTTGAAATTAATTTTAGATTTTACTTGAGAACTTCACAAC<br>TTCATATAATTTTAAAAACTGAAGACCAGATTGTGGAATCATAAAATCTAAATC | |

(continued)

| Region | Base sequence of target region | SEQ ID NO |
|---|---|---|
| R6 | Region in vicinity of acceptor of intron 46 (in range of -400 to +50 bases from 3' end of intron 46 as basing point)<br>ATAATGCAATTTCTCAGCAGCAAGCTACGGTATGCTATGGCATGCTATGATACCCAAGAGG<br>CTGATGAATTTGTTCACATTGTTCTATTTCTGATAGAGAGATAGGTTTTCAGACACTAACT<br>TTATTTGGAGTGTTGCTTTACCATCTCACATTTTTCTCTTAAAAAATTTATGAGGGATAAT<br>ATAATCGTTTATTTTCTACAGAGATTTATCTACTGAGGGGGTGAGTGTTTCAGTCAATCAG<br>CTCTGTGCTCAGATAGAAAACTGTTGGTATTTGAGGTACCACTGGGCCCTCGGTCAAGTCG<br>CTTCATTTTGATAGACTAATCAATAGAAGCAAAGACAAGGTAGTTGGAATTGTGCTGTAAT<br>TCATTTTAAACGTTGTTGCATTTGTCTGTTTCAGTTACTGGTGGAAGAGTTGCCCCTGCGC<br>CAGGGAATTCTCAAACAATTAAA | SEQ No. 238 |
| R7 | Region in vicinity of donor of intron 47 (in range of -20 to +400 bases from 5' end of intron 47 as basing point)<br>CAAATCTCCAGTGGATAAAGGTTAGACATTAACCATCTCTTCCGTCACATGTGTTAAATGT<br>TGCAAGTATTTGTATGTATTTTGTTTCCTGGGTGCTTCATTGGTCGGGGAGGAGGCTGGTA<br>TGTGGATTGTTGTTTTGTTTTGTTTTTTTAACCTGACCGTTTGCTTTGGCTATATGTTTTG<br>TTGTGGCTAGAAAAAATGATGATGGTGAATGGCTTTACATTAATGACCAAATGCCAAAATT<br>TATACCACAATTTTTTGCATAAATTATTCTGAAGAATCAGACTGAAGAAATGGCGAAGTAT<br>TTAATTCAGTGGCCAGGCATGTACTGACAGTATTTAAGCTGAAAGGACGTGGTCTGGTTCT<br>AGTTAAACAAGTGTCATAAATCAAAATTAATTATTCACACCTGTGGTATGGACT | SEQ No. 239 |
| R8 | Region in vicinity of acceptor of intron 47 (in range of -400 to +50 bases from 3' end of intron 47 as basing point)<br>TATTCATTTTTATAACTGCAAAGGAAGCGCGTATGGCATATAATACACAACACACCAGTAT<br>ATTTAGTAACTGAGTGAATAAATGAAAGATGTATTTCTTTACTTTATCAGTTGCAGTTGGC<br>TATGCCTTTGTGTAAGGTGTGTGTTTTGAAATTCCAAAAAGGTATTAGTTTCTTTAAAGCA<br>AAGAATTTTTGTAGCAGGTTAATGAATAATTTTGAATACATTGGTTAAATCCCAACATGTA<br>ATATATGTAAATAATCAATATTATGCTGCTAAAATAACACAAATCAGTAAGATTCTGTAAT<br>ATTTCATGATAAATAACTTTTGAAAATATATTTTTAAACATTTTGGCTTATGCCTTGAGAA<br>TTATTTACCTTTTTAAAATGTATTTTCCTTTCAGGTTTCCAGAGCTTTACCTGAGAAACAA<br>GGAGAAATTGAAGCTCAAATAAA | SEQ No. 240 |
| R9 | Region in vicinity of donor of intron 48 (in range of -20 to +400 bases from 5' end of intron 48 as basing point)<br>AAGGACCATTTGACGTTCAGGTAGGGAACTTTTTGCTTTAAATATTTTTGTCTTTTTTAAG<br>AAAAATGGCAATATCACTGAATTTTCTCATTTGGTATCATTATTAAAGACAAAATATTACT<br>TGTTAAAGTGTGGTAAGGAAGACTTTATTCAGGATAACCACAATAGGCACAGGGACCACTG<br>CAATGGAGTATTACAGGAGGTTGGATAGAGAGAGATTGGGCTCAACTCTAAATACAGCACA<br>GTGGAAGTAGGAATTTATAGCCAAGGAGCAGTGTAGGAGTCAGTAGATGGAAAATTATTAA<br>GAGGAAACATCAGGGGTAAGTGGGATTCTGGCTAAACCAACCTCACAGGATTCTTGCTGAA<br>GATAGGCCAGGGTTATCTTATCAGACAACCCTTGGGGAATGGTGGAGAATACTG | SEQ No. 241 |
| R10 | Region in vicinity of acceptor of intron 48 (in range of -400 to +50 bases from 3' end of intron 48 as basing point)<br>TACTAAACACAGAATTTTGTAAAACAATAAGTGTATAAAGTAAAATGAACATTAGGATTAT<br>TGAGATTATTGTAGCTAAAACTAGTGTTTATTCATATAAATTATGTTAATAAATTGTATTG<br>TCATTATTGCATTTTACTTTTTTGAAAAGTAGTTAATGCCTGTGTTTCTATATGAGTATTA<br>TATAATTCAAGAAGATATTGGATGAATTTTTTTTAAGTTTAATGTGTTTCACATCTCTGTT<br>TCTTTTCTCTGCACCAAAAGCTACATTTTTGTGCCCTTATGTACCAGGCAGAAATTGATCT<br>GCAATACATGTGGAGTCTCCAAGGGTATATTTAAATTTAGTAATTTTATTGCTAACTGTGA<br>AGTTAATCTGCACTATATGGGTTCTTTTCCCCAGGAAACTGAAATAGCAGTTCAAGCTAAA<br>CAACCGGATGTGGAAGAGATTTT | SEQ No. 242 |

(continued)

| Region | Base sequence of target region | SEQ ID NO |
|---|---|---|
| R11 | Region in vicinity of donor of intron 49 (in range of -20 to +400 bases from 5' end of intron 49 as basing point)<br>CAGCCACTCAGCCAGTGAAGGTAATGAAGCAACCTCTAGCAATATCCATTACCTCATAATG<br>GGTTATGCTTCCCCTGTTGTACATTTGCCATTGACGTGGACTATTTATAATCAGTGAAATA<br>ACTTGTAAGGAAATACTGGCCATACTGTAATAGCAGAGGCAAAGCTGTCTTTTTGATCAGC<br>ATATCCTATTTATATATTGTGATCTTAAGGCTATTAACGAGTCATTGCTTTAAAGGACTCA<br>TTTCTGTCCTGGTGTGCTGCCATCAATACAAAAGTAGTCCCACCTTCAAGGTAGATTAAAT<br>TCTTTGGGGCTTTATTGCTTTGCTTGCCAGCCTTGATGCTTTTCATATTGTTTGGTTTAAT<br>TCAAATCAAGCTACTGCATCATAGTGTCTGTCTCCAACAGCTGTAAAGAATCAC | SEQ No. 243 |
| R12 | Region in vicinity of acceptor of intron 49 (in range of -400 to +50 bases from 3' end of intron 49 as basing point)<br>ATATAATTGACTGGGGGTGAGCCAGTACATTAGGATTTTCCTAAAGTTATCTGGATAATTT<br>TAGTATGCAACCACAATAGATACTCTTCAAGAATTAAGCTAGTTGCTGAGAGGGAACTGTT<br>TTTTGTTGGTTTGTTTTCACTAATGTTTGCACTCTACTTCCTTTAAATAAAATTATGCCTG<br>GAGAAAGGGTTTTTGTATGGAGCAATTGATAAATATTTGTAGGGTGGTTGGCTAAAATAAT<br>TATAATTCCTTTAAAAGAAATTCTACCCACTAAAGTTAATTTAGAAGTAAAATATAATAGA<br>AATCCAATAATATATTCACCAAATGGATTAAGATGTTCATGAATTATCTTCAAAGTGTTAA<br>TCGAATAAGTAATGTGTATGCTTTTCTGTTAAAGAGGAAGTTAGAAGATCTGAGCTCTGAG<br>TGGAAGGCGGTAAACCGTTTACT | SEQ No. 244 |
| R13 | Region in vicinity of donor of intron 50 (in range of -20 to +400 bases from 5' end of intron 50 as basing point)<br>ACTGACCACTATTGGAGCCTGTAAGTATACTGGATCCCATTCTCTTTGGCTCTAGCTATTT<br>GTTCAAAAGTGCAACTATGAAGTGATGACTGGGTGAGAGAGAAAATTTGTTTCAATTCTAA<br>AGATAGAGATAAACCTTTGTGTTATTGACTGTGCAAAAAGTCTTAGAGTACATTCCTTGGA<br>AATTGACTCTGATTCAAAGTGTTGCATGACAACGGGATATGGGGAGTGTTCTCTGGAGATA<br>CACCCACAAGGAAGAGAAGAGCACAAGGGAGATTGTGGAGAGTCTGAAATGTGATTTGTC<br>TGCAGCAGAGGCCTAAGCCAGTCTCGCAGGAGCCCTACATCTGGGCTGGCTGTGCAGAGCT<br>GTCCTGAATTGCAGGCAGTGGGCCTGGCCCTTGTATTCCTGATCCAGCCAGCCA | SEQ No. 245 |
| R14 | Region in vicinity of acceptor oi intron 50 (in range of -400 to +50 bases from 3' end of intron 50 as basing point)<br>TCTTGAATAAAAAAAAAATAAGTAAAATTTATTTCCCTGGCAAGGTCTGAAAACTTTTGTT<br>TTCTTTACCACTTCCACAATGTATATGATTGTTACTGAGAAGGCTTATTTAACTTAAGTTA<br>CTTGTCCAGGCATGAGAATGAGCAAAATCGTTTTTTAAAAAATTGTTAAATGTATATTAAT<br>GAAAAGGTTGAATCTTTTCATTTTCTACCATGTATTGCTAAACAAAGTATCCACATTGTTA<br>GAAAAAGATATATAATGTCATGAATAAGAGTTTGGCTCAAATTGTTACTCTTCAATTAAAT<br>TTGACTTATTGTTATTGAAATTGGCTCTTTAGCTTGTGTTTCTAATTTTTCTTTTTCTTCT<br>TTTTTCCTTTTTGCAAAAACCCAAAATATTTTAGCTCCTACTCAGACTGTTACTCTGGTGA<br>CACAACCTGTGGTTACTAAGGAA | SEQ No. 246 |
| R15 | Region in vicinity of donor of intron 51 (in range of -20 to +400 bases from 5' end of intron 51 as basing point)<br>AGATGATCATCAAGCAGAAGGTATGAGAAAAAATGATAAAAGTTGGCAGAAGTTTTTCTTT<br>AAAATGAAGATTTTCCACCAATCACTTTACTCTCCTAGACCATTTCCCACCAGTTCTTAGG<br>CAACTGTTTCTCTCTCAGCAAACACATTACTCTCACTATTCAGCCTAAGTATAATCAAGGA<br>TATAAATTAATGCAAATAACAAAAGTAGCCATACATTAAAAAGGAAATATACAAAAAAAAA<br>AAAAAAAAAAAGCAGAAACCTTACAAGAATAGTTGTCTCAGTTAAATTTACTAAACAACCT<br>GGTATTTTAAAAATCTATTTTATACCAAATAAGTCACTCAACTGAGCTATTTACATTTAAA<br>CTGTTTGTTTTGGCACTACGCAGCCCAACATATTGCAGAATCAAATATAATAGT | SEQ No. 247 |

(continued)

| Region | Base sequence of target region | SEQ ID NO |
|---|---|---|
| R16 | Region in vicinity of acceptor of intron 51 (in range of -400 to +50 bases from 3' end of intron 51 as basing point)<br>TTTAAAATAAATATACCTTAATTTTGACGTCACACAGAATGATATTATAAGTATAAATAGT<br>TATCTATCTTTTAAATACATTGTCGTAATTCAGAATAACATTTCTTACTCAAGGCATTCAG<br>ACAGTGGTTTAAGTAATCCGAGGTACTCCGGAATGTCTCCATTTGAGCCTTTAAATGAAGA<br>AAATCTATAGTCAAGATTTTCATTTGAAATATTTTTGATATCTAAGAATGAAACATATTTC<br>CTGTTAAATTGTTTTCTATAAACCCTTATACAGTAACATCTTTTTTATTTCTAAAAGTGTT<br>TTGGCTGGTCTCACAATTGTACTTTACTTTGTATTATGTAAAAGGAATACACAACGCTGAA<br>GAACCCTGATACTAAGGGATATTTGTTCTTACAGGCAACAATGCAGGATTTGGAACAGAGG<br>CGTCCCCAGTTGGAAGAACTCAT | SEQ No. 248 |
| R17 | Region in vicinity of donor of intron 52 (in range of -20 to +400 bases from 5' end of intron 52 as basing point)<br>AACAATCATTACGGATCGAAGTAAGTTTTTAACAAGCATGGGACACACAAAGCAAGATGC<br>ATGACAAGTTTCAATAAAAACTTAAGTTCATATATCCCCCTCACATTTATAAAAATAATGT<br>GAAATAATTGTAAATGATAACAATTGTGCTGAGATTTTCAGTCCATAATGTTACCTTTTAA<br>TAAATGAATGTAATTCCATTGAATAGAAGAAATACATTTTTAAATCAATTCAGGGCTTATA<br>TAGTTGCAAAGCATGCATTGATGGGTGTGGTGACCACAGTGTGGCAGAACATTTGTGGCAG<br>AACATTTGTTCTTTAGTTGTCATCTGGGCTGGCATCCATGGAGATGCCAGTCTCTCCCTCA<br>TATCCTTGGCTGTTGGTCCAAGCAGGCAGTGGCTTCTTCCTGGGCCATCTTTCA | SEQ No. 249 |
| R18 | Region in vicinity of acceptor of intron 52 (in range of -400 to +50 bases from 3' end of intron 52 as basing point)<br>CAAACTCCTGTGGCAACAGAAAGCCTTCAGGCAATGAAATGCTGGCACTGGGAAATCAGGC<br>TGATGGGTGCTGAAGTGGCAAGGATGAGGGGATATGGATATTCTGCTGTAGTGCTTTTCTA<br>ACAGATGATTCATATTTGGTTCTAGGGATCAAGAATTGAGTTAAAATTTTATATATATGTT<br>GATGTTCTATGTCACCTTCAGGAAAATAATTTAACAGAAACTAATATTTGCCATCAAAAAA<br>GCAAAGAATCCTGTTGTTCATCATCCTAGCCATAACACAATGAATAATTTTTTAAATAAGC<br>AACATAAATGTGAGATAACGTTTGGAAGTTACATTTAAAATGTCTCCTCCAGACTAGCATT<br>TACTACTATATATTTATTTTTCCTTTTATTCTAGTTGAAAGAATTCAGAATCAGTGGGATG<br>AAGTACAAGAACACCTTCAGAAC | SEQ No. 250 |
| R19 | Region in vicinity of donor of intron 53 (in range of -20 to +400 bases from 5' end of intron 53 as basing point)<br>AGAAAATCACAGAAACCAAGGTTAGTATCAAAGATACCTTTTTAAAATAAAATACTGGTTA<br>CATTTGATAAAATTATACCATAGATTGTAATTTAATGATGTTTAATGTAAAGTTATTAACA<br>GAAAATCACGTTAAAGCTGAAATGAACAGTAGACTTTGTATATTTATTTTCTTAGAGACAG<br>AGTCTCACTGTCACCCAGGCTAAAGTGCAGTGGCACAATCATAGCTCACTGAGCCTTGAAC<br>TCTGGGGCTCAAGCAGTCCTCCTGCCTCAGCCTCCCTAGTAGCTGGGACTACTAGCCAGGC<br>GTGTACCACCACGCCTGGCTAATTTTTTAAAAATTTTTGTTTTCTGTAGAGATGGGTTCTT<br>GAACTCTTGGCCTCAAGCAATTCTCCTTCCTTGGCCTCCCAAAGCACTAGGATT | SEQ No. 251 |
| R20 | Region in vicinity of acceptor of intron 53 (in range of -400 to +50 bases from 3' end of intron 53 as basing point)<br>CAACAAGTTAATGAAGAGGGAAAGAAATGTATGAGGTTTTTTTCGTTCAAATGTTGTTATA<br>TGTCACATATTCAACAATTATATATGAGCTTATTTTTGTAGTTTTTTTCTCTTGTGATAAA<br>AACAATTAAGCCCACTTTATTGCCAATTAATTGCTACTAAGTTGAAATACTTGATACTGGT<br>TATTGCTCAAGATGCTGCATTTGAAAAGTTTGTCCTGAAAGGTGGGTTACCTTATACTGTC<br>ATGATTGACTAAATCATATGGTAGGTTAAAAGCAATCTAATATATGTATTCTGACCTGAGG<br>ATTCAGAAGCTGTTTACGAAGTATTTTAAGACACTCCAACTAGAGATTTCATAAAAAAAAC<br>TGACATTCATTCTCTTTCTCATAAAAATCTATAGCAGTTGGCCAAAGACCTCCGCCAGTGG<br>CAGACAAATGTAGATGTGGCAAA | SEQ No. 252 |

(continued)

| Region | Base sequence of target region | SEQ ID NO |
|---|---|---|
| R21 | Region in vicinity of donor of intron 54 (in range of -20 to +400 bases from 5' end of intron 54 as basing point)<br>TGGAGAAGCATTCATAAAAGGTATGAATTACATTATTTCTAAAACTACTGTTGGCTGTAAT<br>AATGGGGTGGTGAAACTGGATGGACCATGAGGATTTGTTTTTCCAATCCAGCTAAACTGGA<br>GCTTGGGAGGGTTCAAGACGATAAATACCAACTAAACTCACGGACTTGGCTCAGACTTCTA<br>TTTTAAAAACGAGGAACATAAGATCTCATTTGCCCGCTGTCACAAAAGTAGTGACATAACC<br>AAGAGATTAAACAAAAAGCAAAATACTGATTTATAGCTAGAAGAGCCATTTATCAGTCTAC<br>TTTGATAACTCTATCCAAAGGAATATCTTTCTATCTCATCATGGCGCACACTGCCTTACCT<br>GTTATCTGATAAATAAGTCACTTTGGGATTCATGATAGAGTTATAGCTGTACAT | SEQ No. 253 |
| R22 | Region in vicinity of acceptor of intron 54 (in range of -400 to +50 b ase s from 3' end of intron 54 as basing point)<br>TCTCAAATTTGGCAGTATATTAAAAATAAGCTTTCAAAATTGACCAACAAAAACTACAAAA<br>TTGAAAAAAAGGTACTTTGAACTTTCACATGTTCAAATATATGTATATATATTTCACATAT<br>ATATATGAAACCTCCTCTGTGGAGAGGGGTTTATAGAAATCTGTAATTGTCATTCTTGCAT<br>GCCTTCCCCCATACAAACGCCTTTAAGTTAAATAAAAATGAAAGTAAATAGACTGCACAAT<br>ATTATAGTTGTTGCTTAAAGGAAGAGCTGTAGCAACAACTCACCCCATTGTTGGTATATTA<br>CAATTTAGTTCCTCCATCTTTCTCTTTTTATGGAGTTCACTAGGTGCACCATTCTGATATT<br>TAATAATTGCATCTGAACATTTGGTCCTTTGCAGGGTGAGTGAGCGAGAGGCTGCTTTGGA<br>AGAAACTCATAGATTACTGCAAC | SEQ No. 254 |
| R23 | Region in vicinity of donor of intron 55 (in range of -20 to +400 bases from 5' end of intron 55 as basing point)<br>AGCTGATGAAACAATGGCAAGTAAGTCAGGCATTTCCGCTTTAGCACTCTTGTGGATCCAA<br>TTGAACAATTCTCAGCATTTGTACTTGTAACTGACAAGCCAGGGACAAAACAAAATAGTTG<br>CTTTTATACAGCCTGATGTATTTCGGTATTTGGACAAGGAGGAGAGAGGCAGAGGGAGAAG<br>GAAACATCATTTATAATTCCACTTAACACCCTCGTCTTAGAAAAAGTACATGCTCTGACCA<br>GGAAAACATTTGCATATAAAACCAGAGCTTCGGTCAAGGAGAAACTTTGCTCAGAGAAATA<br>ACTTAGGGATTGGTTTATTAAATTTTAAAAGTTGACATTTTTGAGTGTTTATTTAATATTT<br>TACAGGGAAAGCATCTGTATGAATTGTCTGTTTTATTTAGCGTTGCTAACTGAA | SEQ No. 255 |
| R24 | Region in vicinity of acceptor of intron 55 (in range of -400 to +50 bases from 3' end of intron 55 as basing point)<br>ACTTGATCCATATAGTAATGAAATTATTGGCACTGGGGTACACTTTATCATAGAATTTTAT<br>TGCCTATCACTTCCATAAAATAATACATTTTGTCCATAGACTAGAAGATATAACTTGTGAA<br>CTTTATAAAGTTATAAATACATTACTTTCCAACTCATAATGGCAAGGAATAAATCTATTAC<br>AACTAATAAGATGCCCATTTTAAATCTACATAATAACAGGAGAAGGCAATACGCCAAGAAA<br>AGGGATTTGAGATGTATCTTCTTGTTAGTTTAGCCTGATTGAAATGTCTTTTGAACTAATA<br>ATTATTTATATTTTGCAATTCTCCAAATTCACATTCATCGCTTGTTTCTTTTGTTTGGTAA<br>TTCTGCACATATTCTTCTTCCTGCTGTCCTGTAGGACCTCCAAGGTGAAATTGAAGCTCAC<br>ACAGATGTTTATCACAACCTGGA | SEQ No. 256 |

In an alternative aspect, specific examples of the base sequences of the regions R2, R6, R8, R10, R12, R14, R16, R18, R20, and R22 are as follows.

[Table 2]

Table 2

| Region | Base sequence of target region | SEQ ID NO |
|---|---|---|
| R2 | Region in vicinity of acceptor of intron 44 (in range of -600 to +19 bases from 3' end of intron 44 as basing point)<br>TCTTGATGGGATGCTCCTGAAAGCAATTAATTCTCAGTTTTTGTGGCTTCTAATGCAAAA<br>TACATTGACGCAGACAGAATTTGAAATGAATTTTCTTCTAATATAGCAATTAATTTTATTT<br>AAATATCTCTAGAGTTTTTTTTTAATACTGTGACTAACCTATGTTTGTTCTTTTTCACCTC<br>TCGTATCCACGATCACTAAGAAACCCAAATACTTTGTTCATGTTTAAATTTTACAACATTT<br>CATAGACTATTAAACATGGAACATCCTTGTGGGGACAAGAAATCGAATTTGCTCTTGAAAA<br>GGTTTCCAACTAATTGATTTGTAGGACATTATAACATCCTCTAGCTGACAAGCTTACAAAA<br>ATAAAAACTGGAGCTAACCGAGAGGGTGCTTTTTTCCCTGACACATAAAAGGTGTCTTTCT<br>GTCTTGTATCCTTTGGATATGGGCATGTCAGTTTCATAGGGAAATTTTCACATGGAGCTTT<br>TGTATTTCTTTCTTTGCCAGTACAACTGCATGTGGTAGCACACTGTTTAATCTTTTCTCAA<br>ATAAAAAGACATGGGGCTTCATTTTTGTTTTGCCTTTTTGGTATCTTACAGGAACTCCAGG<br>ATGGCATTG | SEQ No. 341 |
| R6 | Region in vicinity of acceptor of intron 46 (in range of -400 to +37 bases from 3' end of intron 46 as basing point)<br>ATAATGCAATTTCTCAGCAGCAAGCTACGGTATGCTATGGCATGCTATGATACCCAAGAGG<br>CTGATGAATTTGTTCACATTGTTCTATTTCTGATAGAGAGATAGGTTTTCAGACACTAACT<br>TTATTTGGAGTGTTGCTTTACCATCTCACATTTTTCTCTTAAAAAATTTATGAGGGATAAT<br>ATAATCGTTTATTTTCTACAGAGATTTATCTACTGAGGGGGTGAGTGTTTCAGTCAATCAG<br>CTCTGTGCTCAGATAGAAAACTGTTGGTATTTGAGGTACCACTGGGCCCTCGGTCAAGTCG<br>CTTCATTTTGATAGACTAATCAATAGAAGCAAAGACAAGGTAGTTGGAATTGTGCTGTAAT<br>TCATTTTAAACGTTGTTGCATTTGTCTGTTTCAGTTACTGGTGGAAGAGTTGCCCCTGCGC<br>CAGGGAATTC | SEQ No. 385 |
| R8 | Region in vicinity of acceptor of intron 47 (in range of -400 to +19 bases from 3' end of intron 47 as basing point)<br>TATTCATTTTTATAACTGCAAAGGAAGCGCGTATGGCATATAATACACAACACACCAGTAT<br>ATTTAGTAACTGAGTGAATAAATGAAAGATGTATTTCTTTACTTTATCAGTTGCAGTTGGC<br>TATGCCTTTGTGTAAGGTGTGTGTTTTGAAATTCCAAAAAGGTATTAGTTTCTTTAAAGCA<br>AAGAATTTTTGTAGCAGGTTAATGAATAATTTTGAATACATTGGTTAAATCCCAACATGTA<br>ATATATGTAAATAATCAATATTATGCTGCTAAAATAACACAAATCAGTAAGATTCTGTAAT<br>ATTTCATGATAAATAACTTTTGAAAATATATTTTTAAACATTTTGGCTTATGCCTTGAGAA<br>TTATTTACCTTTTTAAAATGTATTTTCCTTTCAGGTTTCCAGAGCTTTACCTG | SEQ No. 386 |
| R10 | Region in vicinity of acceptor of intron 48 (in range of -400 to +42 bases from 3' end of intron 48 as basing point)<br>TACTAAACACAGAATTTTGTAAAACAATAAGTGTATAAAGTAAAATGAACATTAGGATTAT<br>TGAGATTATTGTAGCTAAAACTAGTGTTTATTCATATAAATTATGTTAATAAATTGTATTG<br>TCATTATTGCATTTTACTTTTTTGAAAAGTAGTTAATGCCTGTGTTTCTATATGAGTATTA<br>TATAATTCAAGAAGATATTGGATGAATTTTTTTTAAGTTTAATGTGTTTCACATCTCTGTT<br>TCTTTTCTCTGCACCAAAAGCTACATTTTTGTGCCCTTATGTACCAGGCAGAAATTGATCT<br>GCAATACATGTGGAGTCTCCAAGGGTATATTTAAATTTAGTAATTTTATTGCTAACTGTGA<br>AGTTAATCTGCACTATATGGGTTCTTTTCCCCAGGAAACTGAAATAGCAGTTCAAGCTAAA<br>CAACCGGATGTGGAA | SEQ No. 387 |
| R12 | Region in vicinity of acceptor of intron 49 (in range of -400 to +44 bases from 3' end of intron 49 as basing point)<br>ATATAATTGACTGGGGGTGAGCCAGTACATTAGGATTTTCCTAAAGTTATCTGGATAATTT<br>TAGTATGCAACCACAATAGATACTCTTCAAGAATTAAGCTAGTTGCTGAGAGGGAACTGTT<br>TTTTGTTGGTTTGTTTTCACTAATGTTTGCACTCTACTTCCTTTAAATAAAATTATGCCTG<br>GAGAAAGGGTTTTTGTATGGAGCAATTGATAAATATTTGTAGGGTGGTTGGCTAAAATAAT<br>TATAATTCCTTTAAAAGAAATTCTACCCACTAAAGTTAATTTAGAAGTAAAATATAATAGA | SEQ No. 388 |

(continued)

| Region | Base sequence of target region | SEQ ID NO |
|---|---|---|
| | AATCCAATAATATATTCACCAAATGGATTAAGATGTTCATGAATTATCTTCAAAGTGTTAA<br>TCGAATAAGTAATGTGTATGCTTTTCTGTTAAAGAGGAAGTTAGAAGATCTGAGCTCTGAG<br>TGGAAGGCGGTAAACCG | |
| R14 | Region in vicinity of acceptor of intron 50 (in range of -400 to +25 bases from 3' end of intron 50 as basing point)<br>TCTTGAATAAAAAAAAAATAAGTAAAATTTATTTCCCTGGCAAGGTCTGAAAACTTTTGTT<br>TTCTTTACCACTTCCACAATGTATATGATTGTTACTGAGAAGGCTTATTTAACTTAAGTTA<br>CTTGTCCAGGCATGAGAATGAGCAAAATCGTTTTTTAAAAAATTGTTAAATGTATATTAAT<br>GAAAAGGTTGAATCTTTTCATTTTCTACCATGTATTGCTAAACAAAGTATCCACATTGTTA<br>GAAAAAGATATATAATGTCATGAATAAGAGTTTGGCTCAAATTGTTACTCTTCAATTAAAT<br>TTGACTTATTGTTATTGAAATTGGCTCTTTAGCTTGTGTTTCTAATTTTTCTTTTTCTTCT<br>TTTTTCCTTTTTGCAAAAACCCAAAATATTTTAGCTCCTACTCAGACTGTTACTCTGGTGA<br>CAC | **SEQ** No. 342 |
| R16 | Region in vicinity of acceptor of intron 51 (in range of -400 to +24 bases from 3' end of intron 51 as basing point)<br>TTTAAAATAAATATACCTTAATTTTGACGTCACACAGAATGATATTATAAGTATAAATAGT<br>TATCTATCTTTTAAATACATTGTCGTAATTCAGAATAACATTTCTTACTCAAGGCATTCAG<br>ACAGTGGTTTAAGTAATCCGAGGTACTCCGGAATGTCTCCATTTGAGCCTTTAAATGAAGA<br>AAATCTATAGTCAAGATTTTCATTTGAAATATTTTTGATATCTAAGAATGAAACATATTTC<br>CTGTTAAATTGTTTTCTATAAACCCTTATACAGTAACATCTTTTTTATTTCTAAAAGTGTT<br>TTGGCTGGTCTCACAATTGTACTTTACTTTGTATTATGTAAAAGGAATACACAACGCTGAA<br>GAACCCTGATACTAAGGGATATTTGTTCTTACAGGCAACAATGCAGGATTTGGAACAGAGG<br>CGTCCCCAGTTGGAAGAACTCA | SEQ No. 343 |
| R18 | Region in vicinity of acceptor of intron 52 (in range of -400 to +34 bases from 3' end of intron 52 as basing point)<br>CAAACTCCTGTGGCAACAGAAAGCCTTCAGGCAATGAAATGCTGGCACTGGGAAATCAGGC<br>TGATGGGTGCTGAAGTGGCAAGGATGAGGGGATATGGATATTCTGCTGTAGTGCTTTTCTA<br>ACAGATGATTCATATTTGGTTCTAGGGATCAAGAATTGAGTTAAAATTTTATATATATGTT<br>GATGTTCTATGTCACCTTCAGGAAAATAATTTAACAGAAACTAATATTTGCCATCAAAAAA<br>GCAAAGAATCCTGTTGTTCATCATCCTAGCCATAACACAATGAATAATTTTTTAAATAAGC<br>AACATAAATGTGAGATAACGTTTGGAAGTTACATTTAAAATGTCTCCTCCAGACTAGCATT<br>TACTACTATATATTTATTTTTCCTTTTATTCTAGTTGAAAGAATTCAGAATCAGTGGGATG<br>AAGTACA | SEQ No. 344 |
| R20 | Region in vicinity of acceptor of intron 53 (in range of -400 to +43 bases from 3' end of intron 53 as basing point)<br>CAACAAGTTAATGAAGAGGGAAAGAAATGTATGAGGTTTTTTTCGTTCAAATGTTGTTATA<br>TGTCACATATTCAACAATTATATATGAGCTTATTTTTGTAGTTTTTTTCTCTTGTGATAAA<br>AACAATTAAGCCCACTTTATTGCCAATTAATTGCTACTAAGTTGAAATACTTGATACTGGT<br>TATTGCTCAAGATGCTGCATTTGAAAAGTTTGTCCTGAAAGGTGGGTTACCTTATACTGTC<br>ATGATTGACTAAATCATATGGTAGGTTAAAAGCAATCTAATATATGTATTCTGACCTGAGG<br>ATTCAGAAGCTGTTTACGAAGTATTTTAAGACACTCCAACTAGAGATTTCATAAAAAAAAC<br>TGACATTCATTCTCTTTCTCATAAAAATCTATAGCAGTTGGCCAAAGACCTCCGCCAGTGG<br>CAGACAAATGTAGATG | SEQ No. 389 |

(continued)

| Region | Base sequence of target region | SEQ ID NO |
|---|---|---|
| R22 | Region in vicinity of acceptor of intron 54 (in range of -400 to +25 bases from 3' end of intron 54 as basing point)<br>TCTCAAATTTGGCAGTATATTAAAAATAAGCTTTCAAAATTGACCAACAAAAACTACAAAA<br>TTGAAAAAAAGGTACTTTGAACTTTCACATGTTCAAATATATGTATATATATTTCACATAT<br>ATATATGAAACCTCCTCTGTGGAGAGGGGTTTATAGAAATCTGTAATTGTCATTCTTGCAT<br>GCCTTCCCCCATACAAACGCCTTTAAGTTAAATAAAAATGAAAGTAAATAGACTGCACAAT<br>ATTATAGTTGTTGCTTAAAGGAAGAGCTGTAGCAACAACTCACCCCATTGTTGGTATATTA<br>CAATTTAGTTCCTCCATCTTTCTCTTTTTATGGAGTTCACTAGGTGCACCATTCTGATATT<br>TAATAATTGCATCTGAACATTTGGTCCTTTGCAGGGTGAGTGAGCGAGAGGCTGCTTTG | SEQ No. 345 |

In a further alternative aspect, the antisense oligomer of the present invention targets at least any one region selected from the group consisting of regions R1 to R24 represented by

region Rn (wherein n is an odd number of 1 to 23) which consists of a base sequence of NY bases in the downstream direction from the 5' end of the NBth intron in the human dystrophin pre-mRNA, and
region Rn (wherein n is an even number of 2 to 24) which consists of a base sequence of NX bases in the upstream direction from the 3' end of the NAth intron in the human dystrophin pre-mRNA.

In the foregoing embodiment, examples of the target region Rn of the present invention include, but not limited to, regions wherein

when n is 1, NB = 44 and NY = 400,
when n is 2, NA = 44 and NX = 600,
when n is 3, NB = 45 and NY = 400,
when n is 4, NA = 45 and NX = 400,
when n is 5, NB = 46 and NY = 400,
when n is 6, NA = 46 and NX = 400,
when n is 7, NB = 47 and NY = 400,
when n is 8, NA = 47 and NX = 400,
when n is 9, NB = 48 and NY = 400,
when n is 10, NA = 48 and NX = 400,
when n is 11, NB = 49 and NY = 400,
when n is 12, NA = 49 and NX = 400,
when n is 13, NB = 50 and NY = 400,
when n is 14, NA = 50 and NX = 400,
when n is 15, NB = 51 and NY = 400,
when n is 16, NA = 51 and NX = 400,
when n is 17, NB = 52 and NY = 400,
when n is 18, NA = 52 and NX = 400,
when n is 19, NB = 53 and NY = 400,
when n is 20, NA = 53 and NX = 400,
when n is 21, NB = 54 and NY = 400,
when n is 22, NA = 54 and NX = 400,
when n is 23, NB = 55 and NY = 400, or
when n is 24, NA = 55 and NX = 400.

R1 to R24 which are examples of the target region Rn of the present invention according to the foregoing embodiment are, but not limited to, as follows:

for example, in a certain embodiment,
the region R1 is a region that consists of a base sequence of 400 bases in the downstream direction from the 5' end of the 44th intron in the human dystrophin pre-mRNA,

the region R2 is a region that consists of a base sequence of 600 bases in the upstream direction from the 3' end of the 44th intron in the human dystrophin pre-mRNA,
the region R3 is a region that consists of a base sequence of 400 bases in the downstream direction from the 5' end of the 45th intron in the human dystrophin pre-mRNA,
the region R4 is a region that consists of a base sequence of 400 bases in the upstream direction from the 3' end of the 45th intron in the human dystrophin pre-mRNA,
the region R5 is a region that consists of a base sequence of 400 bases in the downstream direction from the 5' end of the 46th intron in the human dystrophin pre-mRNA,
the region R6 is a region that consists of a base sequence of 400 bases in the upstream direction from the 3' end of the 46th intron in the human dystrophin pre-mRNA,
the region R7 is a region that consists of a base sequence of 400 bases in the downstream direction from the 5' end of the 47th intron in the human dystrophin pre-mRNA,
the region R8 is a region that consists of a base sequence of 400 bases in the upstream direction from the 3' end of the 47th intron in the human dystrophin pre-mRNA,
the region R9 is a region that consists of a base sequence of 400 bases in the downstream direction from the 5' end of the 48th intron in the human dystrophin pre-mRNA,
the region R10 is a region that consists of a base sequence of 400 bases in the upstream direction from the 3' end of the 48th intron in the human dystrophin pre-mRNA,
the region R11 is a region that consists of a base sequence of 400 bases in the downstream direction from the 5' end of the 49th intron in the human dystrophin pre-mRNA,
the region R12 is a region that consists of a base sequence of 400 bases in the upstream direction from the 3' end of the 49th intron in the human dystrophin pre-mRNA,
the region R13 is a region that consists of a base sequence of 400 bases in the downstream direction from the 5' end of the 50th intron in the human dystrophin pre-mRNA,
the region R14 is a region that consists of a base sequence of 400 bases in the upstream direction from the 3' end of the 50th intron in the human dystrophin pre-mRNA,
the region R15 is a region that consists of a base sequence of 400 bases in the downstream direction from the 5' end of the 51st intron in the human dystrophin pre-mRNA,
the region R16 is a region that consists of a base sequence of 400 bases in the upstream direction from the 3' end of the 51st intron in the human dystrophin pre-mRNA,
the region R17 is a region that consists of a base sequence of 400 bases in the downstream direction from the 5' end of the 52nd intron in the human dystrophin pre-mRNA,
the region R18 is a region that consists of a base sequence of 400 bases in the upstream direction from the 3' end of the 52nd intron in the human dystrophin pre-mRNA,
the region R19 is a region that consists of a base sequence of 400 bases in the downstream direction from the 5' end of the 53rd intron in the human dystrophin pre-mRNA,
the region R20 is a region that consists of a base sequence of 400 bases in the upstream direction from the 3' end of the 53rd intron in the human dystrophin pre-mRNA,
the region R21 is a region that consists of a base sequence of 400 bases in the downstream direction from the 5' end of the 54th intron in the human dystrophin pre-mRNA,
the region R22 is a region that consists of a base sequence of 400 bases in the upstream direction from the 3' end of the 54th intron in the human dystrophin pre-mRNA,
the region R23 is a region that consists of a base sequence of 400 bases in the downstream direction from the 5' end of the 55th intron in the human dystrophin pre-mRNA, or
the region R24 is a region that consists of a base sequence of 400 bases in the upstream direction from the 3' end of the 55th intron in the human dystrophin pre-mRNA.

In another embodiment, R1 to R24 which are examples of the target region Rn of the present invention according to the foregoing embodiment are, but not limited to, as follows:

(A1) Region R1
The region R1 is a region indicated by the range of +1 base to +400 bases when the boundary between the 3' end of exon 44 and the 5' end of intron 44 is defined as basing point 0 and a base sequence region on the 3' side (downstream) from the basing point is indicated by "+". In this respect, the region indicated by the range of +1 base to +400 bases belongs to intron 44.
(A2) Region R2
The region R2 is a region indicated by the range of -600 bases to -1 base when the boundary between the 3' end of intron 44 and the 5' end of exon 45 is defined as basing point 0 and a base sequence region on the 5' side

(upstream) from the basing point is indicated by "-" (minus). In this respect, the region indicated by the range of -600 bases to -1 base belongs to intron 44.

(A3) Region R3

The region R3 is a region indicated by the range of +1 base to +400 bases when the boundary between the 3' end of exon 45 and the 5' end of intron 45 is defined as basing point 0 and a base sequence region on the 3' side (downstream) from the basing point is indicated by "+". In this respect, the region indicated by the range of +1 base to +400 bases belongs to intron 45.

(A4) Region R4

The region R4 is a region indicated by the range of -400 bases to -1 base when the boundary between the 3' end of intron 45 and the 5' end of exon 46 is defined as basing point 0 and a base sequence region on the 5' side (upstream) from the basing point is indicated by "-" (minus). In this respect, the region indicated by the range of -400 bases to -1 base belongs to intron 45.

(A5) Region R5

The region R5 is a region indicated by the range of +1 base to +400 bases when the boundary between the 3' end of exon 46 and the 5' end of intron 46 is defined as basing point 0 and a base sequence region on the 3' side (downstream) from the basing point is indicated by "+". In this respect, the region indicated by the range of +1 base to +400 bases belongs to intron 46.

(A6) Region R6

The region R6 is a region indicated by the range of -400 bases to -1 base when the boundary between the 3' end of intron 46 and the 5' end of exon 47 is defined as basing point 0 and a base sequence region on the 5' side (upstream) from the basing point is indicated by "-" (minus). In this respect, the region indicated by the range of -400 bases to -1 base belongs to intron 46.

(A7) Region R7

The region R7 is a region indicated by the range of +1 base to +400 bases when the boundary between the 3' end of exon 47 and the 5' end of intron 47 is defined as basing point 0 and a base sequence region on the 3' side (downstream) from the basing point is indicated by "+". In this respect, the region indicated by the range of +1 base to +400 bases belongs to intron 47.

(A8) Region R8

The region R8 is a region indicated by the range of -400 bases to -1 base when the boundary between the 3' end of intron 47 and the 5' end of exon 48 is defined as basing point 0 and a base sequence region on the 5' side (upstream) from the basing point is indicated by "-" (minus). In this respect, the region indicated by the range of -400 bases to -1 base belongs to intron 47.

(A9) Region R9

The region R9 is a region indicated by the range of +1 base to +400 bases when the boundary between the 3' end of exon 48 and the 5' end of intron 48 is defined as basing point 0 and a base sequence region on the 3' side (downstream) from the basing point is indicated by "+". In this respect, the region indicated by the range of +1 base to +400 bases belongs to intron 48.

(A10) Region R10

The region R10 is a region indicated by the range of -400 bases to -1 base when the boundary between the 3' end of intron 48 and the 5' end of exon 49 is defined as basing point 0 and a base sequence region on the 5' side (upstream) from the basing point is indicated by "-" (minus). In this respect, the region indicated by the range of -400 bases to -1 base belongs to intron 48.

(A11) Region R11

The region R11 is a region indicated by the range of +1 base to +400 bases when the boundary between the 3' end of exon 49 and the 5' end of intron 49 is defined as basing point 0 and a base sequence region on the 3' side (downstream) from the basing point is indicated by "+". In this respect, the region indicated by the range of +1 base to +400 bases belongs to intron 49.

(A12) Region R12

The region R12 is a region indicated by the range of -400 bases to -1 base when the boundary between the 3' end of intron 49 and the 5' end of exon 50 is defined as basing point 0 and a base sequence region on the 5' side (upstream) from the basing point is indicated by "-" (minus). In this respect, the region indicated by the range of -400 bases to -1 base belongs to intron 49.

(A13) Region R13

The region R13 is a region indicated by the range of +1 base to +400 bases when the boundary between the 3' end of exon 50 and the 5' end of intron 50 is defined as basing point 0 and a base sequence region on the 3' side (downstream) from the basing point is indicated by "+". In this respect, the region indicated by the range of +1 base to +400 bases belongs to intron 50.

(A14) Region R14

The region R14 is a region indicated by the range of -400 bases to -1 base when the boundary between the 3' end of intron 50 and the 5' end of exon 51 is defined as basing point 0 and a base sequence region on the 5' side (upstream) from the basing point is indicated by "-" (minus). In this respect, the region indicated by the range of -400 bases to -1 base belongs to intron 50.

(A15) Region R15

The region R15 is a region indicated by the range of +1 base to +400 bases when the boundary between the 3' end of exon 51 and the 5' end of intron 51 is defined as basing point 0 and a base sequence region on the 3' side (downstream) from the basing point is indicated by "+". In this respect, the region indicated by the range of +1 base to +400 bases belongs to intron 51.

(A16) Region R16

The region R16 is a region indicated by the range of -400 bases to -1 base when the boundary between the 3' end of intron 51 and the 5' end of exon 52 is defined as basing point 0 and a base sequence region on the 5' side (upstream) from the basing point is indicated by "-" (minus). In this respect, the region indicated by the range of -400 bases to -1 base belongs to intron 51.

(A17) Region R17

The region R17 is a region indicated by the range of +1 base to +400 bases when the boundary between the 3' end of exon 52 and the 5' end of intron 52 is defined as basing point 0 and a base sequence region on the 3' side (downstream) from the basing point is indicated by "+". In this respect, the region indicated by the range of +1 base to +400 bases belongs to intron 52.

(A18) Region R18

The region R18 is a region indicated by the range of -400 bases to -1 base when the boundary between the 3' end of intron 52 and the 5' end of exon 53 is defined as basing point 0 and a base sequence region on the 5' side (upstream) from the basing point is indicated by "-" (minus). In this respect, the region indicated by the range of -400 bases to -1 base belongs to intron 52.

(A19) Region R19

The region R19 is a region indicated by the range of +1 base to +400 bases when the boundary between the 3' end of exon 53 and the 5' end of intron 53 is defined as basing point 0 and a base sequence region on the 3' side (downstream) from the basing point is indicated by "+". In this respect, the region indicated by the range of +1 base to +400 bases belongs to intron 53.

(A20) Region R20

The region R20 is a region indicated by the range of -400 bases to -1 base when the boundary between the 3' end of intron 53 and the 5' end of exon 54 is defined as basing point 0 and a base sequence region on the 5' side (upstream) from the basing point is indicated by "-" (minus). In this respect, the region indicated by the range of -400 bases to -1 base belongs to intron 53.

(A21) Region R21

The region R21 is a region indicated by the range of +1 base to +400 bases when the boundary between the 3' end of exon 54 and the 5' end of intron 54 is defined as basing point 0 and a base sequence region on the 3' side (downstream) from the basing point is indicated by "+". In this respect, the region indicated by the range of +1 base to +400 bases belongs to intron 54.

(A22) Region R22

The region R22 is a region indicated by the range of -400 bases to -1 base when the boundary between the 3' end of intron 54 and the 5' end of exon 55 is defined as basing point 0 and a base sequence region on the 5' side (upstream) from the basing point is indicated by "-" (minus). In this respect, the region indicated by the range of -400 bases to -1 base belongs to intron 54.

(A23) Region R23

The region R23 is a region indicated by the range of +1 base to +400 bases when the boundary between the 3' end of exon 55 and the 5' end of intron 55 is defined as basing point 0 and a base sequence region on the 3' side (downstream) from the basing point is indicated by "+". In this respect, the region indicated by the range of +1 base to +400 bases belongs to intron 55.

(A24) Region R24

The region R24 is a region indicated by the range of -400 bases to -1 base when the boundary between the 3' end of intron 55 and the 5' end of exon 56 is defined as basing point 0 and a base sequence region on the 5' side (upstream) from the basing point is indicated by "-" (minus). In this respect, the region indicated by the range of -400 bases to -1 base belongs to intron 55.

In the foregoing embodiment, specific examples of the base sequences of the regions R1 to R24 are as follows.

[Table 3]

Table 3

| Region | Base sequence of target region | SEQ ID NO |
|---|---|---|
| R1 | Region in vicinity of donor of intron 44 (in range of +1 to +400 bases from 5' end of intron 44 as basing point)<br>GTAAGTCTTTGATTTGTTTTTTCGAAATTGTATTTATCTTCAGCACATCTGGACTCTTTAA<br>CTTCTTAAAGATCAGGTTCTGAAGGGTGATGGAAATTACTTTTGACTGTTGTTGTCATCAT<br>TATATTACTAGAAAGAAAATTATCATAATGATAATATTAGAGCACGGTGCTATGGACTTTT<br>TGTGTCAGGATGAGAGAGTTTGCCTGGACGGAGCTGGTTTATCTGATAAACTGCAAAATAT<br>AATTGAATCTGTGACAGAGGGAAGCATCGTAACAGCAAGGTGTTTTGTGGCTTTGGGGCAG<br>TGTGTATTTCGGCTTTATGTTGGAACCTTTCCAGAAGGAGAACTTGTGGCATACTTAGCTA<br>AAATGAAGTTGCTAGAAATATCCATCATGATAAA | SEQ No. 346 |
| R2 | Region in vicinity of acceptor of intron 44 (in range of -600 to -1 bases from 3' end of intron 44 as basing point)<br>TCTTGATGGGATGCTCCTGAAAGCAATTAATTCTCAGTTTTTTGTGGCTTCTAATGCAAAA<br>TACATTGACGCAGACAGAATTTGAAATGAATTTTCTTCTAATATAGCAATTAATTTTATTT<br>AAATATCTCTAGAGTTTTTTTTTAATACTGTGACTAACCTATGTTTGTTCTTTTTCACCTC<br>TCGTATCCACGATCACTAAGAAACCCAAATACTTTGTTCATGTTTAAATTTTACAACATTT<br>CATAGACTATTAAACATGGAACATCCTTGTGGGGACAAGAAATCGAATTTGCTCTTGAAAA<br>GGTTTCCAACTAATTGATTTGTAGGACATTATAACATCCTCTAGCTGACAAGCTTACAAAA<br>ATAAAAACTGGAGCTAACCGAGAGGGTGCTTTTTTCCCTGACACATAAAAGGTGTCTTTCT<br>GTCTTGTATCCTTTGGATATGGGCATGTCAGTTTCATAGGGAAATTTTCACATGGAGCTTT<br>TGTATTTCTTTCTTTGCCAGTACAACTGCATGTGGTAGCACACTGTTTAATCTTTTCTCAA<br>ATAAAAAGACATGGGGCTTCATTTTTGTTTTGCCTTTTTGGTATCTTACAG | SEQ No. 347 |
| R3 | Region in vicinity of donor of intron 45 (in range of +1 to +400 bases from 5' end of intron 45 as basing point)<br>GTAGGGCGACAGATCTAATAGGAATGAAAACATTTTAGCAGACTTTTTAAGCTTTCTTTAG<br>AAGAATATTTCATGAGAGATTATAAGCAGGGTGAAAGGCACTAACATTAAAGAACCTATCA<br>ACCATTAATCAACAGCAGTAAAGAAATTTTTTATTTCTTTTTTTCATATACTAAAATATAT<br>ACTTGTGGCTAGTTAGTGGTTTTCTGCTATTTTAAACTTGAAGTTTGCTTTAAAAATCACC<br>CATGATTGCTTAAAGGTGAATATCTTCAATATATTTTAACTTCAACAAGCTGAATCTCAGT<br>TGTTTTTCAAGAAGATTTTAGAAAGCAATTATAAATGATTGTTTTGTAGGAAAGACAGATC<br>TTTGCTTAGTTTTAAAAATAGCTATGAATATGAC | SEQ No. 348 |
| R4 | Region in vicinity of acceptor of intron 45 (in range of -400 to -1 bases from 3' end of intron 45 as basing point)<br>ATTACAGGCGCCTGCCACCAAACCTGGCAAATTTTTGTATTTTTAGTGTAGACGGGGTTTC<br>ACCATATTTGCCAGGCTGGTCGCAAACTCCTGACCTCAAGTGATCCGCCCACATCGGCCTC<br>CCTAAGCGCTAGGGTTACAGGCATGAGCCACTGCGCCTGGCCAGGAATTTTTGAATCAGAA<br>TTTTTCTTGTTCGATTTTAATCTCTTATCATTTAGAGATTCTTGAAATATTGAAATTACTT<br>TGTTCAAAGTGAATGAATTTTCTTAAATTATGTATGGTTAACATCTTTTAAATTGCTTATT<br>TTTAAATTGCCATGTTTGTGTCCCAGTTTGCATTAACAAATAGTTTGAGAACTATGTTGGA<br>AAAAAAAATAACAATTTTATTCTTCTTTCTCCAG | SEQ No. 349 |

(continued)

| Region | Base sequence of target region | SEQ ID NO |
|---|---|---|
| R5 | Region in vicinity of donor of intron 46 (in range of +1 to +400 bases from 5' end of intron 46 as basing point)<br>GTAATTTTATTTTCTCAAATCCCCCAGGGCCTGCTTGCATAAAGAAGTATATGAATCTATT<br>TTTTAATTCAATCATTGGTTTTCTGCCCATTAGGTTATTCATAGTTCCTTGCTAAAGTGTT<br>TTTCTCACAACTTTATTTCTTCTTAACCCTGCAGTTCTGAACCAGTGCACATAAGAACATA<br>TGTATATATGTGTGTGTGTGTATTTATATATACACACACACATATTGCATCTATACATCTA<br>CACATATAGATGTATAGATTCAATATGTCTAAATGTATATAATTCACAGTTTTTATCTTTG<br>ATTTGAAATTAATTTTAGATTTTACTTGAGAACTTCACAACTTCATATAATTTTAAAAACT<br>GAAGACCAGATTGTGGAATCATAAAATCTAAATC | SEQ No. 350 |
| R6 | Region in vicinity of acceptor of intron 46 (in range of -400 to -1 n bases from 3' end of intron 46 as basing point)<br>ATAATGCAATTTCTCAGCAGCAAGCTACGGTATGCTATGGCATGCTATGATACCCAAGAGG<br>CTGATGAATTTGTTCACATTGTTCTATTTCTGATAGAGAGATAGGTTTTCAGACACTAACT<br>TTATTTGGAGTGTTGCTTTACCATCTCACATTTTTCTCTTAAAAAATTTATGAGGGATAAT<br>ATAATCGTTTATTTTCTACAGAGATTTATCTACTGAGGGGGTGAGTGTTTCAGTCAATCAG<br>CTCTGTGCTCAGATAGAAAACTGTTGGTATTTGAGGTACCACTGGGCCCTCGGTCAAGTCG<br>CTTCATTTTGATAGACTAATCAATAGAAGCAAAGACAAGGTAGTTGGAATTGTGCTGTAAT<br>TCATTTTAAACGTTGTTGCATTTGTCTGTTTCAG | SEQ No. 351 |
| R7 | Region in vicinity of donor of intron 47 (in range of +1 to +400 bases from 5' end of intron 47 as basing point)<br>GTTAGACATTAACCATCTCTTCCGTCACATGTGTTAAATGTTGCAAGTATTTGTATGTATT<br>TTGTTTCCTGGGTGCTTCATTGGTCGGGGAGGAGGCTGGTATGTGGATTGTTGTTTTGTTT<br>TGTTTTTTTAACCTGACCGTTTGCTTTGGCTATATGTTTTGTTGTGGCTAGAAAAAATGAT<br>GATGGTGAATGGCTTTACATTAATGACCAAATGCCAAAATTTATACCACAATTTTTTGCAT<br>AAATTATTCTGAAGAATCAGACTGAAGAAATGGCGAAGTATTTAATTCAGTGGCCAGGCAT<br>GTACTGACAGTATTTAAGCTGAAAGGACGTGGTCTGGTTCTAGTTAAACAAGTGTCATAAA<br>TCAAAATTAATTATTCACACCTGTGGTATGGACT | SEQ No. 352 |
| R8 | Region in vicinity of acceptor of intron 47 (in range of -400 to -1 bases from 3' end of intron 47 as basing point)<br>TATTCATTTTTATAACTGCAAAGGAAGCGCGTATGGCATATAATACACAACACACCAGTAT<br>ATTTAGTAACTGAGTGAATAAATGAAAGATGTATTTCTTTACTTTATCAGTTGCAGTTGGC<br>TATGCCTTTGTGTAAGGTGTGTGTTTTGAAATTCCAAAAAGGTATTAGTTTCTTTAAAGCA<br>AAGAATTTTTGTAGCAGGTTAATGAATAATTTTGAATACATTGGTTAAATCCCAACATGTA<br>ATATATGTAAATAATCAATATTATGCTGCTAAAATAACACAAATCAGTAAGATTCTGTAAT<br>ATTTCATGATAAATAACTTTTGAAAATATATTTTTAAACATTTTGGCTTATGCCTTGAGAA<br>TTATTTACCTTTTTAAAATGTATTTTCCTTTCAG | SEQ No. 353 |
| R9 | Region in vicinity of donor of intron 48 (in range of +1 to +400 bases from 5' end of intron 48 as basing point)<br>GTAGGGAACTTTTTGCTTTAAATATTTTTGTCTTTTTTAAGAAAAATGGCAATATCACTGA<br>ATTTTCTCATTTGGTATCATTATTAAAGACAAAATATTACTTGTTAAAGTGTGGTAAGGAA<br>GACTTTATTCAGGATAACCACAATAGGCACAGGGACCACTGCAATGGAGTATTACAGGAGG<br>TTGGATAGAGAGAGATTGGGCTCAACTCTAAATACAGCACAGTGGAAGTAGGAATTTATAG<br>CCAAGGAGCAGTGTAGGAGTCAGTAGATGGAAAATTATTAAGAGGAAACATCAGGGGTAAG<br>TGGGATTCTGGCTAAACCAACCTCACAGGATTCTTGCTGAAGATAGGCCAGGGTTATCTTA<br>TCAGACAACCCTTGGGGAATGGTGGAGAATACTG | SEQ No. 354 |

(continued)

| Region | Base sequence of target region | SEQ ID NO |
|---|---|---|
| R10 | Region in vicinity of acceptor of intron 48 (in range of -400 to -1 bases from 3' end of intron 48 as basing point)<br>TACTAAACACAGAATTTTGTAAAACAATAAGTGTATAAAGTAAAATGAACATTAGGATTAT<br>TGAGATTATTGTAGCTAAAACTAGTGTTTATTCATATAAATTATGTTAATAAATTGTATTG<br>TCATTATTGCATTTTACTTTTTTGAAAAGTAGTTAATGCCTGTGTTTCTATATGAGTATTA<br>TATAATTCAAGAAGATATTGGATGAATTTTTTTTAAGTTTAATGTGTTTCACATCTCTGTT<br>TCTTTTCTCTGCACCAAAAGCTACATTTTTGTGCCCTTATGTACCAGGCAGAAATTGATCT<br>GCAATACATGTGGAGTCTCCAAGGGTATATTTAAATTTAGTAATTTTATTGCTAACTGTGA<br>AGTTAATCTGCACTATATGGGTTCTTTTCCCCAG | SEQ No. 355 |
| R11 | Region in vicinity of donor of intron 49 (in range of +1 to +400 bases from 5' end of intron 49 as basing point)<br>GTAATGAAGCAACCTCTAGCAATATCCATTACCTCATAATGGGTTATGCTTCCCCTGTTGT<br>ACATTTGCCATTGACGTGGACTATTTATAATCAGTGAAATAACTTGTAAGGAAATACTGGC<br>CATACTGTAATAGCAGAGGCAAAGCTGTCTTTTTGATCAGCATATCCTATTTATATATTGT<br>GATCTTAAGGCTATTAACGAGTCATTGCTTTAAAGGACTCATTTCTGTCCTGGTGTGCTGC<br>CATCAATACAAAAGTAGTCCCACCTTCAAGGTAGATTAAATTCTTTGGGGCTTTATTGCTT<br>TGCTTGCCAGCCTTGATGCTTTTCATATTGTTTGGTTTAATTCAAATCAAGCTACTGCATC<br>ATAGTGTCTGTCTCCAACAGCTGTAAAGAATCAC | SEQ No. 356 |
| R12 | Region in vicinity of acceptor of intron 49 (in range of -400 to -1 bases from 3' end of intron 49 as basing point)<br>ATATAATTGACTGGGGGTGAGCCAGTACATTAGGATTTTCCTAAAGTTATCTGGATAATTT<br>TAGTATGCAACCACAATAGATACTCTTCAAGAATTAAGCTAGTTGCTGAGAGGGAACTGTT<br>TTTTGTTGGTTTGTTTTCACTAATGTTTGCACTCTACTTCCTTTAAATAAAATTATGCCTG<br>GAGAAAGGGTTTTTGTATGGAGCAATTGATAAATATTTGTAGGGTGGTTGGCTAAAATAAT<br>TATAATTCCTTTAAAAGAAATTCTACCCACTAAAGTTAATTTAGAAGTAAAATATAATAGA<br>AATCCAATAATATATTCACCAAATGGATTAAGATGTTCATGAATTATCTTCAAAGTGTTAA<br>TCGAATAAGTAATGTGTATGCTTTTCTGTTAAAG | SEQ No. 357 |
| R13 | Region in vicinity of donor of intron 50 (in range of +1 to +400 bases from 5' end of intron 50 as basing point)<br>GTAAGTATACTGGATCCCATTCTCTTTGGCTCTAGCTATTTGTTCAAAAGTGCAACTATGA<br>AGTGATGACTGGGTGAGAGAGAAAATTTGTTTCAATTCTAAAGATAGAGATAAACCTTTGT<br>GTTATTGACTGTGCAAAAAGTCTTAGAGTACATTCCTTGGAAATTGACTCTGATTCAAAGT<br>GTTGCATGACAACGGGATATGGGGAGTGTTCTCTGGAGATACACCCACAAGGAAGAGAAGA<br>GCACAAGGGAGATTGTGGGAGAGTCTGAAATGTGATTTGTCTGCAGCAGAGGCCTAAGCCA<br>GTCTCGCAGGAGCCCTACATCTGGGCTGGCTGTGCAGAGCTGTCCTGAATTGCAGGCAGTG<br>GGCCTGGCCCTTGTATTCCTGATCCAGCCAGCCA | SEQ No. 358 |
| R14 | Region in vicinity of acceptor of intron 50 (in range of -400 to -1 bases from 3' end of intron 50 as basing point)<br>TCTTGAATAAAAAAAAAATAAGTAAAATTTATTTCCCTGGCAAGGTCTGAAAACTTTTGTT<br>TTCTTTACCACTTCCACAATGTATATGATTGTTACTGAGAAGGCTTATTTAACTTAAGTTA<br>CTTGTCCAGGCATGAGAATGAGCAAAATCGTTTTTTAAAAAATTGTTAAATGTATATTAAT<br>GAAAAGGTTGAATCTTTTCATTTTCTACCATGTATTGCTAAACAAAGTATCCACATTGTTA<br>GAAAAAGATATATAATGTCATGAATAAGAGTTTGGCTCAAATTGTTACTCTTCAATTAAAT<br>TTGACTTATTGTTATTGAAATTGGCTCTTTAGCTTGTGTTTCTAATTTTTCTTTTTCTTCT<br>TTTTTCCTTTTTGCAAAAACCCAAAATATTTTAG | SEQ No. 359 |

(continued)

| Region | Base sequence of target region | SEQ ID NO |
|---|---|---|
| R15 | Region in vicinity of donor of intron 51 (in range of +1 to +400 bases from 5' end of intron 51 as basing point)<br>GTATGAGAAAAAATGATAAAAGTTGGCAGAAGTTTTTCTTTAAAATGAAGATTTTCCACCA<br>ATCACTTTACTCTCCTAGACCATTTCCCACCAGTTCTTAGGCAACTGTTTCTCTCTCAGCA<br>AACACATTACTCTCACTATTCAGCCTAAGTATAATCAAGGATATAAATTAATGCAAATAAC<br>AAAAGTAGCCATACATTAAAAAGGAAATATACAAAAAAAAAAAAAAAAAAAGCAGAAACC<br>TTACAAGAATAGTTGTCTCAGTTAAATTTACTAAACAACCTGGTATTTTAAAAATCTATTT<br>TATACCAAATAAGTCACTCAACTGAGCTATTTACATTTAAACTGTTTGTTTTGGCACTACG<br>CAGCCCAACATATTGCAGAATCAAATATAATAGT | SEQ No. 360 |
| R16 | Region in vicinity of acceptor of intron 51 (in range of -400 to -1 bases from 3' end of intron 51 as basing point)<br>TTTAAAATAAATATACCTTAATTTTGACGTCACACAGAATGATATTATAAGTATAAATAGT<br>TATCTATCTTTTAAATACATTGTCGTAATTCAGAATAACATTTCTTACTCAAGGCATTCAG<br>ACAGTGGTTTAAGTAATCCGAGGTACTCCGGAATGTCTCCATTTGAGCCTTTAAATGAAGA<br>AAATCTATAGTCAAGATTTTCATTTGAAATATTTTTGATATCTAAGAATGAAACATATTTC<br>CTGTTAAATTGTTTTCTATAAACCCTTATACAGTAACATCTTTTTTATTTCTAAAAGTGTT<br>TTGGCTGGTCTCACAATTGTACTTTACTTTGTATTATGTAAAAGGAATACACAACGCTGAA<br>GAACCCTGATACTAAGGGATATTTGTTCTTACAG | SEQ No. 361 |
| R17 | Region in vicinity of donor of intron 52 (in range of +1 to +400 bases from 5' end of intron 52 as basing point)<br>GTAAGTTTTTTAACAAGCATGGGACACACAAAGCAAGATGCATGACAAGTTTCAATAAAAA<br>CTTAAGTTCATATATCCCCCTCACATTTATAAAAATAATGTGAAATAATTGTAAATGATAA<br>CAATTGTGCTGAGATTTTCAGTCCATAATGTTACCTTTTAATAAATGAATGTAATTCCATT<br>GAATAGAAGAAATACATTTTTAAATCAATTCAGGGCTTATATAGTTGCAAAGCATGCATTG<br>ATGGGTGTGGTGACCACAGTGTGGCAGAACATTTGTGGCAGAACATTTGTTCTTTAGTTGT<br>CATCTGGGCTGGCATCCATGGAGATGCCAGTCTCTCCCTCATATCCTTGGCTGTTGGTCCA<br>AGCAGGCAGTGGCTTCTTCCTGGGCCATCTTTCA | SEQ No. 362 |
| R18 | Region in vicinity of acceptor of intron 52 (in range of -400 to -1 bases from 3' end of intron 52 as basing point)<br>CAAACTCCTGTGGCAACAGAAAGCCTTCAGGCAATGAAATGCTGGCACTGGGAAATCAGGC<br>TGATGGGTGCTGAAGTGGCAAGGATGAGGGGATATGGATATTCTGCTGTAGTGCTTTTCTA<br>ACAGATGATTCATATTTGGTTCTAGGGATCAAGAATTGAGTTAAAATTTTATATATATGTT<br>GATGTTCTATGTCACCTTCAGGAAAATAATTTAACAGAAACTAATATTTGCCATCAAAAAA<br>GCAAAGAATCCTGTTGTTCATCATCCTAGCCATAACACAATGAATAATTTTTTAAATAAGC<br>AACATAAATGTGAGATAACGTTTGGAAGTTACATTTAAAATGTCTCCTCCAGACTAGCATT<br>TACTACTATATATTTATTTTTCCTTTTATTCTAG | SEQ No. 363 |
| R19 | Region in vicinity of donor of intron 53 (in range of +1 to +400 bases from 5' end of intron 53 as basing point)<br>GTTAGTATCAAAGATACCTTTTTAAAATAAAATACTGGTTACATTTGATAAAATTATACCA<br>TAGATTGTAATTTAATGATGTTTAATGTAAAGTTATTAACAGAAAATCACGTTAAAGCTGA<br>AATGAACAGTAGACTTTGTATATTTATTTTCTTAGAGACAGAGTCTCACTGTCACCCAGGC<br>TAAAGTGCAGTGGCACAATCATAGCTCACTGAGCCTTGAACTCTGGGGCTCAAGCAGTCCT<br>CCTGCCTCAGCCTCCCTAGTAGCTGGGACTACTAGCCAGGCGTGTACCACCACGCCTGGCT<br>AATTTTTTAAAAATTTTTGTTTTCTGTAGAGATGGGTTCTTGAACTCTTGGCCTCAAGCAA<br>TTCTCCTTCCTTGGCCTCCCAAAGCACTAGGATT | SEQ No. 364 |

(continued)

| Region | Base sequence of target region | SEQ ID NO |
|---|---|---|
| R20 | Region in vicinity of acceptor of intron 53 (in range of -400 to -1 bases from 3' end of intron 53 as basing point)<br>CAACAAGTTAATGAAGAGGGAAAGAAATGTATGAGGTTTTTTTCGTTCAAATGTTGTTATA<br>TGTCACATATTCAACAATTATATATGAGCTTATTTTTGTAGTTTTTTTCTCTTGTGATAAA<br>AACAATTAAGCCCACTTTATTGCCAATTAATTGCTACTAAGTTGAAATACTTGATACTGGT<br>TATTGCTCAAGATGCTGCATTTGAAAAGTTTGTCCTGAAAGGTGGGTTACCTTATACTGTC<br>ATGATTGACTAAATCATATGGTAGGTTAAAAGCAATCTAATATATGTATTCTGACCTGAGG<br>ATTCAGAAGCTGTTTACGAAGTATTTTAAGACACTCCAACTAGAGATTTCATAAAAAAAAC<br>TGACATTCATTCTCTTTCTCATAAAAATCTATAG | SEQ No. 365 |
| R21 | Region in vicinity of donor of intron 54 (in range of +1 to +400 bases from 5' end of intron 54 as basing point)<br>GTATGAATTACATTATTTCTAAAACTACTGTTGGCTGTAATAATGGGGTGGTGAAACTGGA<br>TGGACCATGAGGATTTGTTTTTCCAATCCAGCTAAACTGGAGCTTGGGAGGGTTCAAGACG<br>ATAAATACCAACTAAACTCACGGACTTGGCTCAGACTTCTATTTTAAAAACGAGGAACATA<br>AGATCTCATTTGCCCGCTGTCACAAAAGTAGTGACATAACCAAGAGATTAAACAAAAAGCA<br>AAATACTGATTTATAGCTAGAAGAGCCATTTATCAGTCTACTTTGATAACTCTATCCAAAG<br>GAATATCTTTCTATCTCATCATGGCGCACACTGCCTTACCTGTTATCTGATAAATAAGTCA<br>CTTTGGGATTCATGATAGAGTTATAGCTGTACAT | SEQ No. 366 |
| R22 | Region in vicinity of acceptor of intron 54 (in range of -400 to -1 bases from 3' end of intron 54 as basing point)<br>TCTCAAATTTGGCAGTATATTAAAAATAAGCTTTCAAAATTGACCAACAAAAACTACAAAA<br>TTGAAAAAAAGGTACTTTGAACTTTCACATGTTCAAATATATGTATATATATTTCACATAT<br>ATATATGAAACCTCCTCTGTGGAGAGGGGTTTATAGAAATCTGTAATTGTCATTCTTGCAT<br>GCCTTCCCCCATACAAACGCCTTTAAGTTAAATAAAAATGAAAGTAAATAGACTGCACAAT<br>ATTATAGTTGTTGCTTAAAGGAAGAGCTGTAGCAACAACTCACCCCATTGTTGGTATATTA<br>CAATTTAGTTCCTCCATCTTTCTCTTTTTATGGAGTTCACTAGGTGCACCATTCTGATATT<br>TAATAATTGCATCTGAACATTTGGTCCTTTGCAG | SEQ No. 367 |
| R23 | Region in vicinity of donor of intron 55 (in range of +1 to +400 nucleotides from 5' end of intron 55 as basing point)<br>GTAAGTCAGGCATTTCCGCTTTAGCACTCTTGTGGATCCAATTGAACAATTCTCAGCATTT<br>GTACTTGTAACTGACAAGCCAGGGACAAAACAAAATAGTTGCTTTTATACAGCCTGATGTA<br>TTTCGGTATTTGGACAAGGAGGAGAGAGGCAGAGGGAGAAGGAAACATCATTTATAATTCC<br>ACTTAACACCCTCGTCTTAGAAAAAGTACATGCTCTGACCAGGAAAACATTTGCATATAAA<br>ACCAGAGCTTCGGTCAAGGAGAAACTTTGCTCAGAGAAATAACTTAGGGATTGGTTTATTA<br>AATTTTAAAAGTTGACATTTTTGAGTGTTTATTTAATATTTTACAGGGAAAGCATCTGTAT<br>GAATTGTCTGTTTTATTTAGCGTTGCTAACTGAA | SEQ No. 368 |
| R24 | Region in vicinity of acceptor of intron 55 (in range of -400 to -1 bases from 3' end of intron 55 as basing point)<br>ACTTGATCCATATAGTAATGAAATTATTGGCACTGGGGTACACTTTATCATAGAATTTTAT<br>TGCCTATCACTTCCATAAAATAATACATTTTGTCCATAGACTAGAAGATATAACTTGTGAA<br>CTTTATAAAGTTATAAATACATTACTTTCCAACTCATAATGGCAAGGAATAAATCTATTAC<br>AACTAATAAGATGCCCATTTTAAATCTACATAATAACAGGAGAAGGCAATACGCCAAGAAA<br>AGGGATTTGAGATGTATCTTCTTGTTAGTTTAGCCTGATTGAAATGTCTTTTGAACTAATA<br>ATTATTTATATTTTGCAATTCTCCAAATTCACATTCATCGCTTGTTTCTTTTGTTTGGTAA<br>TTCTGCACATATTCTTCTTCCTGCTGTCCTGTAG | SEQ No. 369 |

The target regions R1 to R24 of the antisense oligomer of the present invention include both wild (e.g., the regions represented by SEQ ID NOs: 233 to 256, 341 to 369, and 385 to 389) and mutant types in relation to the human dystrophin pre-mRNA. Such a mutant type specifically has any one base sequence selected from the group consisting

of base sequences (B0) and (B1) to (B16) below:

(B0) a base sequence that hybridizes under stringent conditions to a base sequence complementary to any one base sequence selected from the group consisting of SEQ ID NOs: 233 to 256, 341 to 369, and 385 to 389;
(B1) a base sequence that has at least 85% identity with any one base sequence selected from the group consisting of SEQ ID NOs: 233 to 256, 341 to 369, and 385 to 389,
and has a length within ±15% of the length of the any one base sequence selected;
(B2) a base sequence that has at least 86% identity with any one base sequence selected from the group consisting of SEQ ID NOs: 233 to 256, 341 to 369, and 385 to 389, and has a length within ±14% of the length of the any one base sequence selected;
(B3) a base sequence that has at least 87% identity with any one base sequence selected from the group consisting of SEQ ID NOs: 233 to 256, 341 to 369, and 385 to 389, and has a length within ±13% of the length of the any one base sequence selected;
(B4) a base sequence that has at least 88% identity with any one base sequence selected from the group consisting of SEQ ID NOs: 233 to 256, 341 to 369, and 385 to 389, and has a length within ±12% of the length of the any one base sequence selected;
(B5) a base sequence that has at least 89% identity with any one base sequence selected from the group consisting of SEQ ID NOs: 233 to 256, 341 to 369, and 385 to 389, and has a length within ±11% of the length of the any one base sequence selected;
(B6) a base sequence that has at least 90% identity with any one base sequence selected from the group consisting of SEQ ID NOs: 233 to 256, 341 to 369, and 385 to 389, and has a length within ±10% of the length of the any one base sequence selected;
(B7) a base sequence that has at least 91% identity with any one base sequence selected from the group consisting of SEQ ID NOs: 233 to 256, 341 to 369, and 385 to 389, and has a length within ±9% of the length of the any one base sequence selected;
(B8) a base sequence that has at least 92% identity with any one base sequence selected from the group consisting of SEQ ID NOs: 233 to 256, 341 to 369, and 385 to 389, and has a length within ±8% of the length of the any one base sequence selected;
(B9) a base sequence that has at least 93% identity with any one base sequence selected from the group consisting of SEQ ID NOs: 233 to 256, 341 to 369, and 385 to 389, and has a length within ±7% of the length of the any one base sequence selected;
(B10) a base sequence that has at least 94% identity with any one base sequence selected from the group consisting of SEQ ID NOs: 233 to 256, 341 to 369, and 385 to 389, and has a length within ±6% of the length of the any one base sequence selected;
(B11) a base sequence that has at least 95% identity with any one base sequence selected from the group consisting of SEQ ID NOs: 233 to 256, 341 to 369, and 385 to 389, and has a length within ±5% of the length of the any one base sequence selected;
(B12) a base sequence that has at least 96% identity with any one base sequence selected from the group consisting of SEQ ID NOs: 233 to 256, 341 to 369, and 385 to 389, and has a length within ±4% of the length of the any one base sequence selected;
(B13) a base sequence that has at least 97% identity with any one base sequence selected from the group consisting of SEQ ID NOs: 233 to 256, 341 to 369, and 385 to 389, and has a length within ±3% of the length of the any one base sequence selected;
(B14) a base sequence that has at least 98% identity with any one base sequence selected from the group consisting of SEQ ID NOs: 233 to 256, 341 to 369, and 385 to 389, and has a length within ±2% of the length of the any one base sequence selected;
(B15) a base sequence that has at least 99% identity with any one base sequence selected from the group consisting of SEQ ID NOs: 233 to 256, 341 to 369, and 385 to 389, and has a length within ±1% of the length of the any one base sequence selected;
and
(B16) a base sequence that has at least 99.5% identity with any one base sequence selected from the group consisting of SEQ ID NOs: 233 to 256, 341 to 369, and 385 to 389, and has a length within ±0.5% of the length of the any one base sequence selected.

As used herein, the term "base sequence that hybridizes under stringent conditions" refers to, for example, a base sequence obtained by colony hybridization, plaque hybridization, Southern hybridization or the like, using as a probe all or part of a base sequence complementary to, *e.g.,* any one base sequence selected from the group consisting of SEQ ID NOs: 233 to 256, 341 to 369, and 385 to 389. The hybridization method which may be used includes methods

described in, for example, "Sambrook & Russell, Molecular Cloning: A Laboratory Manual Vol. 3, Cold Spring Harbor, Laboratory Press, 2001," "Ausubel, Current Protocols in Molecular Biology, John Wiley & Sons, 1987-1997," etc.

As used herein, the term "complementary base sequence" is not limited to a base sequence that forms Watson-Crick pairs with an intended base sequence, and also includes a base sequence that forms wobble base pairs therewith. Herein, the Watson-Crick pair means a base pair that forms a hydrogen bond between adenine and thymine, between adenine and uracil, or between guanine and cytosine, and the wobble base pair means a base pair that forms a hydrogen bond between guanine and uracil, between inosine and uracil, between inosine and adenine, or between inosine and cytosine. The term "complementary base sequence" does not have to have 100% complementarity with the intended base sequence and may contain, for example, 1, 2, 3, 4, or 5 noncomplementary bases to the intended base sequence or may be a base sequence shorter by 1 base, 2 bases, 3 bases, 4 bases, or 5 bases than the intended base sequence.

As used herein, the term "stringent conditions" may be any of low stringent conditions, moderate stringent conditions or high stringent conditions. The term "low stringent condition" is, for example, 5 × SSC, 5 × Denhardt's solution, 0.5% SDS, 50% formamide at 32°C. The term "moderate stringent condition" is, for example, 5 × SSC, 5 × Denhardt's solution, 0.5% SDS, 50% formamide at 42°C, or 5 × SSC, 1% SDS, 50 mM Tris-HCl (pH 7.5), 50% formamide at 42°C. The term "high stringent condition" is, for example, 5 × SSC, 5 × Denhardt's solution, 0.5% SDS, 50% formamide at 50°C, or 0.2 × SSC, 0.1% SDS at 65°C. Under these conditions, base sequences with higher identity are expected to be obtained efficiently at higher temperatures, although multiple factors are involved in hybridization stringency including temperature, probe concentration, probe length, ionic strength, time, salt concentration and others, and those skilled in the art may appropriately select these factors to achieve similar stringency.

When commercially available kits are used for hybridization, for example, an Alkphos Direct Labelling and Detection System (GE Healthcare) may be used. In this case, according to the attached protocol, after incubation with a labeled probe overnight, the membrane can be washed with a primary wash buffer containing 0.1% (w/v) SDS at 55°C, thereby detecting hybridization. Alternatively, when the probe is labeled with digoxigenin (DIG) using a commercially available reagent (e.g., a PCR Labelling Mix (Roche Diagnostics), etc.) in producing a probe based on all or part of the complementary sequence to any one base sequence selected from the group consisting of SEQ ID NOs: 233 to 256, 341 to 369, and 385 to 389, hybridization can be detected with a DIG Nucleic Acid Detection Kit (Roche Diagnostics) or the like.

The identity between base sequences may be determined using algorithm BLAST (Basic Local Alignment Search Tool) by Karlin and Altschul (Proc. Natl. Acad. Sci. USA 872264-2268, 1990; Proc. Natl. Acad. Sci. USA 90: 5873, 1993). Programs called BLASTN and BLASTX based on the BLAST algorithm have been developed (Altschul SF, et al: J. Mol. Biol. 215: 403, 1990). When a base sequence is analyzed using BLASTN, the parameters are, for example, score = 100 and wordlength = 12. When BLAST and Gapped BLAST programs are used, the default parameters for each program are employed.

The antisense oligomer of the present invention comprises a base sequence complementary to a base sequence of at least any one region selected from the group consisting of the target regions R1 to R24 of the present invention, or a partial base sequence thereof. The term "partial" means a region, except for the full length, of at least any one region selected from the group consisting of the target regions R1 to R24, i.e., a partial region of at least any one region selected from the group consisting of the target regions R1 to R24. The partial region may be 10 to 60 bases long, 10 to 55 bases long, 10 to 50 bases long, 10 to 45 bases long, 10 to 40 bases long, 10 to 35 bases long, 10 to 30 bases long, 10 to 25 bases long, 15 to 60 bases long, 15 to 55 bases long, 15 to 50 bases long, 15 to 45 bases long, 15 to 40 bases long, 15 to 35 bases long, 15 to 30 bases long, 15 to 25 bases long, 16 to 60 bases long, 16 to 55 bases long, 16 to 50 bases long, 16 to 45 bases long, 16 to 40 bases long, 16 to 35 bases long, 16 to 30 bases long, 16 to 25 bases long, 17 to 60 bases long, 17 to 55 bases long, 17 to 50 bases long, 17 to 45 bases long, 17 to 40 bases long, 17 to 35 bases long, 17 to 30 bases long, 17 to 25 bases long, 18 to 60 bases long, 18 to 55 bases long, 18 to 50 bases long, 18 to 45 bases long, 18 to 40 bases long, 18 to 35 bases long, 18 to 30 bases long, 18 to 25 bases long, 19 to 60 bases long, 19 to 55 bases long, 19 to 50 bases long, 19 to 45 bases long, 19 to 40 bases long, 19 to 35 bases long, 19 to 30 bases long, 19 to 25 bases long, 20 to 60 bases long, 20 to 55 bases long, 20 to 50 bases long, 20 to 45 bases long, 20 to 40 bases long, 20 to 35 bases long, 20 to 30 bases long, 20 to 25 bases long, 15 to 30 bases long, 15 to 29 bases long, 15 to 28 bases long, 15 to 27 bases long, 15 to 26 bases long, 15 to 25 bases long, 15 to 24 bases long, 15 to 23 bases long, 15 to 22 bases long, 15 to 21 bases long, 15 to 20 bases long, 15 to 19 bases long, 15 to 18 bases long, 16 to 30 bases long, 16 to 29 bases long, 16 to 28 bases long, 16 to 27 bases long, 16 to 26 bases long, 16 to 25 bases long, 16 to 24 bases long, 16 to 23 bases long, 16 to 22 bases long, 16 to 21 bases long, 16 to 20 bases long, 16 to 19 bases long, 16 to 18 bases long, 17 to 30 bases long, 17 to 29 bases long, 17 to 28 bases long, 17 to 27 bases long, 17 to 26 bases long, 17 to 25 bases long, 17 to 24 bases long, 17 to 23 bases long, 17 to 22 bases long, 17 to 21 bases long, 17 to 20 bases long, 17 to 19 bases long, 17 to 18 bases long, 18 to 30 bases long, 18 to 29 bases long, 18 to 28 bases long, 18 to 27 bases long, 18 to 26 bases long, 18 to 25 bases long, 18 to 24 bases long, 18 to 23 bases long, 18 to 22 bases long, 18 to 21 bases long, 18 to 20 bases long, 18 to 19 bases long, 19 to 30 bases long, 19 to 29 bases long, 19 to 28 bases long, 19 to 27 bases long, 19 to 26 bases long, 19 to 25 bases long, 19 to 24 bases long, 19 to 23

bases long, 19 to 22 bases long, 19 to 21 bases long, 19 to 20 bases long, 20 to 30 bases long, 20 to 29 bases long, 20 to 28 bases long, 20 to 27 bases long, 20 to 26 bases long, 20 to 25 bases long, 20 to 24 bases long, 20 to 23 bases long, 20 to 22 bases long, 20 to 21 bases long, 5 to 25 bases long, 5 to 24 bases long, 5 to 23 bases long, 5 to 22 bases long, 5 to 21 bases long, 5 to 20 bases long, 5 to 19 bases long, 5 to 18 bases long, 5 to 17 bases long, 5 to 16 bases long, 5 to 15 bases long, 5 to 14 bases long, 5 to 13 bases long, 5 to 12 bases long, 7 to 25 bases long, 7 to 24 bases long, 7 to 23 bases long, 7 to 22 bases long, 7 to 21 bases long, 7 to 20 bases long, 7 to 19 bases long, 7 to 18 bases long, 7 to 17 bases long, 7 to 16 bases long, 7 to 15 bases long, 7 to 14 bases long, 7 to 13 bases long, 7 to 12 bases long, 9 to 25 bases long, 9 to 24 bases long, 9 to 23 bases long, 9 to 22 bases long, 9 to 21 bases long, 9 to 20 bases long, 9 to 19 bases long, 9 to 18 bases long, 9 to 17 bases long, 9 to 16 bases long, 9 to 15 bases long, 9 to 14 bases long, 9 to 13 bases long, 9 to 12 bases long, 10 to 25 bases long, 10 to 24 bases long, 10 to 23 bases long, 10 to 22 bases long, 10 to 21 bases long, 10 to 20 bases long, 10 to 19 bases long, 10 to 18 bases long, 10 to 17 bases long, 10 to 16 bases long, 10 to 15 bases long, 10 to 14 bases long, 10 to 13 bases long, 10 to 12 bases long, 60 bases long, 59 bases long, 58 bases long, 57 bases long, 56 bases long, 55 bases long, 54 bases long, 53 bases long, 52 bases long, 51 bases long, 50 bases long, 49 bases long, 48 bases long, 47 bases long, 46 bases long, 45 bases long, 44 bases long, 43 bases long, 42 bases long, 41 bases long, 40 bases long, 39 bases long, 38 bases long, 37 bases long, 36 bases long, 35 bases long, 34 bases long, 33 bases long, 32 bases long, 31 bases long, 30 bases long, 29 bases long, 28 bases long, 27 bases long, 26 bases long, 25 bases long, 24 bases long, 23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, 10 bases long, 9 bases long, 8 bases long, 7 bases long, 6 bases long, or 5 bases long, but not limited thereto. These lengths may be increased or decreased by 1, 2, or 3 bases.

The antisense oligomer of the present invention has an activity to cause simultaneous skipping of any two or more numerically consecutive exons selected from the group consisting of the 45th exon to the 55th exon in human dystrophin pre-mRNA. As used herein, such skipping of two or more numerically consecutive exons from pre-mRNA of interest is referred to as "multi-exon skipping" or "multi-skipping", and this activity is referred to as "multi-exon skipping activity" or "multi-skipping activity".

Herein, the any two or more numerically consecutive exons selected from the group consisting of the 45th exon to the 55th exon mean a plurality of exons that increase one by one in exon number among 11 exons from the 45th exon to the 55th exon included in pre-mRNA. The exon number means a number assigned to exons in order from the 5' end to the 3' end with an exon at the most upstream position of pre-mRNA defined as the first exon, followed by the second, the third, ..., and the 79th exons among 79 exons included in human dystrophin pre-mRNA. An intron is numbered as the same number as that of an exon positioned on the 5' side thereof. Specifically, the 45th intron is flanked by the 45th exon positioned on the 5' side thereof and the 46th exon positioned on the 3' side thereof. As used herein, the "nth" exon or intron means the nth exon or intron counted from the 5' end toward the 3' end in pre-mRNA.

Table 4 shows combinations of exons included in the any two or more numerically consecutive exons selected from the group consisting of the 45th exon to the 55th exon.

[Table 4]

| Combination | Exons included | Combination | Exons included |
|---|---|---|---|
| Combination 1 | 45, 46 | Combination 29 | 48-50 |
| Combination 2 | 45-47 | Combination 30 | 48-51 |
| Combination 3 | 45-48 | Combination 31 | 48-52 |
| Combination 4 | 45-49 | Combination 32 | 48-53 |
| Combination 5 | 45-50 | Combination 33 | 48-54 |
| Combination 6 | 45-51 | Combination 34 | 48-55 |
| Combination 7 | 45-52 | Combination 35 | 49, 50 |
| Combination 8 | 45-53 | Combination 36 | 49-51 |
| Combination 9 | 45-54 | Combination 37 | 49-52 |
| Combination 10 | 45-55 | Combination 38 | 49-53 |
| Combination 11 | 46, 47 | Combination 39 | 49-54 |
| Combination 12 | 46-48 | Combination 40 | 49-55 |
| Combination 13 | 46-49 | Combination 41 | 50, 51 |

(continued)

| Combination | Exons included | Combination | Exons included |
|---|---|---|---|
| Combination 14 | 46-50 | Combination 42 | 50-52 |
| Combination 15 | 46-51 | Combination 43 | 50-53 |
| Combination 16 | 46-52 | Combination 44 | 50-54 |
| Combination 17 | 46-53 | Combination 45 | 50-55 |
| Combination 18 | 46-54 | Combination 46 | 51, 52 |
| Combination 19 | 46-55 | Combination 47 | 51-53 |
| Combination 20 | 47, 48 | Combination 48 | 51-54 |
| Combination 21 | 47-49 | Combination 49 | 51-55 |
| Combination 22 | 47-50 | Combination 50 | 52, 53 |
| Combination 23 | 47-51 | Combination 51 | 52-54 |
| Combination 24 | 47-52 | Combination 52 | 52-55 |
| Combination 25 | 47-53 | Combination 53 | 53, 54 |
| Combination 26 | 47-54 | Combination 54 | 53-55 |
| Combination 27 | 47-55 | Combination 55 | 54, 55 |
| Combination 28 | 48, 49 | | |

Among the combinations of exons described in Table 4, for example, the combination 1, 2, 3, 4, 6, 8, 10, 18, 20, 21, 23, 25, 27, 28, 30, 32, 34, 36, 38, 40, 41, 43, 45, 46, 50, 52, or 55 is a skipping pattern expected to exert higher therapeutic effects on DMD. Multi-exon skipping in such a combination is expected to exert therapeutic effects on more patients with DMD.

The any two or more numerically consecutive exons selected from the group consisting of the 45th exon to the 55th exon may include a plurality of groups of consecutive exons and may be, for example, but not limited to, (example 1) exons 45 and 46 (first exon group) and exons 48 to 53 (second exon group), or (example 2) exons 46 and 47 (first exon group), exons 49 and 50 (second exon group), and exons 52 to 54 (third exon group).

In the present invention, the term "activity to cause skipping" (i.e., multi-skipping activity) means, when human dystrophin pre-mRNA is taken as an example, an activity to produce human dystrophin mRNA having deletion of any two or more numerically consecutive exons selected from the group consisting of the 45th exon to the 55th exon in the human dystrophin pre-mRNA.

In other words, this activity means that by binding of the antisense oligomer of the present invention to a target site in human dystrophin pre-mRNA, the 5'-terminal nucleotide of an exon immediately downstream of the exons to be deleted is linked to the 3'-terminal nucleotide of an exon immediately upstream of the exons to be deleted when the pre-mRNA undergoes splicing, thus resulting in formation of mature mRNA which is free of codon frame shift (i.e., mature mRNA having deletion of the exons without frame shift).

The antisense oligomer of the present invention exhibits a multi-skipping activity under physiological conditions. The term "under physiological conditions" refers to conditions set to mimic the *in vivo* environment in terms of pH, salt composition and temperature. The conditions are, for example, 25 to 40°C, preferably 37°C, pH 5 to 8, preferably pH 7.4 and 150 mM of sodium chloride concentration. When multi-skipping is induced, one or more, for example, 2, 3, 4, 5, 6, 7, 8, 9 or 10 antisense oligomers, can be used in combination as the antisense oligomer of the present invention.

Whether multi-skipping is caused or not can be confirmed by introducing the antisense oligomer of the present invention into a dystrophin expression cell (e.g., human rhabdomyosarcoma cells), amplifying the region surrounding exons 45 to 55 of mRNA of the human dystrophin gene from the total RNA of the dystrophin expression cell by RT-PCR and performing nested PCR or sequence analysis on the PCR amplified product. The multi-skipping efficiency can be determined as follows. The mRNA for the human dystrophin gene is collected from test cells; in the mRNA, the polynucleotide level "A" of the band where any two or more numerically consecutive exons among exons 45 to 55 are skipped, the polynucleotide level "B" of the band where any one exon among exons 45 to 55 is skipped, and the polynucleotide level "C" of the band where no skipping is caused are measured. Using these measurement values of "A", "B", and "C", the efficiency is calculated by the following equation.

$$\text{Skipping efficiency (\%)} = A / (A + B + C) \times 100$$

For example, the multi-skipping efficiency of exons 45 to 55 can be determined by using a forward primer for exon 44 and a reverse primer for exon 56 to measure the polynucleotide level "A" of the band where exons 45 to 55 are multi-skipped, using the forward primer for exon 44 and a reverse primer for exon 46 to measure the polynucleotide level "B" of the band where exon 45 is single-skipped, and using the forward primer for exon 44 and the reverse primer for exon 46 to measure the polynucleotide level "C" of the band where no skipping is caused, followed by calculation by the equation using these measurement values of "A", "B", and "C".

The number of exons to be deleted in human dystrophin mRNA by the antisense oligomer of the present invention is 2, 3, 4, 5, 6, 7, 8, 9, 10, or 11. This is referred to as a deletion pattern, and various deletion patterns may exist in admixture in results obtained in one skipping experiment or skipping treatment. For example, mRNA admixture having deletion of 2, 3, 4, 5, 6, 7, 8, 9, 10, or 11 exons is obtained by introducing the antisense oligomer of the present invention to cells expressing human dystrophin pre-mRNA, and collecting its mRNA.

In a certain embodiment, the term "activity to cause skipping" can be defined as (C1) to (C10) below.

(C1) Any two numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the two numerically consecutive exons may be the 45th and the 46th exons, the 46th and the 47th exons, the 47th and the 48th exons, the 48th and the 49th exons, the 49th and the 50th exons, the 50th and the 51st exons, the 51st and the 52nd exons, the 52nd and the 53rd exons, the 53rd and the 54th exons, or the 54th and the 55th exons.

(C2) Any three numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the three numerically consecutive exons may be the 45th to the 47th exons, the 46th to the 48th exons, the 47th to the 49th exons, the 48th to the 50th exons, the 49th to the 51st exons, the 50th to the 52nd exons, the 51st to the 53rd exons, the 52nd to the 54th exons, or the 53rd to the 55th exons.

(C3) Any four numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the four numerically consecutive exons may be the 45th to the 48th exons, the 46th to the 49th exons, the 47th to the 50th exons, the 48th to the 51st exons, the 49th to the 52nd exons, the 50th to the 53rd exons, the 51st to the 54th exons, or the 52nd to the 55th exons.

(C4) Any five numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the five numerically consecutive exons may be the 45th to the 49th exons, the 46th to the 50th exons, the 47th to the 51st exons, the 48th to the 52nd exons, the 49th to the 53rd exons, the 50th to the 54th exons, or the 51st to the 55th exons.

(C5) Any six numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the six numerically consecutive exons may be the 45th to the 50th exons, the 46th to the 51st exons, the 47th to the 52nd exons, the 48th to the 53rd exons, the 49th to the 54th exons, or the 50th to the 55th exons.

(C6) Any seven numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or

higher, 60% or higher, 65% or higher, 70% or higher, 75% or higher, 80% or higher, 85% or higher, 90% or higher, or 95% or higher.

Herein, the seven numerically consecutive exons may be the 45th to the 51st exons, the 46th to the 52nd exons, the 47th to the 53rd exons, the 48th to the 54th exons, or the 49th to the 55th exons.

(C7) Any eight numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the eight numerically consecutive exons may be the 45th to the 52nd exons, the 46th to the 53rd exons, the 47th to the 54th exons, or the 48th to the 55th exons.

(C8) Any nine numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the nine numerically consecutive exons may be the 45th to the 53rd exons, the 46th to the 54th exons, or the 47th to the 55th exons.

(C9) Any ten numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the ten numerically consecutive exons may be the 45th to the 54th exons, or the 46th to the 55th exons.

(C10) Eleven numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the eleven numerically consecutive exons may be the 45th to the 55th exons.

The antisense oligomer of the present invention may be 10 to 60 bases long, 10 to 55 bases long, 10 to 50 bases long, 10 to 45 bases long, 10 to 40 bases long, 10 to 35 bases long, 10 to 30 bases long, 10 to 25 bases long, 15 to 60 bases long, 15 to 55 bases long, 15 to 50 bases long, 15 to 45 bases long, 15 to 40 bases long, 15 to 35 bases long, 15 to 30 bases long, 15 to 25 bases long, 16 to 60 bases long, 16 to 55 bases long, 16 to 50 bases long, 16 to 45 bases long, 16 to 40 bases long, 16 to 35 bases long, 16 to 30 bases long, 16 to 25 bases long, 17 to 60 bases long, 17 to 55 bases long, 17 to 50 bases long, 17 to 45 bases long, 17 to 40 bases long, 17 to 35 bases long, 17 to 30 bases long, 17 to 25 bases long, 18 to 60 bases long, 18 to 55 bases long, 18 to 50 bases long, 18 to 45 bases long, 18 to 40 bases long, 18 to 35 bases long, 18 to 30 bases long, 18 to 25 bases long, 19 to 60 bases long, 19 to 55 bases long, 19 to 50 bases long, 19 to 45 bases long, 19 to 40 bases long, 19 to 35 bases long, 19 to 30 bases long, 19 to 25 bases long, 20 to 60 bases long, 20 to 55 bases long, 20 to 50 bases long, 20 to 45 bases long, 20 to 40 bases long, 20 to 35 bases long, 20 to 30 bases long, 20 to 25 bases long, 15 to 30 bases long, 15 to 29 bases long, 15 to 28 bases long, 15 to 27 bases long, 15 to 26 bases long, 15 to 25 bases long, 15 to 24 bases long, 15 to 23 bases long, 15 to 22 bases long, 15 to 21 bases long, 15 to 20 bases long, 15 to 19 bases long, 15 to 18 bases long, 16 to 30 bases long, 16 to 29 bases long, 16 to 28 bases long, 16 to 27 bases long, 16 to 26 bases long, 16 to 25 bases long, 16 to 24 bases long, 16 to 23 bases long, 16 to 22 bases long, 16 to 21 bases long, 16 to 20 bases long, 16 to 19 bases long, 16 to 18 bases long, 17 to 30 bases long, 17 to 29 bases long, 17 to 28 bases long, 17 to 27 bases long, 17 to 26 bases long, 17 to 25 bases long, 17 to 24 bases long, 17 to 23 bases long, 17 to 22 bases long, 17 to 21 bases long, 17 to 20 bases long, 17 to 19 bases long, 17 to 18 bases long, 18 to 30 bases long, 18 to 29 bases long, 18 to 28 bases long, 18 to 27 bases long, 18 to 26 bases long, 18 to 25 bases long, 18 to 24 bases long, 18 to 23 bases long, 18 to 22 bases long, 18 to 21 bases long, 18 to 20 bases long, 18 to 19 bases long, 19 to 30 bases long, 19 to 29 bases long, 19 to 28 bases long, 19 to 27 bases long, 19 to 26 bases long, 19 to 25 bases long, 19 to 24 bases long, 19 to 23 bases long, 19 to 22 bases long, 19 to 21 bases long, 19 to 20 bases long, 20 to 30 bases long, 20 to 29 bases long, 20 to 28 bases long, 20 to 27 bases long, 20 to 26 bases long, 20 to 25 bases long, 20 to 24 bases long, 20 to 23 bases long, 20 to 22 bases long, 20 to 21 bases long, 60 bases long, 59 bases long, 58 bases long, 57 bases long, 56 bases long, 55 bases long, 54 bases long, 53 bases long, 52 bases long, 51 bases long, 50 bases long, 49 bases long, 48 bases long, 47 bases long, 46 bases long, 45 bases long, 44 bases long, 43 bases long, 42 bases long, 41 bases long, 40 bases long, 39 bases long, 38 bases long, 37 bases long, 36 bases long, 35 bases long, 34 bases long, 33 bases long, 32 bases long, 31 bases long, 30 bases long, 29 bases long, 28 bases long, 27 bases long, 26 bases long, 25 bases long, 24 bases long,

23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, or 10 bases long, but not limited thereto. These lengths may be increased or decreased by 1, 2, or 3 bases.

One embodiment of the antisense oligomer of the present invention is a linked-type antisense oligomer configured to comprise a plurality of unit oligomers linked to each other, a pharmaceutically acceptable salt thereof, or hydrate thereof (hereinafter, referred to as the "linked-type antisense oligomer of the present invention"). The unit oligomers mean respective oligomers constituting the linked-type antisense oligomer of the present invention. Specifically, the unit oligomers mean moieties (units) comprising base sequences that hybridize with target base sequences having consecutive base sequences when the linked-type antisense oligomer of the present invention binds to the target base sequences in human dystrophin pre-mRNA.

Specifically, the linked-type antisense oligomer of the present invention is an antisense oligomer comprising two or more unit oligomers linked to each other, or a pharmaceutically acceptable salt thereof, or hydrate thereof, wherein each of the unit oligomers comprises a base sequence complementary to a base sequence of any one region selected from the group consisting of the regions R1 to R24 (which may include the wild-type regions represented by SEQ ID NOs: 233 to 256, 341 to 369, and 385 to 389 and their mutant types), or a partial base sequence thereof, and the respective base sequences of the unit oligomers are neither consecutive nor overlapped with each other.

The unit oligomers may be linked via a linker that does not contribute to hybridization, or may be linked directly without the mediation of a linker. When the unit oligomers are linked directly to each other, the 3' end of the unit positioned on the 5' side and the 5' end of the unit positioned on the 3' side form a phosphate bond or any one of the following groups.

[Formula 3]

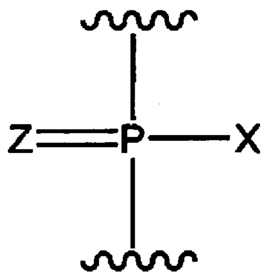
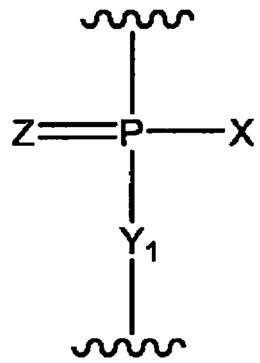
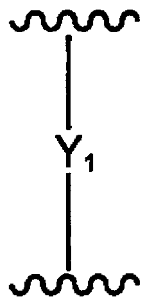
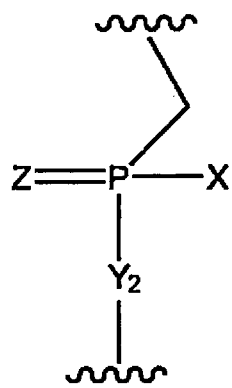

wherein X represents -OH, $-CH_2R^1$, $-O-CH_2R^1$, $-S-CH_2R^1$, $-NR^2R^3$ or F;

- $R^1$ represents H or an alkyl;
- $R^2$ and $R^3$, which may be the same or different, each represents H, an alkyl, a cycloalkyl or an aryl;
- $Y_1$ represents O, S, $CH_2$, or $NR^1$;
- $Y_2$ represents O, S, or $NR^1$;
- Z represents O or S.

In the linked-type antisense oligomer of the present invention, the unit oligomers may respectively target base sequences included in separate regions among the regions R1 to R24, or may respectively target base sequences included in the same region. In the linked-type antisense oligomer of the present invention, the respective base sequences of the unit oligomers are neither consecutive nor overlapped with each other. The respective base sequences of the unit oligomers that are not consecutive mean that the respective target base sequences of the unit oligomers constituting the linked-type antisense oligomer of the present invention are not consecutive as base sequences in human dystrophin pre-mRNA. Thus, when the respective target base sequences of the unit oligomers are compared to the base sequence of human dystrophin pre-mRNA, at least one base intervenes between the target base sequences as the base sequence of human dystrophin pre-mRNA. On the other hand, the respective base sequences of the unit oligomers that do not overlapped with each other mean that the respective target base sequences of the unit oligomers do not overlapped with each other as base sequences in human dystrophin pre-mRNA. The accidental presence of a match of about 1, 2, 3, 4, 5, 6, 7, 8, 9, or 10 bases between the respective base sequences of the unit oligomers is accepted.

The linked-type antisense oligomer of the present invention may be configured such that each of the unit oligomers comprises a base sequence complementary to

(D1) any one base sequence selected from the group consisting of SEQ ID NOs: 233 to 256, 341 to 369, and 385 to 389,
(D2) a base sequence that has at least 85% identity with any one base sequence selected from the group consisting of SEQ ID NOs: 233 to 256, 341 to 369, and 385 to 389, and has a length within ±15% of the length of the any one base sequence selected,
(D3) a base sequence that hybridizes under stringent conditions to a base sequence complementary to any one base sequence selected from the group consisting of SEQ ID NOs: 233 to 256, 341 to 369, and 385 to 389, or
(D4) a partial base sequence of any one base sequence selected from the group consisting of the base sequences (D1), (D2), and (D3), and the respective base sequences of the unit oligomers are neither consecutive nor overlapped with each other.

The linked-type antisense oligomer of the present invention may comprise, as described above, a base sequence complementary to any one base sequence selected from the group consisting of (D1), (D2), (D3), and (D4). The base sequence (D2) is a mutant of the base sequence (D1), and examples thereof also include

(D2-1) a base sequence that has at least 85% identity with any one base sequence selected from the group consisting of SEQ ID NOs: 233 to 256, 341 to 369, and 385 to 389, and has a length within ±15% of the length of the any one base sequence selected,
(D2-2) a base sequence that has at least 86% identity with any one base sequence selected from the group consisting of SEQ ID NOs: 233 to 256, 341 to 369, and 385 to 389, and has a length within ±14% of the length of the any one base sequence selected,
(D2-3) a base sequence that has at least 87% identity with any one base sequence selected from the group consisting of SEQ ID NOs: 233 to 256, 341 to 369, and 385 to 389, and has a length within ±13% of the length of the any one base sequence selected,
(D2-4) a base sequence that has at least 88% identity with any one base sequence selected from the group consisting of SEQ ID NOs: 233 to 256, 341 to 369, and 385 to 389, and has a length within ±12% of the length of the any one base sequence selected,
(D2-5) a base sequence that has at least 89% identity with any one base sequence selected from the group consisting of SEQ ID NOs: 233 to 256, 341 to 369, and 385 to 389, and has a length within ±11% of the length of the any one base sequence selected,
(D2-6) a base sequence that has at least 90% identity with any one base sequence selected from the group consisting of SEQ ID NOs: 233 to 256, 341 to 369, and 385 to 389, and has a length within ±10% of the length of the any one base sequence selected,
(D2-7) a base sequence that has at least 91% identity with any one base sequence selected from the group consisting of SEQ ID NOs: 233 to 256, 341 to 369, and 385 to 389, and has a length within ±9% of the length of the any one base sequence selected,
(D2-8) a base sequence that has at least 92% identity with any one base sequence selected from the group consisting of SEQ ID NOs: 233 to 256, 341 to 369, and 385 to 389, and has a length within ±8% of the length of the any one base sequence selected,
(D2-9) a base sequence that has at least 93% identity with any one base sequence selected from the group consisting of SEQ ID NOs: 233 to 256, 341 to 369, and 385 to 389, and has a length within ±7% of the length of the any one base sequence selected,
(D2-10) a base sequence that has at least 94% identity with any one base sequence selected from the group consisting of SEQ ID NOs: 233 to 256, 341 to 369, and 385 to 389, and has a length within ±6% of the length of the any one base sequence selected,
(D2-11) a base sequence that has at least 95% identity with any one base sequence selected from the group consisting of SEQ ID NOs: 233 to 256, 341 to 369, and 385 to 389, and has a length within ±5% of the length of the any one base sequence selected,
(D2-12) a base sequence that has at least 96% identity with any one base sequence selected from the group consisting of SEQ ID NOs: 233 to 256, 341 to 369, and 385 to 389, and has a length within ±4% of the length of the any one base sequence selected,
(D2-13) a base sequence that has at least 97% identity with any one base sequence selected from the group consisting of SEQ ID NOs: 233 to 256, 341 to 369, and 385 to 389, and has a length within ±3% of the length of the any one base sequence selected,
(D2-14) a base sequence that has at least 98% identity with any one base sequence selected from the group consisting of SEQ ID NOs: 233 to 256, 341 to 369, and 385 to 389, and has a length within ±2% of the length of the any one base sequence selected,
(D2-15) a base sequence that has at least 99% identity with any one base sequence selected from the group

consisting of SEQ ID NOs: 233 to 256, 341 to 369, and 385 to 389, and has a length within ±1% of the length of the any one base sequence selected, and

(D2-16) a base sequence that has at least 99.5% identity with any one base sequence selected from the group consisting of SEQ ID NOs: 233 to 256, 341 to 369, and 385 to 389, and has a length within ±0.5% of the length of the any one base sequence selected.

Thus, each of the unit oligomers constituting the linked-type antisense oligomer of the present invention may comprise a base sequence complementary to any one base sequence selected from the group consisting of the base sequences (D1), (D2-1) to (D2-16), (D3), and (D4).

The linked-type antisense oligomer of the present invention may comprise, as described above, a base sequence complementary to any one base sequence selected from the group consisting of (D1), (D2), (D3), and (D4). The base sequence (D4) relates to a partial base sequence of any one base sequence selected from the group consisting of the base sequences (D1), (D2), and (D3). In relation to (D4), the term "partial" means a partial region of consecutive bases, except for the full length, of (D1), (D2), or (D3). The partial region may be 5 to 25 bases long, 5 to 24 bases long, 5 to 23 bases long, 5 to 22 bases long, 5 to 21 bases long, 5 to 20 bases long, 5 to 19 bases long, 5 to 18 bases long, 5 to 17 bases long, 5 to 16 bases long, 5 to 15 bases long, 5 to 14 bases long, 5 to 13 bases long, 5 to 12 bases long, 7 to 25 bases long, 7 to 24 bases long, 7 to 23 bases long, 7 to 22 bases long, 7 to 21 bases long, 7 to 20 bases long, 7 to 19 bases long, 7 to 18 bases long, 7 to 17 bases long, 7 to 16 bases long, 7 to 15 bases long, 7 to 14 bases long, 7 to 13 bases long, 7 to 12 bases long, 9 to 25 bases long, 9 to 24 bases long, 9 to 23 bases long, 9 to 22 bases long, 9 to 21 bases long, 9 to 20 bases long, 9 to 19 bases long, 9 to 18 bases long, 9 to 17 bases long, 9 to 16 bases long, 9 to 15 bases long, 9 to 14 bases long, 9 to 13 bases long, 9 to 12 bases long, 10 to 25 bases long, 10 to 24 bases long, 10 to 23 bases long, 10 to 22 bases long, 10 to 21 bases long, 10 to 20 bases long, 10 to 19 bases long, 10 to 18 bases long, 10 to 17 bases long, 10 to 16 bases long, 10 to 15 bases long, 10 to 14 bases long, 10 to 13 bases long, 10 to 12 bases long, 25 bases long, 24 bases long, 23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, 10 bases long, 9 bases long, 8 bases long, 7 bases long, 6 bases long, or 5 bases long, but not limited thereto. These lengths may be increased or decreased by 1, 2, or 3 bases.

The size of each unit oligomer may be 5 to 30 bases long, 5 to 29 bases long, 5 to 28 bases long, 5 to 27 bases long, 5 to 26 bases long, 5 to 25 bases long, 5 to 24 bases long, 5 to 23 bases long, 5 to 22 bases long, 5 to 21 bases long, 5 to 20 bases long, 5 to 19 bases long, 5 to 18 bases long, 5 to 17 bases long, 5 to 16 bases long, 5 to 15 bases long, 5 to 14 bases long, 5 to 13 bases long, 5 to 12 bases long, 7 to 30 bases long, 7 to 29 bases long, 7 to 28 bases long, 7 to 27 bases long, 7 to 26 bases long, 7 to 25 bases long, 7 to 24 bases long, 7 to 23 bases long, 7 to 22 bases long, 7 to 21 bases long, 7 to 20 bases long, 7 to 19 bases long, 7 to 18 bases long, 7 to 17 bases long, 7 to 16 bases long, 7 to 15 bases long, 7 to 14 bases long, 7 to 13 bases long, 7 to 12 bases long, 9 to 30 bases long, 9 to 29 bases long, 9 to 28 bases long, 9 to 27 bases long, 9 to 26 bases long, 9 to 25 bases long, 9 to 24 bases long, 9 to 23 bases long, 9 to 22 bases long, 9 to 21 bases long, 9 to 20 bases long, 9 to 19 bases long, 9 to 18 bases long, 9 to 17 bases long, 9 to 16 bases long, 9 to 15 bases long, 9 to 14 bases long, 9 to 13 bases long, 9 to 12 bases long, 10 to 30 bases long, 10 to 29 bases long, 10 to 28 bases long, 10 to 27 bases long, 10 to 26 bases long, 10 to 25 bases long, 10 to 24 bases long, 10 to 23 bases long, 10 to 22 bases long, 10 to 21 bases long, 10 to 20 bases long, 10 to 19 bases long, 10 to 18 bases long, 10 to 17 bases long, 10 to 16 bases long, 10 to 15 bases long, 10 to 14 bases long, 10 to 13 bases long, 10 to 12 bases long, 30 bases long, 29 bases long, 28 bases long, 27 bases long, 26 bases long, 25 bases long, 24 bases long, 23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, 10 bases long, 9 bases long, 8 bases long, 7 bases long, 6 bases long, 5 bases long, but not limited thereto. These lengths may be increased or decreased by 1, 2, or 3 bases. The unit oligomers may have the same size or different sizes.

In a certain embodiment, the linked-type antisense oligomer of the present invention may be an antisense oligomer consisting of

(E1) any one base sequence selected from the group consisting of SEQ ID NOs: 18 to 21, 80 to 111, and 225 to 232, or

(E2) a base sequence that has at least 85% identity with any one base sequence selected from the group consisting of SEQ ID NOs: 18 to 21, 80 to 111, and 225 to 232, and has a length within ±15% of the length of the any one base sequence selected, or

a pharmaceutically acceptable salt thereof, or hydrate thereof.

Herein, the base sequence (E2) is a mutant type of the base sequence (E1), and examples of such a mutant type also include

(E2-1) a base sequence that has at least 85% identity with any one base sequence selected from the group consisting

of SEQ ID NOs: 18 to 21, 80 to 111, and 225 to 232, and has a length within ±15% of the length of the any one base sequence selected,

(E2-2) a base sequence that has at least 86% identity with any one base sequence selected from the group consisting of SEQ ID NOs: 18 to 21, 80 to 111, and 225 to 232, and has a length within ±14% of the length of the any one base sequence selected,

(E2-3) a base sequence that has at least 87% identity with any one base sequence selected from the group consisting of SEQ ID NOs: 18 to 21, 80 to 111, and 225 to 232, and has a length within ±13% of the length of the any one base sequence selected,

(E2-4) a base sequence that has at least 88% identity with any one base sequence selected from the group consisting of SEQ ID NOs: 18 to 21, 80 to 111, and 225 to 232, and has a length within ±12% of the length of the any one base sequence selected,

(E2-5) a base sequence that has at least 89% identity with any one base sequence selected from the group consisting of SEQ ID NOs: 18 to 21, 80 to 111, and 225 to 232, and has a length within ±11% of the length of the any one base sequence selected,

(E2-6) a base sequence that has at least 90% identity with any one base sequence selected from the group consisting of SEQ ID NOs: 18 to 21, 80 to 111, and 225 to 232, and has a length within ±10% of the length of the any one base sequence selected,

(E2-7) a base sequence that has at least 91% identity with any one base sequence selected from the group consisting of SEQ ID NOs: 18 to 21, 80 to 111, and 225 to 232, and has a length within ±9% of the length of the any one base sequence selected,

(E2-8) a base sequence that has at least 92% identity with any one base sequence selected from the group consisting of SEQ ID NOs: 18 to 21, 80 to 111, and 225 to 232, and has a length within ±8% of the length of the any one base sequence selected,

(E2-9) a base sequence that has at least 93% identity with any one base sequence selected from the group consisting of SEQ ID NOs: 18 to 21, 80 to 111, and 225 to 232, and has a length within ±7% of the length of the any one base sequence selected,

(E2-10) a base sequence that has at least 94% identity with any one base sequence selected from the group consisting of SEQ ID NOs: 18 to 21, 80 to 111, and 225 to 232, and has a length within ±6% of the length of the any one base sequence selected,

(E2-11) a base sequence that has at least 95% identity with any one base sequence selected from the group consisting of SEQ ID NOs: 18 to 21, 80 to 111, and 225 to 232, and has a length within ±5% of the length of the any one base sequence selected,

(E2-12) a base sequence that has at least 96% identity with any one base sequence selected from the group consisting of SEQ ID NOs: 18 to 21, 80 to 111, and 225 to 232, and has a length within ±4% of the length of the any one base sequence selected,

(E2-13) a base sequence that has at least 97% identity with any one base sequence selected from the group consisting of SEQ ID NOs: 18 to 21, 80 to 111, and 225 to 232, and has a length within ±3% of the length of the any one base sequence selected,

(E2-14) a base sequence that has at least 98% identity with any one base sequence selected from the group consisting of SEQ ID NOs: 18 to 21, 80 to 111, and 225 to 232, and has a length within ±2% of the length of the any one base sequence selected,

(E2-15) a base sequence that has at least 99% identity with any one base sequence selected from the group consisting of SEQ ID NOs: 18 to 21, 80 to 111, and 225 to 232, and has a length within ±1% of the length of the any one base sequence selected, and

(E2-16) a base sequence that has at least 99.5% identity with any one base sequence selected from the group consisting of SEQ ID NOs: 18 to 21, 80 to 111, and 225 to 232, and has a length within ±0.5% of the length of the any one base sequence selected.

Thus, the linked-type antisense oligomer of the present invention may consist of any one base sequence selected from the group consisting of the base sequences (E1) and (E2-1) to (E2-16).

Preferably, the linked-type antisense oligomer of the present invention comprises any one base sequence selected from the group consisting of SEQ ID NOs: 18 to 21, 80 to 111, and 225 to 232. In a certain embodiment, the linked-type antisense oligomer of the present invention consists of any one base sequence selected from the group consisting of SEQ ID NOs: 18 to 21, 80 to 111, and 225 to 232.

Further, the antisense oligomer of the present invention may be an antisense oligomer consisting of

(E'1) any one base sequence selected from the group consisting of SEQ ID NOs: 1 to 111, 113 to 177, 179, 180, 182, 183, 185 to 193, 195 to 198, 200 to 223, and 225 to 232, or

(E'2) a base sequence that has at least 85% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±15% of the length of the any one base sequence selected, or
a pharmaceutically acceptable salt thereof, or hydrate thereof.

Herein, the base sequence (E'2) is a mutant type of the base sequence (E'1), and examples of such a mutant type also include

(E'2-1) a base sequence that has at least 85% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±15% of the length of the any one base sequence selected,
(E'2-2) a base sequence that has at least 86% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±14% of the length of the any one base sequence selected,
(E'2-3) a base sequence that has at least 87% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±13% of the length of the any one base sequence selected,
(E'2-4) a base sequence that has at least 88% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±12% of the length of the any one base sequence selected,
(E'2-5) a base sequence that has at least 89% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±11% of the length of the any one base sequence selected,
(E'2-6) a base sequence that has at least 90% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±10% of the length of the any one base sequence selected,
(E'2-7) a base sequence that has at least 91% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±9% of the length of the any one base sequence selected,
(E'2-8) a base sequence that has at least 92% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±8% of the length of the any one base sequence selected,
(E'2-9) a base sequence that has at least 93% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±7% of the length of the any one base sequence selected,
(E'2-10) a base sequence that has at least 94% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±6% of the length of the any one base sequence selected,
(E'2-11) a base sequence that has at least 95% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±5% of the length of the any one base sequence selected,
(E'2-12) a base sequence that has at least 96% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±4% of the length of the any one base sequence selected,
(E'2-13) a base sequence that has at least 97% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±3% of the length of the any one base sequence selected,
(E'2-14) a base sequence that has at least 98% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±2% of the length of the any one base sequence selected,
(E'2-15) a base sequence that has at least 99% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±1% of the length of the any one base sequence selected, and
(E'2-16) a base sequence that has at least 99.5% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±0.5% of the length of the any one base sequence selected.

Thus, the antisense oligomer of the present invention may consist of any one base sequence selected from

the group consisting of the base sequences (E'1) and (E'2-1) to (E'2-16).

Preferably, the antisense oligomer of the present invention comprises any one base sequence selected from the group consisting of SEQ ID NOs: 1 to 111, 113 to 177, 179, 180, 182, 183, 185 to 193, 195 to 198, 200 to 223, and 225 to 232. In a certain embodiment, the antisense oligomer of the present invention consists of any one base sequence selected from the group consisting of SEQ ID NOs: 1 to 111, 113 to 177, 179, 180, 182, 183, 185 to 193, 195 to 198, 200 to 223, and 225 to 232.

In a certain embodiment, the antisense oligomer of the present invention may be an antisense oligomer consisting of

(E"1) any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, or (E"2) a base sequence that has at least 85% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±15% of the length of the any one base sequence selected,
or a pharmaceutically acceptable salt thereof, or hydrate thereof.

Herein, the base sequence (E"2) is a mutant type of the base sequence (E"1), and examples of such a mutant type also include

(E"2-1) a base sequence that has at least 85% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±15% of the length of the any one base sequence selected,
(E"2-2) a base sequence that has at least 86% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±14% of the length of the any one base sequence selected,
(E' '2-3) a base sequence that has at least 87% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±13% of the length of the any one base sequence selected,
(E"2-4) a base sequence that has at least 88% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±12% of the length of the any one base sequence selected,
(E"2-5) a base sequence that has at least 89% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±11% of the length of the any one base sequence selected,
(E"2-6) a base sequence that has at least 90% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±10% of the length of the any one base sequence selected,
(E"2-7) a base sequence that has at least 91% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±9% of the length of the any one base sequence selected,

(E"2-8) a base sequence that has at least 92% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±8% of the length of the any one base sequence selected,
(E"2-9) a base sequence that has at least 93% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±7% of the length of the any one base sequence selected,
(E"2-10) a base sequence that has at least 94% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±6% of the length of the any one base sequence selected,
(E"2-11) a base sequence that has at least 95% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±5% of the length of the any one base sequence selected,
(E"2-12) a base sequence that has at least 96% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±4% of the length of the any one base sequence selected,
(E"2-13) a base sequence that has at least 97% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±3% of the length of the any one base sequence selected,
(E"2-14) a base sequence that has at least 98% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±2% of the length of the any one base sequence selected,
(E''2-15) a base sequence that has at least 99% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±1% of the length of the any one base sequence selected, and
(E''2-16) a base sequence that has at least 99.5% identity with any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and has a length within ±0.5% of the length of the any one base sequence selected.

Thus, the antisense oligomer of the present invention may consist of any one base sequence selected from the group consisting of the base sequences (E"1) and (E"2-1) to (E"2-16).

In a certain embodiment, the antisense oligomer of the present invention may be an antisense oligomer consisting of

(E'''1) any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, or
(E'''2) a base sequence that has at least 85% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±15% of the length

of the any one base sequence selected,
or a pharmaceutically acceptable salt thereof, or hydrate thereof.

Herein, the base sequence (E‴2) is a mutant type of the base sequence (E‴1), and examples of such a mutant type also include

(E‴2-1) a base sequence that has at least 85% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±15% of the length of the any one base sequence selected,
(E‴2-2) a base sequence that has at least 86% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±14% of the length of the any one base sequence selected,
(E‴2-3) a base sequence that has at least 87% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±13% of the length of the any one base sequence selected,
(E‴2-4) a base sequence that has at least 88% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±12% of the length of the any one base sequence selected,
(E‴2-5) a base sequence that has at least 89% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±11% of the length of the any one base sequence selected,
(E‴2-6) a base sequence that has at least 90% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±10% of the length of the any one base sequence selected,
(E‴2-7) a base sequence that has at least 91% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±9% of the length of the any one base sequence selected,
(E‴2-8) a base sequence that has at least 92% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±8% of the length of the any one base sequence selected,
(E‴2-9) a base sequence that has at least 93% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±7% of the length of the any one base sequence selected,
(E‴2-10) a base sequence that has at least 94% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±6% of the length of the any one base sequence selected,
(E‴2-11) a base sequence that has at least 95% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±5% of the length of the any one base sequence selected,
(E‴2-12) a base sequence that has at least 96% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±4% of the length of the any one base sequence selected,
(E‴2-13) a base sequence that has at least 97% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±3% of the length of the any one base sequence selected,

(E‴2-14) a base sequence that has at least 98% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±2% of the length of the any one base sequence selected,

(E‴2-15) a base sequence that has at least 99% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±1% of the length of the any one base sequence selected, and

(E‴2-16) a base sequence that has at least 99.5% identity with any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, and has a length within ±0.5% of the length of the any one base sequence selected.

Thus, the antisense oligomer of the present invention may consist of any one base sequence selected from the group consisting of the base sequences (E‴1) and (E‴2-1) to (E‴2-16).

Preferably, the antisense oligomer of the present invention is an antisense oligomer comprising any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, or a pharmaceutically acceptable salt thereof, or hydrate thereof. In a certain embodiment, the antisense oligomer of the present invention is an antisense oligomer consisting of any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, or a pharmaceutically acceptable salt thereof, or hydrate thereof.

More preferably, the antisense oligomer of the present invention is an antisense oligomer comprising any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, or a pharmaceutically acceptable salt thereof, or hydrate thereof. In a certain embodiment, the antisense oligomer of the present invention is an antisense oligomer consisting of any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228, or a pharmaceutically acceptable salt thereof, or hydrate thereof.

In a certain embodiment, the linked-type antisense oligomer of the present invention is an antisense oligomer comprising any one base sequence selected from the group consisting of SEQ ID NOs: 80, 82, 86, 92, 97, 98, 100, 102, 225, 228, 231, and 232, or a pharmaceutically acceptable salt thereof, or hydrate thereof. In a certain embodiment, the linked-type antisense oligomer of the present invention consists of any one base sequence selected from the group consisting of SEQ ID NOs: 80, 82, 86, 92, 97, 98, 100, 102, 225, 228, 231, and 232. Further preferably, the linked-type antisense oligomer of the present invention comprises any one base sequence selected from the group consisting of SEQ ID NOs: 102, 225, and 228. In a certain embodiment, the linked-type antisense oligomer of the present invention is an antisense oligomer consisting of any one base sequence selected from the group consisting of SEQ ID NOs: 102, 225, and 228, or a pharmaceutically acceptable salt thereof, or hydrate thereof.

The antisense oligomer of the present invention comprises a base sequence complementary to a base sequence of at least any one region selected from the group consisting of the regions R1 to R24, or a partial base sequence thereof. Any base sequence selected from the group consisting of (E1) and (E2-1) to (E2-16), the group consisting of (E'1) and (E'2-1) to (E'2-16), the group consisting of (E"1) and (E"2-1) to (E"2-16), or the group consisting of (E‴1) and (E‴2-1) to (E‴2-16) may be selected as the base sequence complementary to the base sequence of each region included in the regions R1 to R24, or a partial base sequence thereof. For example, as the base sequences complementary to the base sequences of the regions R1 to R24, or partial base sequences thereof, a base sequence complementary to the base sequence of each region, or a partial base sequence thereof may be used as to the regions R4 to R24, and a base sequence that is selected from the group consisting of (E1) and (E2-1) to (E2-16) and is included in each of the regions R1 to R3 may be used as to the regions R1 to R3.

The antisense oligomer of the present invention (including the linked-type antisense oligomer of the present invention) may be an oligonucleotide, morpholino oligomer or peptide nucleic acid (PNA) oligomer (hereinafter, also referred to as the "antisense oligonucleotide of the present invention", the "antisense morpholino oligomer of the present invention", or the "antisense peptide nucleic acid oligomer of the present invention").

The antisense oligonucleotide of the present invention is an antisense oligomer composed of nucleotides as

constituent units. Such nucleotides may be any of ribonucleotides, deoxyribonucleotides and modified nucleotides.

The modified nucleotide refers to one having fully or partly modified nucleobases, sugar moieties and/or phosphate bond moieties, which constitute the ribonucleotide or deoxyribonucleotide.

The nucleobase includes, for example, adenine, guanine, hypoxanthine, cytosine, thymine, uracil, and modified bases thereof. Examples of such modified bases include, but not limited to, pseudouracil, 3-methyluracil, dihydrouracil, 5-alkylcytosines (*e.g.,* 5-methylcytosine), 5-alkyluracils (*e.g.,* 5-ethyluracil), 5-halouracils (*e.g.*, 5-bromouracil), 6-azapyrimidine, 6-alkylpyrimidines (*e.g.*, 6-methyluracil), 2-thiouracil, 4-thiouracil, 4-acetylcytosine, 5-(carboxyhydroxymethyl) uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, xanthine, etc.

Modification of the sugar moiety may include, for example, modifications at the 2'-position of ribose and modifications of the other positions of the sugar. The modification at the 2'-position of ribose includes a modification of replacing the 2'-OH of ribose with -OR, - R, -R'OR, -SH, -SR, $-NH_2$, -NHR, $-NR_2$, $-N_3$, -CN, -F, -Cl, - Br or -I, wherein R represents an alkyl or an aryl and R' represents an alkylene.

The modification for the other positions of the sugar includes, for example, replacement of O at the 4' position of ribose or deoxyribose with S, bridging between 2' and 4' positions of the sugar, e.g., LNA (locked nucleic acid) or ENA (2'-O,4'-C-ethylene-bridged nucleic acids), but is not limited thereto.

A modification of the phosphate bond moiety includes, for example, a modification of replacing phosphodiester bond with phosphorothioate bond, phosphorodithioate bond, alkyl phosphonate bond, phosphoramidate bond or boranophosphate bond (cf., e.g., Enya et al: Bioorganic & Medicinal Chemistry, 2008, 18, 9154-9160) (cf., *e.g.,* Japan Domestic Re-Publications of PCT Application Nos. 2006/129594 and 2006/038608).

As used herein, the alkyl is preferably a straight or branched alkyl having 1 to 6 carbon atoms. Specific examples include methyl, ethyl, n-propyl, isopropyl, *n*-butyl, isobutyl, sec-butyl, tert-butyl, n-pentyl, isopentyl, neopentyl, tert-pentyl, n-hexyl and isohexyl. The alkyl may optionally be substituted. Examples of such substituents are a halogen, an alkoxy, cyano and nitro. The alkyl may be substituted with 1 to 3 substituents.

As used herein, the cycloalkyl is preferably a cycloalkyl having 3 to 12 carbon atoms. Specific examples include cyclopropyl, cyclobutyl, cyclopentyl, cyclohexyl, cycloheptyl, cyclooctyl, cyclodecyl and cyclododecyl.

As used herein, the halogen includes fluorine, chlorine, bromine and iodine.

As used herein, the alkoxy is a straight or branched alkoxy having 1 to 6 carbon atoms such as methoxy, ethoxy, n-propoxy, isopropoxy, n-butoxy, isobutoxy, *sec*-butoxy, tert-butoxy, n-pentyloxy, isopentyloxy, n-hexyloxy, isohexyloxy, etc. Among others, an alkoxy having 1 to 3 carbon atoms is preferred.

As used herein, the aryl is preferably an aryl having 6 to 10 carbon atoms. Specific examples include phenyl, $\alpha$-naphthyl and $\beta$-naphthyl. Among others, phenyl is preferred. The aryl may optionally be substituted. Examples of such substituents are an alkyl, a halogen, an alkoxy, cyano and nitro. The aryl may be substituted with one to three of such substituents.

As used herein, the alkylene is preferably a straight or branched alkylene having 1 to 6 carbon atoms. Specific examples include methylene, ethylene, trimethylene, tetramethylene, pentamethylene, hexamethylene, 2-(ethyl) trimethylene and 1-(methyl) tetramethylene.

As used herein, the acyl includes a straight or branched alkanoyl or aroyl. Examples of the alkanoyl include formyl, acetyl, 2-methylacetyl, 2,2-dimethylacetyl, propionyl, butyryl, isobutyryl, pentanoyl, 2,2-dimethylpropionyl, hexanoyl, etc. Examples of the aroyl include benzoyl, toluoyl and naphthoyl. The aroyl may optionally be substituted at substitutable positions and may be substituted with an alkyl(s).

Preferably, the antisense oligonucleotide of the present invention is the antisense oligomer of the present invention having a group represented by general formula below as a constituent unit wherein the -OH group at position 2' of ribose is substituted with methoxy and the phosphate bond moiety is a phosphorothioate bond:

[Formula 4]

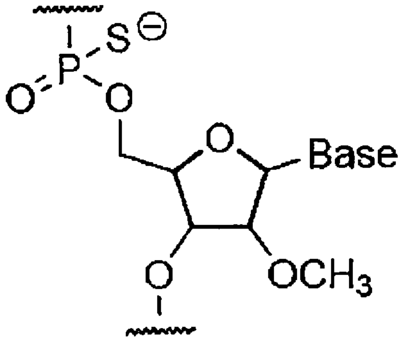

wherein Base represents a nucleobase.

The antisense oligonucleotide of the present invention may be easily synthesized using various automated synthesizer (e.g., AKTA oligopilot plus 10/100 (GE Healthcare)). Alternatively, the synthesis may also be entrusted to a third-party organization (e.g., Promega Corp. or Takara Co.), etc.

The antisense morpholino oligomer of the present invention is an antisense oligomer comprising the constituent unit represented by general formula below:

[Formula 5]

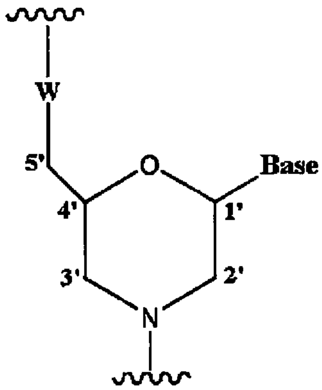

wherein Base has the same significance as defined above, and,

W represents a group shown by any one of the following groups:

[Formula 6]

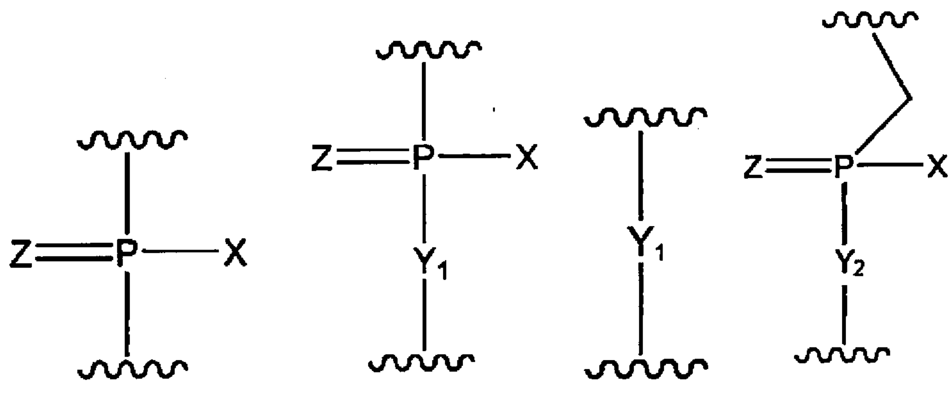

wherein X represents $-CH_2R^1$, $-O-CH_2R^1$, $-S-CH_2R^1$, $-NR^2R^3$, or F;

$R^1$ represents H or an alkyl;

$R^2$ and $R^3$, which may be the same or different, each represents H, an alkyl, a cycloalkyl, or an aryl;

$Y_1$ represents O, S, $CH_2$, or $NR^1$;

$Y_2$ represents O, S, or $NR^1$;

Z represents O or S.

Examples of morpholino monomer compounds that are used in synthesis of the antisense morpholino oligomer of the present invention include, but not limited to, the following morpholino monomer compound (A), morpholino monomer compound (C), morpholino monomer compound (T), and morpholino monomer compound (G) shown in Table 5.

[Table 5]

Table 5

| Morpholino monomer compound (A) | Morpholino monomer compound (C) | Morpholino monomer compound (T) | Morpholino monomer compound (G) |
|---|---|---|---|
| 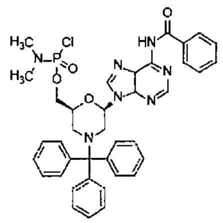 | 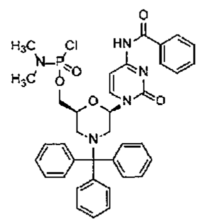 | 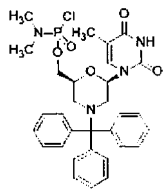 | 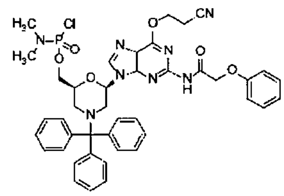 |

In the present invention, preferably, the morpholino oligomer is an oligomer having a group represented by general formula below as a constituent unit (phosphorodiamidate morpholino oligomer (hereinafter referred to as "PMO")).

[Formula 7]

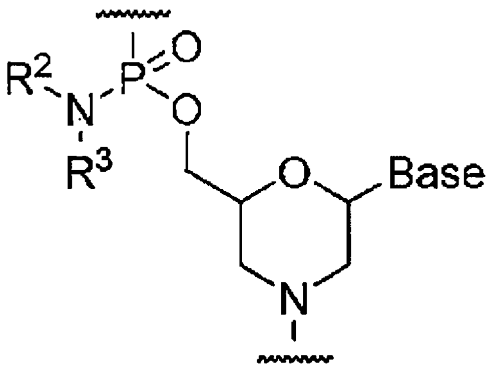

wherein Base, $R^2$ and $R^3$ have the same significance as defined above.

The morpholino oligomer may be produced by the procedure described in, *e.g.,* WO 1991/009033 or WO 2009/064471. In particular, PMO can be produced by the procedure described in WO 2009/064471 or WO2013/100190.

The antisense peptide nucleic acid oligomer of the present invention is an antisense oligomer having a group represented by general formula below as a constituent unit:

[Formula 8]

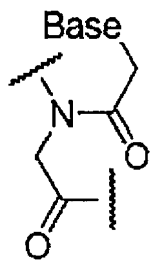

wherein Base has the same significance as defined above.

The peptide nucleic acid oligomer can be produced in accordance with, *e.g.*, the following literatures:

1)P. E. Nielsen, M. Egholm, R. H. Berg, O. Buchardt, Science, 254, 1497 (1991)
2) M. Egholm, O. Buchardt, P. E. Nielsen, R. H. Berg, JACS, 114, 1895 (1992)
3) K. L. Dueholm, M. Egholm, C. Behrens, L. Christensen, H. F. Hansen, T. Vulpius, K. H. Petersen, R. H. Berg, P. E. Nielsen, O. Buchardt, J. Org. Chem., 59, 5767 (1994)
4) L. Christensen, R. Fitzpatrick, B. Gildea, K. H. Petersen, H. F. Hansen, T. Koch, M. Egholm, O. Buchardt, P. E. Nielsen, J. Coull, R. H. Berg, J. Pept. Sci., 1, 175 (1995)
5) T. Koch, H. F. Hansen, P. Andersen, T. Larsen, H. G. Batz, K. Otteson, H. Orum, J. Pept. Res., 49, 80 (1997)

The antisense oligomer of the present invention (including the linked-type antisense oligomer of the present invention) may be in the form of a pharmaceutically acceptable salt thereof, in the form of a hydrate thereof, or in the form of a hydrate of the pharmaceutically acceptable salt.

Examples of the pharmaceutically acceptable salt of the antisense oligomer of the present invention are alkali metal salts such as salts of sodium, potassium and lithium; alkaline earth metal salts such as salts of calcium and magnesium; metal salts such as salts of aluminum, iron, zinc, copper, nickel, cobalt, etc.; ammonium salts; organic amine salts such as salts of t-octylamine, dibenzylamine, morpholine, glucosamine, phenylglycine alkyl ester, ethylenediamine, *N*-methylglucamine, guanidine, diethylamine, triethylamine, dicyclohexylamine, N,N' -dibenzylethylenediamine, chloroprocaine, procaine, diethanolamine, N-benzyl-phenethylamine, piperazine, tetramethylammonium, tris(hydroxymethyl)aminomethane; hydrohalide salts such as salts of hydrofluorides, hydrochlorides, hydrobromides and hydroiodides; inorganic acid salts such as nitrates, perchlorates, sulfates, phosphates, etc.; lower alkane sulfonates such as methanesulfonates, trifluoromethanesulfonates and ethanesulfonates; arylsulfonates such as benzenesulfonates and p-toluenesulfonates; organic acid salts such as acetates, malates, fumarates, succinates, citrates, tartarates, oxalates, maleates, etc.; and, amino acid salts such as salts of glycine, lysine, arginine, ornithine, glutamic acid and aspartic acid. These salts may be produced by known methods. Alternatively, the antisense oligomer of the present invention may be in the form of a hydrate thereof.

## 2. Suppressor antisense oligomer

Another embodiment of the present invention provides a suppressor antisense oligomer which suppresses single exon skipping (hereinafter, referred to as "single skipping"). The suppressor antisense oligomer can suppress single skipping and thereby enhance an effect of multi-exon skipping by an antisense oligomer. The single skipping relates to skipping of only one exon, not simultaneous skipping of a plurality of exons.

Specifically, the present invention provides a suppressor antisense oligomer or a pharmaceutically acceptable salt thereof, or hydrate thereof which suppresses single skipping of any one exon selected from the group consisting of the 45th exon to the 55th exon in human dystrophin pre-mRNA. Hereinafter, the suppressor antisense oligomer and the pharmaceutically acceptable salt thereof and the hydrate of the antisense oligomer or the salt are also collectively referred to as the "suppressor antisense oligomer of the present invention". The suppressor antisense oligomer of the present invention may refer to each of the suppressor antisense oligomer or the pharmaceutically acceptable salt thereof, or hydrate thereof.

The suppressor antisense oligomer of the present invention suppresses single skipping by targeting the site of a splicing silencer sequence, a branch site sequence, or a splice site sequence in human dystrophin pre-mRNA and inhibiting splicing. The suppressor antisense oligomer of the present invention reduces the efficiency of single skipping of an intended exon as compared to a control.

For example, the suppressor antisense oligomer that suppresses single skipping of any one exon selected from the group consisting of the 45th exon to the 55th exon targets a splicing silencer sequence of any one of the 44th to 56th exons, or a splicing silencer sequence, a branch site sequence, or a splice site sequence of any one of the 44th

to 55th introns.

As used herein, the splicing silencer sequence is a base sequence element that functions to suppress recognition of an exon in pre-mRNA. Examples of the splicing silencer sequence include recognition sequences of proteins or protein complexes, such as heterogeneous nuclear ribonucleoprotein A1 (hnRNP A1), hnRNP A2/B1, DAZAP1, hnRNP I, Fox-1, Fox-2, hnRNP H1, hnRNP H2, hnRNP H3, hnRNP L, Sam68, and SRp40.

As used herein, the splice site is the boundary between an exon and an intron. The splice site can be predicted from the 5' end of the intron starting at GU, AU, or the like, and the 3' end of the intron ending at AG, AC, or the like.

As used herein, the branch site is a sequence within an intronthat initiates an attack on a 5' splice site during splicing reaction and forms a covalent bond. The branch site sequence refers to a base sequence in an intron containing the branch site.

Examples of the splicing silencer sequence, the branch site sequence, or the splice site sequence which is the target (hereinafter, referred to as a "suppressor sequence") of the suppressor antisense oligomer of the present invention include base sequences given below (SEQ ID NOs: 370 to 384 and 390). The positions of the suppressor sequence, the branch site sequence, and the splice site sequence can be detected using software such as SpliceAid (Francesco Piva et al., Bioinformatics, 25 (9), 1211-1213, 2009), SpliceAid-2 (Francesco Piva et al., Human Mutation, 33 (1), 81-85, 2012), SpliceAid-F (Matteo Giulietti et al., Nucleic Acids Res., 41, D125-D131, 2012), ATtRACT-a (Girolamo Giudice et al., Database (Oxford), baw035, 2016), SROOGLE (Schraga Schwartz et al., Nucleic Acids Res., 37, W189-W192, 2009), Reg RNA (Hsi-Yuan Huang et al., Nucleic Acids Res., 34, W429-W434, 2006), Reg RNA 2.0 (Tzu-Hao Chang et al., BMC bioinformatics, 14 (Suppl 2), S4, 2013), Human Splicing Finder (Francois-Olivier Desmet et al., Nucleic Acids Res., 37, 9, e67, 2009), or SVM-BPfinder (Andre Corvelo et al., PLoS Comput Biol., 6, 11, e1001016, 2010).

[Table 6]

Table 6

| SEQ ID NO | Name of protein or protein complex or sequence site to be bound | Base sequence |
|---|---|---|
| 370 | hnRNP A1 | UAGGA |
| 371 | | UAGGAU |
| 372 | | UAGGAA |
| 373 | | UAGGCA |
| 374 | | UAGGCU |
| 375 | | UAGGGA |
| 376 | | UAGGGC |
| 377 | | UAGGGU |
| 378 | | GGUAGGGC |
| 379 | | AGAAC |
| 380 | Fox-1 | AGCAUG |
| 381 | | UGACUG |
| 382 | | UGCAUG |
| 383 | Branch site | yUnAy |
| 384 | Splice site | GU |
| 390 | | AG |
| y: A or U, n: A, G, C, U, or T | | |

Herein, specific examples of the splicing silencer sequence include SEQ ID NOs: 370 to 382. Examples of mutants of SEQ ID NOs: 370 to 382 also include

(F1) a base sequence that has at least 85% identity with any one base sequence selected from the group consisting

of SEQ ID NOs: 370 to 382, and has a length within ±15% of the length of the any one base sequence selected,
(F2) a base sequence that has at least 86% identity with any one base sequence selected from the group consisting of SEQ ID NOs: 370 to 382, and has a length within ±14% of the length of the any one base sequence selected,
(F3) a base sequence that has at least 87% identity with any one base sequence selected from the group consisting of SEQ ID NOs: 370 to 382, and has a length within ±13% of the length of the any one base sequence selected,
(F4) a base sequence that has at least 88% identity with any one base sequence selected from the group consisting of SEQ ID NOs: 370 to 382, and has a length within ±12% of the length of the any one base sequence selected,
(F5) a base sequence that has at least 89% identity with any one base sequence selected from the group consisting of SEQ ID NOs: 370 to 382, and has a length within ±11% of the length of the any one base sequence selected,
(F6) a base sequence that has at least 90% identity with any one base sequence selected from the group consisting of SEQ ID NOs: 370 to 382, and has a length within ±10% of the length of the any one base sequence selected,
(F7) a base sequence that has at least 91% identity with any one base sequence selected from the group consisting of SEQ ID NOs: 370 to 382, and has a length within ±9% of the length of the any one base sequence selected,
(F8) a base sequence that has at least 92% identity with any one base sequence selected from the group consisting of SEQ ID NOs: 370 to 382, and has a length within ±8% of the length of the any one base sequence selected,
(F9) a base sequence that has at least 93% identity with any one base sequence selected from the group consisting of SEQ ID NOs: 370 to 382, and has a length within ±7% of the length of the any one base sequence selected,
(F10) a base sequence that has at least 94% identity with any one base sequence selected from the group consisting of SEQ ID NOs: 370 to 382, and has a length within ±6% of the length of the any one base sequence selected,
(F11) a base sequence that has at least 95% identity with any one base sequence selected from the group consisting of SEQ ID NOs: 370 to 382, and has a length within ±5% of the length of the any one base sequence selected,
(F12) a base sequence that has at least 96% identity with any one base sequence selected from the group consisting of SEQ ID NOs: 370 to 382, and has a length within ±4% of the length of the any one base sequence selected,
(F13) a base sequence that has at least 97% identity with any one base sequence selected from the group consisting of SEQ ID NOs: 370 to 382, and has a length within ±3% of the length of the any one base sequence selected,
(F14) a base sequence that has at least 98% identity with any one base sequence selected from the group consisting of SEQ ID NOs: 370 to 382, and has a length within ±2% of the length of the any one base sequence selected,
(F15) a base sequence that has at least 99% identity with any one base sequence selected from the group consisting of SEQ ID NOs: 370 to 382, and has a length within ±1% of the length of the any one base sequence selected, and
(F16) a base sequence that has at least 99.5% identity with any one base sequence selected from the group consisting of SEQ ID NOs: 370 to 382, and has a length within ±0.5% of the length of the any one base sequence selected.

In a certain embodiment of the present invention, the splicing silencer sequence may be a recognition sequence of heterogeneous nuclear ribonucleoprotein A1 (hnRNP A1). Examples of the recognition sequence of human hnRNP A1 are shown in SEQ ID NOs: 370 to 379.

Herein, specific examples of the splice site sequence include SEQ ID NOs: 384 and 390. Examples of mutants of SEQ ID NOs: 384 and 390 also include (G1) a base sequence that has at least 80% identity with the base sequence of SEQ ID NO: 384 or 390, and has a length within ±20% of the length of the any one base sequence selected.

Herein, specific examples of the branch site sequence include SEQ ID NO: 383. Examples of a mutant of SEQ ID NO: 383 also include
(H1) a base sequence that has at least 80% identity with the base sequence of SEQ ID NO: 383, and has a length within ±20% of the length of the any one base sequence selected.

As for a mutant type of the suppressor sequence, the following target regions are also included in the target base sequence of the suppressor antisense oligomer of the present invention:

(I1) a base sequence that has at least 85% identity with any one base sequence selected from the group consisting of SEQ ID NOs: 370 to 384 and 390, and has a length within ±15% of the length of the any one base sequence selected,
(12) a base sequence that has at least 86% identity with any one base sequence selected from the group consisting of SEQ ID NOs: 370 to 384 and 390, and has a length within ±14% of the length of the any one base sequence selected,
(13) a base sequence that has at least 87% identity with any one base sequence selected from the group consisting of SEQ ID NOs: 370 to 384 and 390, and has a length within ±13% of the length of the any one base sequence selected,
(14) a base sequence that has at least 88% identity with any one base sequence selected from the group consisting of SEQ ID NOs: 370 to 384 and 390, and has a length within ±12% of the length of the any one base sequence selected,

(I5) a base sequence that has at least 89% identity with any one base sequence selected from the group consisting of SEQ ID NOs: 370 to 384 and 390, and has a length within ±11% of the length of the any one base sequence selected,
(I6) a base sequence that has at least 90% identity with any one base sequence selected from the group consisting of SEQ ID NOs: 370 to 384 and 390, and has a length within ±10% of the length of the any one base sequence selected,
(I7) a base sequence that has at least 91% identity with any one base sequence selected from the group consisting of SEQ ID NOs: 370 to 384 and 390, and has a length within ±9% of the length of the any one base sequence selected,
(I8) a base sequence that has at least 92% identity with any one base sequence selected from the group consisting of SEQ ID NOs: 370 to 384 and 390, and has a length within ±8% of the length of the any one base sequence selected,
(I9) a base sequence that has at least 93% identity with any one base sequence selected from the group consisting of SEQ ID NOs: 370 to 384 and 390, and has a length within ±7% of the length of the any one base sequence selected,
(I10) a base sequence that has at least 94% identity with any one base sequence selected from the group consisting of SEQ ID NOs: 370 to 384 and 390, and has a length within ±6% of the length of the any one base sequence selected,
(I11) a base sequence that has at least 95% identity with any one base sequence selected from the group consisting of SEQ ID NOs: 370 to 384 and 390, and has a length within ±5% of the length of the any one base sequence selected,
(I12) a base sequence that has at least 96% identity with any one base sequence selected from the group consisting of SEQ ID NOs: 370 to 384 and 390, and has a length within ±4% of the length of the any one base sequence selected,
(I13) a base sequence that has at least 97% identity with any one base sequence selected from the group consisting of SEQ ID NOs: 370 to 384 and 390, and has a length within ±3% of the length of the any one base sequence selected,
(I14) a base sequence that has at least 98% identity with any one base sequence selected from the group consisting of SEQ ID NOs: 370 to 384 and 390, and has a length within ±2% of the length of the any one base sequence selected,
(I15) a base sequence that has at least 99% identity with any one base sequence selected from the group consisting of SEQ ID NOs: 370 to 384 and 390, and has a length within ±1% of the length of the any one base sequence selected, and
(I16) a base sequence that has at least 99.5% identity with any one base sequence selected from the group consisting of SEQ ID NOs: 370 to 384 and 390, and has a length within ±0.5% of the length of the any one base sequence selected.

In one embodiment, the suppressor antisense oligomer of the present invention comprises a base sequence complementary to any one base sequence of a base sequence selected from the group consisting of the base sequences represented by SEQ ID NOs: 370 to 384 and 390 and the mutant type base sequences shown in (I1) to (I16) above, or a partial base sequence thereof. In another embodiment, the suppressor antisense oligomer of the present invention consists of a base sequence complementary to any one base sequence of a base sequence selected from the group consisting of the base sequences represented by SEQ ID NOs: 370 to 384 and 390, or a partial base sequence thereof.

Herein, the term "partial" is as defined in the section about the antisense oligomer of the present invention.

As a further embodiment, the suppressor antisense oligomer of the present invention is a suppressor antisense oligomer which consists of

(J1) any one base sequence selected from the group consisting of SEQ ID NOs: 257 to 275, or
(J2) a base sequence that has at least 85% identity with any one base sequence selected from the group consisting of SEQ ID NOs: 257 to 275, and has a length within ±15% of the length of the any one base sequence selected,
or a pharmaceutically acceptable salt thereof, or hydrate thereof.

Herein, the base sequence (J2) is a mutant type of the base sequence (J1). Such a mutant type also includes

(J2-1) a base sequence that has at least 85% identity with any one base sequence selected from the group consisting of SEQ ID NOs: 257 to 275, and has a length within ±15% of the length of the any one base sequence selected,
(J2-2) a base sequence that has at least 86% identity with any one base sequence selected from the group consisting of SEQ ID NOs: 257 to 275, and has a length within ±14% of the length of the any one base sequence selected,
(J2-3) a base sequence that has at least 87% identity with any one base sequence selected from the group consisting of SEQ ID NOs: 257 to 275, and has a length within ±13% of the length of the any one base sequence selected,
(J2-4) a base sequence that has at least 88% identity with any one base sequence selected from the group consisting of SEQ ID NOs: 257 to 275, and has a length within ±12% of the length of the any one base sequence selected,
(J2-5) a base sequence that has at least 89% identity with any one base sequence selected from the group consisting of SEQ ID NOs: 257 to 275, and has a length within ±11% of the length of the any one base sequence selected,
(J2-6) a base sequence that has at least 90% identity with any one base sequence selected from the group consisting of SEQ ID NOs: 257 to 275, and has a length within ±10% of the length of the any one base sequence selected,

(J2-7) a base sequence that has at least 91% identity with any one base sequence selected from the group consisting of SEQ ID NOs: 257 to 275, and has a length within ±9% of the length of the any one base sequence selected,
(J2-8) a base sequence that has at least 92% identity with any one base sequence selected from the group consisting of SEQ ID NOs: 257 to 275, and has a length within ±8% of the length of the any one base sequence selected,
(J2-9) a base sequence that has at least 93% identity with any one base sequence selected from the group consisting of SEQ ID NOs: 257 to 275, and has a length within ±7% of the length of the any one base sequence selected,
(J2-10) a base sequence that has at least 94% identity with any one base sequence selected from the group consisting of SEQ ID NOs: 257 to 275, and has a length within ±6% of the length of the any one base sequence selected,
(J2-11) a base sequence that has at least 95% identity with any one base sequence selected from the group consisting of SEQ ID NOs: 257 to 275, and has a length within ±5% of the length of the any one base sequence selected,
(J2-12) a base sequence that has at least 96% identity with any one base sequence selected from the group consisting of SEQ ID NOs: 257 to 275, and has a length within ±4% of the length of the any one base sequence selected,
(J2-13) a base sequence that has at least 97% identity with any one base sequence selected from the group consisting of SEQ ID NOs: 257 to 275, and has a length within ±3% of the length of the any one base sequence selected,
(J2-14) a base sequence that has at least 98% identity with any one base sequence selected from the group consisting of SEQ ID NOs: 257 to 275, and has a length within ±2% of the length of the any one base sequence selected,
(J2-15) a base sequence that has at least 99% identity with any one base sequence selected from the group consisting of SEQ ID NOs: 257 to 275, and has a length within ±1% of the length of the any one base sequence selected, and
(J2-16) a base sequence that has at least 99.5% identity with any one base sequence selected from the group consisting of SEQ ID NOs: 257 to 275, and has a length within ±0.5% of the length of the any one base sequence selected.

Thus, a further embodiment of the suppressor antisense oligomer of the present invention is a suppressor antisense oligomer consisting of any one base sequence selected from the group consisting of (J1) and (J2-1) to (J2-16) or a pharmaceutically acceptable salt thereof, or hydrate thereof.
A further embodiment of the suppressor antisense oligomer of the present invention is a suppressor antisense oligomer consisting of

(J'1) any one base sequence selected from the group consisting of SEQ ID NO: 260, 261, and 263, or
(J'2) a base sequence that has at least 85% identity with any one base sequence selected from the group consisting of SEQ ID NOs: 260, 261, and 263, and has a length within ±15% of the length of the any one base sequence selected, or

a pharmaceutically acceptable salt thereof, or hydrate thereof.
Herein, the base sequence (J'2) is a mutant type of the base sequence (J'1). Such a mutant type also includes

(J'2-1) a base sequence that has at least 85% identity with any one base sequence selected from the group consisting of SEQ ID NOs: 260, 261, and 263, and has a length within ±15% of the length of the any one base sequence selected,
(J'2-2) a base sequence that has at least 86% identity with any one base sequence selected from the group consisting of SEQ ID NOs: 260, 261, and 263, and has a length within ±14% of the length of the any one base sequence selected,
(J'2-3) a base sequence that has at least 87% identity with any one base sequence selected from the group consisting of SEQ ID NOs: 260, 261, and 263, and has a length within ±13% of the length of the any one base sequence selected,
(J'2-4) a base sequence that has at least 88% identity with any one base sequence selected from the group consisting of SEQ ID NOs: 260, 261, and 263, and has a length within ±12% of the length of the any one base sequence selected,
(J'2-5) a base sequence that has at least 89% identity with any one base sequence selected from the group consisting of SEQ ID NOs: 260, 261, and 263, and has a length within ±11% of the length of the any one base sequence selected,
(J'2-6) a base sequence that has at least 90% identity with any one base sequence selected from the group consisting of SEQ ID NOs: 260, 261, and 263, and has a length within ±10% of the length of the any one base sequence selected,
(J'2-7) a base sequence that has at least 91% identity with any one base sequence selected from the group consisting of SEQ ID NOs: 260, 261, and 263, and has a length within ±9% of the length of the any one base sequence selected,
(J'2-8) a base sequence that has at least 92% identity with any one base sequence selected from the group consisting of SEQ ID NOs: 260, 261, and 263, and has a length within ±8% of the length of the any one base sequence selected,

(J'2-9) a base sequence that has at least 93% identity with any one base sequence selected from the group consisting of SEQ ID NOs: 260, 261, and 263, and has a length within ±7% of the length of the any one base sequence selected,
(J'2-10) a base sequence that has at least 94% identity with any one base sequence selected from the group consisting of SEQ ID NOs: 260, 261, and 263, and has a length within ±6% of the length of the any one base sequence selected,
(J'2-11) a base sequence that has at least 95% identity with any one base sequence selected from the group consisting of SEQ ID NOs: 260, 261, and 263, and has a length within ±5% of the length of the any one base sequence selected,
(J'2-12) a base sequence that has at least 96% identity with any one base sequence selected from the group consisting of SEQ ID NOs: 260, 261, and 263, and has a length within ±4% of the length of the any one base sequence selected,
(J'2-13) a base sequence that has at least 97% identity with any one base sequence selected from the group consisting of SEQ ID NOs: 260, 261, and 263, and has a length within ±3% of the length of the any one base sequence selected,
(J'2-14) a base sequence that has at least 98% identity with any one base sequence selected from the group consisting of SEQ ID NOs: 260, 261, and 263, and has a length within ±2% of the length of the any one base sequence selected,
(J'2-15) a base sequence that has at least 99% identity with any one base sequence selected from the group consisting of SEQ ID NOs: 260, 261, and 263, and has a length within ±1% of the length of the any one base sequence selected, and
(J'2-16) a base sequence that has at least 99.5% identity with any one base sequence selected from the group consisting of SEQ ID NOs: 260, 261, and 263, and has a length within ±0.5% of the length of the any one base sequence selected.

Thus, a further embodiment of the suppressor antisense oligomer of the present invention is a suppressor antisense oligomer consisting of any one base sequence selected from the group consisting of (J'1) and (J'2-1) to (J'2-16) or a pharmaceutically acceptable salt thereof, or hydrate thereof.

The suppressor antisense oligomer of the present invention enhances a multi-skipping effect under physiological conditions. The term "under physiological conditions" refers to conditions set to mimic the *in vivo* environment in terms of pH, salt composition and temperature. The conditions are, for example, 25 to 40°C, preferably 37°C, pH 5 to 8, preferably pH 7.4 and 150 mM of sodium chloride concentration.

Whether the suppressor antisense oligomer of the present invention enhances a multi-exon skipping effect or not can be confirmed by providing (i) an experimental system for multi-exon skipping using only the antisense oligomer of the present invention alone and (ii) an experimental system for multi-exon skipping using the antisense oligomer of the present invention and the suppressor antisense oligomer of the present invention such that the other conditions are the same therebetween, and observing the difference between a multi-exon skipping effect obtained in the experimental system (ii) and a multi-exon skipping effect obtained in the experimental system (i).

The multi-skipping effect is measured as mentioned in the section "1. Antisense oligomer" about the antisense oligomer of the present invention.

The suppressor antisense oligomer of the present invention is 10 to 60 bases long and may be 10 to 55 bases long, 10 to 50 bases long, 10 to 45 bases long, 10 to 40 bases long, 10 to 35 bases long, 10 to 30 bases long, 10 to 25 bases long, 15 to 60 bases long, 15 to 55 bases long, 15 to 50 bases long, 15 to 45 bases long, 15 to 40 bases long, 15 to 35 bases long, 15 to 30 bases long, 15 to 25 bases long, 16 to 60 bases long, 16 to 55 bases long, 16 to 50 bases long, 16 to 45 bases long, 16 to 40 bases long, 16 to 35 bases long, 16 to 30 bases long, 16 to 25 bases long, 17 to 60 bases long, 17 to 55 bases long, 17 to 50 bases long, 17 to 45 bases long, 17 to 40 bases long, 17 to 35 bases long, 17 to 30 bases long, 17 to 25 bases long, 18 to 60 bases long, 18 to 55 bases long, 18 to 50 bases long, 18 to 45 bases long, 18 to 40 bases long, 18 to 35 bases long, 18 to 30 bases long, 18 to 25 bases long, 19 to 60 bases long, 19 to 55 bases long, 19 to 50 bases long, 19 to 45 bases long, 19 to 40 bases long, 19 to 35 bases long, 19 to 30 bases long, 19 to 25 bases long, 20 to 60 bases long, 20 to 55 bases long, 20 to 50 bases long, 20 to 45 bases long, 20 to 40 bases long, 20 to 35 bases long, 20 to 30 bases long, 20 to 25 bases long, 15 to 30 bases long, 15 to 29 bases long, 15 to 28 bases long, 15 to 27 bases long, 15 to 26 bases long, 15 to 25 bases long, 15 to 24 bases long, 15 to 23 bases long, 15 to 22 bases long, 15 to 21 bases long, 15 to 20 bases long, 15 to 19 bases long, 15 to 18 bases long, 16 to 30 bases long, 16 to 29 bases long, 16 to 28 bases long, 16 to 27 bases long, 16 to 26 bases long, 16 to 25 bases long, 16 to 24 bases long, 16 to 23 bases long, 16 to 22 bases long, 16 to 21 bases long, 16 to 20 bases long, 16 to 19 bases long, 16 to 18 bases long, 17 to 30 bases long, 17 to 29 bases long, 17 to 28 bases long, 17 to 27 bases long, 17 to 26 bases long, 17 to 25 bases long, 17 to 24 bases long, 17 to 23 bases long, 17 to 22 bases long, 17 to 21 bases long, 17 to 20 bases long, 17 to 19 bases long, 17 to 18 bases long, 18 to 30 bases long, 18 to 29 bases long, 18 to 28 bases long, 18 to 27 bases long, 18 to 26 bases long, 18 to 25 bases long, 18 to 24 bases long, 18 to 23 bases long, 18 to 22 bases

long, 18 to 21 bases long, 18 to 20 bases long, 18 to 19 bases long, 19 to 30 bases long, 19 to 29 bases long, 19 to 28 bases long, 19 to 27 bases long, 19 to 26 bases long, 19 to 25 bases long, 19 to 24 bases long, 19 to 23 bases long, 19 to 22 bases long, 19 to 21 bases long, 19 to 20 bases long, 20 to 30 bases long, 20 to 29 bases long, 20 to 28 bases long, 20 to 27 bases long, 20 to 26 bases long, 20 to 25 bases long, 20 to 24 bases long, 20 to 23 bases long, 20 to 22 bases long, 20 to 21 bases long, 60 bases long, 59 bases long, 58 bases long, 57 bases long, 56 bases long, 55 bases long, 54 bases long, 53 bases long, 52 bases long, 51 bases long, 50 bases long, 49 bases long, 48 bases long, 47 bases long, 46 bases long, 45 bases long, 44 bases long, 43 bases long, 42 bases long, 41 bases long, 40 bases long, 39 bases long, 38 bases long, 37 bases long, 36 bases long, 35 bases long, 34 bases long, 33 bases long, 32 bases long, 31 bases long, 30 bases long, 29 bases long, 28 bases long, 27 bases long, 26 bases long, 25 bases long, 24 bases long, 23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, or 10 bases long, but not limited thereto. These lengths may be increased or decreased by 1, 2, or 3 bases.

The suppressor antisense oligomer of the present invention may be a linked-type antisense oligomer configured to comprise a plurality of unit oligomers linked to each other, or a pharmaceutically acceptable salt thereof, or hydrate thereof. The unit oligomers may be linked via a linker that does not contribute to hybridization, or may be linked directly without the mediation of a linker, as mentioned above. When the suppressor antisense oligomer of the present invention is a linked type, the respective base sequences of the unit oligomers are neither consecutive nor overlapped with each other.

The suppressor antisense oligomer of the present invention may be an oligonucleotide, morpholino oligomer, or peptide nucleic acid (PNA) oligomer.

When the suppressor antisense oligomer of the present invention is an oligonucleotide (hereinafter, referred to as the "suppressor antisense oligonucleotide of the present invention"), the suppressor antisense oligonucleotide of the present invention is a suppressor antisense oligomer composed of nucleotides as constituent units. Such nucleotides may be any of ribonucleotides, deoxyribonucleotides and modified nucleotides.

The modified nucleotide is as mentioned above.

Preferably, the suppressor antisense oligonucleotide of the present invention is the suppressor antisense oligomer of the present invention having a group represented by general formula below as a constituent unit wherein the -OH group at position 2' of ribose is substituted with methoxy and the phosphate bond moiety is a phosphorothioate bond:

[Formula 9]

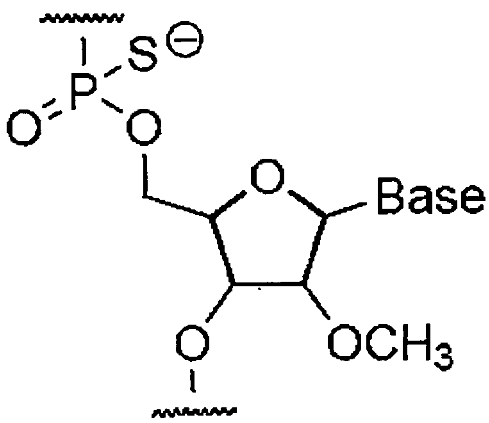

wherein Base has the same significance as defined above.

The suppressor antisense oligonucleotide of the present invention may be easily synthesized using various automated synthesizer (e.g., AKTA oligopilot plus 10/100 (GE Healthcare)). Alternatively, the synthesis may also be entrusted to a third-party organization (e.g., Promega Corp. or Takara Co.), etc.

When the suppressor antisense oligomer of the present invention is a morpholino oligomer (hereinafter, referred to as the "suppressor antisense morpholino oligomer of the present invention"), the suppressor antisense morpholino oligomer of the present invention is a suppressor antisense oligomer having a group represented by general formula below as a constituent unit:

[Formula 10]

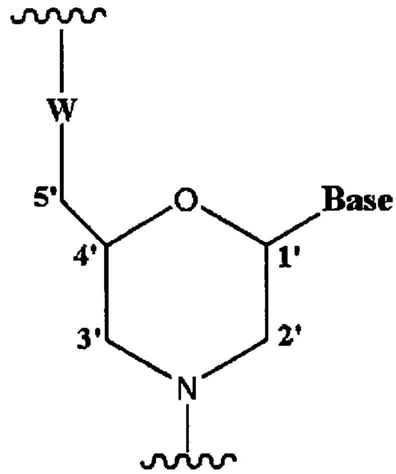

wherein Base and W have the same significance as defined above.

Examples of morpholino monomer compounds that are used in synthesis of the suppressor antisense morpholino oligomer of the present invention include, but not limited to, the following morpholino monomer compound (A), morpholino monomer compound (C), morpholino monomer compound (T), and morpholino monomer compound (G) shown in Table 7.

[Table 7]

Table 7

| Morpholino monomer compound (A) | Morpholino monomer compound (C) | Morpholino monomer compound (T) | Morpholino monomer compound (G) |
|---|---|---|---|
| 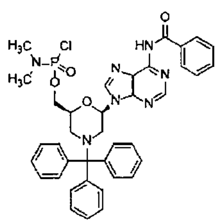 | 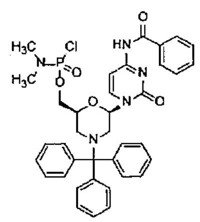 | 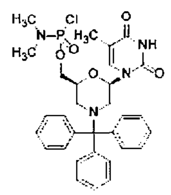 | 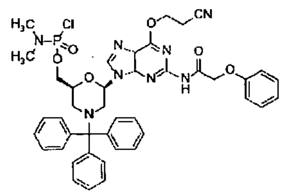 |

In the suppressor antisense morpholino oligomer of the present invention, preferably the morpholino oligomer is PMO.

The morpholino oligomer may be produced by the procedure described in, *e.g.,* WO 1991/009033 or WO 2009/064471. In particular, PMO can be produced by the procedure described in WO 2009/064471 or WO2013/100190.

When the suppressor antisense oligomer of the present invention is a peptide nucleic acid oligomer (hereinafter, referred to as the "suppressor antisense peptide nucleic acid oligomer of the present invention"), the suppressor antisense peptide nucleic acid oligomer of the present invention is a suppressor antisense oligomer having a group represented by general formula (I) below as a constituent unit:

[Formula 11]

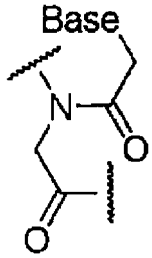

wherein Base has the same significance as defined above.

The peptide nucleic acid oligomer can be produced as mentioned above.

The suppressor antisense oligomer of the present invention may be in the form of a pharmaceutically acceptable salt thereof, in the form of a hydrate thereof, or in the form of a hydrate of the pharmaceutically acceptable salt.

Examples of the pharmaceutically acceptable salt of the suppressor antisense oligomer of the present invention are alkali metal salts such as salts of sodium, potassium and lithium; alkaline earth metal salts such as salts of calcium and magnesium; metal salts such as salts of aluminum, iron, zinc, copper, nickel, cobalt, etc.; ammonium salts; organic amine salts such as salts of t-octylamine, dibenzylamine, morpholine, glucosamine, phenylglycine alkyl ester, ethylenediamine, N-methylglucamine, guanidine, diethylamine, triethylamine, dicyclohexylamine, *N,N'* -dibenzylethylenediamine, chloroprocaine, procaine, diethanolamine, *N*-benzyl-phenethylamine, piperazine, tetramethylammonium, tris(hydroxymethyl)aminomethane; hydrohalide salts such as salts of hydrofluorides, hydrochlorides, hydrobromides and hydroiodides; inorganic acid salts such as nitrates, perchlorates, sulfates, phosphates, etc.; lower alkane sulfonates such as methanesulfonates, trifluoromethanesulfonates and ethanesulfonates; arylsulfonates such as benzenesulfonates and p-toluenesulfonates; organic acid salts such as acetates, malates, fumarates, succinates, citrates, tartarates, oxalates, maleates, etc.; and, amino acid salts such as salts of glycine, lysine, arginine, ornithine, glutamic acid and aspartic acid. These salts may be produced by known methods. Alternatively, the suppressor antisense oligomer of the present invention may be in the form of a hydrate thereof.

[Method for producing PMO]

Each of the antisense oligomer of the present invention and the suppressor antisense oligomer of the present invention may be PMO. An embodiment of PMO is, for example, the compound represented by general formula (I) below (hereinafter, referred to as PMO (I)).

[Formula 12]

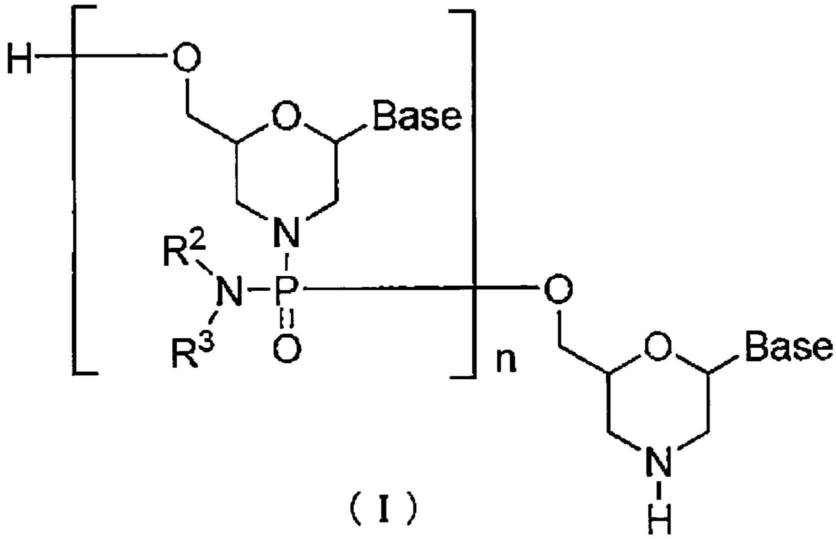

(I)

wherein Base, $R^2$ and $R^3$ have the same significance as defined above; and,

n is a given integer of 1 to 99, preferably a given integer of 18 to 28.

PMO (I) can be produced in accordance with a known method (cf., *e.g.,* WO2009/064471 or WO2013/100190).

In the antisense oligomer of the present invention or the suppressor antisense oligomer of the present invention, the 5' end may be a group represented by any of chemical structures (1) to (3) below, and preferably is (3)-OH.

[Formula 13]

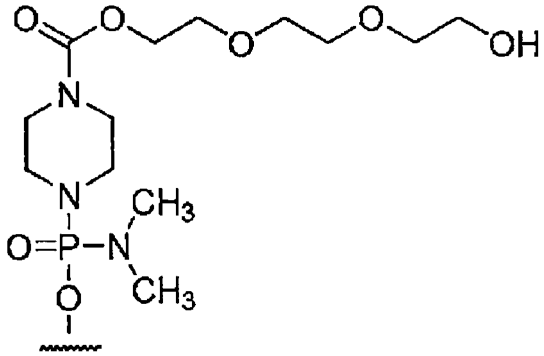

(1)

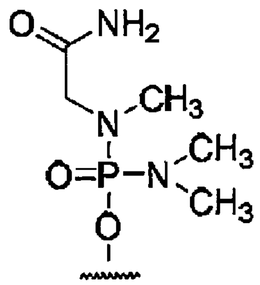

(2)

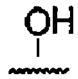

(3)

Hereinafter, the groups shown by (1), (2) and (3) above are referred to as "Group (1)," "Group (2)" and "Group (3)," respectively.

3. Medical application

The present invention provides a pharmaceutical composition comprising the antisense oligomer of the present invention (also including the pharmaceutically acceptable salt thereof, or hydrate thereof) (hereinafter, referred to as the "pharmaceutical composition of the present invention"). The pharmaceutical composition of the present invention may further comprise the suppressor antisense oligomer of the present invention (also including the pharmaceutically acceptable salt thereof, or hydrate thereof) and/or a pharmaceutically acceptable carrier.

The present invention also provides a pharmaceutical composition comprising the antisense oligomer of the present invention (also including the pharmaceutically acceptable salt thereof, or hydrate thereof) and the suppressor antisense oligomer of the present invention (also including the pharmaceutically acceptable salt thereof, or hydrate thereof) (hereinafter, referred to as the "pharmaceutical combination of the present invention").

When the pharmaceutical composition of the present invention comprises the antisense oligomer of the present invention and the suppressor antisense oligomer of the present invention, any combination of these oligomers is included. In the pharmaceutical combination of the present invention, any combination of the antisense oligomer of the present invention and the suppressor antisense oligomer of the present invention is included. The antisense oligomer contained in the pharmaceutical composition of the present invention or the pharmaceutical combination of the present invention can be any antisense oligomer of the present invention and is, but not particularly limited to, preferably an antisense oligomer consisting of any one base sequence selected from the group consisting of SEQ ID NOs: 1 to 111, 113 to 177, 179, 180, 182, 183, 185 to 193, 195 to 198, 200 to 223, and 225 to 232, more preferably an antisense oligomer consisting of any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232, and further preferably an antisense oligomer consisting of any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228.

The suppressor antisense oligomer contained in the pharmaceutical composition of the present invention or the pharmaceutical combination of the present invention can be any suppressor antisense oligomer of the present invention and is, but not particularly limited to, preferably a suppressor antisense oligomer consisting of any one base sequence selected from the group consisting of SEQ ID NOs: 257 to 275, more preferably SEQ ID NO: 1, 5, 6, 7, 8, 10, 11, 14, 26, or 27, and further preferably a suppressor antisense oligomer consisting of any one base sequence selected from the group consisting of SEQ ID NOs: 260, 261, and 263.

When the pharmaceutical composition of the present invention comprises the antisense oligomer of the present invention and the suppressor antisense oligomer of the present invention, preferably, the pharmaceutical composition comprises a combination of the antisense oligomer of the present invention and the suppressor antisense oligomer of the present invention described above. Preferably, the pharmaceutical combination of the present invention comprises a combination of the antisense oligomer of the present invention and the suppressor antisense oligomer of the present invention described above.

In the pharmaceutical combination of the present invention, examples of the combination of the antisense oligomer of the present invention and the suppressor antisense oligomer of the present invention include

(K1) a combination in which the antisense oligomer is an oligomer consisting of SEQ ID NO: 75 and the suppressor antisense oligomer is an oligomer consisting of SEQ ID NO: 260,
(K2) a combination in which the antisense oligomer is an oligomer consisting of SEQ ID NO: 75 and the suppressor antisense oligomer is an oligomer consisting of SEQ ID NO: 261,
(K3) a combination in which the antisense oligomer is an oligomer consisting of SEQ ID NO: 75 and the suppressor antisense oligomer is an oligomer consisting of SEQ ID NO: 263,
(K4) a combination in which the antisense oligomer is an oligomer consisting of SEQ ID NO: 65 and the suppressor antisense oligomer is an oligomer consisting of SEQ ID NO: 260,
(K5) a combination in which the antisense oligomer is an oligomer consisting of SEQ ID NO: 65 and the suppressor antisense oligomer is an oligomer consisting of SEQ ID NO: 261,
(K6) a combination in which the antisense oligomer is an oligomer consisting of SEQ ID NO: 65 and the suppressor antisense oligomer is an oligomer consisting of SEQ ID NO: 263,
(K7) a combination in which the antisense oligomer is a combination of oligomers consisting of SEQ ID NOs: 55 and 59 and the suppressor antisense oligomer is an oligomer consisting of SEQ ID NO: 260,
(K8) a combination in which the antisense oligomer is a combination of oligomers consisting of SEQ ID NOs: 55 and 59 and the suppressor antisense oligomer is an oligomer consisting of SEQ ID NO: 261, and
(K9) a combination in which the antisense oligomer is a combination of oligomers consisting of SEQ ID NOs: 55 and 59 and the suppressor antisense oligomer is an oligomer consisting of SEQ ID NO: 263.

The pharmaceutical composition of the present invention may comprise the antisense oligomer of the present invention and the suppressor antisense oligomer of the present invention in these combinations.

The pharmaceutical composition of the present invention and the pharmaceutical combination of the present invention can each be used for the treatment of, for example, Duchenne muscular dystrophy, Becker muscular dystrophy, limb-girdle muscular dystrophy (LGMD), congenital muscular dystrophy, Emery-Dreifuss muscular dystrophy, facioscapulohumeral muscular dystrophy, oculopharyngeal muscular dystrophy, cerebral autosomal dominant arteriopathy with subcortical infarct and leukoencephalopathy (CADASIL), and Alport's syndrome. The pharmaceutical combination of the present invention and the pharmaceutical composition of the present invention can each be administered to a human patient and in particular, a human patient with muscular dystrophy. The patient to receive the pharmaceutical combination of the present invention or the pharmaceutical composition of the present invention may be a human patient having a mutation that is amenable to skipping of two or more exons selected from the group consisting of exons 45 to 55 in the dystrophin gene.

One embodiment of the present invention provides a method for treatment of muscular dystrophy, which comprises administering to a patient with muscular dystrophy the antisense oligomer of the present invention or a combination of the antisense oligomer of the present invention and the suppressor antisense oligomer of the present invention.

Another embodiment of the present invention provides a method for treatment of muscular dystrophy, which comprises administering to a patient with muscular dystrophy the pharmaceutical composition of the present invention or the pharmaceutical combination of the present invention.

The method for treatment may involve performing skipping of any two or more numerically consecutive exons selected from the group consisting of the 45th exon to the 55th exon in human dystrophin pre-mRNA. In the method for treatment, the patient with muscular dystrophy may be a patient having a mutation that is amenable to exon 45 to 55 skipping in the dystrophin gene. The patient may be a human and may be a human patient having a mutation that is amenable to exon 45 to 55 skipping in the dystrophin gene.

The present invention further provides use of the antisense oligomer of the present invention or a combination of the antisense oligomer of the present invention and the suppressor antisense oligomer of the present invention, or the pharmaceutical composition of the present invention or the pharmaceutical combination of the present invention in manufacturing of a medicament for the treatment of muscular dystrophy.

The present invention further provides the antisense oligomer of the present invention or a combination of the antisense oligomer of the present invention and the suppressor antisense oligomer of the present invention, or the pharmaceutical composition of the present invention or the pharmaceutical combination of the present invention for use

in the treatment of muscular dystrophy. The treatment may involve performing skipping of any two or more numerically consecutive exons selected from the group consisting of the 45th exon to the 55th exon in human dystrophin pre-mRNA. In the treatment, the patient with muscular dystrophy may be a patient having a mutation that is amenable to exon 45 to 55 skipping in the dystrophin gene. The patient may be a human and may be a human patient having a mutation that is amenable to exon 45 to 55 skipping in the dystrophin gene.

Administration route for the antisense oligomer of the present invention or the combination of the antisense oligomer of the present invention and the suppressor antisense oligomer of the present invention, or the pharmaceutical composition of the present invention or the pharmaceutical combination of the present invention is not particularly limited so long as it is pharmaceutically acceptable route for administration, and can be chosen depending upon method of treatment. In view of easiness in delivery to muscle tissues, preferred are intravenous administration, intraarterial administration, intramuscular administration, subcutaneous administration, oral administration, tissue administration, transdermal administration, etc. Also, dosage forms which are available for the composition of the present invention are not particularly limited, and include, for example, various injections, oral agents, drips, inhalations, ointments, lotions, etc.

In administration of the antisense oligomer of the present invention and/or the suppressor antisense oligomer of the present invention to patients with muscular dystrophy, preferably, the composition of the present invention contains a carrier to promote delivery of the oligomer to muscle tissues. Such a carrier is not particularly limited as far as it is pharmaceutically acceptable, and examples include cationic carriers such as cationic liposomes, cationic polymers, etc., or carriers using viral envelope. The cationic liposomes are, for example, liposomes composed of 2-O-(2-diethylaminoethyl)carbamoyl-1,3-O-dioleoylglycerol and phospholipids as the essential constituents (hereinafter referred to as "liposome A"), Oligofectamine (registered trademark) (manufactured by Invitrogen Corp.), Lipofectin (registered trademark) (manufactured by Invitrogen Corp.), Lipofectamine (registered trademark) (manufactured by Invitrogen Corp.), Lipofectamine 2000 (registered trademark) (manufactured by Invitrogen Corp.), DMRIE-C (registered trademark) (manufactured by Invitrogen Corp.), GeneSilencer (registered trademark) (manufactured by Gene Therapy Systems), TransMessenger (registered trademark) (manufactured by QIAGEN, Inc.), TransIT TKO (registered trademark) (manufactured by Mirus) and Nucleofector II (Lonza). Among others, liposome A is preferred. Examples of cationic polymers are JetSI (registered trademark) (manufactured by Qbiogene, Inc.) and Jet-PEI (registered trademark) (polyethylenimine, manufactured by Qbiogene, Inc.). An example of carriers using viral envelop is GenomeOne (registered trademark) (HVJ-E liposome, manufactured by Ishihara Sangyo). Alternatively, the medical devices described in Japanese Patent Nos. 2924179 and the cationic carriers described in Japanese Domestic RePublication PCT Nos. 2006/129594 and 2008/096690 may be used as well.

A concentration of the antisense oligomer of the present invention contained in the pharmaceutical composition of the present invention and/or the pharmaceutical combination of the present invention may vary depending on kind of the carrier, etc., and is appropriately in a range of 0.1 nM to 100 μM, preferably in a range of 1 nM to 10 μM, and more preferably in a range of 10 nM to 1 μM. A weight ratio of the antisense oligomer of the present invention contained in the composition of the present invention and the carrier (carrier/antisense oligomer of the present invention) may vary depending on property of the oligomer, type of the carrier, etc., and is appropriately in a range of 0.1 to 100, preferably in a range of 1 to 50, and more preferably in a range of 10 to 20.

A concentration of the suppressor antisense oligomer of the present invention contained in the pharmaceutical composition of the present invention and/or the pharmaceutical combination of the present invention may vary depending on kind of the carrier, etc., and is appropriately in a range of 0.1 nM to 100 μM, preferably in a range of 1 nM to 10 μM, and more preferably in a range of 10 nM to 1 μM. A weight ratio of the suppressor antisense oligomer of the present invention contained in the composition of the present invention and the carrier (carrier/suppressor antisense oligomer of the present invention) may vary depending on property of the oligomer, type of the carrier, etc., and is appropriately in a range of 0.1 to 100, preferably in a range of 1 to 50, and more preferably in a range of 10 to 20.

The pharmaceutical composition of the present invention and/or the pharmaceutical combination of the present invention may be in the form of an aqueous solution. In this case, the pharmaceutical composition of the present invention and/or the pharmaceutical combination of the present invention may comprise the antisense oligomer of the present invention in a concentration of 2.5 to 500 mg/mL, 5 to 450 mg/mL, 10 to 400 mg/mL, 15 to 350 mg/mL, 20 to 300 mg/mL, 20 to 250 mg/mL, 20 to 200 mg/mL, 20 to 150 mg/mL, 20 to 100 mg/mL, 20 to 50 mg/mL, 20 to 40 mg/mL, 20 to 30 mg/mL, 23 to 27 mg/mL, 24 to 26 mg/mL, or 25 mg/mL. Alternatively, the pharmaceutical composition of the present invention and/or the pharmaceutical combination of the present invention may comprise the antisense oligomer of the present invention in a concentration of 10 to 100 mg/mL, 15 to 95 mg/mL, 20 to 80 mg/mL, 25 to 75 mg/mL, 30 to 70 mg/mL, 35 to 65 mg/mL, 40 to 60 mg/mL, 45 to 55 mg/mL, 47 to 53 mg/mL, 48 to 52 mg/mL, 49 to 51 mg/mL, or 50 mg/mL.

The pharmaceutical composition of the present invention and/or the pharmaceutical combination of the present invention in the form of an aqueous solution may comprise the suppressor oligomer of the present invention in a concentration of 2.5 to 500 mg/mL, 5 to 450 mg/mL, 10 to 400 mg/mL, 15 to 350 mg/mL, 20 to 300 mg/mL, 20 to 250 mg/mL, 20 to 200 mg/mL, 20 to 150 mg/mL, 20 to 100 mg/mL, 20 to 50 mg/mL, 20 to 40 mg/mL, 20 to 30 mg/mL, 23 to 27 mg/mL, 24 to 26 mg/mL, or 25 mg/mL. Alternatively, the pharmaceutical composition of the present invention and/or the

pharmaceutical combination of the present invention may comprise the suppressor oligomer of the present invention in a concentration of 10 to 100 mg/mL, 15 to 95 mg/mL, 20 to 80 mg/mL, 25 to 75 mg/mL, 30 to 70 mg/mL, 35 to 65 mg/mL, 40 to 60 mg/mL, 45 to 55 mg/mL, 47 to 53 mg/mL, 48 to 52 mg/mL, 49 to 51 mg/mL, or 50 mg/mL.

The pharmaceutical composition of the present invention and/or the pharmaceutical combination of the present invention may be in a dry form. In this case, in order to prepare the pharmaceutical composition of the present invention and/or the pharmaceutical combination of the present invention in an aqueous solution form, for example, 125 mg or 250 mg of the antisense oligomer of the present invention in a dry form may be mixed with 0.5 mL to 100 mL of water (which corresponds to the antisense oligomer of the present invention in a concentration of 1.25 mg/mL to 250 mg/mL or 2.5 mg/mL to 500 mg/mL), preferably with 1 mL to 50 mL of water (which corresponds to the antisense oligomer of the present invention in a concentration of 2.5 mg/mL to 125 mg/mL or 5 mg/mL to 250 mg/mL), more preferably with 5 mL to 10 mL of water (which correspond to the antisense oligomer of the present invention in a concentration of 12.5 mg/mL to 25 mg/mL or 25 mg/mL to 50 mg/mL) and used.

When the pharmaceutical composition of the present invention and/or the pharmaceutical combination of the present invention is in a dry form, in order to prepare the pharmaceutical composition of the present invention and/or the pharmaceutical combination of the present invention in an aqueous solution form, for example, 125 mg or 250 mg of the suppressor oligomer of the present invention in a dry form may be mixed with 0.5 mL to 100 mL of water (which corresponds to the suppressor oligomer of the present invention in a concentration of 1.25 mg/mL to 250 mg/mL or 2.5 mg/mL to 500 mg/mL), preferably with 1 mL to 50 mL of water (which corresponds to the suppressor oligomer of the present invention in a concentration of 2.5 mg/mL to 125 mg/mL or 5 mg/mL to 250 mg/mL), more preferably with 5 mL to 10 mL of water (which correspond to the suppressor oligomer of the present invention in a concentration of 12.5 mg/mL to 25 mg/mL or 25 mg/mL to 50 mg/mL) and used.

The concentrations of the antisense oligomer of the present invention and the suppressor oligomer of the present invention contained in the pharmaceutical composition of the present invention and/or the pharmaceutical combination of the present invention may be the respective concentrations of the antisense oligomer of the present invention and the suppressor oligomer of the present invention or may be the total concentration of the antisense oligomer of the present invention and the suppressor oligomer of the present invention.

In addition to the antisense oligomer of the present invention and/or the suppressor antisense oligomer of the present invention and the carrier described above, pharmaceutically acceptable additives may also be optionally formulated in the pharmaceutical composition of the present invention and/or the pharmaceutical combination of the present invention. Examples of such additives are emulsification aids (e.g., fatty acids having 6 to 22 carbon atoms and their pharmaceutically acceptable salts, albumin and dextran), stabilizers (*e.g.,* cholesterol, phosphatidic acid, mannitol, and sorbitol), isotonizing agents (e.g., sodium chloride, glucose, maltose, lactose, sucrose, and trehalose), and pH controlling agents (e.g., hydrochloric acid, sulfuric acid, phosphoric acid, acetic acid, sodium hydroxide, potassium hydroxide and triethanolamine). One or more of these additives can be used. The content of the additive in the composition of the present invention is appropriately 90 wt% or less, preferably 70 wt% or less and more preferably, 50 wt% or less.

The pharmaceutical composition of the present invention and/or the pharmaceutical combination of the present invention can be prepared by adding the antisense oligomer of the present invention and/or the suppressor antisense oligomer of the present invention to a carrier dispersion and adequately stirring the mixture. Additives may be added at an appropriate step either before or after addition of the antisense oligomer of the present invention and/or the suppressor antisense oligomer of the present invention. An aqueous solvent that can be used in adding the antisense oligomer of the present invention and/or the suppressor antisense oligomer of the present invention is not particularly limited as far as it is pharmaceutically acceptable, and examples are injectable water or injectable distilled water, electrolyte fluid such as physiological saline, etc., and sugar fluid such as glucose fluid, maltose fluid, etc. A person skilled in the art can appropriately choose conditions for pH and temperature for such matter.

The pharmaceutical composition of the present invention and/or the pharmaceutical combination of the present invention may be prepared into, e.g., a liquid form and its lyophilized preparation. The lyophilized preparation can be prepared by lyophilizing the composition of the present invention in a liquid form in a conventional manner. The lyophilization can be performed, for example, by appropriately sterilizing the composition of the present invention in a liquid form, dispensing an aliquot into a vial container, performing preliminary freezing for 2 hours at conditions in a range of about -40°C to -20°C, performing a primary drying in a range of about 0°C to 10°C under reduced pressure, and then performing a secondary drying in a range of about 15°C to 25°C under reduced pressure. In general, the lyophilized preparation of the composition of the present invention can be obtained by replacing the content of the vial with nitrogen gas and capping.

The lyophilized preparation of the pharmaceutical composition of the present invention and/or the pharmaceutical combination of the present invention can be used in general upon reconstitution by adding an optional suitable solution (reconstitution liquid) and redissolving the preparation. Such a reconstitution liquid includes injectable water, physiological saline and other infusion fluids. A volume of the reconstitution liquid may vary depending on the intended use, etc., is not particularly limited, and is suitably 0.5-fold to 2-fold greater than the volume prior to lyophilization or no

more than 500 mL.

It is desired to control a dose of the pharmaceutical composition of the present invention and/or the pharmaceutical combination of the present invention to be administered, by taking the following factors into account: the type and dosage form of the antisense oligomer of the present invention and/or the suppressor antisense oligomer of the present invention contained; patients' conditions including age, body weight, etc.; administration route; and the characteristics and extent of the disease. A single dose calculated as the amount of the antisense oligomer of the present invention and/or the suppressor antisense oligomer of the present invention can be 0.1 mg to 1 g per kg body weight, preferably 1 mg to 100 mg per kg body weight, more preferably 1 mg to 90 mg per kg body weight, and further preferably 1 mg to 80 mg per kg body weight. The frequency of administration may be once per 1 to 3 days, once per week, or once per 2 to 3 weeks. This numerical range may vary occasionally depending on type of the target disease, administration route and target molecule. Therefore, a dose or frequency of administration lower than the range may be sufficient in some occasion and conversely, a dose or frequency of administration higher than the range may be required occasionally.

In still another embodiment of the pharmaceutical composition of the present invention and/or the pharmaceutical combination of the present invention, there is provided a pharmaceutical composition comprising a vector capable of expressing the antisense oligomer of the present invention and/or the suppressor antisense oligomer of the present invention and the carrier described above. Such an expression vector may be a vector capable of expressing a plurality of the antisense oligomers of the present invention and/or the suppressor antisense oligomers of the present invention. The composition may be formulated with pharmaceutically acceptable additives as in the case with the composition of the present invention containing the antisense oligomer of the present invention and/or the suppressor antisense oligomer of the present invention. A concentration of the expression vector contained in the composition may vary depending upon type of the career, etc., and is appropriately in a range of 0.1 nM to 100 μM, preferably in a range of 1 nM to 10 μM, and more preferably in a range of 10 nM to 1 μM. A weight ratio of the expression vector contained in the composition and the carrier (carrier/expression vector) may vary depending on property of the expression vector, type of the carrier, etc., and is appropriately in a range of 0.1 to 100, preferably in a range of 1 to 50, and more preferably in a range of 10 to 20. The content of the carrier contained in the composition is the same as in the case with the composition of the present invention containing the antisense oligomer of the present invention and/or the suppressor antisense oligomer of the present invention, and a method for producing the same is also the same as in the case with the composition of the present invention.

4. pre-mRNA of interest other than human dystrophin pre-mRNA

As used herein, the case where the pre-mRNA of interest is human dystrophin pre-mRNA is specifically described, but is not limited thereto. As for pre-mRNA of interest other than human dystrophin pre-mRNA, methods for designing, producing, and using an antisense oligomer and a suppressor antisense oligomer can be carried out in accordance with techniques and methods disclosed herein or known in the art. Those skilled in the art can also design, produce and use an antisense oligomer or a suppressor antisense oligomer for pre-mRNA of interest other than human dystrophin pre-mRNA on the basis of techniques and methods disclosed herein or known in the art. Likewise, those skilled in the art can perform multi-exon skipping of the pre-mRNA of interest using the antisense oligomer and enhance the efficiency of multi-exon skipping using the suppressor antisense oligomer on the basis of techniques and methods disclosed herein or known in the art.

As used herein, examples of the pre-mRNA of interest include human γ-sarcoglycan (SGCG) pre-mRNA, human Notch3 pre-mRNA, and human dystrophin pre-mRNA. In a certain embodiment of the present invention, the pre-mRNA of interest may be human dystrophin pre-mRNA. In another embodiment of the present invention, the pre-mRNA of interest may be SGCG or human Notch3 pre-mRNA.

The base sequences of target regions of the antisense oligomer for use in multi-exon skipping in the pre-mRNA of interest of human SGCG or human Notch3 are as given below.

In the present invention, the target regions in human SGCG pre-mRNA are shown as SEQ ID NOs: 283 and 284 below.

[Table 8]

Table 8

| Target region | Base sequence of target region | SEQ ID NO |
|---|---|---|
| Region in vicinity of acceptor of intron 3 (region indicated by range of -400 bases to +50 bases with 3' end of intron 3 defined as basing point 0) | ATTACAAAAATATGGAAATAGAGAGTAGCAGGAAAAAGTATATGGCATT<br>TCTATTACCTAGAACCCCACTGTTGTAGGTGTCTTTCCTTGACATAGCT<br>GTGACCATATAGCTCTGTTAAATTGCATATTTGTCTAAATAATTCATAT<br>TTTTTTCTATCCCTATCTTGCGTTTGGAGCTCATTTTATGATTGTTATT<br>CTTTTATCTTTCAATAAATACTCAAAATGTTAAAAAAATATGATTCAGG<br>AATTTTTAATTGTCTTAGCCACAAATTTATAGGATTTCCAGGATCTGTA<br>ACAATGGATAAATAATTTTATAAAAATCCTAAATTTACACAGAATTATA<br>AAGATATAATCATTTTAAACAGCACCTATTTTGCAAATTTTATAAATCT<br>CTTTCTAGGACTCATCTCTGCTTCTACAATCAACCCAGAATGTGACTGT<br>AAATGCGCG | 283 |
| Region in vicinity of donor of intron 4 (region indicated by range of -20 bases to +400 bases with 5' end of intron 4 defined as basing point 0) | CACAGGCAGGTTAAAAGTCGGTGAGTCCAGCTTCATCATGGTGCTTTGC<br>ATGCATGTTGTCCATGAATAGTGCTAAATGAATGCATTGTTTTTTCTTC<br>TAAAGAAATCAAAGCTACTTATGAACAAAATATGAATTTTCTAAATATC<br>ATGCTGTGTTGACCACAGACTAGCACCACAGAGTGGGGTGGGGGGTGAG<br>GGGACAGCCTGAAGTGCTTTGATTAGAGTTATTTTTGTTCCAAGAAATA<br>GAGGACAATTAAAGCTAGTACAAGAAACAAAATGTTTATTGTGGGTTAC<br>CAAAGACTCTTGCAGCAACCAAAGGGAAGGAACCCAAGTAGCTGGCCTC<br>AAAGAGCACAGAGACCAGTGGCTGAGAGCCACGAATACATTCAGTTCCC<br>TCCCCGTTCTTTCTTCTCTTTTCTCTCC | 284 |

In the present invention, the target regions in human Notch3 pre-mRNA are shown below.

[Table 9]

Table 9

| Target region | Base sequence of target region | SEQ ID NO |
|---|---|---|
| Region in vicinity of acceptor of intron 3 (region indicated by range of -400 bases to +50 bases with 3' end of intron 3 defined as basing point 0) | AAGTGATCTGCCCGCCGCAGCCTCCCAAAGTTCTGGGATTACAGGCGTG<br>AGCCACTGCTCCCAGCAAATGTGTGTTTGCTGCTCTGTTTCCCTGCGTG<br>TTTCTTGCCTGTCTTGTGTGTATCTTTGTGTCTGGGGCCATCCTGCCCT<br>GTGCTGCCCAACCAAGCCATCTCTGCCCACAGGTGCCCGCCTGGCTGGG<br>TGGGTGAGCGGTGTCAGCTGGAGGACCCCTGTCACTCAGGCCCCTGTGC<br>TGGCCGTGGTGTCTGCCAGAGTTCAGTGGTGGCTGGCACCGCCCGATTC<br>TCATGCCGGTGCCCCCGTGGCTTCCGAGGTGAGAGGGGAAGAGTCTGGA<br>GGGGAGGTAGTCGGGGGTGTGGTCAGTCCTAAACTCACCCTGTCCTGGT<br>CCCTCCAGGCCCTGACTGCTCCCTGCCAGATCCCTGCCTCAGCAGCCCT<br>TGTGCCCAC | 285 |
| Region in vicinity of donor of intron 4 (region indicated by range of -20 bases to +400 bases with 5' en of intron 4 defined as basing point 0) | CGACTGTGCCTGTCTTCCTGGTGAGTGAGCCCTACTCAGGAGAGTCAGA<br>GGGGTGGGCGTGGGGACAGCAGGCCAGCCCGGCGGTGACCATCCTTGCC<br>CCCTTCCCTGCTAGGGTTTGAGGGTCAGAATTGTGAAGTGAACGTGGAC<br>GACTGTCCAGGACACCGATGTCTCAATGGGGGACATGCGTGGATGGCG<br>TCAACACCTATAACTGCCAGTGCCCTCCTGAGTGGACAGGTGGGCACTG<br>CGGCCAGAGGGAGCGGGGAGGCAGGCCTCGGGTGGACATGCGCCAGGTG<br>GCTGGACTGCTGCATCTGTGTGCCACAGGCCAGTTCTGCACGGAGGACG<br>TGGATGAGTGTCAGCTGCAGCCCAACGCCTGCCACAATGGGGGTACCTG<br>CTTCAACACGCTGGGTGGCCACAGCTGC | 286 |

The antisense oligomer for human SGCG that induces multi-exon skipping from human SGCG pre-mRNA targets, for example, the range of -400 to +50 bases with the 3' end of intron 3 defined as a basing point, and as a specific example, is an antisense oligomer for human SGCG consisting of

(L1) any one base sequence selected from the group consisting of SEQ ID NOs: 287 to 308, or
(L2) a base sequence that has at least 85% identity with any one base sequence selected from the group consisting of SEQ ID NOs: 287 to 308, and has a length within ±15% of the length of the any one base sequence selected, or a pharmaceutically acceptable salt thereof, or hydrate thereof.

Herein, the base sequence (L2) is a mutant type of the base sequence (L1). Such a mutant type also includes

(L2-1) a base sequence that has at least 85% identity with any one base sequence selected from the group consisting of SEQ ID NOs: 287 to 308, and has a length within ±15% of the length of the any one base sequence selected,
(L2-2) a base sequence that has at least 86% identity with any one base sequence selected from the group consisting of SEQ ID NOs: 287 to 308, and has a length within ±14% of the length of the any one base sequence selected,
(L2-3) a base sequence that has at least 87% identity with any one base sequence selected from the group consisting of SEQ ID NOs: 287 to 308, and has a length within ±13% of the length of the any one base sequence selected,
(L2-4) a base sequence that has at least 88% identity with any one base sequence selected from the group consisting of SEQ ID NOs: 287 to 308, and has a length within ±12% of the length of the any one base sequence selected,
(L2-5) a base sequence that has at least 89% identity with any one base sequence selected from the group consisting of SEQ ID NOs: 287 to 308, and has a length within ±11% of the length of the any one base sequence selected,
(L2-6) a base sequence that has at least 90% identity with any one base sequence selected from the group consisting of SEQ ID NOs: 287 to 308, and has a length within ±10% of the length of the any one base sequence selected,
(L2-7) a base sequence that has at least 91% identity with any one base sequence selected from the group consisting of SEQ ID NOs: 287 to 308, and has a length within ±9% of the length of the any one base sequence selected,
(L2-8) a base sequence that has at least 92% identity with any one base sequence selected from the group consisting of SEQ ID NOs: 287 to 308, and has a length within ±8% of the length of the any one base sequence selected,
(L2-9) a base sequence that has at least 93% identity with any one base sequence selected from the group consisting of SEQ ID NOs: 287 to 308, and has a length within ±7% of the length of the any one base sequence selected,
(L2-10) a base sequence that has at least 94% identity with any one base sequence selected from the group consisting of SEQ ID NOs: 287 to 308, and has a length within ±6% of the length of the any one base sequence selected,
(L2-11) a base sequence that has at least 95% identity with any one base sequence selected from the group consisting of SEQ ID NOs: 287 to 308, and has a length within ±5% of the length of the any one base sequence selected,
(L2-12) a base sequence that has at least 96% identity with any one base sequence selected from the group consisting of SEQ ID NOs: 287 to 308, and has a length within ±4% of the length of the any one base sequence selected,
(L2-13) a base sequence that has at least 97% identity with any one base sequence selected from the group consisting of SEQ ID NOs: 287 to 308, and has a length within ±3% of the length of the any one base sequence selected,
(L2-14) a base sequence that has at least 98% identity with any one base sequence selected from the group consisting of SEQ ID NOs: 287 to 308, and has a length within ±2% of the length of the any one base sequence selected,
(L2-15) a base sequence that has at least 99% identity with any one base sequence selected from the group consisting of SEQ ID NOs: 287 to 308, and has a length within ±1% of the length of the any one base sequence selected, and
(L2-16) a base sequence that has at least 99.5% identity with any one base sequence selected from the group consisting of SEQ ID NOs: 287 to 308, and has a length within ±0.5% of the length of the any one base sequence selected.

Thus, a further embodiment of the antisense oligomer for human SGCG is an antisense oligomer for human SGCG consisting of any one base sequence selected from the group consisting of (L1) and (L2-1) to (L2-16), or a pharmaceutically acceptable salt thereof, or hydrate thereof. The antisense oligomer for human SGCG is included in the present invention.

The suppressor antisense oligomer that suppresses single skipping from human SGCG pre-mRNA (suppressor antisense oligomer for human SGCG) targets, for example, a splicing silencer sequence of intron 4 or a splice site of single skipping, and as a specific example, is a suppressor antisense oligomer for human SGCG consisting of

(M1) any one base sequence selected from the group consisting of SEQ ID NOs: 331 to 335, or
(M2) a base sequence that has at least 85% identity with any one base sequence selected from the group consisting of SEQ ID NOs: 331 to 335, and has a length within ±15% of the length of the any one base sequence selected, or a pharmaceutically acceptable salt thereof, or hydrate thereof.

Herein, the base sequence (M2) is a mutant type of the base sequence (M1). Such a mutant type also includes

(M2-1) a base sequence that has at least 85% identity with any one base sequence selected from the group consisting of SEQ ID NOs: 331 to 335, and has a length within ±15% of the length of the any one base sequence selected,
(M2-2) a base sequence that has at least 86% identity with any one base sequence selected from the group consisting of SEQ ID NOs: 331 to 335, and has a length within ±14% of the length of the any one base sequence selected,
(M2-3) a base sequence that has at least 87% identity with any one base sequence selected from the group consisting of SEQ ID NOs: 331 to 335, and has a length within ±13% of the length of the any one base sequence selected,
(M2-4) a base sequence that has at least 88% identity with any one base sequence selected from the group consisting of SEQ ID NOs: 331 to 335, and has a length within ±12% of the length of the any one base sequence selected,
(M2-5) a base sequence that has at least 89% identity with any one base sequence selected from the group consisting of SEQ ID NOs: 331 to 335, and has a length within ±11% of the length of the any one base sequence selected,
(M2-6) a base sequence that has at least 90% identity with any one base sequence selected from the group consisting of SEQ ID NOs: 331 to 335, and has a length within ±10% of the length of the any one base sequence selected,
(M2-7) a base sequence that has at least 91% identity with any one base sequence selected from the group consisting of SEQ ID NOs: 331 to 335, and has a length within ±9% of the length of the any one base sequence selected,
(M2-8) a base sequence that has at least 92% identity with any one base sequence selected from the group consisting of SEQ ID NOs: 331 to 335, and has a length within ±8% of the length of the any one base sequence selected,
(M2-9) a base sequence that has at least 93% identity with any one base sequence selected from the group consisting of SEQ ID NOs: 331 to 335, and has a length within ±7% of the length of the any one base sequence selected,
(M2-10) a base sequence that has at least 94% identity with any one base sequence selected from the group consisting of SEQ ID NOs: 331 to 335, and has a length within ±6% of the length of the any one base sequence selected,
(M2-11) a base sequence that has at least 95% identity with any one base sequence selected from the group consisting of SEQ ID NOs: 331 to 335, and has a length within ±5% of the length of the any one base sequence selected,
(M2-12) a base sequence that has at least 96% identity with any one base sequence selected from the group consisting of SEQ ID NOs: 331 to 335, and has a length within ±4% of the length of the any one base sequence selected,
(M2-13) a base sequence that has at least 97% identity with any one base sequence selected from the group consisting of SEQ ID NOs: 331 to 335, and has a length within ±3% of the length of the any one base sequence selected,
(M2-14) a base sequence that has at least 98% identity with any one base sequence selected from the group consisting of SEQ ID NOs: 331 to 335, and has a length within ±2% of the length of the any one base sequence selected,
(M2-15) a base sequence that has at least 99% identity with any one base sequence selected from the group consisting of SEQ ID NOs: 331 to 335, and has a length within ±1% of the length of the any one base sequence selected, and
(M2-16) a base sequence that has at least 99.5% identity with any one base sequence selected from the group consisting of SEQ ID NOs: 331 to 335, and has a length within ±0.5% of the length of the any one base sequence selected.

Thus, a further embodiment of the suppressor antisense oligomer for human SGCG is a suppressor antisense oligomer for human SGCG consisting of any one base sequence selected from the group consisting of (M1) and (M2-1) to (M2-16), or a pharmaceutically acceptable salt thereof, or hydrate thereof. The suppressor antisense oligomer for human SGCG is included in the present invention. The suppressor antisense oligomer for human SGCG is included in the present invention. The suppressor antisense oligomer for human SGCG is capable of enhancing the efficiency of multi-skipping from SGCG pre-mRNA.

The antisense oligomer for human Notch3 that induces multi-exon skipping from human Notch3 pre-mRNA targets, for example, a region in the vicinity of an acceptor of intron 3, i.e., the range of -400 to +50 bases with the 3' end of intron 3 defined as a basing point, and is, for example, an antisense oligomer for human Notch3 consisting of

(N1) any one base sequence selected from the group consisting of SEQ ID NOs: 309 to 330, or

(N2) a base sequence that has at least 85% identity with any one base sequence selected from the group consisting of SEQ ID NOs: 309 to 330, and has a length within ±15% of the length of the any one base sequence selected, or a pharmaceutically acceptable salt thereof, or hydrate thereof.

Herein, the base sequence (N2) is a mutant type of the base sequence (N1). Such a mutant type also includes

(N2-1) a base sequence that has at least 85% identity with any one base sequence selected from the group consisting of SEQ ID NOs: 309 to 330, and has a length within ±15% of the length of the any one base sequence selected,
(N2-2) a base sequence that has at least 86% identity with any one base sequence selected from the group consisting of SEQ ID NOs: 309 to 330, and has a length within ±14% of the length of the any one base sequence selected,
(N2-3) a base sequence that has at least 87% identity with any one base sequence selected from the group consisting of SEQ ID NOs: 309 to 330, and has a length within ±13% of the length of the any one base sequence selected,
(N2-4) a base sequence that has at least 88% identity with any one base sequence selected from the group consisting of SEQ ID NOs: 309 to 330, and has a length within ±12% of the length of the any one base sequence selected,
(N2-5) a base sequence that has at least 89% identity with any one base sequence selected from the group consisting of SEQ ID NOs: 309 to 330, and has a length within ±11% of the length of the any one base sequence selected,
(N2-6) a base sequence that has at least 90% identity with any one base sequence selected from the group consisting of SEQ ID NOs: 309 to 330, and has a length within ±10% of the length of the any one base sequence selected,
(N2-7) a base sequence that has at least 91% identity with any one base sequence selected from the group consisting of SEQ ID NOs: 309 to 330, and has a length within ±9% of the length of the any one base sequence selected,
(N2-8) a base sequence that has at least 92% identity with any one base sequence selected from the group consisting of SEQ ID NOs: 309 to 330, and has a length within ±8% of the length of the any one base sequence selected,
(N2-9) a base sequence that has at least 93% identity with any one base sequence selected from the group consisting of SEQ ID NOs: 309 to 330, and has a length within ±7% of the length of the any one base sequence selected,
(N2-10) a base sequence that has at least 94% identity with any one base sequence selected from the group consisting of SEQ ID NOs: 309 to 330, and has a length within ±6% of the length of the any one base sequence selected,
(N2-11) a base sequence that has at least 95% identity with any one base sequence selected from the group consisting of SEQ ID NOs: 309 to 330, and has a length within ±5% of the length of the any one base sequence selected,
(N2-12) a base sequence that has at least 96% identity with any one base sequence selected from the group consisting of SEQ ID NOs: 309 to 330, and has a length within ±4% of the length of the any one base sequence selected,
(N2-13) a base sequence that has at least 97% identity with any one base sequence selected from the group consisting of SEQ ID NOs: 309 to 330, and has a length within ±3% of the length of the any one base sequence selected,
(N2-14) a base sequence that has at least 98% identity with any one base sequence selected from the group consisting of SEQ ID NOs: 309 to 330, and has a length within ±2% of the length of the any one base sequence selected,
(N2-15) a base sequence that has at least 99% identity with any one base sequence selected from the group consisting of SEQ ID NOs: 309 to 330, and has a length within ±1% of the length of the any one base sequence selected, and
(N2-16) a base sequence that has at least 99.5% identity with any one base sequence selected from the group consisting of SEQ ID NOs: 309 to 330, and has a length within ±0.5% of the length of the any one base sequence selected.

Thus, a further embodiment of the antisense oligomer for human Notch3 is an antisense oligomer for human Notch3 consisting of any one base sequence selected from the group consisting of (N1) and (N2-1) to (N2-16), or a pharmaceutically acceptable salt thereof, or hydrate thereof. The antisense oligomer for human Notch3 is included in the present invention.

The suppressor antisense oligomer that suppresses single skipping from human Notch3 pre-mRNA (suppressor antisense oligomer for human Notch3) targets, for example, a splicing silencer sequence of intron 4 or a splice site of single skipping, and as a specific example, is a suppressor antisense oligomer for human Notch3 consisting of

(O1) any one base sequence selected from the group consisting of SEQ ID NOs: 336 to 340, or
(O2) a base sequence that has at least 85% identity with any one base sequence selected from the group consisting of SEQ ID NOs: 336 to 340, and has a length within ±15% of the length of the any one base sequence selected, or a pharmaceutically acceptable salt thereof, or hydrate thereof.

Herein, the base sequence (O2) is a mutant type of the base sequence (O1). Such a mutant type also includes

(O2-1) a base sequence that has at least 85% identity with any one base sequence selected from the group consisting of SEQ ID NOs: 336 to 340, and has a length within ±15% of the length of the any one base sequence selected,
(O2-2) a base sequence that has at least 86% identity with any one base sequence selected from the group consisting of SEQ ID NOs: 336 to 340, and has a length within ±14% of the length of the any one base sequence selected,
(O2-3) a base sequence that has at least 87% identity with any one base sequence selected from the group consisting of SEQ ID NOs: 336 to 340, and has a length within ±13% of the length of the any one base sequence selected,
(O2-4) a base sequence that has at least 88% identity with any one base sequence selected from the group consisting of SEQ ID NOs: 336 to 340, and has a length within ±12% of the length of the any one base sequence selected,
(O2-5) a base sequence that has at least 89% identity with any one base sequence selected from the group consisting of SEQ ID NOs: 336 to 340, and has a length within ±11% of the length of the any one base sequence selected,
(O2-6) a base sequence that has at least 90% identity with any one base sequence selected from the group consisting of SEQ ID NOs: 336 to 340, and has a length within ±10% of the length of the any one base sequence selected,
(O2-7) a base sequence that has at least 91% identity with any one base sequence selected from the group consisting of SEQ ID NOs: 336 to 340, and has a length within ±9% of the length of the any one base sequence selected,
(O2-8) a base sequence that has at least 92% identity with any one base sequence selected from the group consisting of SEQ ID NOs: 336 to 340, and has a length within ±8% of the length of the any one base sequence selected,
(O2-9) a base sequence that has at least 93% identity with any one base sequence selected from the group consisting of SEQ ID NOs: 336 to 340, and has a length within ±7% of the length of the any one base sequence selected,
(O2-10) a base sequence that has at least 94% identity with any one base sequence selected from the group consisting of SEQ ID NOs: 336 to 340, and has a length within ±6% of the length of the any one base sequence selected,
(O2-11) a base sequence that has at least 95% identity with any one base sequence selected from the group consisting of SEQ ID NOs: 336 to 340, and has a length within ±5% of the length of the any one base sequence selected,
(O2-12) a base sequence that has at least 96% identity with any one base sequence selected from the group consisting of SEQ ID NOs: 336 to 340, and has a length within ±4% of the length of the any one base sequence selected,
(O2-13) a base sequence that has at least 97% identity with any one base sequence selected from the group consisting of SEQ ID NOs: 336 to 340, and has a length within ±3% of the length of the any one base sequence selected,
(O2-14) a base sequence that has at least 98% identity with any one base sequence selected from the group consisting of SEQ ID NOs: 336 to 340, and has a length within ±2% of the length of the any one base sequence selected,
(O2-15) a base sequence that has at least 99% identity with any one base sequence selected from the group consisting of SEQ ID NOs: 336 to 340, and has a length within ±1% of the length of the any one base sequence selected, and
(O2-16) a base sequence that has at least 99.5% identity with any one base sequence selected from the group consisting of SEQ ID NOs: 336 to 340, and has a length within ±0.5% of the length of the any one base sequence selected.

Thus, a further embodiment of the suppressor antisense oligomer for human Notch3 is a suppressor antisense oligomer for human Notch3 consisting of any one base sequence selected from the group consisting of (O1) and (O2-1) to (02-16), or a pharmaceutically acceptable salt thereof, or hydrate thereof. The suppressor antisense oligomer for human Notch3 is included in the present invention. The suppressor antisense oligomer for human Notch3 is capable of enhancing the efficiency of multi-skipping from Notch3 pre-mRNA.
The base sequences of SEQ ID NOs: 287 to 340 will be shown below.

| SEQ ID NO | Target sequence | Base sequence |
|---|---|---|
| 287 | SCGCex4_ (-400) - (-371) | TGCTACTCTCTATTTCCATATTTTTGTAAT |
| 288 | SCGCex4_ (-380) - (-351) | AAATGCCATATACTTTTTCCTGCTACTCTC |
| 289 | SCGCex4_ (-360) - (-331) | AGTGGGGTTCTAGGTAATAGAAATGCCATA |
| 290 | SCGCex4_ (-340) - (-311) | CAAGGAAAGACACCTACAACAGTGGGGTTC |
| 291 | SCGCex4_ (-320) - (-291) | CTATATGGTCACAGCTATGTCAAGGAAAGA |

(continued)

| SEQ ID NO | Target sequence | Base sequence |
|---|---|---|
| 292 | SCGCex4_ (-300) - (-271) | AAATATGCAATTTAACAGAGCTATATGGTC |
| 293 | SCGCex4_ (-280) - (-251) | AAAATATGAATTATTTAGACAAATATGCAA |
| 294 | SCGCex4_ (-260) - (-231) | ACGCAAGATAGGGATAGAAAAAAATATGAA |
| 295 | SCGCex4_ (-240) - (-211) | AATCATAAAATGAGCTCCAAACGCAAGATA |
| 296 | SCGCex4_ (-220) - (-191) | TTGAAAGATAAAAGAATAACAATCATAAAA |
| 297 | SCGCex4_ (-200) - (-171) | TTTAACATTTTGAGTATTTATTGAAAGATA |
| 298 | SCGCex4_ (-180) - (-151) | AAATTCCTGAATCATATTTTTTTAACATTT |
| 299 | SCGCex4_(-160)-(-131) | TTTGTGGCTAAGACAATTAAAAATTCCTGA |
| 300 | SCGCex4_(-140)-(-111) | GATCCTGGAAATCCTATAAATTTGTGGCTA |
| 301 | SCGCex4_(-180)-(-91) | ATTATTTATCCATTGTTACAGATCCTGGAA |
| 302 | SCGCex4_(-100)-(-71) | AAATTTAGGATTTTTATAAAATTATTTATC |
| 303 | SCGCex4_(-80)-(-51) | ATATCTTTATAATTCTGTGTAAATTTAGGA |
| 304 | SCGCex4_(-60)-(-31) | AGGTGCTGTTTAAAATGATTATATCTTTAT |
| 305 | SCGCex4_(-40)-(-11) | ATTTATAAAATTTGCAAAATAGGTGCTGTT |
| 306 | SCGCex4_(-20)-10 | GAGATGAGTCCTAGAAAGAGATTTATAAAA |
| 307 | SCGCex4_1-30 | CTGGGTTGATTGTAGAAGCAGAGATGAGTC |
| 308 | SCGCex4-21-50 | CGCGCATTTACAGTCACATTCTGGGTTGAT |
| 309 | Notch3ex4_(-400)-(-371) | CTTTGGGAGGCTGCGGCGGGCAGATCACTT |
| 310 | Noteh3ex4_(-380)-(-351) | TCACGCCTGTAATCCCAGAACTTTGGGAGG |
| 311 | Notch3ex4_(-360)-(-331) CATTTGCTGGGAGCAGTGGCTCACGCCTGT | |
| 312 | Notch3ex4_(-340)-(-311) | GAAACAGAGCAGCAAACACACATTTGCTGG |
| 313 | Notch3ex4_(-320)-(-291) | ACAGGCAAGAAACACGCAGGGAAACAGAGC |
| 314 | Notch3ex4_(-300)-(-271) | GACACAAAGATACACACAAGACAGGCAAGA |
| 315 | Notch3ex4_(-280)-(-251) | CACAGGGCAGGATGGCCCCAGACACAAAGA |
| 316 | Notch3ex4_(-260)-(-231) | GAGATGGCTTGGTTGGGCAGCACAGGGCAG |
| 317 | Notch3ex4_(-240)-(-211) | CAGGCGGGCACCTGTGGGCAGAGATGGCTT |
| 318 | Notch3ex4_(-220)-(-191) | ACACCGCTCACCCACCCAGCCAGGCGGGCA |
| 319 | Notch3ex4_ (-200) - (-171) | TGACAGGGGTCCTCCAGCTGACACCGCTCA |
| 320 | Notch3ex4_ (-180) - (-151) | GGCCAGCACAGGGGCCTGAGTGACAGGGGT |
| 321 | Notch3ex4_ (-160) - (-131) | TGAACTCTGGCAGACACCACGGCCAGCACA |
| 322 | Notch3ex4_ (-140) - (-111) | CGGGCGGTGCCAGCCACCACTGAACTCTGG |
| 323 | Notch3ex4_(-120)-(-91) | GGGGGCACCGGCATGAGAATCGGGCGGTGC |
| 324 | Notch3ex4_ (-100) - (-71) | CCTCTCACCTCGGAAGCCACGGGGGCACCG |
| 325 | Notch3ex4_ (-80) - (-51) | CCTCCCCTCCAGACTCTTCCCCTCTCACCT |
| 326 | Notch3ex4_ (-60) - (-31) | ACTGACCACACCCCCGACTACCTCCCCTCC |
| 327 | Notch3ex4_ (-40) - (-11) | CAGGACAGGGTGAGTTTAGGACTGACCACA |
| 328 | Notch3ex4_ (-20) - 10 | CAGTCAGGGCCTGGAGGGACCAGGACAGGG |
| 329 | Notch3ex4_1-36 | GGCAGGGATCTGGCAGGGAGCAGTCAGGGC |

(continued)

| SEQ ID NO | Target sequence | Base sequence |
|---|---|---|
| 330 | Notch3ex4_21-50 | GTGGGCACAAGGGCTGCTGAGGCAGGGATC |
| 331 | SGCGex4_216-246 | TGTGGTGCTAGTCTGTGGTCAACACAGCAT |
| 332 | SGCGex4_226-256 | CACCCCACTCTGTGGTGCTAGTCTGTGGTC |
| 333 | SGCGex4_236-266 | CCTCACCCCCACCCCACTCTGTGGTGCTA |
| 334 | SGCGex4_246-276 | CAGGCTGTCCCCTCACCCCCACCCCACTC |
| 335 | SGCGex4_256-286 | CAAAGCACTTCAGGCTGTCCCCTCACCCCC |
| 336 | Notch3ex4_397-425 | AGGGAAGGGGGCAAGGATGGTCACCGCCGG |
| 337 | Notch3ex4_407-436 | CAAACCCTAGCAGGGAAGGGGGCAAGGATG |
| 338 | Notch3ex4_417-446 | TCTGACCCTCAAACCCTAGCAGGGAAGGGG |
| 339 | Notch3ex4_427-456 | ACTTCACAATTCTGACCCTCAAACCCTAGC |
| 340 | Notch3ex4_437-466 | GTCCACGTTCACTTCACAATTCTGACCCTC |

5. Method for enhancing efficiency of multi-exon skipping

The present invention provides a method for enhancing the efficiency of skipping of two or more numerically consecutive exons, which comprises inhibiting a splicing silencer sequence, a splice site sequence, or a branch site sequence of pre-mRNA of interest when the two or more numerically consecutive exons are skipped from the pre-mRNA of interest (hereinafter, referred to as the "enhancement method of the present invention").

In the enhancement method of the present invention, the skipping of two or more numerically consecutive exons in the pre-mRNA of interest can be performed using an antisense oligomer that induces multi-exon skipping, for example, the antisense oligomer of the present invention or the pharmaceutically acceptable salt thereof, or hydrate thereof.

The enhancement method of the present invention can enhance the efficiency of multi-exon skipping by inhibiting a splicing silencer sequence, a splice site sequence, or a branch site sequence in pre-mRNA of interest, and thereby suppressing single skipping. The enhancement method of the present invention comprises inhibiting a function of the splicing silencer sequence, the splice site sequence, or the branch site sequence in the pre-mRNA of interest, and more specifically, comprises suppressing single exon skipping by targeting these sequences using a suppressor antisense oligomer.

In the enhancement method of the present invention, the inhibition of the splicing silencer sequence, the splice site, or the branch site sequence can be performed using a suppressor antisense oligomer such as the suppressor antisense oligomer of the present invention, or a pharmaceutically acceptable salt thereof, or hydrate thereof.

In the enhancement method of the present invention, an oligomer comprising a base sequence complementary to the splicing silencer sequence, the branch site sequence, or the splice site sequence in the pre-mRNA of interest can be used as the suppressor antisense oligomer in the inhibition of the splicing silencer sequence, the branch site sequence, or the splice site sequence.

The splicing silencer sequence, the branch site sequence, and the splice site sequence are as mentioned above. In a certain embodiment of the enhancement method of the present invention, the splicing silencer sequence may be a recognition sequence of heterogeneous nuclear ribonucleoprotein A1 (hnRNP A1). Examples of the recognition sequence of human hnRNP A1 are shown in SEQ ID NOs: 370 to 379.

The term "using" or "used" in relation to the antisense oligomer and the suppressor antisense oligomer in the enhancement method of the present invention means that cells expressing the pre-mRNA of interest are allowed to incorporate the antisense oligomer and the suppressor antisense oligomer so that exon skipping is caused in the pre-mRNA of interest. Examples of the method for allowing the cells to incorporate the antisense oligomer and the suppressor antisense oligomer include introduction methods using cationic carriers such as cationic liposomes, cationic polymers, etc., or carriers using viral envelope. The cationic liposomes are, for example, liposomes composed of 2-O-(2-diethylaminoethyl)carbamoyl-1,3-O-dioleoylglycerol and phospholipids as the essential constituents (hereinafter referred to as "liposome A"), Oligofectamine (registered trademark) (manufactured by Invitrogen Corp.), Lipofectin (registered trademark) (manufactured by Invitrogen Corp.), Lipofectamine (registered trademark) (manufactured by Invitrogen Corp.), Lipofectamine 2000 (registered trademark) (manufactured by Invitrogen Corp.), DMRIE-C (registered trademark) (manufactured by Invitrogen Corp.), GeneSilencer (registered trademark) (manufactured by Gene Therapy Systems), Trans-Messenger (registered trademark) (manufactured by QIAGEN, Inc.), TransIT TKO (registered trademark) (manufactured by Mirus) and Nucleofector II (Lonza). Among others, liposome A is preferred. Examples of cationic polymers are JetSI (registered trademark) (manufactured by Qbiogene, Inc.) and Jet-PEI (registered trademark) (polyethylenimine, manufactured by Qbiogene, Inc.). An example of carriers using viral envelop is GenomeOne (registered trademark) (HVJ-E liposome, manufactured by Ishihara Sangyo). Alternatively, the medical devices described in Japanese Patent Nos. 2924179 and the cationic carriers described in Japanese Domestic RePublication PCT Nos. 2006/129594 and 2008/096690 may be used as well.

For the method for allowing cells expressing pre-mRNA of interest in the body of a patient to incorporate the antisense oligomer and the suppressor antisense oligomer, the administration method described in the section "3. Medical application" can be referred.

In the enhancement method of the present invention, the inhibition of the splicing silencer sequence, the splice site sequence, or the branch site sequence in the pre-mRNA of interest enhances the efficiency of multi-exon skipping by 2% or higher, 4% or higher, 6% or higher, 8% or higher, 10% or higher, 12% or higher, 14% or higher, 16% or higher, 18% or higher, 20% or higher, 22% or higher, 24% or higher, 26% or higher, 28% or higher, 30% or higher, 32% or higher, 34% or higher, 36% or higher, 38% or higher, 40% or higher, 42% or higher, 44% or higher, 46% or higher, 48% or higher, 50% or higher, 52% or higher, 54% or higher, 56% or higher, 58% or higher, 60% or higher, 62% or higher, 64% or higher, 66% or higher, 68% or higher, 70% or higher, 72% or higher, 74% or higher, 76% or higher, 78% or higher, 80% or higher, 82% or higher, 84% or higher, 86% or higher, 88% or higher, 90% or higher, 92% or higher, 94% or higher, 96% or higher, 98% or higher, or 100% or higher when the efficiency obtained without the inhibition is defined as 100.

In the enhancement method of the present invention, examples of the pre-mRNA of interest include human γ-sarcoglycan (SGCG) pre-mRNA, human Notch3 pre-mRNA, and human dystrophin pre-mRNA. In a certain embodiment of the present invention, the pre-mRNA of interest is human dystrophin pre-mRNA. When the pre-mRNA of interest is human dystrophin pre-mRNA, the two or more numerically consecutive exons are exons selected from the group consisting of the 45th exon to the 55th exon in the human dystrophin pre-mRNA.

## Examples

Hereinafter, the present invention will be described in more detail with reference to Examples and Test Examples below, but is not deemed to be limited thereto.

### [Example 1: Production of antisense oligomer]

<u>Production of 4-{[(2S,6R)-6-(4-benzamido-2-oxopyrimidin-1-yl)-4-tritylmorpholin-2-yl]methoxy)-4-oxobutanoic acid supported on amino polystyrene resin (Compound 1)</u>

Step 1: Production of 4-{[(2S,6R)-6-(4-benzamido-2-oxopyrimidin-1(2H)-yl)-4-tritylmorpholin-2-yl]methoxy}-4-oxobutanoic acid

Under argon atmosphere, 3.44 g of N-{1-[(2R,6S)-6-(hydroxymethyl)-4-tritylmorpholin-2-yl]-2-oxo-1,2-dihydropyrimidin-4-yl}benzamide and 1.1 g of 4-dimethylaminopyridine (4-DMAP) were suspended in 50 mL of dichloromethane, and 0.90 g of succinic anhydride was added to the suspension, followed by stirring at room temperature for 3 hours. To the reaction mixture, 10 mL of methanol was added, and the mixture was concentrated under reduced pressure. The residue was extracted using ethyl acetate and 0.5 M aqueous potassium dihydrogenphosphate solution. The resulting organic layer was washed sequentially with 0.5 M aqueous potassium dihydrogenphosphate solution, water and brine in the order mentioned. The resulting organic layer was dried over sodium sulfate and concentrated under reduced pressure to give 4.0 g of the product.

Step 2; Production of 4-{[(2S,6R)-6-(4-benzamido-2-oxopyrimidin-1-yl)-4-tritylmorpholin-2-yl]methoxy}-4-oxobutanoic acid supported on amino polystyrene resin

After 4.0 g of 4-{[(2S,6R)-6-(4-benzamido-2-oxopyrimidin-1(2H)-yl)-4-tritylmorpholin-2-yl]methoxy}-4-oxobutanoic acid was dissolved in 200 mL of pyridine (dehydrated), 0.73 g of 4-DMAP and 11.5 g of 1-ethyl-3-(3-dimethylaminopropyl)carbodiimide hydrochloride were added to the solution. Then, 25.0 g of amino polystyrene resin Primer support 200 amino (manufactured by GE Healthcare Japan Co., Ltd., 17-5214-97) and 8.5 mL of triethylamine were added to the mixture, followed by shaking at room temperature for 4 days. After completion of the reaction, the resin was taken out by filtration. The resulting resin was washed sequentially with pyridine, methanol and dichloromethane in the order

mentioned, and dried under reduced pressure. To the resulting resin, 200 mL of tetrahydrofuran (dehydrate), 15 mL of acetic anhydride and 15 mL of 2,6-lutidine were added, and the mixture was shaken at room temperature for 2 hours. The resin was taken out by filtration, washed sequentially with pyridine, methanol and dichloromethane in the order mentioned and dried under reduced pressure to give 26.7 g of the product of interest.

The loading amount of the product was determined from the molar amount of the trityl per g resin by measuring UV absorbance at 409 nm using a known method. The loading amount of the resin was 129.2 μmol/g.

Conditions of UV measurement

Apparatus: U-2910 (Hitachi, Ltd.)
Solvent: methanesulfonic acid
Wavelength: 409 nm
ε value: 45000

Production of 4-{[(2S,6R)-6-(5-methyl-2,4-dioxopyrimidin-1-yl)-4-tritylmorpholin-2-yl]methoxy}-4-oxobutanoic acid supported on amino polystyrene resin (Compound 2)

The title compound was produced in the same manner as in Compound 1 except that 1-[(2R,6S)-6-(hydroxymethyl)-4-tritylmorpholin-2-yl]-5-methylpyrimidine-2,4(1H,3H)-dione was used in this step instead of N-{1-[(2R,6S)-6-(hydroxymethyl)-4-tritylmorpholin-2-yl]-2-oxo-1,2-dihydropyrimidin-4-yl}benzamide used in Step 1 in the production of Compound 1.

The loading amount of the product was determined from the molar amount of the trityl per g resin by measuring UV absorbance at 409 nm using a known method. The loading amount of the resin was 164.0 μmol/g.

Production of 4-{[(2S,6R)-6-(6-benzamidopurin-9-yl)-4-tritylmorpholin-2-yl]methoxy}-4-oxobutanoic acid supported on amino polystyrene resin (Compound 3)

The title compound was produced in the same manner as in Compound 1 except that N-{9-[(2R,6S)-6-(hydroxymethyl)-4-tritylmorpholin-2-yl]purin-6-yl}benzamide was used in this step instead of N-{1-[(2R,6S)-6-(hydroxymethyl)-4-tritylmorpholin-2-yl]-2-oxo-1,2-dihydropyrimidin-4-yl}benzamide used in Step 1 in the production of Compound 1.

The loading amount of the product was determined from the molar amount of the trityl per g resin by measuring UV absorbance at 409 nm using a known method. The loading amount of the resin was 185.7 μmol/g.

Production of 4-{{(2S,6R)-6-{6-(2-cyanoethoxy)-2-[(2-phenoxyacetyl)amino]purin-9-yl}-4-tritylmorpholin-2-yl}methoxy}-4-oxobutanoic acid supported on amino polystyrene resin (Compound 4)

The title compound was produced in the same manner as in Compound 1 except that N-{6-(2-cyanoethoxy)-9-[(2R,6S)-6-(hydroxymethyl)-4-tritylmorpholin-2-yl]purin-2-yl}-2-phenoxyacetamide was used in this step instead of N-{1-[(2R,6S)-6-(hydroxymethyl)-4-tritylmorpholin-2-yl]-2-oxo-1,2-dihydropyrimidin-4-yl}benzamide used in Step 1 in the production of Compound 1.

The loading amount of the product was determined from the molar amount of the trityl per g resin by measuring UV absorbance at 409 nm using a known method. The loading amount of the resin was 164.8 μmol/g.

Production of antisense PMO targeting region in vicinity of donor or acceptor of intron in human dystrophin gene

According to the description given below, PMOs having the base sequences of PMO Nos. 1 to 232 (SEQ ID NOs: 1 to 232) shown in Table 11 which targeted regions in the vicinity of donors or acceptors of introns 44 to 55 in human dystrophin pre-mRNA shown in Table 10 were synthesized. The 5' end of each PMO is Group (3) below. The synthesized PMO was dissolved in water for injection (manufactured by Otsuka Pharmaceutical Factory, Inc.).

[Formula 14]

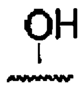

(3)

The target base sequence of the antisense oligomer of the present invention or the suppressor antisense oligomer of the present invention was described as "Ha$_1$_b$_1$-c$_1$", "Ha$_2$_b$_2$-c$_2$_Ha$_3$_b$_3$-c$_3$", or "Ha$_4$_b$_4$-c$_4$_Ha$_5$_b$_5$-C$_5$_Ha$_6$_b$_6$-c$_6$" .

"Ha$_1$_b$_1$-c$_1$" is the target base sequence of the antisense oligomer of the first embodiment used herein. "Ha$_1$" represents the ath exon of the human dystrophin gene, "b$_1$" represents the 5'-terminal base of the target base sequence, and "c$_1$" represents the 3'-terminal base of the target base sequence. "Ha$_1$_b$_1$-c$_1$" may have one additional base at its 3' end, and such a target base sequence is represented by "Ha$_1$_b$_1$-c$_1$_N" ("N" represents a given base).

When "b$_1$" and "c$_1$" are positive integers, "b$_1$" and "c$_1$" each represent a base number in the downstream direction when the 5'-terminal base of the ath exon is counted as the 1st base. On the other hand, when "b$_1$" and "c$_1$" are negative numbers, "b$_1$" and "c$_1$" each represent a base number in the upstream direction when the 3'-terminal base of the (a - 1)th intron is counted as the 1st base.

For example, "H45_(-10)-15" means a base sequence in which the 5' end of the target base sequence is the 10th base in the upstream direction from the 3' end of the 44th intron and the 3' end of the target base sequence is the 15th base in the downstream direction from the 5' end of the 45th exon.

"Ha$_2$_b$_2$-c$_2$_Ha$_3$_b$_3$-c$_3$" is the target base sequence of the antisense oligomer of the second embodiment used herein. "Ha$_2$_b$_2$-c$_2$" which is the first part of "Ha$_2$_b$_2$-c$_2$_Ha$_3$_b$_3$-c$_3$" means the target base sequence of a 3' unit oligomer constituting the antisense oligomer, and the second part "Ha$_3$_b$_3$-c$_3$" means the target base sequence of a 5' unit oligomer constituting the antisense oligomer. "Ha$_3$_b$_3$-c$_3$" may have one additional base at its 3' end, and such a target base sequence is represented by "Ha$_2$_b$_2$-c$_2$-Ha$_3$_b$_3$-c$_3$_N" ("N" represents a given base). Each of "Ha$_2$_b$_2$-c$_2$" and "Ha$_3$_b$_3$_-c$_3$" abides by the same notation as that of "Ha$_1$_b$_1$-c$_1$".

When "Ha$_2$" and "Ha$_3$" are the same, the "_Ha$_3$" part may be omitted.

For example, "H45_(-5)-5_25-35" or "H45_(-5)-5_H45_25-35" means a base sequence in which the target base sequence of the 3' unit oligomer constituting the antisense oligomer is "H45_(-5)-5" and the target base sequence of the 5' unit oligomer constituting the antisense oligomer is "H45_25-35".

"Ha$_4$_b$_4$-c$_4$_Ha$_5$_b$_5$-c$_5$_Ha$_6$_b$_6$-c$_6$" is the target base sequence of the antisense oligomer of the third embodiment used herein. "Ha$_4$_b$_4$-c$_4$" which is the first part of "Ha$_4$_b$_4$-c$_4$_Ha$_5$_b$_5$-c$_5$_Ha$_6$_b$_6$-c$_6$" means the target base sequence of a 3' unit oligomer constituting the antisense oligomer, the second part "Ha$_5$_b$_5$-c$_5$" means the target base sequence of an intermediate unit oligomer constituting the antisense oligomer, and the third part "Ha$_6$_b$_6$-c$_6$" means the target base sequence of a 5' unit oligomer constituting the antisense oligomer. "Ha$_6$_b$_6$-c$_6$" may have one additional base at its 3' end, and such a target base sequence is represented by "Ha$_4$_b$_4$-c$_4$_Ha$_5$_b$_5$-c$_5$-Ha$_6$_b$_6$-c$_6$_N" ("N" represents a given base) . Each of "Ha$_4$_b$_4$-c$_4$", "Ha$_5$_b$_5$_-c$_5$", and "Ha$_6$_b$_6$_-c$_6$" abides by the same notation as that of "Ha$_1$_b$_1$-c$_1$".

When "Ha$_4$" is the same with "Ha$_5$" and "Ha$_6$", the "_Ha$_5$" and "_Ha$_6$" parts may be omitted.

For example, "H45_(-5)-5_25-35_60-70" or "H45_(-5)-5_H45_25-35_H45_60-70" means a base sequence in which the target base sequence of the 3' unit oligomer constituting the antisense oligomer is "H45_(-5)-5", the target base sequence of the intermediate unit oligomer is "H45_25-35", and the target base sequence of the 5' unit oligomer is "H45_60-70".

[Table 10]

Table 10 Target region in human dystrophin pre-mRNA

| Target region | Range of target region | SEQ ID NO |
|---|---|---|
| (R1) Region in vicinity of donor of intron 44 | Region indicated by range of -20 bases to +400 bases with 5' end of intron 44 defined as basing point 0 | 233 |

(continued)

| Target region | Range of target region | SEQ ID NO |
|---|---|---|
| (R2) Region in vicinity of acceptor of intron 44 | Region indicated by range of -600 bases to +50 bases with 3' end of intron 44 defined as basing point 0 | 234 |
| (R3) Region in vicinity of donor of intron 45 | Region indicated by range of -20 bases to +400 bases with 5' end of intron 45 defined as basing point 0 | 235 |
| (R4) Region in vicinity of acceptor of intron 45 | Region indicated by range of -400 bases to +50 bases with 3' end of intron 45 defined as basing point 0 | 236 |
| (R5) Region in vicinity of donor of intron 46 | Region indicated by range of -20 bases to +400 bases with 5' end of intron 46 defined as basing point 0 | 237 |
| (R6) Region in vicinity of acceptor of intron 46 | Region indicated by range of -400 bases to +50 bases with 3' end of intron 46 defined as basing point 0 | 238 |
| (R7) Region in vicinity of donor of intron 47 | Region indicated by range of -20 bases to +400 bases with 5' end of intron 47 defined as basing point 0 | 239 |
| (R8) Region in vicinity of acceptor of intron 47 | Region indicated by range of -400 bases to +50 bases with 3' end of intron 47 defined as basing point 0 | 240 |
| (R9) Region in vicinity of donor of intron 48 | Region indicated by range of -20 bases to +400 bases with 5' end of intron 48 defined as basing point 0 | 241 |
| (R10) Region in vicinity of acceptor of intron 48 | Region indicated by range of -400 bases to +50 bases with 3' end of intron 48 defined as basing point 0 | 242 |
| (R11) Region in vicinity of donor of intron 49 | Region indicated by range of -20 bases to +400 bases with 5' end of intron 49 defined as basing point 0 | 243 |
| (R12) Region in vicinity of acceptor of intron 49 | Region indicated by range of -400 bases to +50 bases with 3' end of intron 49 defined as basing point 0 | 244 |
| (R13) Region in vicinity of donor of intron 50 | Region indicated by range of -20 bases to +400 bases with 5' end of intron 50 defined as basing point 0 | 245 |
| (R14) Region in vicinity of acceptor of intron 50 | Region indicated by range of -400 bases to +50 bases with 3' end of intron 50 defined as basing point 0 | 246 |
| (R15) Region in vicinity of donor of intron 51 | Region indicated by range of -20 bases to +400 bases with 5' end of intron 51 defined as basing point 0 | 247 |
| (R16) Region in vicinity of acceptor of intron 51 | Region indicated by range of -400 bases to +50 bases with 3' end of intron 51 defined as basing point 0 | 248 |
| (R17) Region in vicinity of donor of intron 52 | Region indicated by range of -20 bases to +400 bases with 5' end of intron 52 defined as basing point 0 | 249 |
| (R18) Region in vicinity of acceptor of intron 52 | Region indicated by range of -400 bases to +50 bases with 3' end of intron 52 defined as basing point 0 | 250 |
| (R19) Region in vicinity of donor of intron 53 | Region indicated by range of -20 bases to +400 bases with 5' end of intron 53 defined as basing point 0 | 251 |
| (R20) Region in vicinity of acceptor of intron 53 | Region indicated by range of -400 bases to +50 bases with 3' end of intron 53 defined as basing point 0 | 252 |
| (R21) Region in vicinity of donor of intron 54 | Region indicated by range of -20 bases to +400 bases with 5' end of intron 54 defined as basing point 0 | 253 |
| (R22) Region in vicinity of acceptor of intron 54 | Region indicated by range of -400 bases to +50 bases with 3' end of intron 54 defined as basing point 0 | 254 |
| (R23) Region in vicinity of donor of intron 55 | Region indicated by range of -20 bases to +400 bases with 5' end of intron 55 defined as basing point 0 | 255 |

(continued)

| Target region | Range of target region | SEQ ID NO |
|---|---|---|
| (R24) Region in vicinity of acceptor of intron 55 | Region indicated by range of -400 bases to +50 bases with 3' end of intron 55 defined as basing point 0 | 256 |

[Table 11]

Table 11

| PMO No. | Target region | Target base sequence | Base sequence of PMO | SEQ ID NO |
|---|---|---|---|---|
| 1 | R1 | H44_150-175 | TTTCGAAAAAACAAATCAAAGACTTA | 1 |
| 2 | | H44_157-196 | ATGTGCTGAAGATAAATACAATTTCGAAAAAACAAATCAA | 2 |
| 3 | | H44_159-193 | TGCTGAAGATAAATACAATTTCGAAAAAACAAATC | 3 |
| 4 | | H44_170-199 | CAGATGTGCTGAAGATAAATACAATTTCGA | 4 |
| 5 | | H44_179-206 | AAGAGTCCAGATGTGCTGAAGATAAATA | 5 |
| 6 | | H44_209-238 | ACCCTTCAGAACCTGATCTTTAAGAAGT | 6 |
| 7 | | H44_239-266 | TGACAACAACAGTCAAAAGTAATTTCCA | 7 |
| 8 | | H44_269-296 | ATGATAATTTTCTTTCTAGTAATATAAT | 8 |
| 9 | | H44_299-326 | TCCATAGCACCGTGCTCTAATATTATCA | 9 |
| 10 | | H44_330-355 | GGCAAACTCTCTCATCCTGACACAAA | 10 |
| 11 | | H44_354-381 | TTTATCAGATAAACCAGCTCCGTCCAGG | 11 |
| 12 | | H44_389-416 | CTTCCCTCTGTCACAGATTCAATTATAT | 12 |
| 13 | | H44_413-438 | AAAACACCTTGCTGTTACGATGCTTC | 13 |
| 14 | | H44_423-452 | TGCCCCAAAGCCACAAAACACCTTGCTGTT | 14 |
| 15 | | H44_455-480 | GGTTCCAACATAAAGCCGAAATACAC | 15 |
| 16 | | H44_479-506 | TATGCCACAAGTTCTCCTTCTGGAAAGG | 16 |
| 17 | | H44_509-536 | GATATTTCTAGCAACTTCATTTTAGCTA | 17 |
| 18 | | H44_150-164_179-193 | TGCTGAAGATAAATACAAATCAAAGACTTA | 18 |
| 19 | | H44_150-164_192-206 | AAGAGTCCAGATGTGCAAATCAAAGACTTA | 19 |
| 20 | | H44_161-175_179-193 | TGCTGAAGATAAATATTTCGAAAAAACAAA | 20 |
| 21 | | H44_161-175_192-206 | AAGAGTCCAGATGTGTTTCGAAAAAACAAA | 21 |

(continued)

| PMO No. | Target region | Target base sequence | Base sequence of PMO | SEQ ID NO |
|---|---|---|---|---|
| 22 | | H45_ (-598) - (-571) | TTAATTGCTTTCAGGAGCATCCCATCAA | 22 |
| 23 | | H45_ (-568) - (-541) | TTTGCATTAGAAGCCACAAAAAACTGAG | 23 |
| 24 | | H45_ (-538) - (-511) | TCATTTCAAATTCTGTCTGCGTCAATGT | 24 |
| 25 | | H45_ (-518) - (-491) | TTGCTATATTAGAAGAAAATTCATTTCA | 25 |
| 26 | | H45_ (-515) - (-488) | TAATTGCTATATTAGAAGAAAATTCATT | 26 |
| 27 | | H45_ (-508) - (-479) | AAATAAAATTAATTGCTATATTAGAAGAAA | 27 |
| 28 | | H45_ (-478) - (-449) | CAGTATTAAAAAAAAACTCTAGAGATATTT | 28 |
| 29 | | H45_ (-470) - (-443) | TAGTCACAGTATTAAAAAAAAACTCTAG | 29 |
| 30 | | H45_ (-448) - (-421) | GTGAAAAAGAACAAACATAGGTTAGTCA | 30 |
| 31 | | H45_ (-439) - (-412) | ATACGAGAGGTGAAAAAGAACAAACATA | 31 |
| 32 | | H45_ (-436) - (-409) | TGGATACGAGAGGTGAAAAAGAACAAAC | 32 |
| 33 | R2 | H45_ (-422) - (-395) | TTTCTTAGTGATCGTGGATACGAGAGGT | 33 |
| 34 | | H45_ (-418) - (-394) | GTTTCTTAGTGATCGTGGATACGAG | 34 |
| 35 | | H45_ (-418) - (-391) | TGGGTTTCTTAGTGATCGTGGATACGAG | 35 |
| 36 | | H45_ (-415) - (-391) | TGGGTTTCTTAGTGATCGTGGATAC | 36 |
| 37 | | H45_ (-413) - (-386) | GTATTTGGGTTTCTTAGTGATCGTGGAT | 37 |
| 38 | | H45_ (-405) - (-378) | TGAACAAAGTATTTGGGTTTCTTAGTGA | 38 |
| 39 | | H45_ (-389) - (-362) | TTGTAAAATTTAAACATGAACAAAGTAT | 39 |
| 40 | | H45_ (-349) - (-322) | TCCCCACAAGGATGTTCCATGTTTAATA | 40 |
| 41 | | H45_(-320)-(-293) | ACCTTTTCAAGAGCAAATTCGATTTCTT | 41 |
| 42 | | H45_ (-305) - (-280) | CAATTAGTTGGAAACCTTTTCAAGAG | 42 |
| 43 | | H45_ (-290) - (-263) | TATAATGTCCTACAAATCAATTAGTTGG | 43 |
| 44 | | H45_ (-275) - (-250) | AGCTAGAGGATGTTATAATGTCCTAC | 44 |

(continued)

| PMO No. | Target region | Target base sequence | Base sequence of PMO | SEQ ID NO |
|---|---|---|---|---|
| 45 | | H45_ (-260) - (-233) | ATTTTTGTAAGCTTGTCAGCTAGAGGAT | 45 |
| 46 | | H45_ (-230) - (-203) | AAAGCACCCTCTCGGTTAGCTCCAGTTT | 46 |
| 47 | | H45_ (-200) - (-173) | CAGAAAGACACCTTTTATGTGTCAGGGA | 47 |
| 48 | | H45_ (-190) - (-163) | GGATACAAGACAGAAAGACACCTTTTAT | 48 |
| 49 | | H45_ (-185) - (-160) | AAAGGATACAAGACAGAAAGACACCT | 49 |
| 50 | | H45_ (-171) - (-144) | TGACATGCCCATATCCAAAGGATACAAG | 50 |
| 51 | | H45_ (-168) - (-141) | AACTGACATGCCCATATCCAAAGGATAC | 51 |
| 52 | | H45_ (-142) - (-115) | AGCTCGATGTGAAAATTTCCCTATCAAA | 52 |
| 53 | | H45_ (-110) - (-83) | TGCAGTTGTACTGGCAAAGAAAGAAATA | 53 |
| 54 | | H45_ (-80) - (-53) | TGAGAAAAGATTAAACAGTGTGCTACCA | 54 |
| 55 | | H45_ (-78) - (-51) | TTTGAGAAAAGATTAAACAGTGTGCTAC | 55 |
| 56 | | H45_ (-70) - (-43) | TCTTTTTATTTGAGAAAAGATTAAACAG | 56 |
| 57 | | H45_ (-60) - (-33) | AAGCCCCATGTCTTTTTATTTGAGAAAA | 57 |
| 58 | | H45_ (-50) - (-23) | AACAAAAATGAAGCCCCATGTCTTTTTA | 58 |
| 59 | | H45_ (-44) - (-17) | AGGCAAAACAAAAATGAAGCCCCATGTC | 59 |
| 60 | | H45_ (-25) - 3 | TTCCTGTAAGATACCAAAAAGGCAAAAC | 60 |
| 61 | | H45_ (-23) - 5 | AGTTCCTGTAAGATACCAAAAAGGCAAA | 61 |
| 62 | | H45_ (-22) - 3 | TTCCTGTAAGATACCAAAAAGGCAA | 62 |
| 63 | | H45_ (-21) - 7 | GGAGTTCCTGTAAGATACCAAAAAGGCA | 63 |
| 64 | | H45_ (-20) - 5 | AGTTCCTGTAAGATACCAAAAAGGC | 64 |
| 65 | | H45_ (-20) - 8 | TGGAGTTCCTGTAAGATACCAAAAAGGC | 65 |
| 66 | | H45_ (-18) - 10 | CCTGGAGTTCCTGTAAGATACCAAAAAG | 66 |
| 67 | | H45_ (-17) - 8 | TGGAGTTCCTGTAAGATACCAAAAA | 67 |
| 68 | | H45_ (-16) - 12 | ATCCTGGAGTTCCTGTAAGATACCAAAA | 68 |
| 69 | | H45_ (-15) - 10 | CCTGGAGTTCCTGTAAGATACCAAA | 69 |
| 70 | | H45_ (-14) - 14 | CCATCCTGGAGTTCCTGTAAGATACCAA | 70 |
| 71 | | H45_ (-10) - 16 | TGCCATCCTGGAGTTCCTGTAAGATA | 71 |
| 72 | | H45_ (-5) - 20 | CCAATGCCATCCTGGAGTTCCTGTA | 72 |
| 73 | | H45_1-25 | GCTGCCCAATGCCATCCTGGAGTTC | 73 |
| 74 | | H45_6-30 | TTGCCGCTGCCCAATGCCATCCTGG | 74 |
| 75 | | H45_11-35 | ACAGTTTGCCGCTGCCCAATGCCAT | 75 |
| 76 | | H45_11-40 | TGACAACAGTTTGCCGCTGCCCAATGCCAT | 76 |
| 77 | | H45_16-35 | ACAGTTTGCCGCTGCCCAAT | 77 |
| 78 | | H45_16-40 | TGACAACAGTTTGCCGCTGCCCAAT | 78 |
| 79 | | H45_21-45 | TGTTCTGACAACAGTTTGCCGCTGC | 79 |
| 80 | | H45_ (-423) - (-412) _16-33 | AGTTTGCCGCTGCCCAATATACGAGAGGTG | 80 |
| 81 | | H45_ (-416) - (-402) _16- | TTGCCGCTGCCCAATGTGATCGTGGATACG | 81 |
| 82 | | H45_ (-411) - (-400) _16- | AGTTTGCCGCTGCCCAATTAGTGATCGTGG | 82 |
| 83 | | H45_ (-405) - (-391) _16- | TTGCCGCTGCCCAATTGGGTTTCTTAGTGA | 83 |
| 84 | | H45_ (-402) - (-391) _16-33 | AGTTTGCCGCTGCCCAATTGGGTTTCTTAG | 84 |
| 85 | | H45_ (-156) - (-143) - (-141) - (-128) | AATTTCCCTATGAACTGACATGCCCATA | 85 |
| 86 | | H45_ (-78) - (-65) _ (-44) - (-31) | TGAAGCCCCATGTCAAACAGTGTGCTAC | 86 |
| 87 | | H45_ (-78) - (-65) _ (-34) - (-21) | AAAACAAAAATGAAGAACAGTGTGCTAC | 87 |

(continued)

| PMO No. | Target region | Target base sequence | Base sequence of PMO | SEQ ID NO |
|---|---|---|---|---|
| 88 | | H45_ (-78) - (-65) _16-29 | TGCCGCTGCCCAATAAACAGTGTGCTAC | 88 |
| 89 | | H45_ (-78) - (-65) _21-34 | CAGTTTGCCGCTGCAAACAGTGTGCTAC | 89 |
| 90 | | H45_ (-78) - (-65) _27-40 | TGACAACAGTTTGCAAACAGTGTGCTAC | 90 |
| 91 | | H45_ (-74) - (-62) _ (-40) - (-28) | AAATGAAGCCCCAATTAAACAGTGTG | 91 |
| 92 | | H45_ (-72) - (-58) _ (-38) - (-26) | AAAAATGAAGCCCAAAGATTAAACAGTG | 92 |
| 93 | | H45_ (-71) - (-58) _ (-38) - (-25) | CAAAAATGAAGCCCAAAGATTAAACAGT | 93 |
| 94 | | H45_ (-71) - (-58) _16-29 | TGCCGCTGCCCAATAAAGATTAAACAGT | 94 |
| 95 | | H45_ (-71) - (-58) _21-34 | CAGTTTGCCGCTGCAAAGATTAAACAGT | 95 |
| 96 | | H45_ (-71) - (-58) _27-40 | TGACAACAGTTTGCAAAGATTAAACAGT | 96 |
| 97 | | H45_ (-68) - (-60) _ (-36) - (-28) _19-28 | GCCGCTGCCC_AAATGAAGCAGATTAAAC | 97 |
| 98 | | H45_ (-64) - (-51) _ (-44) - (-31) | TGAAGCCCCATGTCTTTGAGAAAAGATT | 98 |
| 99 | | H45_ (-64) - (-51) _ (-35) - (-22) | AAACAAAAATGAAGTTTGAGAAAAGATT | 99 |
| 100 | | H45_ (-64) - (-51) _16-29 | TGCCGCTGCCCAATTTTGAGAAAAGATT | 100 |
| 101 | | H45_ (-64) - (-51) _21-34 | CAGTTTGCCGCTGCTTTGAGAAAAGATT | 101 |
| 102 | | H45_ (-64) - (-51) _27-40 | TGACAACAGTTTGCTTTGAGAAAAGATT | 102 |
| 103 | | H45_ (-44) - (-31) _16-29 | TGCCGCTGCCCAATTGAAGCCCCATGTC | 103 |
| 104 | | H45_ (-44) - (-31) _27-40 | TGACAACAGTTTGCTGAAGCCCCATGTC | 104 |
| 105 | | H45_ (-44) - (-31) _21-33_T | TAGTTTGCCGCTGCTGAAGCCCCATGTC | 105 |
| 106 | | H45_ (-38) - (-25) _16-29 | TGCCGCTGCCCAATCAAAAATGAAGCCC | 106 |
| 107 | | H45_ (-38) - (-25) _21-34 | CAGTTTGCCGCTGCCAAAAATGAAGCCC | 107 |
| 108 | | H45_ (-38) - (-25) _27-40 | TGACAACAGTTTGCCAAAAATGAAGCCC | 108 |
| 109 | | H45_ (-34) - (-21) _16-29 | TGCCGCTGCCCAATAAAACAAAAATGAA | 109 |
| 110 | | H45_ (-34) - (-21) _21-34 | CAGTTTGCCGCTGCAAAACAAAAATGAA | 110 |
| 111 | | H45_ (-34) - (-21) _27-40 | TGACAACAGTTTGCAAAACAAAAATGAA | 111 |
| 112 | | H45_1-21 | CCCAATGCCATCCTGGAGTTC | 112 |
| 144 | R3 | H45_167-194 | TTAGATCTGTCGCCCTACCTCTTTTTTC | 144 |
| 145 | | H45_227-254 | TCTCATGAAATATTCTTCTAAAGAAAGC | 145 |
| 146 | | H45_257-284 | ATGTTAGTGCCTTTCACCCTGCTTATAA | 146 |
| 147 | | H45_287-314 | GCTGTTGATTAATGGTTGATAGGTTCTT | 147 |
| 148 | | H45_317-344 | TGAAAAAAAGAAATAAAAAATTTCTTTA | 148 |
| 149 | | H45_347-374 | TAACTAGCCACAAGTATATATTTTAGTA | 149 |

(continued)

| PMO No. | Target region | Target base sequence | Base sequence of PMO | SEQ ID NO |
|---|---|---|---|---|
| 150 | R4 | H46_ (-200) - (-173) | TCAAGAATCTCTAAATGATAAGAGATTA | 150 |
| 151 | | H46_ (-171) - (-144) | TTCACTTTGAACAAAGTAATTTCAATAT | 151 |
| 152 | | H46_ (-146) - (-119) | ACCATACATAATTTAAGAAAATTCATTC | 152 |
| 153 | | H46_(-141)-(-114) | TGTTAACCATACATAATTTAAGAAAATT | 153 |
| 154 | | H46_ (-136) - (-109) | AAAGATGTTAACCATACATAATTTAAGA | 154 |
| 155 | | H46_ (-126) - (-99) | AAGCAATTTAAAAGATGTTAACCATACA | 155 |
| 156 | | H46_ (-116) - (-89) | ATTTAAAAATAAGCAATTTAAAAGATGT | 156 |
| 157 | | H46_ (-111) - (-84) | TGGCAATTTAAAAATAAGCAATTTAAAA | 157 |
| 158 | | H46_ (-166) - (-79) | AAACATGGCAATTTAAAAATAAGCAATT | 158 |
| 159 | | H46_ (-81) - (-54) | TATTTGTTAATGCAAACTGGGACACAAA | 159 |
| 160 | | H46_ (-51) - (-24) | TTATTTTTTTTTCCAACATAGTTCTCAA | 160 |
| 161 | | H46_ (-18) -6 | TCTAGCCTGGAGAAAGAAGAATAA | 161 |
| 162 | | H46_ (-15) -9 | TCTTCTAGCCTGGAGAAAGAAGAA | 162 |
| 163 | | H46_ (-9) -15 | TTTTGTTCTTCTAGCCTGGAGAAA | 163 |
| 164 | R5 | H46_139-166 | TTGAGAAAATAAAATTACCTTGACTTGC | 164 |
| 165 | | H46_169-196 | ACTTCTTTATGCAAGCAGGCCCTGGGGG | 165 |
| 166 | | H46_199-226 | CAATGATTGAATTAAAAAAIAGATTCAT | 166 |
| 167 | | H46_229-256 | GAACTATGAATAACCTAATGGGCAGAAA | 167 |
| 168 | | H46_259-286 | ATAAAGTTGTGAGAAAAACACTTTAGCA | 168 |
| 169 | | H46_289-316 | ACTGGTTCAGAACTGCAGGGTTAAGAAG | 169 |
| 170 | | H46_319-346 | ACACACATATATACATATGTTCTTATGT | 170 |
| 171 | R6 | H47_ (-200) - (-173) | CTCACCCCCTCAGTAGATAAATCTCTGT | 171 |
| 172 | | H47_ (-171) - (-144) | TCTGAGCACAGAGCTGATTGACTGAAAC | 172 |
| 173 | | H47_ (-141) - (-114) | AGTGGTACCTCAAATACCAACAGTTTTC | 173 |
| 174 | | H47_ (-111) - (-84) | ATCAAAATGAAGCGACTTGACCGAGGGC | 174 |
| 175 | | H47_ (-81) - (-54) | TACCTTGTCTTTGCTTCTATTGATTAGT | 175 |
| 176 | | H47_ (-51) - (-24) | GTTTAAAATGAATTACAGCACAATTCCA | 176 |
| 177 | | H47_ (-16) -12 | TTCCACCAGTAACTGAAACAGACAAATG | 177 |
| 178 | | H47_1-29 | TGGCGCAGGGGCAACTCTTCCACCAGTAA | 178 |
| 179 | R7 | H47_135-162 | TTAATGTCTAACCTTTATCCACTGGAGA | 179 |
| 180 | R8 | H48_ (-21) -7 | TGGAAACCTGAAAGGAAAATACATTTTA | 180 |
| 181 | | H48_1-28 | CTTGTTTCTCAGGTAAAGCTCTGGAAAC | 181 |
| 182 | R9 | H48_177-204 | AAGCAAAAAGTTCCCTACCTGAACGTCA | 182 |
| 183 | R10 | H49_ (-21) -7 | CAGTTTCCTGGGGAAAAGAACCCATATA | 183 |
| 184 | | H49_22-47 | ATCTCTTCCACATCCGGTTGTTTAGC | 184 |
| 185 | R11 | H49_93-120 | TAGAGGTTGCTTCATTACCTTCACTGGC | 185 |

(continued)

| PMO No. | Target region | Target base sequence | Base sequence of PMO | SEQ ID NO |
|---|---|---|---|---|
| 186 | R12 | H50_ (-200) - (-173) | ACCCTACAAATATTTATCAATTGCTCCA | 186 |
| 187 | | H50_ (-171) - (-144) | TAAAGGAATTATAATTATTTTAGCCAAC | 187 |
| 188 | | H50_ (-141) - (-114) | TAAATTAACTTTAGTGGGTAGAATTTCT | 188 |
| 189 | | H50_ (-111) - (-84) | TATTATTGGATTTCTATTATATTTTACT | 189 |
| 190 | | H50_ (-81) - (-54) | TCATGAACATCTTAATCCATTTGGTGAA | 190 |
| 191 | | H50_ (-51) - (-24) | TACTTATTCGATTAACACTTTGAAGATA | 191 |
| 192 | | H50_ (-21) -7 | ACTTCCTCTTTAACAGAAAAGCATACAC | 192 |
| 193 | R13 | H50_100-127 | GGGATCCAGTATACTTACAGGCTCCAAT | 193 |
| 194 | | H50_90-114 | CTTACAGGCTCCAATAGTGGTCAGT | 194 |
| 195 | R14 | H51_ (-18) -10 | TGAGTAGGAGCTAAAATATTTTGGGTTT | 195 |
| 196 | R15 | H51_224-251 | TATCATTTTTTCTCATACCTTCTGCTTG | 196 |
| 197 | R16 | H52_ (-47) - (-20) | TTAGTATCAGGGTTCTTCAGCGTTGTGT | 197 |
| 198 | | H52_ (-18) -10 | GCATTGTTGCCTGTAAGAACAAATATCC | 198 |
| 199 | | H52_9-38 | AACTGGGGACGCCTCTGTTCCAAATCCTGC | 199 |
| 200 | R17 | H52_109-136 | GCTTGTTAAAAAACTTACTTCGATCCGT | 200 |
| 201 | R18 | H53_ (-197) - (-170) | AATATTAGTTTCTGTTAAATTATTTTCC | 201 |
| 202 | | H53_ (-167) - (-140) | ACAACAGGATTCTTTGCTTTTTTGATGG | 202 |
| 203 | | H53_ (-137) - (-110) | TTATTCATTGTGTTATGGCTAGGATGAT | 203 |
| 204 | | H53_ (-107) - (-80) | ATCTCACATTTATGTTGCTTATTTAAAA | 204 |
| 205 | | H53_ (-77) - (-50) | GAGACATTTTAAATGTAACTTCCAAACG | 205 |
| 206 | | H53_ (-47) - (-20) | AAATATATAGTAGTAAATGCTAGTCTGG | 206 |
| 207 | | H53_ (-18) -10 | ATTCTTTCAACTAGAATAAAAGGAAAAA | 207 |
| 208 | R19 | H53_203-230 | GGTATCTTTGATACTAACCTTGGTTTCT | 208 |
| 209 | R20 | H54_ (-18) -10 | TGGCCAACTGCTATAGATTTTTATGAGA | 209 |
| 210 | R21 | H54_146-173 | AAATAATGTAATTCATACCTTTTATGAA | 210 |
| 211 | | H54_176-203 | CCCCATTATTACAGCCAACAGTAGTTTT | 211 |
| 212 | | H54_206-233 | CAAATCCTCATGGTCCATCCAGTTTCAC | 212 |
| 213 | | H54_236-263 | CCAAGCTCCAGTTTAGCTGGATTGGAAA | 213 |
| 214 | | H54_266-293 | TTTAGTTGGTATTTATCGTCTTGAACCC | 214 |
| 215 | | H54_296-323 | TAAAATAGAAGTCTGAGCCAAGTCCGTG | 215 |
| 216 | | H54_326-353 | GGGCAAATGAGATCTTATGTTCCTCGTT | 216 |
| 217 | R22 | H55_ (-197) - (-170) | TTTACTTTCATTTTTATTTAACTTAAAG | 217 |
| 218 | | H55_ (-167) - (-140) | TAAGCAACAACTATAATATTGTGCAGTC | 218 |
| 219 | | H55_ (-137) - (-110) | TGGGGTGAGTTGTTGCTACAGCTCTTCC | 219 |
| 220 | | H55_ (-107) - (-80) | TGGAGGAACTAAATTGTAATATACCAAC | 220 |
| 221 | | H55_ (-77) - (-50) | CACCTAGTGAACTCCATAAAAAGAGAAA | 221 |
| 222 | | H55_ (-47) - (-20) | CAGATGCAATTATTAAATATCAGAATGG | 222 |
| 223 | | H55_ (-18) -10 | CTCACTCACCCTGCAAAGGACCAAATGT | 223 |
| 224 | | H55_12-34 | AGTTTCTTCCAAAGCAGCCTCTC | 224 |

(continued)

| PMO No. | Target region | Target base sequence | Base sequence of PMO | SEQ ID NO |
|---|---|---|---|---|
| 113 | R23 | H55_177-204 | AATGCCTGACTTACTTGCCATTGTTTCA | 113 |
| 114 | | H55_191-218 | AGTGCTAAAGCGGAAATGCCTGACTTAC | 114 |
| 115 | | H55_221-248 | TGCTGAGAATTGTTCAATTGGATCCACA | 115 |
| 116 | | H55_251-278 | TGTCCCTGGCTTGTCAGTTACAAGTACA | 116 |
| 117 | | H55_281-308 | TCAGGCTGTATAAAAGCAACTATTTTGT | 117 |
| 118 | | H55_311-338 | CTCTCCTCCTTGTCCAAATACCGAAATA | 118 |
| 119 | | H55_334-361 | GATGTTTCCTTCTCCCTCTGCCTCTCTC | 119 |
| 120 | | H55_341-368 | TATAAATGATGTTTCCTTCTCCCTCTGC | 120 |
| 121 | | H55_371-398 | TTTTTCTAAGACGAGGGTGTTAAGTGGA | 121 |
| 122 | | H55_401-428 | TGCAAATGTTTTCCTGGTCAGAGCATGT | 122 |
| 123 | | H55_431-458 | TTTCTCCTTGACCGAAGCTCTGGTTTTA | 123 |
| 124 | | H55_461-488 | CCAATCCCTAAGTTATTTCTCTGAGCAA | 124 |
| 125 | | H55_491-518 | CAAAAATGTCAACTTTTAAAATTTAATA | 125 |
| 126 | | H55_521-548 | GCTTTCCCTGTAAAATATTAAATAAACA | 126 |
| 127 | | H55_548-575 | TAAATAAAACAGACAATTCATACAGATG | 127 |
| 128 | R24 | H56_ (-400) - (-373) | AATAATTTCATTACTATATGGATCAAGT | 128 |
| 129 | | H56_ (-380) - (-355) | AAAGTGTACCCCAGTGCCAATAATTT | 129 |
| 130 | | H56_ (-345) - (-318) | TATTTTATGGAAGTGATAGGCAATAAAA | 130 |
| 131 | | H56_ (-315) - (-288) | TATATCTTCTAGTCTATGGACAAAATGT | 131 |
| 132 | | H56_ (-285) - (-258) | TGTATTTATAACTTTATAAAGTTCACAA | 132 |
| 133 | | H56_ (-255) - (-228) | TATTCCTTGCCATTATGAGTTGGAAAGT | 133 |
| 134 | | H56_ (-225) - (-198) | AAATGGGCATCTTATTAGTTGTAATAGA | 134 |
| 135 | | H56_ (-195) - (-168) | ATTGCCTTCTCCTGTTATTATGTAGATT | 135 |
| 136 | | H56_ (-165) - (-138) | AGATACATCTCAAATCCCTTTTCTTGGC | 136 |
| 137 | | H56_ (-135) - (-108) | AGACATTTCAATCAGGCTAAACTAACAA | 137 |
| 138 | | H56_ (-105) - (-78) | TTGCAAAATATAAATAATTATTAGTTCA | 138 |
| 139 | | H56_ (-76) - (-51) | ACAAGCGATGAATGTGAATTTGGAGA | 139 |
| 140 | | H56_ (-59) - (-34) | ATTACCAAACAAAAGAAACAAGCGAT | 140 |
| 141 | | H56_ (-39) - (-14) | GGAAGAAGAATATGTGCAGAATTACC | 141 |
| 142 | | H56_ (-31) - (-6) | GGACAGCAGGAAGAAGAATATGTGCA | 142 |
| 143 | | H56_ (-14) -13 | TCACCTTGGAGGTCCTACAGGACAGCAG | 143 |
| 225 | R1 + R24 | H44_188-202_H56_ (-156) - (-142) | ACATCTCAAATCCCTGTCCAGATGTGCTGA | 225 |
| 226 | | H44_188-202_H56_ (-124) - (-110) | ACATTTCAATCAGGCGTCCAGATGTGCTGA | 226 |
| 227 | | H44_188-202_H56_ (-85) - (-71) | TGGAGAATTGCAAAAGTCCAGATGTGCTGA | 227 |
| 228 | | H56_ (-156) - (-142)_ H44_188-202 | GTCCAGATGTGCTGAACATCTCAAATCCCT | 228 |
| 229 | | H56_(-124)-(-110)_ H44_188-202 | GTCCAGATGTGCTGAACATTTCAATCAGGC | 229 |
| 230 | | H56_(-85)-(-71)_H44_188-202 | GTCCAGATGTGCTGATGGAGAATTGCAAAA | 230 |

(continued)

| PMO No. | Target region | Target base sequence | Base sequence of PMO | SEQ ID NO |
|---|---|---|---|---|
| 231 | R2+R4 | H45_16-35_H46_(-134)-(-115) | GTTAACCATACATAATTTAAACAGTTTGCC GCTGCCCAAT | 231 |
| 232 | | E45_16-35_H46_(-103)-(-84) | TGGCAATTTAAAAATAAGCAACAGTTTGCC GCTGCCCAAT | 232 |

In the PMO of Table 11,

PMO for the target region R2 includes PMO Nos. 22 to 71, 91 to 93, 98 and 99 in another embodiment, and PMO Nos. 22 to 59, 91 to 93, 98 and 99 in still another embodiment;
PMO for the target region R4 includes PMO Nos. 150 to 163 in another embodiment, and PMO Nos. 150 to 160 in still another embodiment;
PMO for the target region R6 includes PMO Nos. 171 to 177 in another embodiment, and PMO Nos. 171 to 176 in still another embodiment;
PMO for the target region R8 includes PMO No. 180 in another embodiment; PMO for the target region R10 includes PMO No. 183 in another embodiment; PMO for the target region R12 includes PMO Nos. 186 to 191 in another embodiment
PMO for the target region R16 includes PMO Nos. 197 and 198 in another embodiment, and PMO No. 197 in still another embodiment;
PMO for the target region R18 includes PMO Nos. 201 to 206 in another embodiment;
PMO for the target region R22 includes PMO Nos. 217 to 223 in another embodiment, and PMO Nos. 217 to 222 in still another embodiment; and
PMO for the target region R24 includes PMO Nos. 128 to 142 in another embodiment.

0.2 g of 4-{[(2S,6R)-6-(4-benzamide-2-oxopyrimidin-1-yl)-4- tritylmorpholin-2-yl]methoxy}-4-oxobutanoic acid supported on an aminopolystyrene resin (Compound 1), 4-{[(2S,6R)-6-(5-methyl-2,4--dioxopyrimidin-1-yl)-4-tritylmorpholin-2-yl]methoxy}-4-oxobutanoic acid supported on amino polystyrene resin (Compound 2), 4-{[(2S,6R)-6-(6-benzamidopurin-9-yl)-4-tritylmorpholin-2-yl]methoxy}-4-oxobutanoic acid supported on amino polystyrene resin (Compound 3), or 4-{{(2S,6R)-6-{6-(2-cyanoethoxy)-2-[(2-phenoxyacetyl)amino]purin-9-yl}-4-tritylmorpholin-2-yl}methoxy}-4-oxobutanoic acid supported on amino polystyrene resin (Compound 4) appropriate for the 5'-terminal base was filled in a column with a filter tip. Then, the synthetic cycle shown in Table 12 was started using a nucleic acid synthesizing machine (AKTA Oligopilot 10 plus). The desired morpholino monomer compound was added in each coupling cycle to give the base sequence of each PMO described in Table 11 (see Table 12).

[Table 12]

Table 12

| Step | Reagent | Volume (mL) | Time (min) |
|---|---|---|---|
| 1 | deblocking solution | 18-32 | 1.8-3.2 |
| 2 | neutralizing and washing solution | 30 | 1.5 |
| 3 | coupling solution B | 5 | 0.5 |
| 4 | coupling solution A | 1.3 | 0.25 |
| 5 | coupling reaction by the reagents added in the steps 3 and 4 | | 120-300 |
| 6 | acetonitrile | 20 | 1.0 |
| 7 | capping solution | 9 | 2.0 |
| 8 | acetonitrile | 30 | 2.0 |

Note that only for 3'-terminal acetylation, only the steps 1, 2, 7, and 8 were carried out again after the final cycle.

The deblocking solution used was dichloromethane solution containing 3% (w/v) trifluoroacetic acid. The neutralizing and washing solution used was a solution obtained by dissolving N,N-diisopropylethylamine to be 10% (v/v) and tetrahydrofuran to be 5% (v/v) in dichloromethane containing 35% (v/v) acetonitrile. The coupling solution A used was a solution obtained by dissolving the morpholino monomer compound in tetrahydrofuran to be 0.10 M. The coupling solution B used was a solution obtained by dissolving N,N-diisopropylethylamine to be 20% (v/v) and tetrahydrofuran to be 10% (v/v) in acetonitrile. The capping solution used was a solution obtained by dissolving 20% (v/v) acetic anhydride and 30% (v/v) 2,6-lutidine in acetonitrile.

The morpholino monomer compound (A), the morpholino monomer compound (C), the morpholino monomer compound (T), and the morpholino monomer compound (G) described in Table 13 were used as the morpholino monomer compound.

[Table 13]

Table 13

| Morpholino monomer compound (A) | Morpholino monomer compound (C) | Morpholino monomer compound (T) | Morpholino monomer compound (G) |
|---|---|---|---|
| 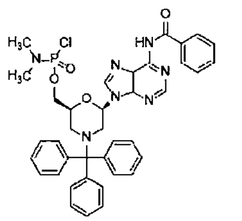 | 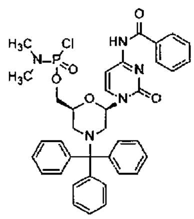 | 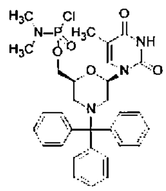 | 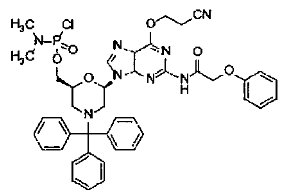 |

The aminopolystyrene resin loaded with the PMO synthesized above was recovered from the reaction vessel and dried at room temperature for at least 2 hours under reduced pressure. The dried PMO loaded onto aminopolystyrene resin was charged in a reaction vessel, and 5 mL of 28% ammonia water-ethanol (1/4) was added thereto. The mixture was stirred at 55°C for 15 hours. The aminopolystyrene resin was separated by filtration and washed with 1 mL of water-ethanol (1/4). The resulting filtrate was concentrated under reduced pressure. The resulting residue was dissolved in 10 mL of a solvent mixture of 20 mM of acetic acid-triethylamine buffer (TEAA buffer) and acetonitrile (4/1) and filtered through a membrane filter. The filtrate obtained was purified by reversed phase HPLC. The conditions used are shown in Table 14.

[Table 14]

Table 14

| | |
|---|---|
| Column | XBridge 5 μm C18 (Waters, ϕ19 × 50 mm, 1 CV = 14 mL) |
| Flow rate | 10 mL/min |
| Column temperature | room temperature |
| Solution A | 20 mM TEAA buffer |
| Solution B | acetonitrile ($CH_3CN$) |
| Gradient | Solution B: (10% → 70%)/15 CV |
| CV: column volume | |

Each fraction was analyzed, and the product of interest was recovered and concentrated under reduced pressure. To the concentrated residue, 0.5 mL of 2 M phosphoric acid aqueous solution was added, and the mixture was stirred for 15 minutes. Furthermore, 2 mL of 2 M sodium hydroxide aqueous solution was added to make the mixture

alkaline, followed by filtration through a membrane filter (0.45 μm).

The resulting aqueous solution containing the product of interest was purified by an anionic exchange resin column. The conditions used are shown in Table 15.

[Table 15]

Table 15

| | |
|---|---|
| Column | Source 15Q (GE Healthcare, ϕ10 × 108 mm, 1 CV = 8.5 mL) |
| Flow rate | 8.5 mL/min |
| Column temperature | room temperature |
| Solution A | 10 mM sodium hydroxide aqueous solution |
| Solution B | 10 mM sodium hydroxide aqueous solution, 1 M sodium chloride aqueous solution |
| Gradient | Solution B: (1% → 50%)/ 40 CV |

Each fraction was analyzed (on HPLC) and the product of interest was obtained as an aqueous solution. To the resulting aqueous solution was added 0.1 M phosphate buffer (pH 6.0) for neutralization. Next, the mixture obtained was demineralized by reversed phase HPLC under the conditions described in Table 16.

[Table 16]

Table 16

| | |
|---|---|
| Column | XBridge 5 μm C18 (Waters, ϕ10 × 50 mm, 1 CV = 4 mL) |
| Flow rate | 4 mL/min |
| Column temperature | 60°C |
| Solution A | water |
| Solution B | $CH_3CN$ |
| Gradient | Solution B: (0% → 50%)/20 CV |

The product of interest was recovered and the mixture was concentrated under reduced pressure. The resulting residue was dissolved in water. The aqueous solution obtained was freeze-dried to give each PMO as the compound of interest as a white cotton-like solid. The calculated and measured values of ESI-TOF-MS are shown in Table 17 below.

[Table 17]

Table 17

| PM0 No. | Target base sequence | Caluculated | Measured |
|---|---|---|---|
| 1 | H44_150-175 | 8610.02 | 8610.48 |
| 2 | H44_157-196 | 13354. 66 | 13354.45 |
| 3 | H44_159-193 | 11652.07 | 11652.09 |
| 4 | H44_170-199 | 10012. 48 | 10013.39 |
| 5 | H44_179-206 | 9392.27 | 9392. 54 |
| 6 | H44_209-236 | 9230. 21 | 9230.92 |

(continued)

| PM0 No. | Target base sequence | Caluculated | Measured |
|---|---|---|---|
| 7 | H44_239-266 | 9256. 24 | 9256. 17 |
| 8 | H44_269-296 | 9264. 2 | 9263.99 |
| 9 | H44_299-326 | 9181. 19 | 9181. 46 |
| 10 | H44_330-355 | 8514. 98 | 8514.36 |
| 11 | H44_354-381 | 9231. 21 | 9231. 24 |
| 12 | H44_389-416 | 9147. 17 | 9147.4 |
| 13 | H44_413-438 | 8551.97 | 8552. 71 |
| 14 | H44_423-452 | 9830. 43 | 9830. 2 |
| 15 | H44_455-480 | 8588. 01 | 8587.53 |
| 16 | H44_479-506 | 9262. 21 | 9262. 15 |
| 17 | H44_509-536 | 9226. 19 | 9225. 7 |
| 18 | H44_150-164_179-193 | 9989. 49 | 9989.23 |
| 19 | H44_150-164_192-206 | 10006. 49 | 10006. 81 |
| 20 | H44_161-175_179-193 | 10013. 5 | 10013. 95 |
| 21 | H44_161-175_192-206 | 10030. 5 | 10031. 48 |
| 22 | H45_(-598)-(-571) | 9221. 2 | 9221. 27 |
| 23 | H45_(-568)-(-541) | 9312. 25 | 9311. 93 |
| 24 | H45_(-538)-(-511) | 9218. 18 | 9218. 33 |
| 25 | H45_(-518)-(-491) | 9283. 22 | 9283. 26 |
| 26 | H45_(-515)-(-488) | 9307. 23 | 9307. 34 |
| 27 | H45_(-508)-(-479) | 10027. 5 | 10027. 98 |
| 28 | H45_(-478)-(-449) | 9979. 48 | 9980. 37 |
| 29 | H45_(-470)-(-443) | 9295. 25 | 9296. 02 |
| 30 | H45_(-448)-(-421) | 9385. 28 | 9385. 45 |
| 31 | H45_(-439)-(-412) | 9403. 3 | 9403. 98 |
| 32 | H45_(-436)-(-409) | 9435. 3 | 9435. 78 |
| 33 | H45_(-422)-(-395) | 9388. 23 | 9388. 34 |
| 34 | H45_(-418)-(-394) | 8363. 88 | 8363. 44 |
| 35 | H45_(-418)-(-391) | 9404. 23 | 9404.6 |
| 36 | H45_(-415)-(-391) | 8354. 87 | 8354.48 |
| 37 | H45_(-413)-(-386) | 9385.21 | 9385. 07 |
| 38 | H45_(-405)-(-378) | 9371. 23 | 9371. 33 |
| 39 | H45_(-389)-(-362) | 9325. 25 | 9325. 6 |
| 40 | H45_(-349)-(-322) | 9221. 2 | 9221. 14 |
| 41 | H45_(-320)-(-293) | 9211. 19 | 9211. 14 |
| 42 | H45_(-305)-(-280) | 8640 | 8640. 11 |
| 43 | H45_(-290)-(-263) | 9284. 22 | 9284. 05 |
| 44 | H45_(-275)-(-250) | 8656 | 8656. 36 |

(continued)

| PM0 No. | Target base sequence | Caluculated | Measured |
|---|---|---|---|
| 45 | H45_(-260)-(-233) | 9347. 22 | 9347 |
| 46 | H45_(-230)-(-203) | 9198. 19 | 9198. 43 |
| 47 | H45_(-200)-(-173) | 9335. 24 | 9335. 21 |
| 48 | H45_(-190)-(-163) | 9312. 25 | 9312. 03 |
| 49 | H45_(-185)-(-166) | 8670. 05 | 8670. 14 |
| 50 | H45_(-171)-(-144) | 9273. 24 | 9273. 53 |
| 51 | H45_(-168)-(-141) | 9233. 23 | 9233. 36 |
| 52 | H45_(-142)-(-115) | 9254. 22 | 9254. 39 |
| 53 | H45_(-110)-(-83) | 9392. 27 | 9392. 55 |
| 54 | H45_(-80)-(-53) | 9352. 26 | 9352. 54 |
| 55 | H45_(-78)-(-51) | 9358. 25 | 9358. 63 |
| 56 | H45_(-70)-(-43) | 9323. 23 | 9323. 51 |
| 57 | H45_(-60)-(-33) | 9260. 21 | 9260. 39 |
| 58 | H45_(-50)-(-23) | 9238.22 | 9238. 79 |
| 59 | H45_(-44)-(-17) | 9291. 26 | 9291. 37 |
| 60 | H45_(-25)-3 | 9281. 25 | 9281. 54 |
| 61 | H45_(-23)-5 | 9321. 26 | 9321. 92 |
| 62 | E45_(-22)-3 | 8287. 9 | 8287. 54 |
| 63 | H45_(-21)-7 | 9353. 26 | 9353. 35 |
| 64 | H45_(-20)-5 | 8303. 9 | 8303. 52 |
| 65 | H45_(-20)-8 | 9344. 25 | 9344. 57 |
| 66 | E45_(-18)-10 | 9304. 24 | 9304. 44 |
| 67 | E45_(-17)-8 | 8318.9 | 8318. 93 |
| 68 | H45_(-16)-12 | 9279. 23 | 9278. 95 |
| 69 | H45_(-15)-10 | 8270. 88 | 8270. 96 |
| 70 | H45_(-14)-14 | 9231. 21 | 9231. 28 |
| 71 | H45_(-10)-16 | 8607. 98 | 8607. 59 |
| 72 | H45_(-5)-20 | 8213. 85 | 8213. 45 |
| 73 | H45_1-25 | 8214. 85 | 8214. 52 |
| 74 | H45_6-30 | 8190.84 | 8190. 04 |
| 75 | H45_11-35 | 8198.85 | 8198. 45 |
| 76 | H45_11-40 | 9877. 43 | 9877. 12 |
| 77 | H45_16-35 | 6544. 28 | 6544. 07 |
| 78 | H45_16-40 | 8222. 86 | 8222. 85 |
| 79 | H45_21-45 | 8244. 85 | 8245. 01 |
| 80 | H45_(-423)-(-412)_16-33 | 9997. 46 | 9997. 49 |
| 81 | H45_(-416)-(-402)_16-30 | 9964. 44 | 9964. 38 |
| 82 | B45_(-411)-(-400)_16-33 | 9979. 44 | 9979. 18 |

(continued)

| PM0 No. | Target base sequence | Caluculated | Measured |
|---|---|---|---|
| 83 | H45_(-405)-(-391)_16-30 | 9945. 42 | 9945. 04 |
| 84 | H45_(-402)-(-391)_16-33 | 9945. 42 | 9945. 07 |
| 85 | H45_(-156)-(-143)-(-141)-(-128) | 9190. 2 | 9189.84 |
| 86 | H45_(-78)-(-65)_(-44)-(-31) | 9256. 22 | 9256.49 |
| 87 | H45_(-78)-(-65)_(-34)-(-21) | 9354. 28 | 9354. 26 |
| 88 | H45_(-78)-(-65)_16-29 | 9232. 21 | 9233. 24 |
| 89 | H45_(-78)-(-65)_21-34 | 9263. 21 | 9263. 82 |
| 90 | H45_(-78)-(-65)_27-40 | 9295. 23 | 9295. 5 |
| 91 | H45_(-74)-(-62)_(-40)-(-28) | 8643. 02 | 8643. 41 |
| 92 | H45_(-72)-(-58)_(-38)-(-26) | 9354. 28 | 9354. 28 |
| 93 | H45_(-71)-(-58)_(-38)- (-25) | 9314. 27 | 9314. 5 |
| 94 | H45_ (-71) -(-58)_16-29 | 9264. 23 | 9262. 81 |
| 95 | H45_(-71)-(-58)_21-34 | 9295. 23 | 9295. 83 |
| 96 | H45_(-71)-(-58)_27-40 | 9327. 25 | 9327. 61 |
| 97 | H45_(-68)-(-60)_(-36)-(-28)_19-28 | 9274. 24 | 9274. 8 |
| 98 | H45_(-64)-(-51)_(-44)- (-31) | 9310.23 | 9310. 1 |
| 99 | H45_(-64)-(-51)_(-35)-(-22) | 9408. 29 | 9408. 3 |
| 100 | H45_(-64)-(-51)_16-29 | 9286. 22 | 9286. 47 |
| 101 | H45_(-64)-(-51)_21-34 | 9317. 22 | 9317.12 |
| 102 | H45_(-64)-(-51) _27-40 | 9349. 24 | 9349. 37 |
| 103 | H45_(-44)-(-31)_16-29 | 9184. 19 | 9183. 98 |
| 104 | H45_(-44)-(-31)_27-40 | 9247. 21 | 9247. 14 |
| 105 | H45_(-44)-(-31)_21-33_T | 9230. 19 | 9230. 7 |
| 106 | H45_(-38)-(-25)_16-29 | 9210. 22 | 9210. 11 |
| 107 | H45_(-38)-(-25)_21-34 | 9241. 22 | 9241. 87 |
| 108 | H45_(-38)-(-25)_27-40 | 9273. 24 | 9273. 24 |
| 109 | H45_(-34)-(-21)_16-29 | 9266. 25 | 9266. 72 |
| 110 | H45_(-34)-(-21)_21-34 | 9297.25 | 9297. 85 |
| 111 | H45_(-34)-(-21)_27-40 | 9329. 27 | 9329. 78 |
| 112 | H45_1-21 | 6859. 387 | 6859. 02 |
| 144 | H45_167-194 | 9121. 14 | 9121. 27 |
| 145 | H45_227-254 | 9253.22 | 9253. 57 |
| 146 | H45_257-284 | 9203. 18 | 9203.09 |
| 147 | H45_287-314 | 9369.21 | 9369. 79 |
| 148 | H45_317-344 | 9342. 27 | 9342. 18 |
| 149 | H45_347-374 | 9268. 22 | 9268. 72 |
| 150 | H46_(-200)-(-173) | 9326. 25 | 9325. 78 |
| 151 | H46_(-171)-(-144) | 9243.21 | 9243. 17 |

(continued)

| PM0 No. | Target base sequence | Caluculated | Measured |
|---|---|---|---|
| 152 | H46_(-146)-(-119) | 9221. 22 | 9221. 07 |
| 153 | H46_(-141)-(-114) | 9276. 23 | 9275. 87 |
| 154 | H46_ (-136)-(-169) | 9310. 25 | 9310. 07 |
| 155 | H46_(-126)-(-99) | 9295. 25 | 9295. 56 |
| 156 | H46_(-116)-(-89) | 9349. 26 | 9349. 16 |
| 157 | H46_(-111)-(-84) | 9334. 26 | 9333.8 |
| 158 | H46_(-106)-(-79) | 9319. 26 | 9319. 07 |
| 159 | H46_(-81)-(-54) | 9318. 24 | 9317. 86 |
| 160 | H46_(-51)-(-24) | 9167. 16 | 9167. 63 |
| 161 | H46_(-18)-6 | 8037. 81 | 8037. 34 |
| 162 | H46_(-15)-9 | 8004. 79 | 8004. 24 |
| 163 | H46_(-9)-15 | 7943. 74 | 7944. 19 |
| 164 | H46_139-166 | 9293. 23 | 9292. 5 |
| 165 | H46_169-196 | 9319.22 | 9319.52 |
| 166 | H46_199-226 | 9325. 25 | 9325.91 |
| 167 | H46_229-256 | 9361. 27 | 9361. 58 |
| 168 | B46_259-286 | 9351. 26 | 9351. 88 |
| 169 | H46_2S9-316 | 9400. 26 | 9400.38 |
| 170 | H46_319-346 | 9219. 2 | 9219. 51 |
| 171 | H47_(-200)-(-173) | 9142. 18 | 9142. 19 |
| 172 | H47_(-171)-(-144) | 9320. 24 | 9320. 43 |
| 173 | H47_(-141)-(-114) | 9230. 21 | 9230. 7 |
| 174 | H47_(-111)-(-84) | 9354. 26 | 9354. 05 |
| 175 | H47_(-81)-(-54) | 9215.16 | 9215.57 |
| 176 | H47_(-51)-(-24) | 9262.23 | 9262.28 |
| 177 | H47_(-16)-12 | 9257. 24 | 9257. 23 |
| 178 | H47_1-29 | 9612. 33 | 9613. 79 |
| 179 | H47_135-162 | 9236. 2 | 9236. 32 |
| 180 | H48_(-21)-7 | 9351. 26 | 9350. 96 |
| 181 | H48_1-28 | 9277. 21 | 9277. 18 |
| 182 | H48_177-204 | 9233.23 | 9232. 72 |
| 183 | 1149_(-21)-7 | 9304. 24 | 9304. 25 |
| 184 | H49_22-47 | 8509. 94 | 8509. 66 |
| 185 | H49_93-120 | 9244. 19 | 9244. 51 |
| 186 | H50_(-200)-(-173) | 9149. 19 | 9148. 87 |
| 187 | H50_(-171)-(-144) | 9292. 23 | 9291. 93 |
| 188 | H50_(-141)-(-114) | 9330. 22 | 9330. 33 |
| 189 | H50_(-111)-(-84) | 9237. 17 | 9236. 8 |

(continued)

| PM0 No. | Target base sequence | Caluculated | Measured |
|---|---|---|---|
| 190 | H50_(-81)-(-59) | 9260. 21 | 9259. 85 |
| 191 | H50_(-51)-(-24) | 9259. 21 | 9268. 85 |
| 192 | H50_(-21)-7 | 9183. 21 | 9183. 31 |
| 193 | H50_100-127 | 9271. 22 | 9271. 25 |
| 194 | H50_90-114 | 8277. 87 | 8278. 28 |
| 195 | H51_(-18)-10 | 9411. 24 | 9411. 6 |
| 196 | H51_224-251 | 9135. 14 | 9135. 51 |
| 197 | H52_(-47)-(-20) | 9330.2 | 9330.03 |
| 198 | H52_(-18)-10 | 9270.22 | 9270.37 |
| 199 | H52_9-38 | 9893.42 | 9893.2 |
| 200 | H52_109-136 | 9236.2 | 9235.9 |
| 201 | H53_(-197)-(-170) | 9231.18 | 9231.18 |
| 202 | H53_(-167)-(-140) | 9298.2 | 9298.58 |
| 203 | H53_(-137)-(-110) | 9353.21 | 9353.6 |
| 204 | H53_(-107)-(-86) | 9225.19 | 9224.55 |
| 205 | H53_(-77)-(-50) | 9278.23 | 9278.35 |
| 206 | H53_(-47)-(-20) | 9373.25 | 9373.48 |
| 207 | H53_(-18)-10 | 9319.26 | 9319 |
| 208 | H53_203-230 | 9249.18 | 9249.06 |
| 209 | H54_(-18)-10 | 9316.22 | 9316.33 |
| 210 | H54_146-173 | 9267. 22 | 9267. 45 |
| 211 | H54_176-203 | 9181. 19 | 9181. 18 |
| 212 | H54_206-233 | 9142.18 | 9142. 41 |
| 213 | H54_236-263 | 9311. 23 | 9311. 57 |
| 214 | H54_266-293 | 9249. 18 | 9248. 66 |
| 215 | H54_296-323 | 9344. 25 | 9344. 57 |
| 216 | E54_326-353 | 9308.21 | 9307.9 |
| 217 | H55_(-197)-(-170) | 9206. 17 | 9206. 95 |
| 218 | H55_(-157)-(-140) | 9278. 23 | 9278. 65 |
| 219 | H55_(-137)-(-110) | 9316. 2 | 9316. 05 |
| 220 | H55_(-107)-(-80) | 9327. 25 | 9326. 87 |
| 221 | H55_(-77)-(-50) | 9290. 26 | 9290. 43 |
| 222 | H55_(-47)-(-20) | 9342. 25 | 9341. 86 |
| 223 | H55_(-18)-10 | 9185. 21 | 9185. 78 |
| 224 | H55_12-34 | 6858. 387 | 6858. 97 |
| 113 | H55_177-204 | 9203.18 | 9203.35 |
| 114 | H55_191-218 | 9320. 24 | 9320. 46 |
| 115 | H55_221-248 | 9301. 22 | 9301. 7 |

(continued)

| PM0 No. | Target base sequence | Caluculated | Measured |
|---|---|---|---|
| 116 | H55_251-278 | 9253. 2 | 9253.12 |
| 117 | H55_281-308 | 9300. 22 | 9300. 57 |
| 118 | H55_311-338 | 9126. 18 | 9126. 01 |
| 119 | H55_334-361 | 9058. 12 | 9057. 77 |
| 120 | H55_341-368 | 9154.16 | 9154. 22 |
| 121 | H55_371-398 | 9412. 24 | 9412. 07 |
| 122 | H55_401-428 | 9308. 21 | 9308. 06 |
| 123 | H55_431-458 | 9210. 17 | 9209. 71 |
| 124 | H55_461-488 | 9181. 19 | 9181. 31 |
| 125 | H55_491-518 | 9260. 23 | 9260. 72 |
| 126 | H55_521-548 | 9237. 22 | 9236.93 |
| 127 | H55_548-575 | 9304.26 | 9304.44 |
| 128 | H56_(-400)-(-373) | 9283. 22 | 9282. 84 |
| 129 | H56_(-380)-(-355) | 8584.99 | 8585.11 |
| 130 | H56_(-345)-(-318) | 9397.26 | 9397.16 |
| 131 | H56_(-315)-(-288) | 9275.21 | 9275.43 |
| 132 | H56_(-285)-(-258) | 9258.21 | 9257.72 |
| 133 | H56_(-255)-(-228) | 9307.21 | 9307.98 |
| 134 | H56_(-225)-(-198) | 9364.24 | 9364.79 |
| 135 | H56_(-195)-(-168) | 9224.17 | 9224.08 |
| 136 | H56_(-165)-(-138) | 9172.18 | 9172.28 |
| 137 | H56_(-135)-(-108) | 9256.24 | 9256.9 |
| 138 | H56_(-105)-(-78) | 9291.23 | 9291.37 |
| 139 | H56_(-76)-(-51) | 8754.04 | 8754.09 |
| 140 | H56_(-59)-(-34) | 8629.04 | 8629.59 |
| 141 | H56_(-39)-(-14) | 8723.04 | 8723.84 |
| 142 | H56_(-31)-(-6) | 8773.06 | 8773.68 |
| 143 | H56_(-14)-13 | 9297.23 | 9296.6 |
| 225 | H44_188-202_H56_(-156)-(-142) | 9876.43 | 9876.87 |
| 226 | H44_188-202_H56_(-124)-(-110) | 9956.45 | 9956.24 |
| 227 | H44_188-202_H56_(-85)-(-71) | 10069.49 | 10070.12 |
| 228 | H56_(-156)-(-142)_H44_188-202 | 9876.43 | 9876.63 |
| 229 | H56_(-124)-(-110)_H44_188-202 | 9956.45 | 9956.72 |
| 230 | H56_(-85)-(-71)_H44_188-202 | 10069.49 | 10069.93 |
| 231 | H45_16-35_H46_(-134)-(-115) | 13207.58 | 13207.56 |
| 232 | H45_16-35_H46_(-103)-(-84) | 13281.61 | 13281.25 |

Production of antisense PMO targeting splicing silencer in intron of human dystrophin gene or splice site involved in single skipping of human dystrophin gene

(i) PMO having the base sequence of PMO No. 233 (SEQ ID NO: 257) targeting a splicing silencer sequence of intron 44 or a splice site involved in single skipping, (ii) PMOs having the base sequences of PMO Nos. 234 to 244 (SEQ ID NOs: 258 to 268) targeting a splicing silencer sequence of intron 45 or a splice site involved in single skipping, (iii) PMOs (linked type) having the base sequences of PMO Nos. 245 to 249 (SEQ ID NOs: 269 to 273) targeting a region (R2) in the vicinity of an acceptor of intron 44 and a splicing silencer sequence of intron 45 or a splice site involved in single skipping, and (iv) PMOs having the base sequences of PMO Nos. 250 to 251 (SEQ ID NOs: 274 to 275) targeting a splicing silencer sequence of exon 45 in the human dystrophin gene were synthesized in the same manner as in the PMO targeting a region in the vicinity of a donor or an acceptor of an intron. The 5' end of each PMO is Group (3), as in the PMO targeting a region in the vicinity of a donor or an acceptor of an intron. Each PMO is shown in Table 18, and the calculated (Clcd) and measured (Found) values of ESI-TOF-MS are shown in Table 19.

[Table 18]

Table 18

| PM0 No. | Target | Target base sequence | Base sequence of PMO | SEQ ID NO |
|---|---|---|---|---|
| 233 | (i) | H45_(-93)-(-70) | GTGTGCTACCACATGCAGTTGTAC | 257 |
| 234 | (ii) | H45_166-189 | TCTGTCGCCCTACCTCTTTTTTCT | 258 |
| 235 | | H45_178-201 | ATTCCTATTAGATCTGTCGCCCTA | 259 |
| 236 | | H45_183-206 | TTTTCATTCCTATTAGATCTGTCG | 260 |
| 237 | | H45_188-211 | AAATGTTTTCATTCCTATTAGATC | 261 |
| 238 | | H45_191-214 | CTAAAATGTTTTCATTCCTATTAG | 262 |
| 239 | | H45_197-220 | AGTCTGCTAAAATGTTTTCATTCC | 263 |
| 240 | | H46_(-35)-(-12) | AGAATAAAATTGTTATTTTTTTTT | 264 |
| 241 | | H46_(-22)_2 | GCCTGGAGAAAGAAGAATAAAATT | 265 |
| 242 | | H46_(-12)_12 | TGTTCTTCTAGCCTGGAGAAAGAA | 266 |
| 243 | | H46_(-2)_22 | GATATTCTTTTGTTCTTCTAGCCT | 267 |
| 244 | | H45_20236-20259 | ATCCATGCATTCTTCCATGCACAG | 268 |
| 245 | (iii) | H45_16-30_191-205 | TTTCATTCCTATTAGTTGCCGCTGCCCAAT | 269 |
| 246 | | H45_16-30_197-211 | AAATGTTTTCATTCCTTGCCGCTGCCCAAT | 270 |
| 247 | | H45_16-33_197-208 | TGTTTTCATTCCAGTTTGCCGCTGCCCAAT | 271 |
| 248 | | H45_16-35_183-192 | AGATCTGTCGACAGTTTGCCGCTGCCCAAT | 272 |
| 249 | | H45_18-35_191-202 | CATTCCTATTAGACAGTTTGCCGCTGCCCA | 273 |
| 250 | (iv) | H45_31-54 | TGCATTCAATGTTCTGACAACAGT | 274 |
| 251 | | H45_131-154 | GTTTGCAGACCTCCTGCCACCGCA | 275 |
| A base sequence presumed to correspond to the splicing silencer sequence in the intron or the splice site involved in single skipping is underlined. | | | | |

A base sequence presumed to correspond to the splicing silencer sequence in the intron or the splice site involved in single skipping is underlined.

[Table 19]

Table 19

| PM0 No. | Target base sequence | Calculated | Measured |
|---|---|---|---|
| 233 | H45_ (-93) - (-70) | 7938. 75 | 7938. 95 |
| 234 | H45_166-189 | 7757. 67 | 7757. 89 |
| 235 | H45_178-201 | 7848. 72 | 7848. 99 |
| 236 | H45_183-206 | 7869.71 | 7869. 94 |
| 237 | H45_188-211 | 7886. 73 | 7886. 24 |
| 238 | H45_191-214 | 7886. 73 | 7886. 37 |
| 239 | H45_197-220 | 7887. 73 | 7887.49 |
| 240 | H46_(-35)-(-12) | 7955.74 | 7955.61 |
| 241 | 846_(-22)_2 | 8061. 82 | 8062.19 |
| 242 | H46_(-12)_12 | 7986. 77 | 7986. 42 |
| 243 | H46_(-2)_22 | 7860. 7 | 7860. 83 |
| 244 | H45_20236-20259 | 7842. 73 | 7842.9 |
| 245 | H45_16-30_191-205 | 9824. 39 | 9824. 66 |
| 246 | H45_16-30_197-211 | 9833.4 | 9833. 32 |
| 247 | H45_16-33_197-208 | 9840. 39 | 9840. 1 |
| 248 | H45_16-35_183-192 | 9908.43 | 9908.14 |
| 249 | H45_18-35_191-202 | 9843. 41 | 9843. 08 |
| 250 | H45_31-54 | 7848. 72 | 7848. 99 |
| 251 | H45_131-154 | 7844.73 | 7844. 85 |

[Example 2: Test on multi-exon skipping activity of antisense oligomer]

<Test Example 1>

*In vitro* assay of multi-exon skipping in human dystrophin gene - (1): induction of multi-exon skipping

Procedures

Each antisense oligomer (PMO Nos. 55, 59, 78, 65, 75, 77, 102, 35, 52, 47, 50, 46, 43, 40, 139, 119, 142, 113, 51, 11, 45, 54, 41, 74, 66, 64, 63, 68, 76, 28, 27, 225, 228, 1, 5, 132, 131, 128, 163, 162, and 161(SEQ ID NO: 55, 59, 78, 65, 75, 77, 102, 35, 52, 47, 50, 46, 43, 40, 139, 119, 142, 113, 51, 11, 45, 54, 41, 74, 66, 64, 63, 68, 76, 28, 27, 225, 228, 1, 5, 132, 131, 128, 163, 162, and 161)) of Table 11 was transfected in a concentration of 25 to 50 μM either singly or in combination of two or three oligomers with $3.5 \times 10^5$ of RD cells (human rhabdomyosarcoma cell line, CCL-136, purchased from ATCC). The reagent used for the transfection was an Amaxa Cell Line Nucleofector Kit L on Nucleofector II (Lonza). The Program T-030 was used.

After transfection, the cells were cultured for three nights in 2 mL of Eagle's minimal essential medium (EMEM) (manufactured by Sigma, hereinafter the same) containing 10% fetal bovine serum (FBS) (manufactured by Invitrogen) under conditions of 37°C and 5% $CO_2$.

The RD cells were washed once with PBS (manufactured by Nissui, hereinafter the same) and 350 μL of Buffer RLT (manufactured by Qiagen) containing 1% 2-mercaptoethanol (manufactured by Nacalai Tesque, Inc.) was added to the cells. After the cells were allowed to stand at room temperature for a few minutes to lyse the cells, the lysate was collected into a QIAshredder homogenizer (manufactured by Qiagen). A homogenate was produced by centrifugation at 15,000 rpm for 2 minutes. The total RNA was extracted according to the protocol attached to RNeasy Mini Kit (manufactured by Qiagen). The concentration of the total RNA extracted was determined using a NanoDrop ND-1000 (manufactured by LMS Co., Ltd.).

One-Step RT-PCR was performed with 800 ng of the extracted total RNA using a QIAGEN One Step RT-PCR Kit (manufactured by Qiagen). A reaction solution was prepared in accordance with the protocol attached to the kit. TaKaRa PCR Thermal Cycler Dice Touch (manufactured by Takara Bio Inc.) was used as the thermal cycler. The RT-PCR program used is as follows.

50°C, 30 mins: reverse transcription reaction
95°C, 15 mins: polymerase activation, reverse transcriptase inactivation, cDNA thermal denaturation
[94°C, 10 seconds; 57°C, 30 seconds; 72°C, 1 minute] × 33 cycles: PCR amplification
72°C, 10 mins: final extension

The base sequences of the forward primer and reverse primer used for RT-PCR are given below.

Forward primer: 5'-ATTTGACAGATCTGTTGAGAAATGG-3' (SEQ ID NO: 276)
Reverse primer 1: 5'-GGCTGTTTTCATCCAGGTTGTG-3' (SEQ ID NO: 277)
Reverse primer 2: 5'-AGTTGCTGCTCTTTTCCAGGT-3' (SEQ ID NO: 278)

Transcripts having multi-exon skipping of exons 45 to 55 can be detected by a combination of the forward primer and the reverse primer 2. Transcripts having no skipping and transcripts having single exon skipping of exon 45 can be detected by a combination of the forward primer and the reverse primer 1.

The reaction product of the PCR above was analyzed using a Bioanalyzer (manufactured by Agilent Technologies, Inc.) and MultiNA (manufactured by Shimadzu Corp.) .

The polynucleotide level "A" of the band with skipping of any two or more numerically consecutive exons among exons 45 to 55, the polynucleotide level "B" of the band with skipping of any one exon among exons 45 to 55, and the polynucleotide level "C" of the band without skipping were measured. Based on these measurement values of "A", "B", and "C", the skipping efficiency of multi-exon skipping was determined by the following equation.

$$\text{Skipping efficiency (\%)} = A / (A + B + C) \times 100$$

Results

The results are shown in Figures 1 to 16. The introduction of PMO Nos. 55, 59, 78, 65, 75, 77, 102, 35, 52, 47, 50, 46, 43, 40, 139, 119, 142, 113, 51, 11, 45, 54, 41, 74, 66, 64, 63, 68, 76, 28, 27, 225, 228, 1, 5, 132, 131, 128, 163, 162, and 161(SEQ ID NO: 55, 59, 78, 65, 75, 77, 102, 35, 52, 47, 50, 46, 43, 40, 139, 119, 142, 113, 51, 11, 45, 54, 41, 74, 66, 64, 63, 68, 76, 28, 27, 225, 228, 1, 5, 132, 131, 128, 163, 162, and 161) singly or in combination induced multi-exon skipping of exons 45 to 55.

These results revealed that the antisense oligomers of the present invention described in Table 11 effectively induce or would effectively induce multi-exon skipping when used singly or in combination.

It was also confirmed that the introduction of PMO Nos. 6, 7, 8, 10, 14, 26, 29, 38, 39, 53, 58, 67, 80, 82, 86, 92, 97, 98, 100, 121, 122, 124, 125, 126, 130, 144, 146, 147, 150, 151, 152, 153, 154, 155, 156, 157, 158, 159, 160, 231, 232 (SEQ ID NO: 6, 7, 8, 10, 14, 26, 29, 38, 39, 53, 58, 67, 80, 82, 86, 92, 97, 98, 100, 121, 122, 124, 125, 126, 130, 144, 146, 147, 150, 151, 152, 153, 154, 155, 156, 157, 158, 159, 160, 231, 232) also induce multi-exon skipping of exons 45 to 55.

<Test Example 2>

*In vitro* assay of multi-exon skipping in human dystrophin gene - (2): promotion of multi-exon skipping

Procedures

The antisense oligomer (PMO No. 75) of Table 11 was added in a concentration of 25 to 50 μM to $3.5 \times 10^5$ of RD cells (human rhabdomyosarcoma cell line, CCL-136, purchased from ATCC), and each antisense oligomer (PMO No. 236, 237, or 239) of Table 18 was introduced in combination therewith in a concentration of 25 to 50 μM. The assay was conducted by the same procedures as in Test Example 1.

Results

The results are shown in Figures 17 and 18. The introduction of PMO No. 75 (SEQ ID NO: 75) in combination

with PMO No. 236, 237, or 239 (SEQ ID NO: 260, 261, or 263) enhanced the skipping efficiency of multi-exon skipping of exons 45 to 55 as compared to the case of PMO No. 75 singly. On the other hand, this approach decreased the skipping efficiency of single exon skipping of exon 45.

These results revealed that the antisense oligomer of the present invention described in Table 11 promote or would promote multi-exon skipping when used in combination with the suppressor antisense oligomer of the present invention described in Table 18.

<Test Example 3>

*In vitro* assay of multi-exon skipping in human dystrophin gene - (3): promotion of overall multi-exon skipping

Procedures

Test Example 3 was conducted by the same procedures as in Test Example 2 except that One-Step RT-PCR was performed as follows.

One-Step RT-PCR was performed with 800 ng of the extracted total RNA using a QIAGEN One Step RT-PCR Kit (manufactured by Qiagen). A reaction solution was prepared in accordance with the protocol attached to the kit. TaKaRa PCR Thermal Cycler Dice Touch (manufactured by Takara Bio Inc.) was used as the thermal cycler. The RT-PCR program used is as follows.

50°C, 30 mins: reverse transcription reaction
95°C, 15 mins: polymerase activation, reverse transcriptase inactivation, cDNA thermal denaturation
[94°C, 10 seconds; 60°C, 30 seconds; 72°C, 2 min] × 40 cycles: PCR amplification
72°C, 10 mins: final extension

The base sequences of the forward primer and reverse primer used for RT-PCR are given below.

Forward primer 2: 5'-ATTTGACAGATCTGTTGAGAAATGG-3' (SEQ ID NO: 279)
Reverse primer 3: 5'-GGCTCCAATAGTGGTCAGTCC-3' (SEQ ID NO: 280)
Forward primer 3: 5'-CCTGAGAATTGGGAACATGC-3' (SEQ ID NO: 281)
Reverse primer 4: 5'-CCTCCTTCCATGACTCAAGC-3' (SEQ ID NO: 282)

Transcripts having multi-exon skipping of exons 45 and 46, exons 45 to 47, exons 45 to 48, or exons 45 to 49 in the human dystrophin gene can be detected by a combination of the forward primer 2 and the reverse primer 3. On the other hand, transcripts having multi-exon skipping of exons 45 and 46, exons 45 to 47, exons 45 to 48, exons 45 to 49, exons 45 to 50, exons 45 to 51, or exons 45 to 52 are detected by a combination of the forward primer 3 and the reverse primer 4.

The reaction product of the PCR above was analyzed using a Bioanalyzer (manufactured by Agilent Technologies, Inc.) and MultiNA (manufactured by Shimadzu Corp.) to quantify the amount of the PCR product of the transcript having each exon skipping.

Results

The results are shown in Figures 19 to 22. The introduction of PMO No. 75 (SEQ ID NO: 75) in combination with PMO No. 236 or 239 (SEQ ID NO: 260 or 263) enhanced the skipping efficiency of multi-exon skipping of exons 45 to 55 (Test Example 2) as well as multi-exon skipping of exons 45 and 46, exons 45 to 47, exons 45 to 48, exons 45 to 49, exons 45 to 50, exons 45 to 51, and exons 45 to 52 as compared to the case of PMO No. 75 singly.

These results revealed that the antisense oligomer of the present invention described in Table 11 promote or would promote overall multi-exon skipping when used in combination with the suppressor antisense oligomer of the present invention described in Table 18.

Industrial Applicability

The present invention provides an antisense oligomer and a suppressor antisense oligomer that cause multi-exon skipping in human dystrophin. Use of the antisense oligomer and the suppressor antisense oligomer of the present invention provides a novel therapeutic agent for DMD and method for treatment of DMD.

## Claims

1. An antisense oligomer or a pharmaceutically acceptable salt thereof, or hydrate thereof which causes simultaneous skipping of any two or more numerically consecutive exons selected from the group consisting of the 45th exon to the 55th exon in human dystrophin pre-mRNA,

   the antisense oligomer comprising a base sequence complementary to a base sequence of at least one region selected from the group consisting of regions R1 to R24 represented by
   region Rn (wherein n is an odd number of 1 to 23) which consists of a base sequence of NX bases in the upstream direction from the 3' end of the NAth exon and a base sequence of NY bases in the downstream direction from the 5' end of the NBth intron in the human dystrophin pre-mRNA, and
   region Rn (wherein n is an even number of 2 to 24) which consists of a base sequence of NX bases in the upstream direction from the 3' end of the NAth intron and a base sequence of NY bases in the downstream

direction from the 5' end of the NBth exon in the human dystrophin pre-mRNA, or a partial base sequence thereof, wherein
when n is 1, NA = 44, NB = 44, NX = 20, and NY = 400,
when n is 2, NA = 44, NB = 45, NX = 600, and NY = 50,
when n is 3, NA = 45, NB = 45, NX = 20, and NY = 400,
when n is 4, NA = 45, NB = 46, NX = 400, and NY = 50,
when n is 5, NA = 46, NB = 46, NX = 20, and NY = 400,
when n is 6, NA = 46, NB = 47, NX = 400, and NY = 50,
when n is 7, NA = 47, NB = 47, NX = 20, and NY = 400,
when n is 8, NA = 47, NB = 48, NX = 400, and NY = 50,
when n is 9, NA = 48, NB = 48, NX = 20, and NY = 400,
when n is 10, NA = 48, NB = 49, NX = 400, and NY = 50,
when n is 11, NA = 49, NB = 49, NX = 20, and NY = 400,
when n is 12, NA = 49, NB = 50, NX = 400, and NY = 50,
when n is 13, NA = 50, NB = 50, NX = 20, and NY = 400,
when n is 14, NA = 50, NB = 51, NX = 400, and NY = 50,
when n is 15, NA = 51, NB = 51, NX = 20, and NY = 400,
when n is 16, NA = 51, NB = 52, NX = 400, and NY = 50,
when n is 17, NA = 52, NB = 52, NX = 20, and NY = 400,
when n is 18, NA = 52, NB = 53, NX = 400, and NY = 50,
when n is 19, NA = 53, NB = 53, NX = 20, and NY = 400,
when n is 20, NA = 53, NB = 54, NX = 400, and NY = 50,
when n is 21, NA = 54, NB = 54, NX = 20, and NY = 400,
when n is 22, NA = 54, NB = 55, NX = 400, and NY = 50,
when n is 23, NA = 55, NB = 55, NX = 20, and NY = 400, or
when n is 24, NA = 55, NB = 56, NX = 400, and NY = 50.

**2.** The antisense oligomer or the pharmaceutically acceptable salt thereof, or hydrate thereof according to claim 1, wherein

the region R1 is a region that consists of a base sequence of 20 bases in the upstream direction from the 3' end of the 44th exon and a base sequence of 400 bases in the downstream direction from the 5' end of the 44th intron in the human dystrophin pre-mRNA,
the region R2 is a region that consists of a base sequence of 600 bases in the upstream direction from the 3' end of the 44th intron and a base sequence of 50 bases in the downstream direction from the 5' end of the 45th exon in the human dystrophin pre-mRNA,
the region R3 is a region that consists of a base sequence of 20 bases in the upstream direction from the 3' end of the 45th exon and a base sequence of 400 bases in the downstream direction from the 5' end of the 45th intron in the human dystrophin pre-mRNA,
the region R4 is a region that consists of a base sequence of 400 bases in the upstream direction from the 3' end of the 45th intron and a base sequence of 50 bases in the downstream direction from the 5' end of the 46th exon in the human dystrophin pre-mRNA,
the region R5 is a region that consists of a base sequence of 20 bases in the upstream direction from the 3' end of the 46th exon and a base sequence of 400 bases in the downstream direction from the 5' end of the 46th intron in the human dystrophin pre-mRNA,
the region R6 is a region that consists of a base sequence of 400 bases in the upstream direction from the 3' end of the 46th intron and a base sequence of 50 bases in the downstream direction from the 5' end of the 47th exon in the human dystrophin pre-mRNA,
the region R7 is a region that consists of a base sequence of 20 bases in the upstream direction from the 3' end of the 47th exon and a base sequence of 400 bases in the downstream direction from the 5' end of the 47th intron in the human dystrophin pre-mRNA,
the region R8 is a region that consists of a base sequence of 400 bases in the upstream direction from the 3' end of the 47th intron and a base sequence of 50 bases in the downstream direction from the 5' end of the 48th exon in the human dystrophin pre-mRNA,
the region R9 is a region that consists of a base sequence of 20 bases in the upstream direction from the 3' end of the 48th exon and a base sequence of 400 bases in the downstream direction from the 5' end of the 48th intron in the human dystrophin pre-mRNA,
the region R10 is a region that consists of a base sequence of 400 bases in the upstream direction from the 3'

end of the 48th intron and a base sequence of 50 bases in the downstream direction from the 5' end of the 49th exon in the human dystrophin pre-mRNA,

the region R11 is a region that consists of a base sequence of 20 bases in the upstream direction from the 3' end of the 49th exon and a base sequence of 400 bases in the downstream direction from the 5' end of the 49th intron in the human dystrophin pre-mRNA,

the region R12 is a region that consists of a base sequence of 400 bases in the upstream direction from the 3' end of the 49th intron and a base sequence of 50 bases in the downstream direction from the 5' end of the 50th exon in the human dystrophin pre-mRNA,

the region R13 is a region that consists of a base sequence of 20 bases in the upstream direction from the 3' end of the 50th exon and a base sequence of 400 bases in the downstream direction from the 5' end of the 50th intron in the human dystrophin pre-mRNA,

the region R14 is a region that consists of a base sequence of 400 bases in the upstream direction from the 3' end of the 50th intron and a base sequence of 50 bases in the downstream direction from the 5' end of the 51st exon in the human dystrophin pre-mRNA,

the region R15 is a region that consists of a base sequence of 20 bases in the upstream direction from the 3' end of the 51st exon and a base sequence of 400 bases in the downstream direction from the 5' end of the 51st intron in the human dystrophin pre-mRNA,

the region R16 is a region that consists of a base sequence of 400 bases in the upstream direction from the 3' end of the 51st intron and a base sequence of 50 bases in the downstream direction from the 5' end of the 52nd exon in the human dystrophin pre-mRNA,

the region R17 is a region that consists of a base sequence of 20 bases in the upstream direction from the 3' end of the 52nd exon and a base sequence of 400 bases in the downstream direction from the 5' end of the 52nd intron in the human dystrophin pre-mRNA,

the region R18 is a region that consists of a base sequence of 400 bases in the upstream direction from the 3' end of the 52nd intron and a base sequence of 50 bases in the downstream direction from the 5' end of the 53rd exon in the human dystrophin pre-mRNA,

the region R19 is a region that consists of a base sequence of 20 bases in the upstream direction from the 3' end of the 53rd exon and a base sequence of 400 bases in the downstream direction from the 5' end of the 53rd intron in the human dystrophin pre-mRNA,

the region R20 is a region that consists of a base sequence of 400 bases in the upstream direction from the 3' end of the 53rd intron and a base sequence of 50 bases in the downstream direction from the 5' end of the 54th exon in the human dystrophin pre-mRNA,

the region R21 is a region that consists of a base sequence of 20 bases in the upstream direction from the 3' end of the 54th exon and a base sequence of 400 bases in the downstream direction from the 5' end of the 54th intron in the human dystrophin pre-mRNA,

the region R22 is a region that consists of a base sequence of 400 bases in the upstream direction from the 3' end of the 54th intron and a base sequence of 50 bases in the downstream direction from the 5' end of the 55th exon in the human dystrophin pre-mRNA,

the region R23 is a region that consists of a base sequence of 20 bases in the upstream direction from the 3' end of the 55th exon and a base sequence of 400 bases in the downstream direction from the 5' end of the 55th intron in the human dystrophin pre-mRNA, or

the region R24 is a region that consists of a base sequence of 400 bases in the upstream direction from the 3' end of the 55th intron and a base sequence of 50 bases in the downstream direction from the 5' end of the 56th exon in the human dystrophin pre-mRNA.

**3.** The antisense oligomer or the pharmaceutically acceptable salt thereof, or hydrate thereof according to claim 1 or 2, wherein

the antisense oligomer comprises a base sequence complementary to

(a) any one base sequence selected from the group consisting of SEQ ID NOs: 233 to 256, 341 to 369, and 385 to 389,

(b) a base sequence that hybridizes under stringent conditions to a base sequence complementary to any one base sequence selected from the group consisting of SEQ ID NOs: 233 to 256, 341 to 369, and 385 to 389,

(c) a base sequence that has at least 85% identity with any one base sequence selected from the group consisting of SEQ ID NOs: 233 to 256, 341 to 369, and 385 to 389, and has a length within ±15% of the length of the any one base sequence selected, or

(d) a partial base sequence of any one base sequence selected from the group consisting of the base sequences (a), (b), and (c) .

4. The antisense oligomer or the pharmaceutically acceptable salt thereof, or hydrate thereof according to any one of claims 1 to 3, wherein

the antisense oligomer is an antisense oligomer comprising two or more unit oligomers linked to each other, wherein
each of the unit oligomers comprises a base sequence complementary to a base sequence of any one region selected from the group consisting of the regions R1 to R24, or a partial base sequence thereof, and the respective base sequences of the unit oligomers are neither consecutive nor overlapped with each other.

5. The antisense oligomer or the pharmaceutically acceptable salt thereof, or hydrate thereof according to any one of claims 1 to 3, wherein

the antisense oligomer is an antisense oligomer comprising two or more unit oligomers linked to each other, wherein
each of the unit oligomers comprises a base sequence complementary to

(a) any one base sequence selected from the group consisting of SEQ ID NOs: 233 to 256, 341 to 369, and 385 to 389,
(b) a base sequence that hybridizes under stringent conditions to a base sequence complementary to any one base sequence selected from the group consisting of SEQ ID NOs: 233 to 256, 341 to 369, and 385 to 389,
(c) a base sequence that has at least 85% identity with any one base sequence selected from the group consisting of SEQ ID NOs: 233 to 256, 341 to 369, and 385 to 389, and has a length within ±15% of the length of the any one base sequence selected, or
(d) a partial base sequence of any one base sequence selected from the group consisting of the base sequences (a), (b), and (c),

and the respective base sequences of the unit oligomers are neither consecutive nor overlapped with each other.

6. The antisense oligomer or the pharmaceutically acceptable salt thereof, or hydrate thereof according to claim 4 or 5, wherein each of the unit oligomers comprises a base sequence complementary to a consecutive base sequence of 5- to 20-base length in the region.

7. The antisense oligomer or the pharmaceutically acceptable salt thereof, or hydrate thereof according to any one of claims 1 to 3, wherein the antisense oligomer consists of

(1) any one base sequence selected from the group consisting of SEQ ID NOs: 1 to 111, 113 to 177, 179, 180, 182, 183, 185 to 193, 195 to 198, 200 to 223, and 225 to 232, or
(2) a base sequence that has at least 85% identity with any one base sequence selected from the group consisting of SEQ ID NOs: 1 to 111, 113 to 177, 179, 180, 182, 183, 185 to 193, 195 to 198, 200 to 223, and 225 to 232, and has a length within ±15% of the length of the any one base sequence selected.

8. The antisense oligomer or the pharmaceutically acceptable salt thereof, or hydrate thereof according to any one of claims 1 to 3 and 7, wherein the antisense oligomer consists of any one base sequence selected from the group consisting of SEQ ID NOs: 1, 5, 6, 7, 8, 10, 11, 14, 26, 27, 28, 29, 35, 38, 39, 40, 41, 43, 45, 46, 47, 50, 51, 52, 53, 54, 55, 58, 59, 63, 64, 65, 66, 67, 68, 74, 75, 76, 77, 78, 80, 82, 86, 92, 97, 98, 100, 102, 113, 119, 121, 122, 124, 125, 126, 128, 130, 131, 132, 139, 142, 144, 146, 147, 150, 151, 152, 153, 154, 155, 156, 157, 158, 159, 160, 161, 162, 163, 225, 228, 231, and 232.

9. The antisense oligomer or the pharmaceutically acceptable salt thereof, or hydrate thereof according to any one of claims 1 to 3, 7 and 8, wherein the antisense oligomer consists of any one base sequence selected from the group consisting of SEQ ID NOs: 1, 5, 11, 27, 28, 35, 40, 41, 43, 45, 46, 47, 50, 51, 52, 54, 55, 59, 63, 64, 65, 66, 68, 74, 75, 76, 77, 78, 102, 113, 119, 128, 131, 132, 139, 142, 161, 162, 163, 225, and 228.

10. The antisense oligomer or the pharmaceutically acceptable salt thereof, or hydrate thereof according to any one of claims 1 to 9, wherein the antisense oligomer is an oligonucleotide.

11. The antisense oligomer or the pharmaceutically acceptable salt thereof, or hydrate thereof according to claim 10,

wherein the sugar moiety and/or the phosphate bond moiety of at least one nucleotide constituting the oligonucleotide is modified.

**12.** The antisense oligomer or the pharmaceutically acceptable salt thereof, or hydrate thereof according to claim 10 or 11, wherein the sugar moiety of at least one nucleotide constituting the oligonucleotide is a ribose in which the 2'-OH group is replaced by any one selected from the group consisting of -OR, -R, -R'OR, -SH, -SR, - $NH_2$, -NHR, -$NR_2$, -$N_3$, -CN, -F, -Cl, -Br, and -I (wherein R is an alkyl or an aryl and R' is an alkylene).

**13.** The antisense oligomer or the pharmaceutically acceptable salt thereof, or hydrate thereof according to any one of claims 10 to 12, wherein the phosphate bond moiety of at least one nucleotide constituting the oligonucleotide is any one selected from the group consisting of a phosphorothioate bond, a phosphorodithioate bond, an alkylphosphonate bond, a phosphoramidate bond and a boranophosphate bond.

**14.** The antisense oligomer or the pharmaceutically acceptable salt thereof, or hydrate thereof according to any one of claims 1 to 9, wherein the antisense oligomer is a morpholino oligomer.

**15.** The antisense oligomer or the pharmaceutically acceptable salt thereof, or hydrate thereof according to claim 14, wherein the antisense oligomer is a phosphorodiamidate morpholino oligomer.

**16.** The antisense oligomer or the pharmaceutically acceptable salt thereof, or hydrate thereof according to claim 14 or 15, wherein the 5' end is any one of chemical formulae (1) to (3) below:

[Formula 15]

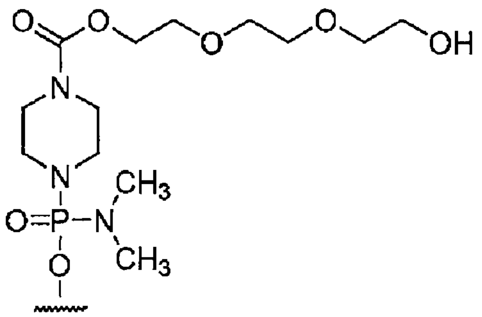

(1)

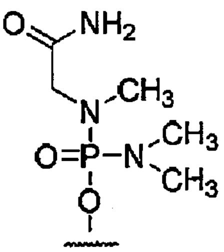

(2)

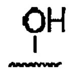

(3)

**17.** A suppressor antisense oligomer or a pharmaceutically acceptable salt thereof, or hydrate thereof which suppresses single skipping of any one exon selected from the group consisting of the 45th exon to the 55th exon in human dystrophin pre-mRNA,
the suppressor antisense oligomer comprising a base sequence complementary to

(a) any one base sequence of a base sequence selected from the group consisting of SEQ ID NOs: 370 to 384,
(b) a base sequence that has at least 85% identity with any one base sequence of a base sequence selected from the group consisting of SEQ ID NOs: 370 to 384, and has a length within ±15% of the length of the any one base sequence selected, or
(c) a partial base sequence of the base sequence (a) or (b) .

**18.** The suppressor antisense oligomer or the pharmaceutically acceptable salt thereof, or hydrate thereof according to claim 17, wherein the suppressor antisense oligomer consists of
(1) any one base sequence selected from the group consisting of SEQ ID NOs: 257 to 275, or (2) a base sequence that has at least 85% identity with any one base sequence selected from the group consisting of SEQ ID NOs: 257 to 275, and has a length within ±15% of the length of the any one base sequence selected.

**19.** The suppressor antisense oligomer or the pharmaceutically acceptable salt thereof, or hydrate thereof according to claim 17 or 18, wherein the suppressor antisense oligomer consists of any one base sequence selected from the

group consisting of SEQ ID NOs: 260, 261, and 263.

**20.** The suppressor antisense oligomer or the pharmaceutically acceptable salt thereof, or hydrate thereof according to any one of claims 17 to 19, wherein the suppressor antisense oligomer is an oligonucleotide.

**21.** The suppressor antisense oligomer or the pharmaceutically acceptable salt thereof, or hydrate thereof according to claim 20, wherein the sugar moiety and/or the phosphate bond moiety of at least one nucleotide constituting the oligonucleotide is modified.

**22.** The suppressor antisense oligomer or the pharmaceutically acceptable salt thereof, or hydrate thereof according to claim 20 or 21, wherein the sugar moiety of at least one nucleotide constituting the oligonucleotide is a ribose in which the 2'-OH group is replaced by any one selected from the group consisting of -OR, -R, -R'OR, -SH, -SR, $-NH_2$, -NHR, $-NR_2$, $-N_3$, -CN, -F, -Cl, -Br, and -I
(wherein R is an alkyl or an aryl and R' is an alkylene).

**23.** The suppressor antisense oligomer or the pharmaceutically acceptable salt thereof, or hydrate thereof according to any one of claims 20 to 22, wherein the phosphate bond moiety of at least one nucleotide constituting the oligonucleotide is any one selected from the group consisting of a phosphorothioate bond, a phosphorodithioate bond, an alkylphosphonate bond, a phosphoramidate bond and a boranophosphate bond.

**24.** The suppressor antisense oligomer or the pharmaceutically acceptable salt thereof, or hydrate thereof according to any one of claims 17 to 19, wherein the suppressor antisense oligomer is a morpholino oligomer.

**25.** The suppressor antisense oligomer or the pharmaceutically acceptable salt thereof, or hydrate thereof according to claim 24, wherein the suppressor antisense oligomer is a phosphorodiamidate morpholino oligomer.

**26.** The suppressor antisense oligomer or the pharmaceutically acceptable salt thereof, or hydrate thereof according to claim 24 or 25, wherein the 5' end is any one of chemical formulae (1) to (3) below:

[Formula 16]

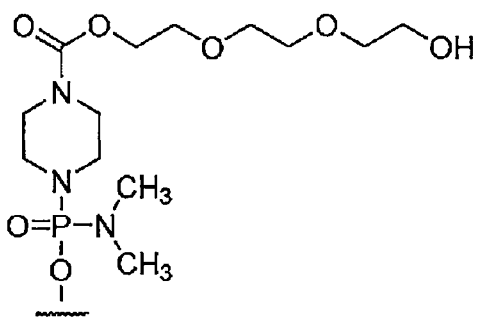

(1)

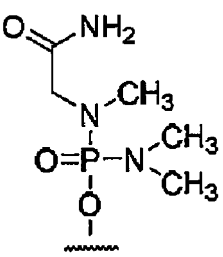

(2)

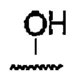

(3)

**27.** A pharmaceutical composition comprising the antisense oligomer or the pharmaceutically acceptable salt thereof, or hydrate thereof according to any one of claims 1 to 16.

**28.** The pharmaceutical composition according to claim 27, further comprising the suppressor antisense oligomer or the pharmaceutically acceptable salt thereof, or hydrate thereof according to any one of claims 17 to 26.

**29.** A pharmaceutical composition comprising the antisense oligomer or the pharmaceutically acceptable salt thereof, or hydrate thereof according to any one of claims 1 to 16, and the suppressor antisense oligomer or the pharmaceutically acceptable salt thereof, or hydrate thereof according to any one of claims 17 to 26.

**30.** The pharmaceutical composition according to claim 28 or 29, wherein

(1) the antisense oligomer is an oligomer consisting of SEQ ID NO: 75, and the suppressor antisense oligomer

is an oligomer consisting of SEQ ID NO: 260,
(2) the antisense oligomer is an oligomer consisting of SEQ ID NO: 75, and the suppressor antisense oligomer is an oligomer consisting of SEQ ID NO: 261, or
(3) the antisense oligomer is an oligomer consisting of SEQ ID NO: 75, and the suppressor antisense oligomer is an oligomer consisting of SEQ ID NO: 263.

**31.** The pharmaceutical composition according to any one of claims 27 to 30, further comprising a pharmaceutically acceptable carrier.

**32.** The pharmaceutical composition according to any one of claims 27 to 31 for treatment of muscular dystrophy.

**33.** The pharmaceutical composition according to any one of claims 27 to 32 for being administered to a human patient.

**34.** A method for treatment of muscular dystrophy, which comprises administering to a patient with muscular dystrophy the antisense oligomer or the pharmaceutically acceptable salt thereof, or hydrate thereof according to any one of claims 1 to 16, or the pharmaceutical composition according to any one of claims 27 to 33.

**35.** The method for treatment according to claim 34, wherein the patient with muscular dystrophy is a patient with a mutation that is amenable to exon 45 to 55 skipping in the dystrophin gene.

**36.** The method for treatment according to claim 34 or 35, wherein the patient is a human.

**37.** Use of the antisense oligomer or the pharmaceutically acceptable salt thereof, or hydrate thereof according to any one of claims 1 to 16, or the pharmaceutical composition according to any one of claims 27 to 33 in manufacturing of a medicament for the treatment of muscular dystrophy.

**38.** The antisense oligomer or the pharmaceutically acceptable salt thereof, or hydrate thereof according to any one of claims 1 to 16, or the pharmaceutical composition according to any one of claims 27 to 33 for use in the treatment of muscular dystrophy.

**39.** The antisense oligomer or the pharmaceutically acceptable salt thereof, or hydrate thereof, or the pharmaceutical composition according to claim 38, wherein the treatment involves performing skipping of any two or more numerically consecutive exons selected from the group consisting of the 45th exon to the 55th exon in human dystrophin pre-mRNA.

**40.** The antisense oligomer or the pharmaceutically acceptable salt thereof, or hydrate thereof, or the pharmaceutical composition according to claim 38 or 39, wherein in the treatment, a patient with muscular dystrophy is a human.

**41.** A method for enhancing the efficiency of skipping of two or more numerically consecutive exons, which comprises inhibiting a splicing silencer sequence, a splice site sequence, or a branch site sequence of pre-mRNA of interest when the two or more numerically consecutive exons are skipped from the pre-mRNA of interest.

**42.** The method according to claim 41, wherein the splicing silencer sequence is a recognition sequence of heterogeneous nuclear ribonucleoprotein A1 (hnRNPAl).

**43.** The method according to claim 41 or 42, wherein the pre-mRNA of interest is human dystrophin pre-mRNA.

**44.** The method according to any one of claims 41 to 43, wherein the two or more numerically consecutive exons are selected from the group consisting of the 45th exon to the 55th exon in human dystrophin pre-mRNA.

**45.** The method according to claim 44, wherein the skipping of the two or more numerically consecutive exons of the pre-mRNA of interest is performed using the antisense oligomer or the pharmaceutically acceptable salt thereof, or hydrate thereof according to any one of claims 1 to 16.

**46.** The method according to claim 44 or 45, wherein specific inhibition of the splicing silencer sequence, the splice site sequence, or the branch site sequence is performed using the suppressor antisense oligomer or the pharmaceutically acceptable salt thereof, or hydrate thereof according to any one of claims 17 to 26.

Fig. 1

A

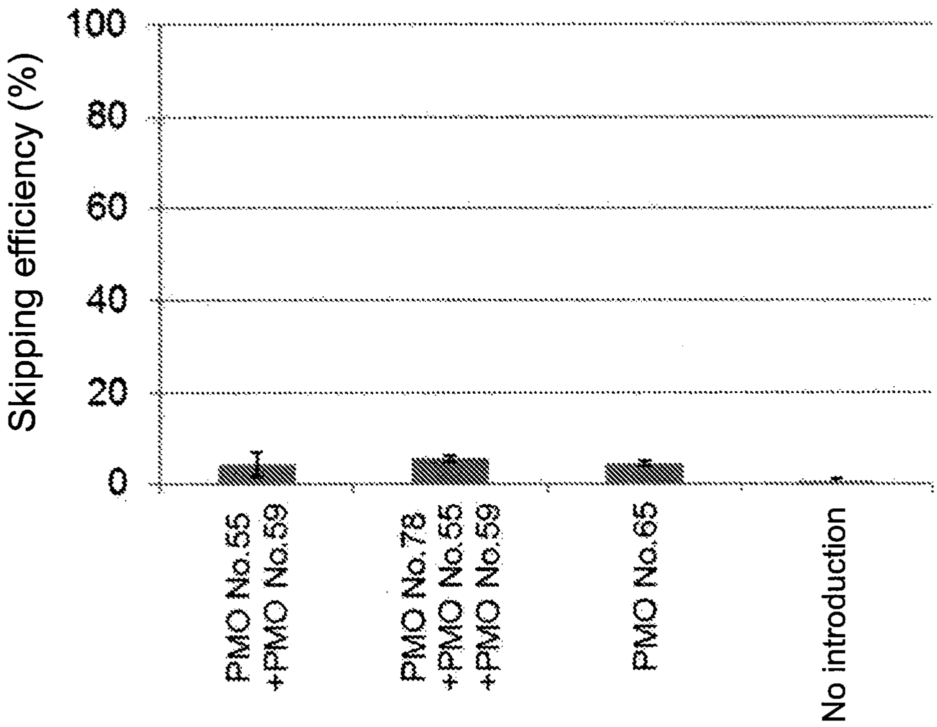

100
80
60
40
20
0
Skipping efficiency (%)
PMO No.55 +PMO No.59
PMO No.78 +PMO No.55 +PMO No.59
PMO No.65
No introduction


B

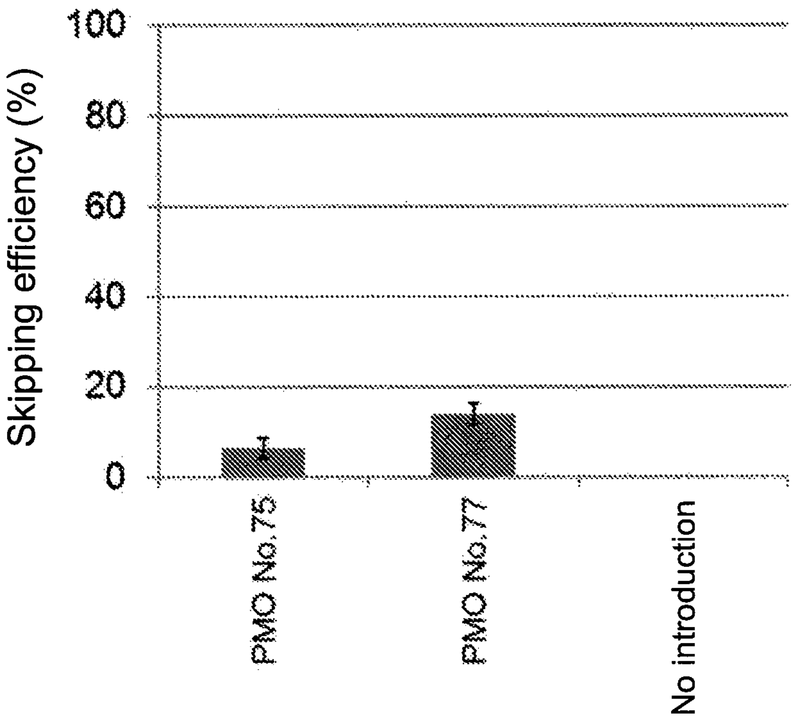

100
80
60
40
20
0
Skipping efficiency (%)
PMO No.75
PMO No.77
No introduction

Fig. 2

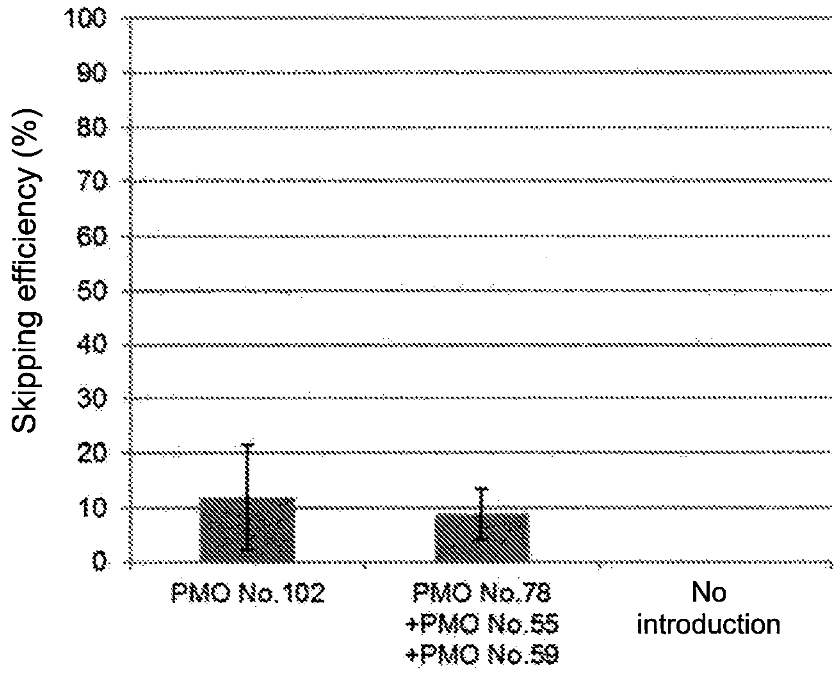
Skipping efficiency (%)
100
90
80
70
60
50
40
30
20
10
0
PMO No.102
PMO No.78
+PMO No.55
+PMO No.59
No
introduction

Fig. 3

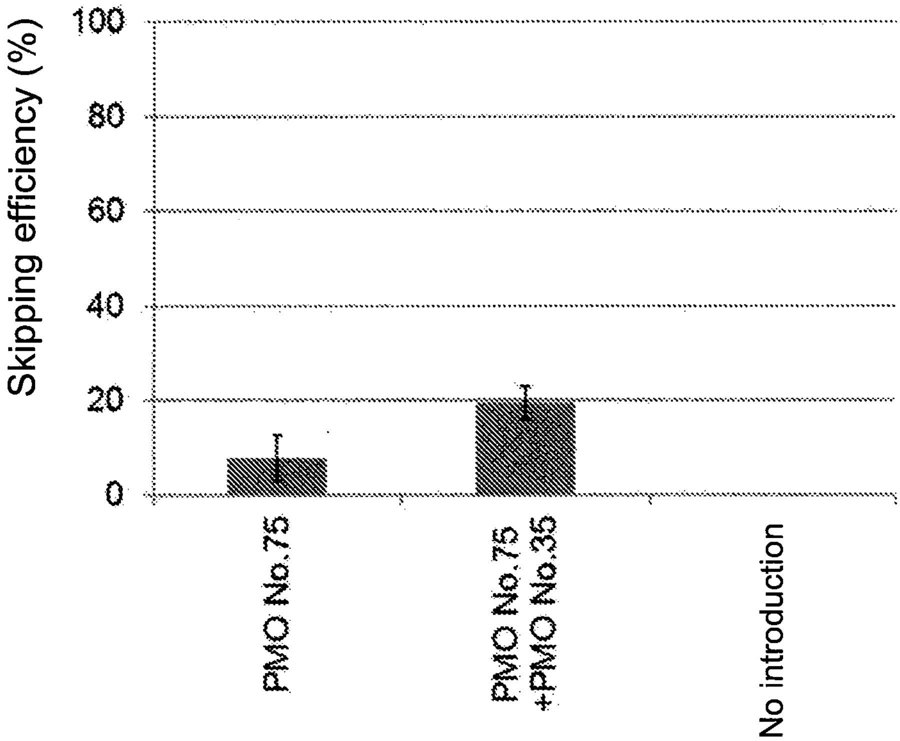
Skipping efficiency (%)
100
80
60
40
20
0
PMO No.75
PMO No.75
+PMO No.35
No introduction

Fig. 4

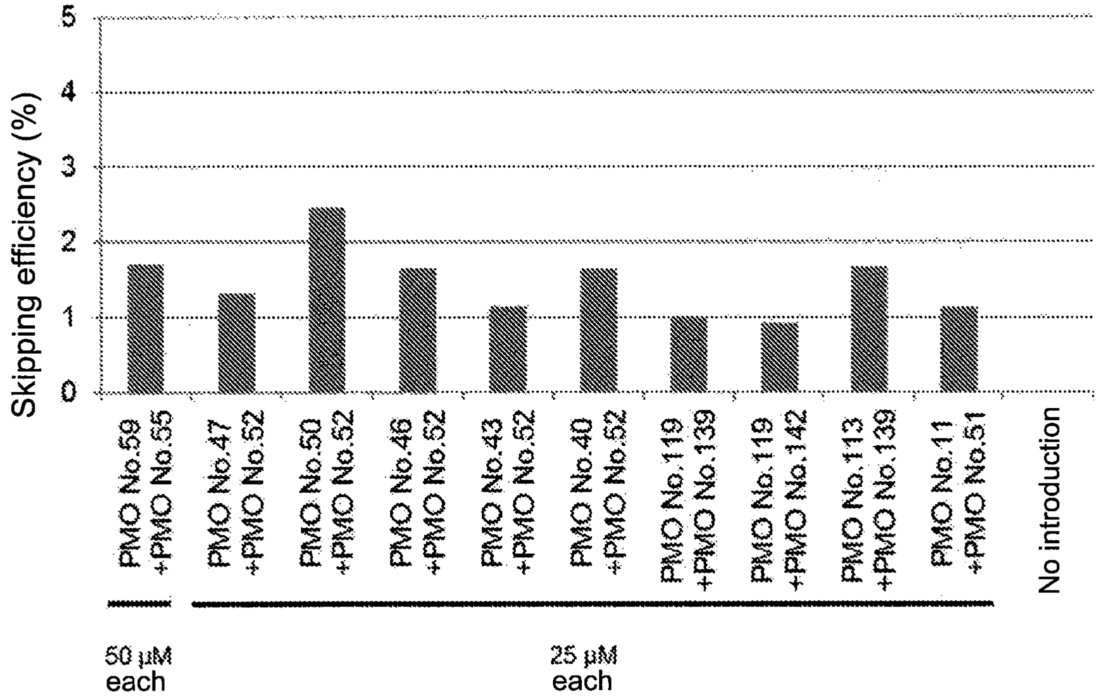
Skipping efficiency (%)
5
4
3
2
1
0
PMO No.59 +PMO No.55
PMO No.47 +PMO No.52
PMO No.50 +PMO No.52
PMO No.46 +PMO No.52
PMO No.43 +PMO No.52
PMO No.40 +PMO No.52
PMO No.119 +PMO No.139
PMO No.119 +PMO No.142
PMO No.113 +PMO No.139
PMO No.11 +PMO No.51
No introduction
50 µM each
25 µM each

Fig. 5

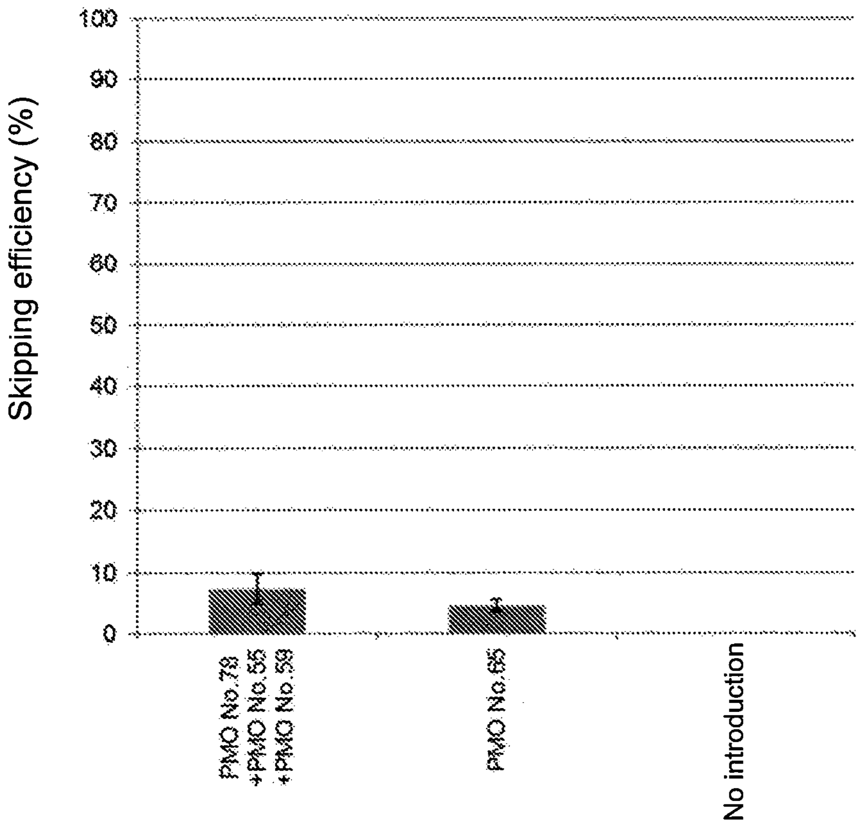
Skipping efficiency (%)
100
90
80
70
60
50
40
30
20
10
0
PMO No.78 +PMO No.55 +PMO No.59
PMO No.65
No introduction

Fig. 6

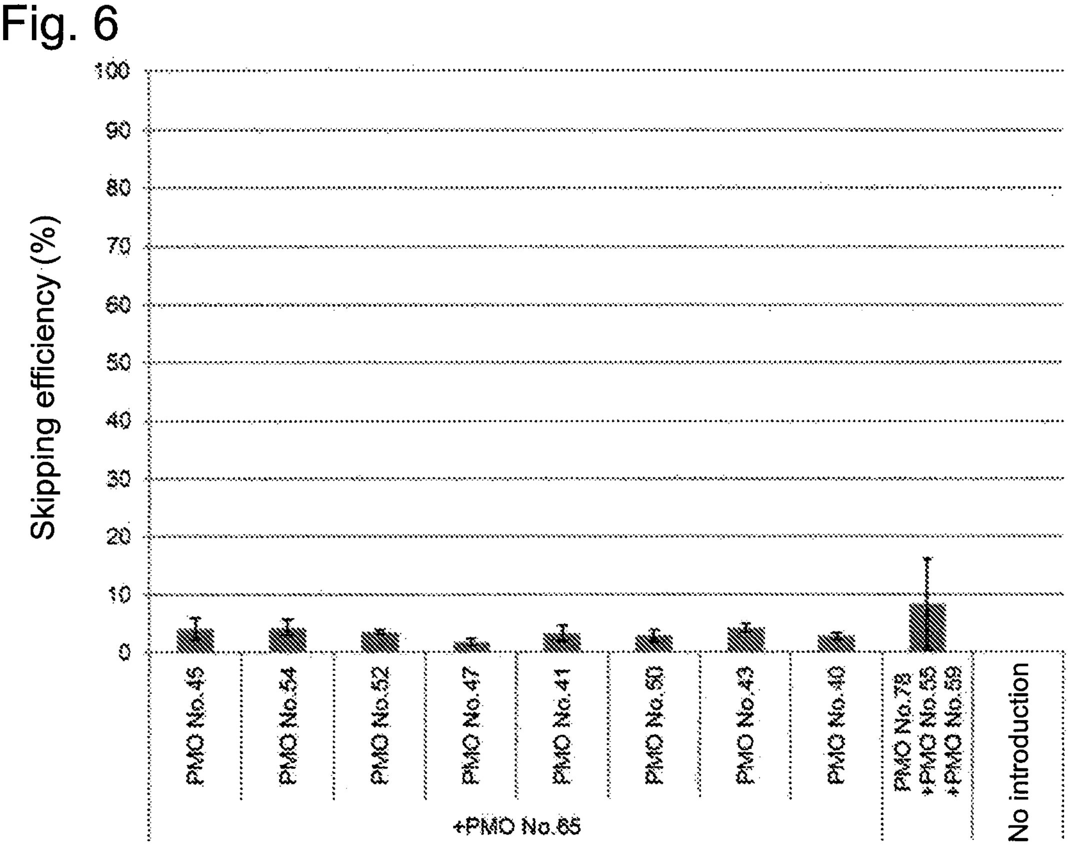
Skipping efficiency (%)
100
90
80
70
60
50
40
30
20
10
0
PMO No.45
PMO No.54
PMO No.52
PMO No.47
PMO No.41
PMO No.50
PMO No.43
PMO No.40
+PMO No.65
PMO No.78 +PMO No.55 +PMO No.59
No introduction

Fig. 7

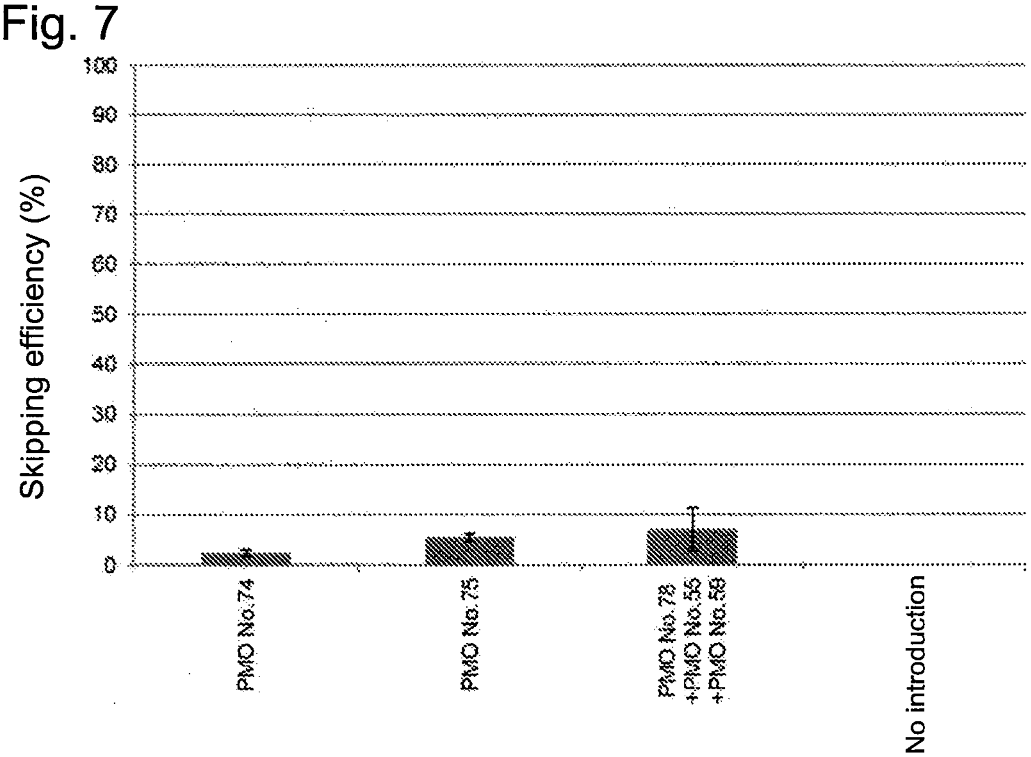
Skipping efficiency (%)
100
90
80
70
60
50
40
30
20
10
0
PMO No.74
PMO No.75
PMO No.78 +PMO No.55 +PMO No.59
No introduction

Fig. 8

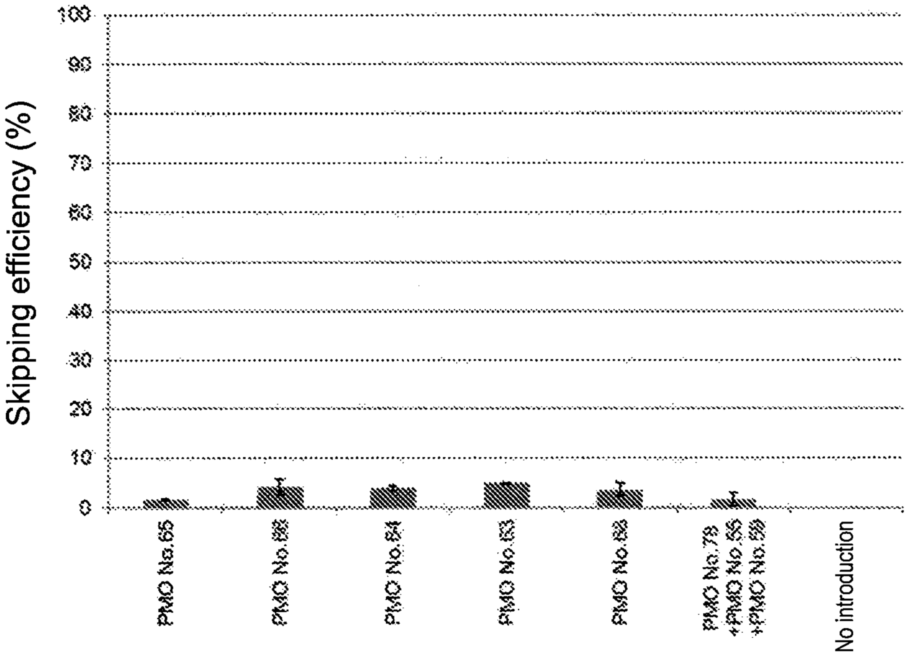
Skipping efficiency (%)
100
90
80
70
60
50
40
30
20
10
0
PMO No.65
PMO No.64
PMO No.63
No introduction

Fig. 9

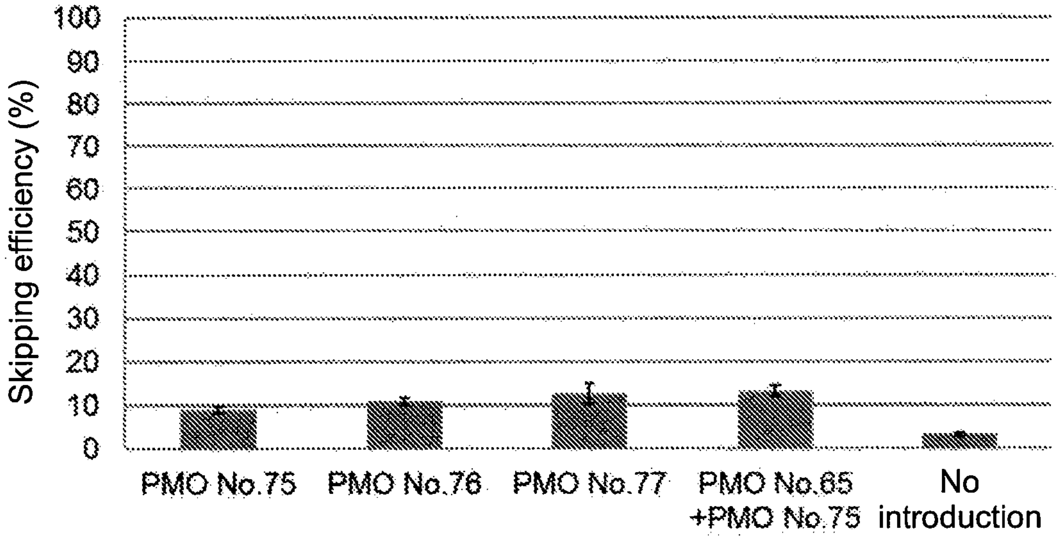
Skipping efficiency (%)
100
90
80
70
60
50
40
30
20
10
0
PMO No.75
PMO No.76
PMO No.77
PMO No.65 +PMO No.75
No introduction

Fig. 10

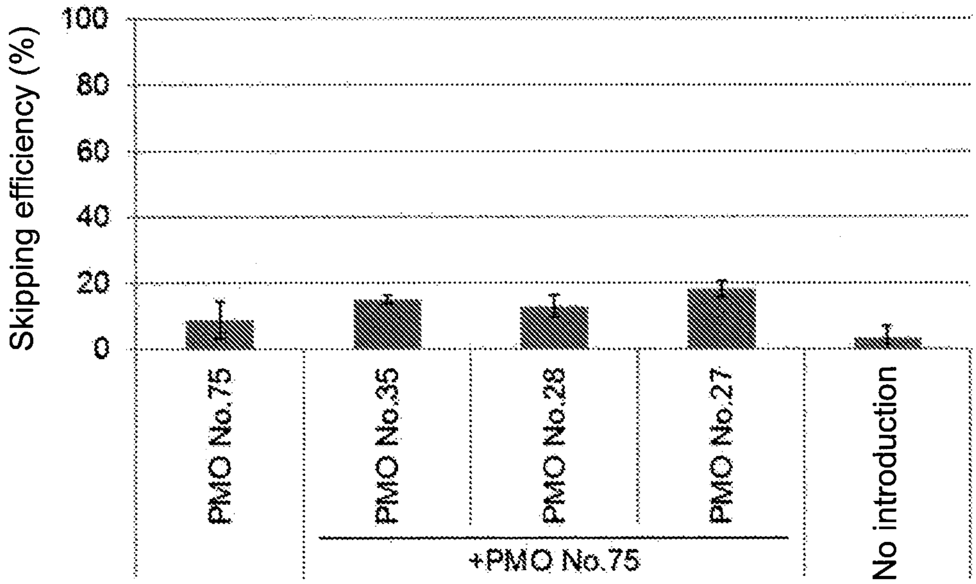
Skipping efficiency (%)
100
80
60
40
20
0
PMO No.75
PMO No.35
PMO No.28
PMO No.27
+PMO No.75
No introduction

Fig. 11

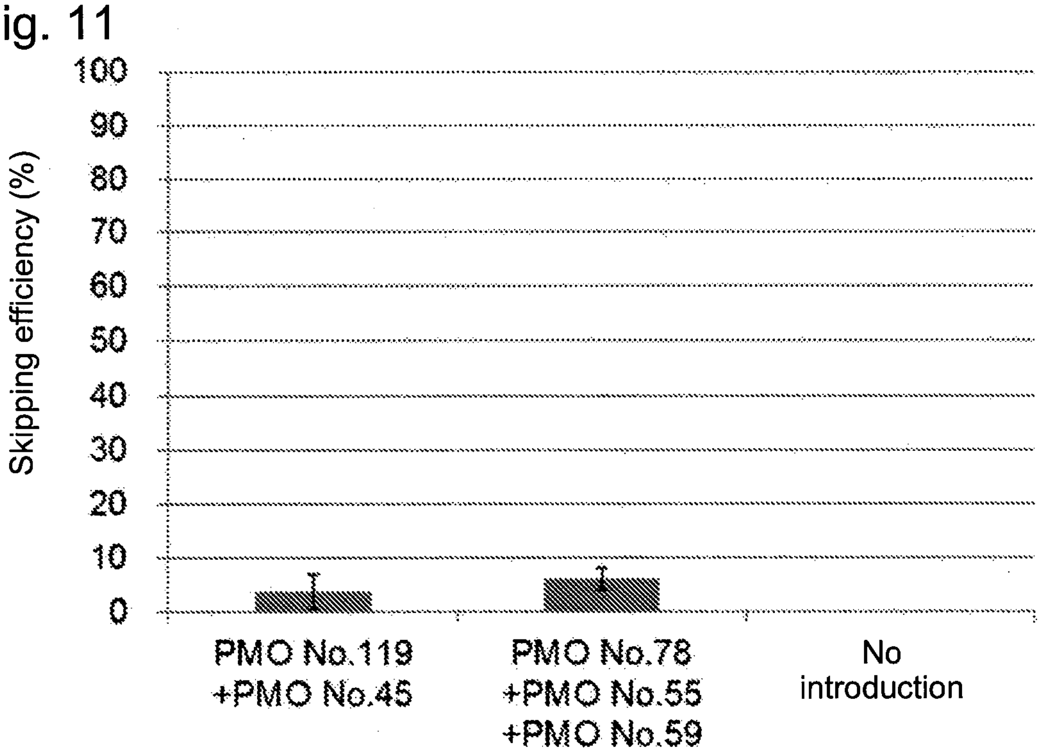
Skipping efficiency (%)
100
90
80
70
60
50
40
30
20
10
0
PMO No.119
+PMO No.45
PMO No.78
+PMO No.55
+PMO No.59
No
introduction

Fig. 12

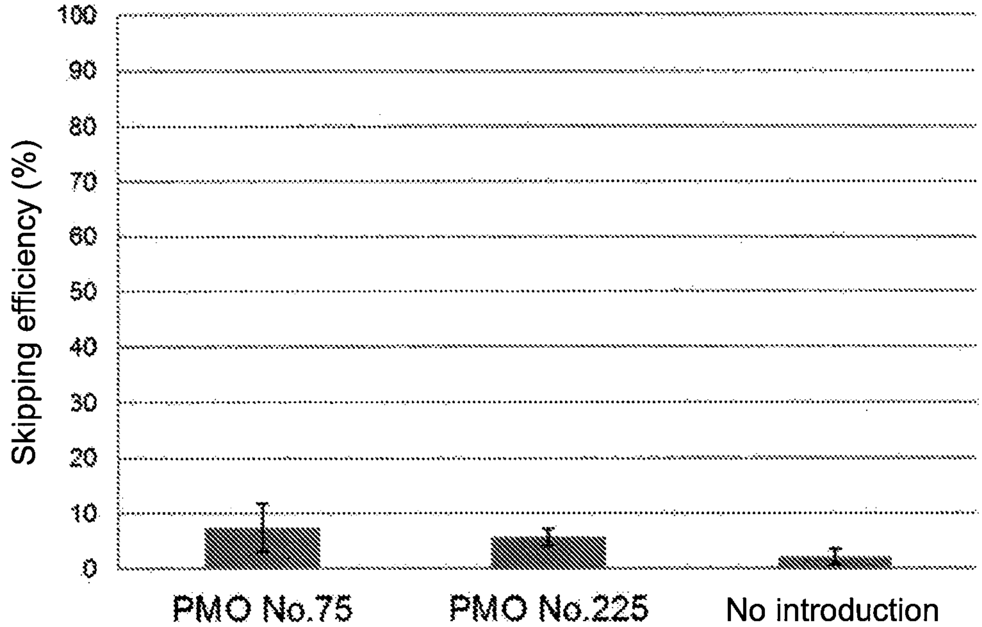
Skipping efficiency (%)
100
90
80
70
60
50
40
30
20
10
0
PMO No.75
PMO No.225
No introduction

Fig. 13

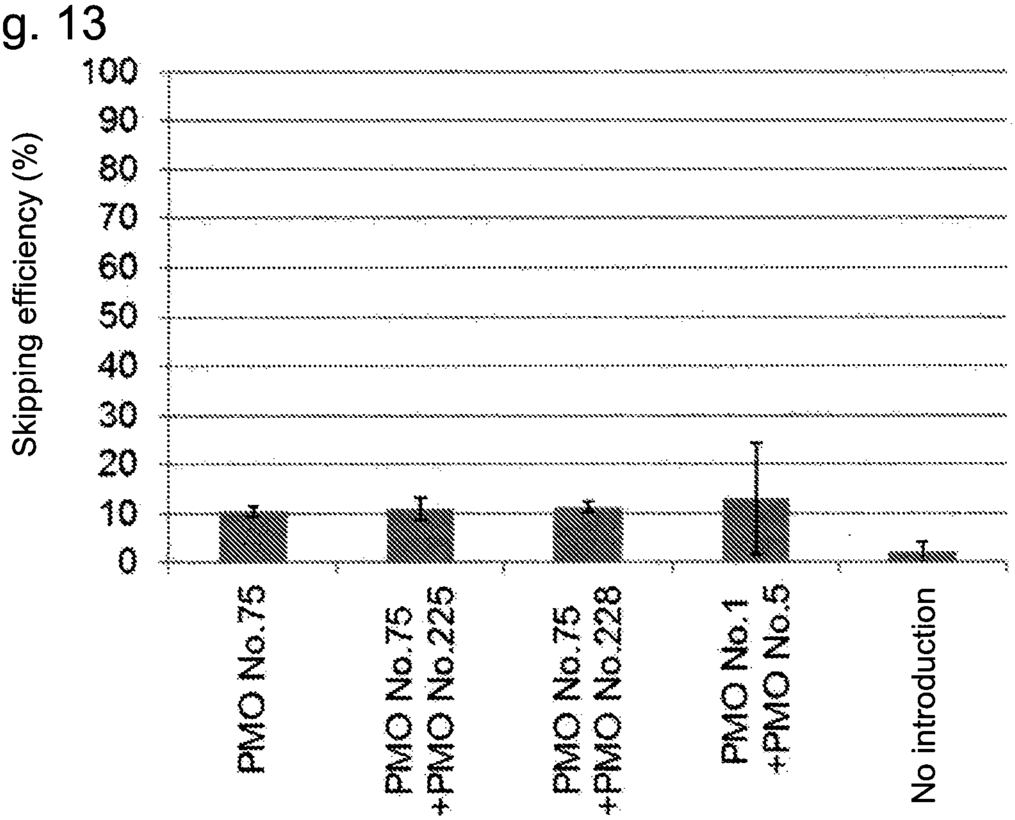
Skipping efficiency (%)
100
90
80
70
60
50
40
30
20
10
0
PMO No.75
PMO No.75 +PMO No.225
PMO No.75 +PMO No.228
PMO No.1 +PMO No.5
No introduction

Fig. 14

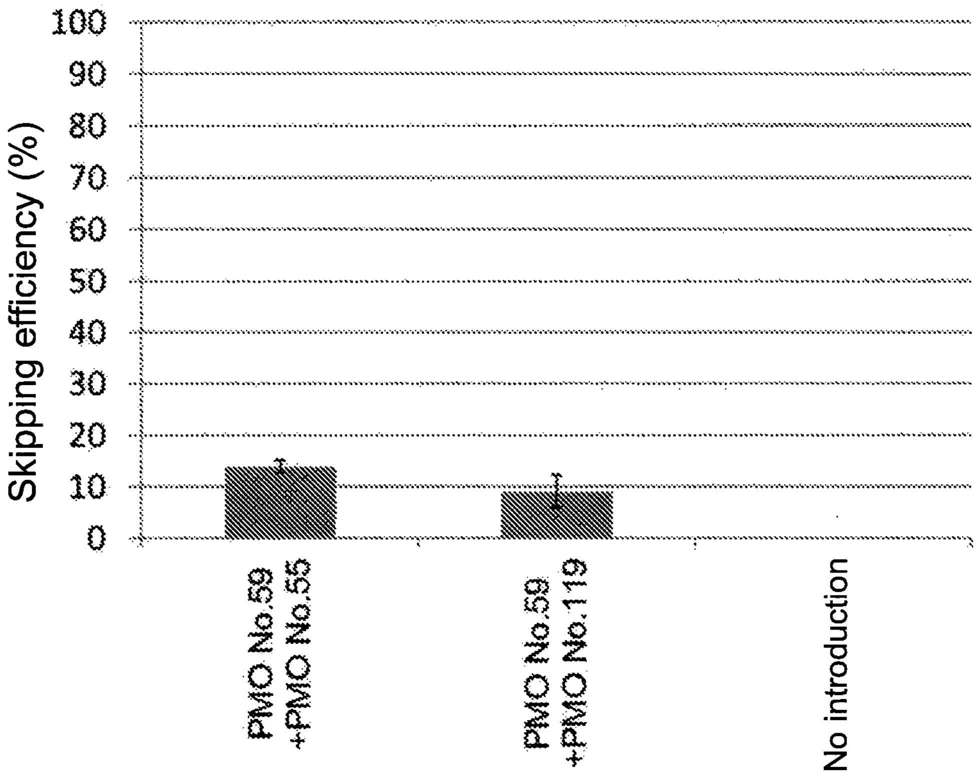
Skipping efficiency (%)
100
90
80
70
60
50
40
30
20
10
0
PMO No.59
+PMO No.55
PMO No.59
+PMO No.119
No introduction

Fig. 15

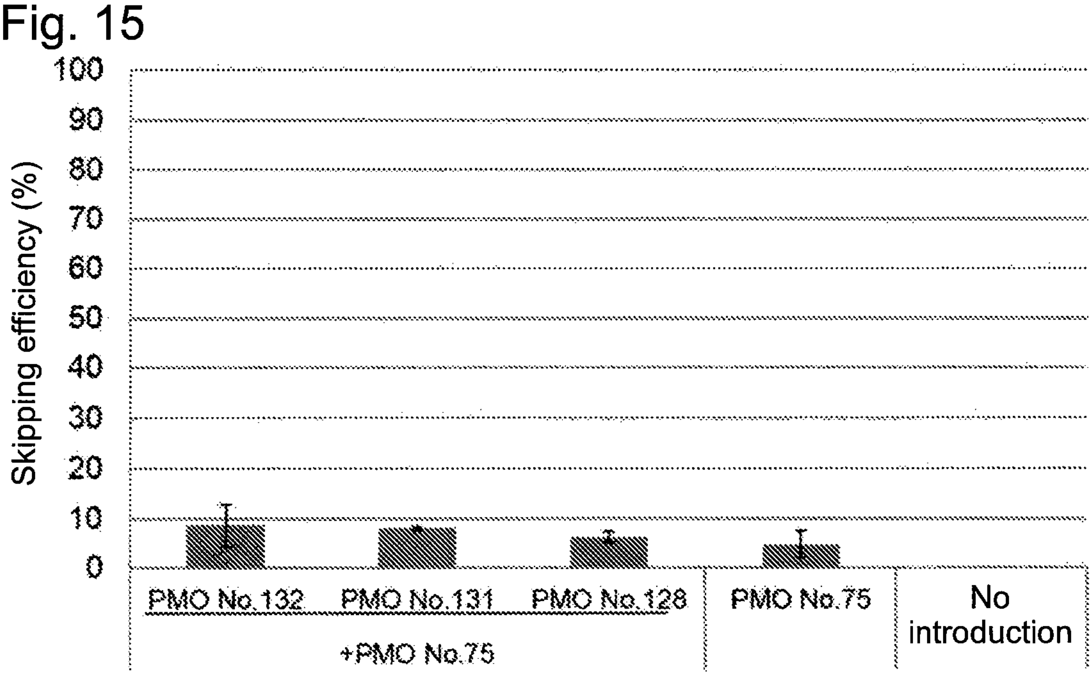
Skipping efficiency (%)
100
90
80
70
60
50
40
30
20
10
0
PMO No.132
PMO No.131
PMO No.128
+PMO No.75
PMO No.75
No
introduction

Fig. 16

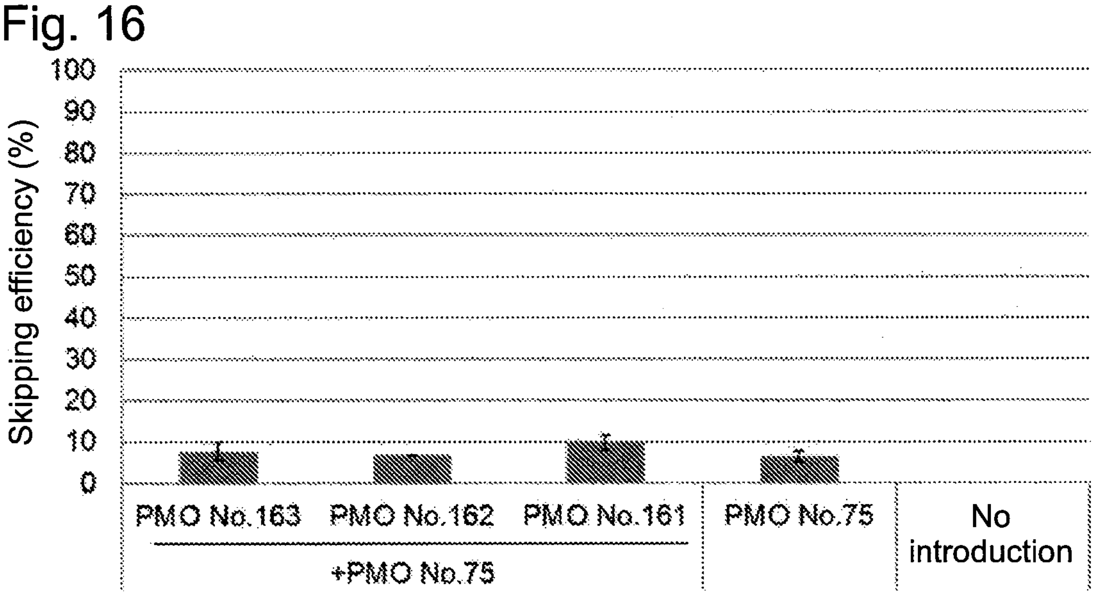
Skipping efficiency (%)
100
90
80
70
60
50
40
30
20
10
0
PMO No.163
PMO No.162
PMO No.161
+PMO No.75
PMO No.75
No introduction

Fig. 17

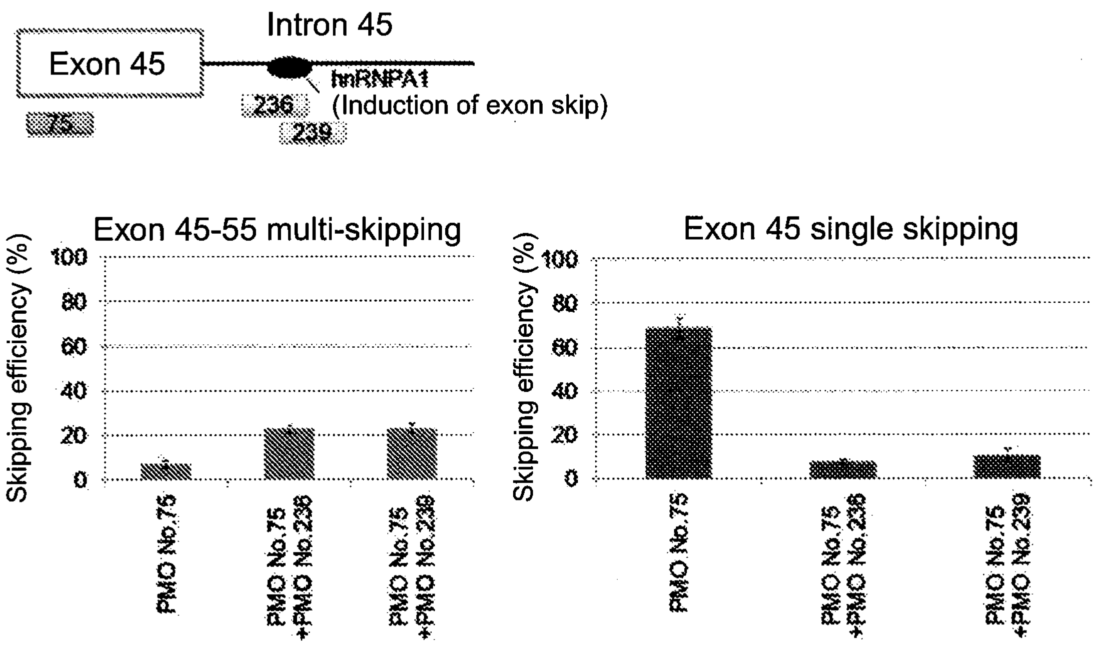
Exon 45
Intron 45
hnRNPA1
(Induction of exon skip)
75
236
239
Exon 45-55 multi-skipping
Exon 45 single skipping
Skipping efficiency (%)
100
80
60
40
20
0
PMO No.75
PMO No.75 +PMO No.236
PMO No.75 +PMO No.239

Fig. 18

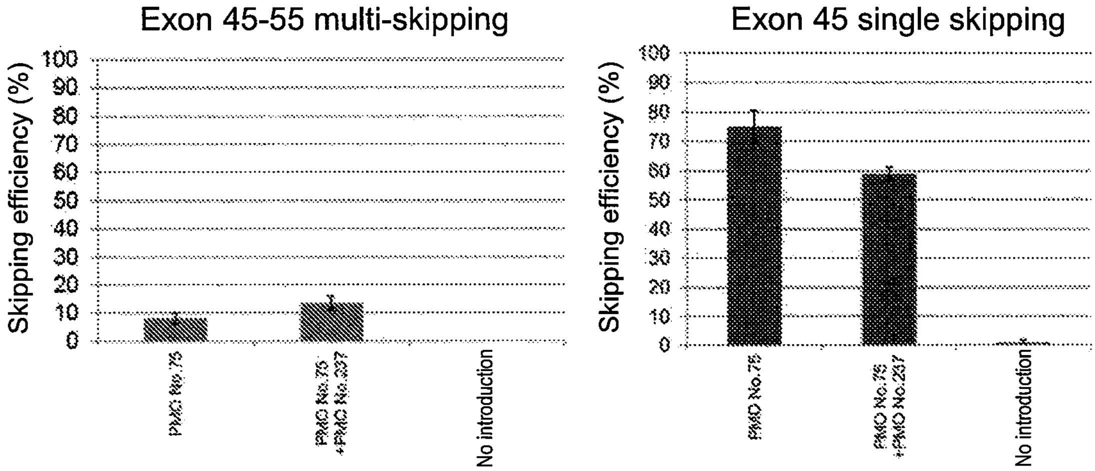
Exon 45-55 multi-skipping
Exon 45 single skipping
Skipping efficiency (%)
100
90
80
70
60
50
40
30
20
10
0
PMO No.75
PMO No.75 +PMO No.237
No introduction

Fig. 19

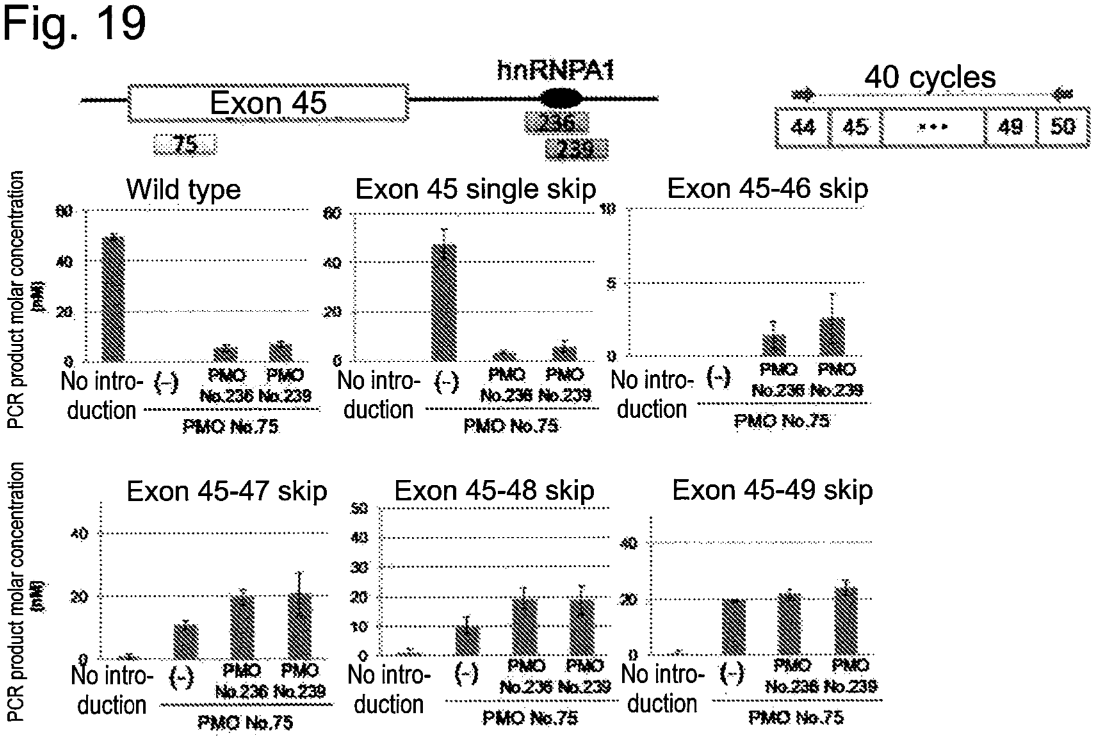
hnRNPA1
Exon 45
75
236
239
40 cycles
44
45
49
50
PCR product molar concentration (nM)
Wild type
Exon 45 single skip
Exon 45-46 skip
Exon 45-47 skip
Exon 45-48 skip
Exon 45-49 skip
No intro-duction
(-)
PMO No.236
PMO No.239
PMO No.75

Fig. 20

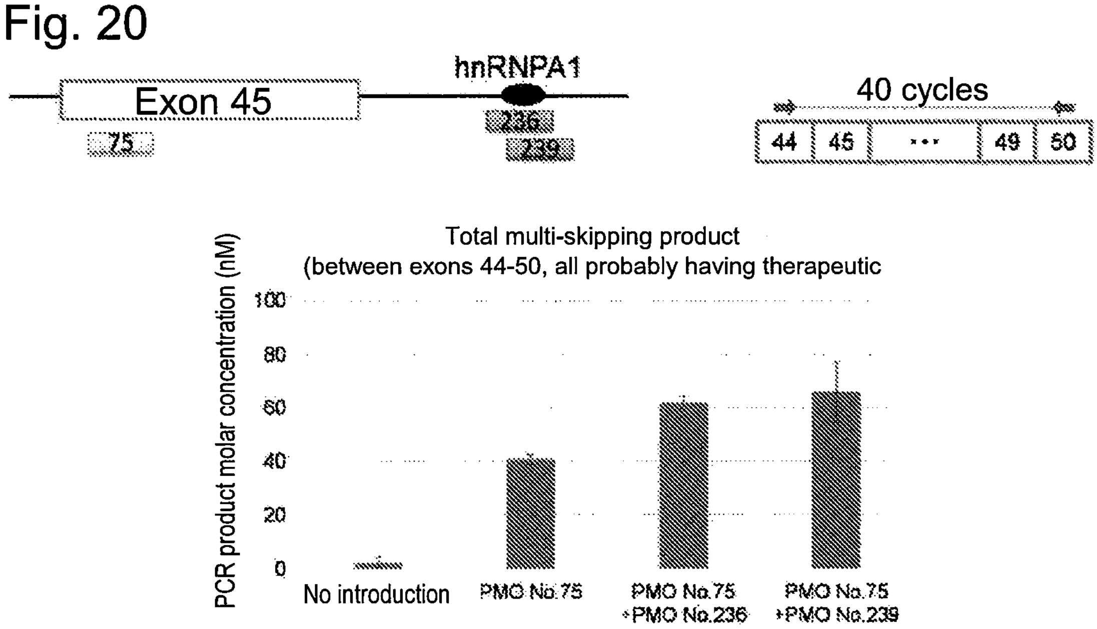


*Total of exon 45-46, exon 45-47, exon 45-48, and exon 45-49 skipping

Fig. 21

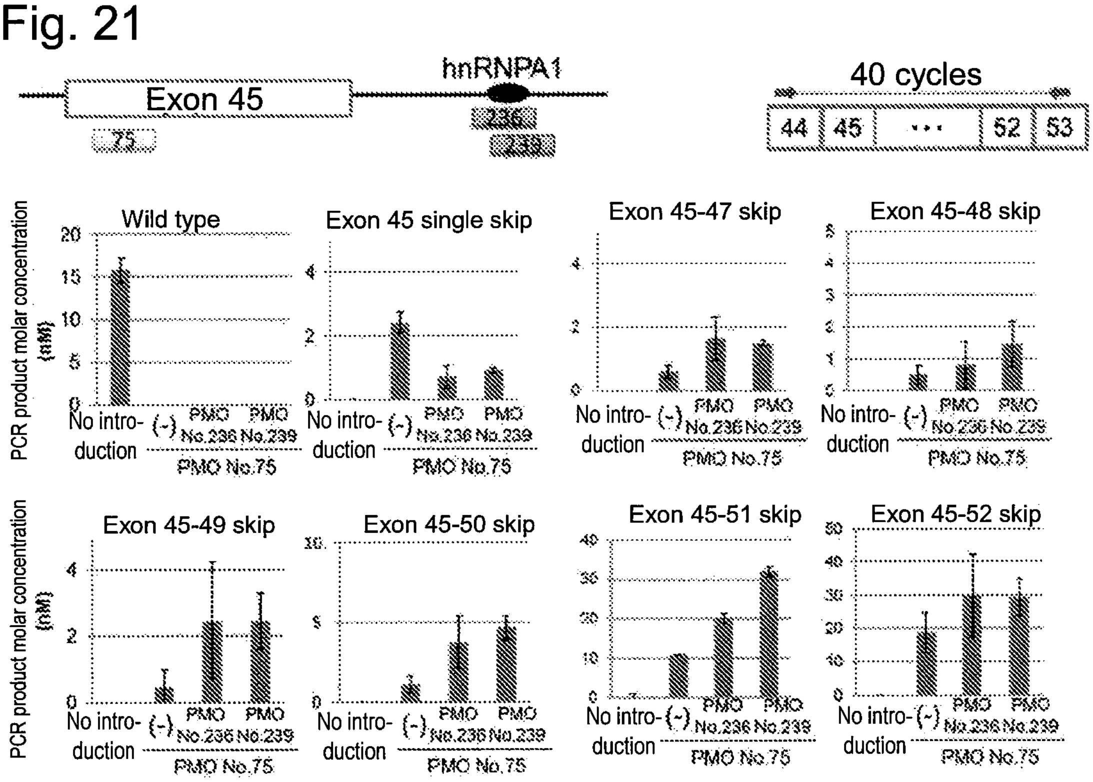

Fig. 22

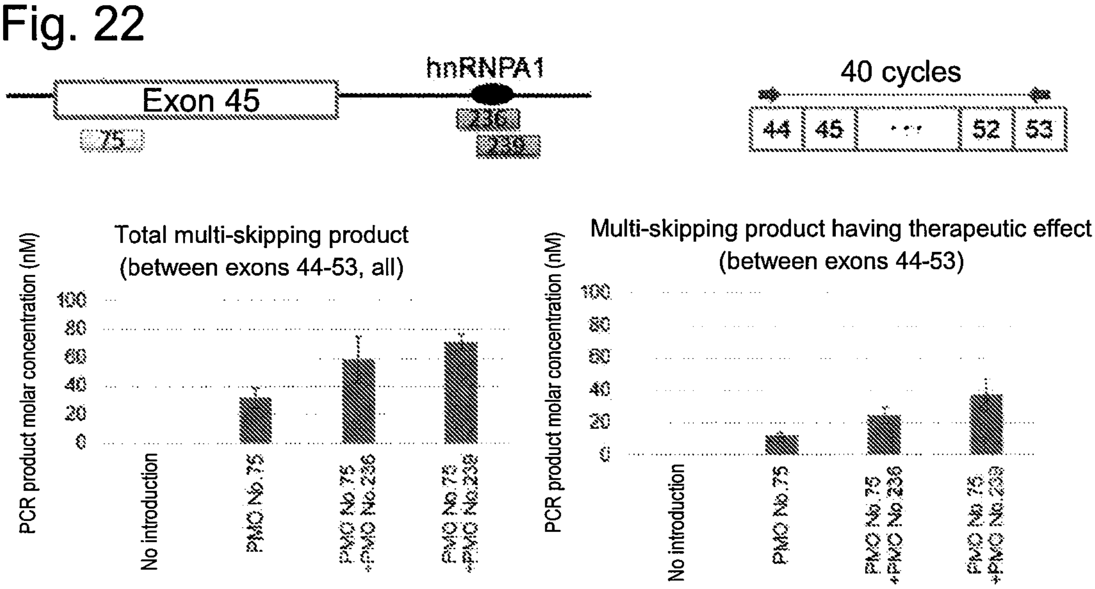


*Total of exon 45-47, exon 45-48, exon 45-49, exon 45-50, exon 45-51 and exon 45-52 skipping

*Total of exon 45-47, exon 45-48, exon 45-49 and exon 45-51 skipping

**INTERNATIONAL SEARCH REPORT**

International application No.
PCT/JP2020/047340

A. CLASSIFICATION OF SUBJECT MATTER

A61K 48/00(2006.01)i; A61P 21/00(2006.01)i; A61P 43/00(2006.01)i; C12N 15/113(2010.01)i; A61K 31/7088(2006.01)i; A61K 31/712(2006.01)i; A61K 31/7125(2006.01)i

FI: C12N15/113 Z ZNA; A61P21/00; A61P43/00 111; A61K48/00; A61P43/00 121; A61K31/7088; A61K31/712; A61K31/712

According to International Patent Classification (IPC) or to both national classification and IPC

B. FIELDS SEARCHED

Minimum documentation searched (classification system followed by classification symbols)

A61K48/00; A61P21/00; A61P43/00; C12N15/113; A61K31/7088; A61K31/712; A61K31/7125

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2018/129384 A1 (AVIDITY BIOSCIENCES LLC) 12 July 2018 (2018-07-12) claims, examples, fig. 1, 2, table 21, in particular, SEQ ID: 370, 371 | 1-16, 27, 31-40<br>11-16, 27, 31-40 |
| X<br>Y | JP 2012-506703 A (AVI BIOPHARMA, INC.) 22 March 2012 (2012-03-22) claims, examples, in particular, SEQ ID NO: 20 | 1-16, 27, 31-40<br>11-16, 27, 31-40 |
| X<br>Y | WO 2019/060775 A1 (AVIDITY BIOSCIENCES LLC) 28 March 2019 (2019-03-28) claims, examples, tables 18, 20A, in particular, SEQ ID: 1079-1082, 1084-1089 | 1-16, 27, 31-40<br>11-16, 27, 31-40 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 26 February 2021 (26.02.2021) | 09 March 2021 (09.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2020/047340

C (Continuation). DOCUMENTS CONSIDERED TO BE RELEVANT

| Category* | Citation of document, with indication, where appropriate, of the relevant passages | Relevant to claim No. |
|---|---|---|
| X | WO 2013/100190 A1 (NIPPON SHINYAKU CO., LTD.) 04 July 2013 (2013-07-04) claims, pp. 58-59, examples, in particular, tables 5, 10, 14, 15 | 1-16, 27, 31-40 |
| X<br>Y | JP 2013-510561 A (THE UNIVERSITY OF WESTERN AUSTRALIA) 28 March 2013 (2013-03-28) claims, paragraph [0054], in particular, SEQ ID NO: 11, 55, 245 | 1-16, 27, 31-40<br>11-16, 27, 31-40 |
| X<br>Y | JP 2012-506697 A (PROSENSA TECHNOLOGIES B.V) 22 March 2012 (2012-03-22) claims, paragraphs [0033], [0049]-[0058], table 1, in particular, SEQ ID NO: 46, 51-56, 61, 67 | 1-16, 27, 31-40<br>11-16, 27, 31-40 |
| X<br>Y | JP 2015-509922 A (PROSENSA TECHNOLOGIES B.V) 02 April 2015 (2015-04-02) claims, paragraphs [0044]-[0056], examples, table 1, in particular, SEQ ID NO: 101, 102, 103 | 1-16, 27, 31-40<br>11-16, 27, 31-40 |
| P, X | WO 2020/028832 A1 (DYNE THERAPEUTICS, INC.) 06 February 2020 (2020-02-06) claims, examples, in particular, SEQ ID 261 | 1-16, 27, 31-40 |
| A | ECHIGOYA, Y. et al. "Multiple Exon Skipping in the Duchenne Muscular Dystrophy Hot Spots: Prospects and Challenges", Journal of Personalized Medicine, 2018, vol. 8, no. 41, pp. 1-28 column 7. | 1-16, 27, 31-40 |
| A | ECHIGOYA, Y. et a., "Quantitative Antisense Screening and Optimization for Exon 51 Skipping in Duchenne Muscular Dystrophy", Molecular Therapy, 2017, vol. 25, no. 11, pp. 2561-2572 table 1 | 1-16, 27, 31-40 |
| A | JP 2016-517279 A (SAREPTA THERAPEUTICS, INC.) 16 June 2016 (2016-06-16) claims, examples | 1-16, 27, 31-40 |
| A | JP 2006-523101 A (ACADEMISCH ZIEKENHUIS LEIDEN) 12 October 2006 (2006-10-12) claims, examples | 1-16, 27, 31-40 |

Form PCT/ISA/210 (continuation of second sheet) (January 2015)

**INTERNATIONAL SEARCH REPORT**

International application No.
PCT/JP2020/047340

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☒ As only some of the required additional search fees were timely paid by the applicant, this international search report covers only those claims for which fees were paid, specifically claims Nos.:
Parts of inventions in claims 1-16, 27, and 31-40 (inventions 1-3)

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.
PCT/JP2020/047340

| Patent Documents referred in the Report | Publication Date | Patent Family | Publication Date |
|---|---|---|---|
| WO 2018/129384 A1 | 12 Jul. 2018 | JP 2020-505330 A<br>claims, examples, fig. 1, 2, table 21, in particular, SEQ ID NO: 370, 371<br>US 2018/0369400 A1<br>EP 3565577 A1 | |
| JP 2012-506703 A | 22 Mar. 2012 | US 2010/0130591 A1<br>claims, examples, in particular, SEQ ID 20<br>WO 2010/048586 A1<br>EP 2350281 A1 | |
| WO 2019/060775 A1 | 28 Mar. 2019 | JP 2020-537497 A<br>claims, examples, tables 18, 20A, in particular, SEQ ID NO: 1079-1082, 1084-1089 | |
| WO 2013/100190 A1 | 04 Jul. 2013 | US 2014/0343266 A1<br>claims, paragraph [0217], examples, in particular, tables 5, 10, 14, 15<br>EP 2799548 A1 | |
| JP 2013-510561 A | 28 Mar. 2013 | US 2012/0270925 A1<br>claims, paragraph [0101], in particular, SEQ ID 11, 55, 245<br>WO 2011/057350 A1<br>EP 2499249 A1 | |
| JP 2012-506697 A | 22 Mar. 2012 | WO 2010/050801 A1<br>claims, pp. 10-11, 16-19, table 1, in particular, SEQ ID 46, 51-56, 61, 67<br>US 2011/294753 A1<br>EP 2344637 A1 | |
| JP 2015-509922 A | 02 Apr. 2015 | WO 2013/112053 A1<br>claims, pp. 12-16, examples, table 1, SEQ ID 101, 102, 103<br>US 2015/0045413 A1<br>EP 2806900 A1 | |
| WO 2020/028832 A1 | 06 Feb. 2020 | (Family: none) | |
| JP 2016-517279 A | 16 Jun. 2016 | US 2014/0323544 A1<br>WO 2014/153220 A2<br>EP 2970963 A2 | |
| JP 2006-523101 A | 12 Oct. 2006 | US 2006/0099616 A1<br>WO 2004/083432 A1<br>EP 1606407 A1 | |

Form PCT/ISA/210 (patent family annex) (January 2015)

INTERNATIONAL SEARCH REPORT

International application No.
PCT/JP2020/047340

<Continuation of Box No. III>

(Invention 1) Invention in claims 1-16, 27, and 31-40 (parts thereof)

As disclosed in documents 1-4, antisense oligomers against human dystrophin pre-mRNA are known, and thus the inventions in claim 1, that is, an antisense oligomer, which includes a base sequence of a region Rn (n=1-24) or a base sequence complementary to a part of the base sequence, or a pharmaceutically acceptable salt thereof, or a hydrate thereof includes at least 24 inventions.

In addition, an invention in which n=1 among the inventions in claim 1, an invention referring to the invention in claim 1 in which n=1 among the inventions in claims 2-16 and 27, and an invention referring to the invention in claim 1 in which n=1 without referring to claims 17-26 among the inventions in claims 31-40 are classified as invention 1.

(Invention 2) Invention in claims 1-16, 27, and 31-40 (parts thereof)

An invention in which n=2 among the inventions in claim 1, an invention referring to the invention in claim 1 in which n=2 among the inventions in claims 2-16 and 27, and an invention referring to the invention in claim 1 in which n=2 without referring to claims 17-26 among the inventions in claims 31-40 are classified as invention 2.

(Invention 3) Invention in claims 1-16, 27, and 31-40 (parts thereof)

An invention in which n=3 among the inventions in claim 1, an invention referring to the invention in claim 1 in which n=3 among the inventions in claims 2-16 and 27, and an invention referring to the invention in claim 1 in which n=3 without referring to claims 17-26 among the inventions in claims 31-40 are classified as invention 3.

(Invention 4) Invention in claims 1-16, 27, and 31-40 (parts thereof)

An invention in which n=4 among the inventions in claim 1, an invention referring to the invention in claim 1 in which n=4 among the inventions in claims 2-16 and 27, and an invention referring to the invention in claim 1 in which n=4 without referring to claims 17-26 among the inventions in claims 31-40 are classified as invention 4.

(Invention 5) Invention in claims 1-16, 27, and 31-40 (parts thereof)

An invention in which n=5 among the inventions in claim 1, an invention referring to the invention in claim 1 in which n=5 among the inventions in claims 2-16 and 27, and an invention referring to the invention in claim 1 in which n=5 without referring to claims 17-26 among the inventions in claims 31-40 are classified as invention 5.

(Invention 6) Invention in claims 1-16, 27, and 31-40 (parts thereof)

An invention in which n=6 among the inventions in claim 1, an invention referring to the invention in claim 1 in which n=6 among the inventions in claims 2-16 and 27, and an invention referring to the invention in claim 1 in which n=6 without referring to claims 17-26 among the inventions in claims 31-40 are classified as invention 6.

(Invention 7) Invention in claims 1-16, 27, and 31-40 (parts thereof)

An invention in which n=7 among the inventions in claim 1, an invention referring to the invention in claim 1 in which n=7 among the inventions in claims 2-16 and 27, and an invention referring to the invention in claim 1 in which n=7 without referring to claims 17-26 among the inventions in claims 31-40 are classified as invention 7.

INTERNATIONAL SEARCH REPORT

International application No. PCT/JP2020/047340

(Invention 8) Invention in claims 1-16, 27, and 31-40 (parts thereof)

An invention in which n=8 among the inventions in claim 1, an invention referring to the invention in claim 1 in which n=8 among the inventions in claims 2-16 and 27, and an invention referring to the invention in claim 1 in which n=8 without referring to claims 17-26 among the inventions in claims 31-40 are classified as invention 8.

(Invention 9) Invention in claims 1-16, 27, and 31-40 (parts thereof)

An invention in which n=9 among the inventions in claim 1, an invention referring to the invention in claim 1 in which n=9 among the inventions in claims 2-16 and 27, and an invention referring to the invention in claim 1 in which n=9 without referring to claims 17-26 among the inventions in claims 31-40 are classified as invention 9.

(Invention 10) Invention in claims 1-16, 27, and 31-40 (parts thereof)

An invention in which n=10 among the inventions in claim 1, an invention referring to the invention in claim 1 in which n=10 among the inventions in claims 2-16 and 27, and an invention referring to the invention in claim 1 in which n=10 without referring to claims 17-26 among the inventions in claims 31-40 are classified as invention 10.

(Invention 11) Invention in claims 1-16, 27, and 31-40 (parts thereof)

An invention in which n=11 among the inventions in claim 1, an invention referring to the invention in claim 1 in which n=11 among the inventions in claims 2-16 and 27, and an invention referring to the invention in claim 1 in which n=11 without referring to claims 17-26 among the inventions in claims 31-40 are classified as invention 11.

(Invention 12) Invention in claims 1-16, 27, and 31-40 (parts thereof)

An invention in which n=12 among the inventions in claim 1, an invention referring to the invention in claim 1 in which n=12 among the inventions in claims 2-16 and 27, and an invention referring to the invention in claim 1 in which n=12 without referring to claims 17-26 among the inventions in claims 31-40 are classified as invention 12.

(Invention 13) Invention in claims 1-16, 27, and 31-40 (parts thereof)

An invention in which n=13 among the inventions in claim 1, an invention referring to the invention in claim 1 in which n=13 among the inventions in claims 2-16 and 27, and an invention referring to the invention in claim 1 in which n=13 without referring to claims 17-26 among the inventions in claims 31-40 are classified as invention 13.

(Invention 14) Invention in claims 1-16, 27, and 31-40 (parts thereof)

An invention in which n=14 among the inventions in claim 1, an invention referring to the invention in claim 1 in which n=14 among the inventions in claims 2-16 and 27, and an invention referring to the invention in claim 1 in which n=14 without referring to claims 17-26 among the inventions in claims 31-40 are classified as invention 14.

(Invention 15) Invention in claims 1-16, 27, and 31-40 (parts thereof)

An invention in which n=15 among the inventions in claim 1, an invention referring to the invention in claim 1 in which n=15 among the inventions in claims 2-16 and 27, and an invention referring to the invention in claim 1 in which n=15 without referring to claims 17-26 among the inventions in claims 31-40 are classified as invention 15.

Form PCT/ISA/210 (extra sheet) (January 2015)

INTERNATIONAL SEARCH REPORT

International application No.
PCT/JP2020/047340

(Invention 16) Invention in claims 1-16, 27, and 31-40 (parts thereof)

An invention in which n=16 among the inventions in claim 1, an invention referring to the invention in claim 1 in which n=16 among the inventions in claims 2-16 and 27, and an invention referring to the invention in claim 1 in which n=16 without referring to claims 17-26 among the inventions in claims 31-40 are classified as invention 16.

(Invention 17) Invention in claims 1-16, 27, and 31-40 (parts thereof)

An invention in which n=17 among the inventions in claim 1, an invention referring to the invention in claim 1 in which n=17 among the inventions in claims 2-16 and 27, and an invention referring to the invention in claim 1 in which n=17 without referring to claims 17-26 among the inventions in claims 31-40 are classified as invention 17.

(Invention 18) Invention in claims 1-16, 27, and 31-40 (parts thereof)

An invention in which n=18 among the inventions in claim 1, an invention referring to the invention in claim 1 in which n=18 among the inventions in claims 2-16 and 27, and an invention referring to the invention in claim 1 in which n=18 without referring to claims 17-26 among the inventions in claims 31-40 are classified as invention 18.

(Invention 19) Invention in claims 1-16, 27, and 31-40 (parts thereof)

An invention in which n=19 among the inventions in claim 1, an invention referring to the invention in claim 1 in which n=19 among the inventions in claims 2-16 and 27, and an invention referring to the invention in claim 1 in which n=19 without referring to claims 17-26 among the inventions in claims 31-40 are classified as invention 19.

(Invention 20) Invention in claims 1-16, 27, and 31-40 (parts thereof)

An invention in which n=20 among the inventions in claim 1, an invention referring to the invention in claim 1 in which n=20 among the inventions in claims 2-16 and 27, and an invention referring to the invention in claim 1 in which n=20 without referring to claims 17-26 among the inventions in claims 31-40 are classified as invention 20.

(Invention 21) Invention in claims 1-16, 27, and 31-40 (parts thereof)

An invention in which n=21 among the inventions in claim 1, an invention referring to the invention in claim 1 in which n=21 among the inventions in claims 2-16 and 27, and an invention referring to the invention in claim 1 in which n=21 without referring to claims 17-26 among the inventions in claims 31-40 are classified as invention 21.

(Invention 22) Invention in claims 1-16, 27, and 31-40 (parts thereof)

An invention in which n=22 among the inventions in claim 1, an invention referring to the invention in claim 1 in which n=22 among the inventions in claims 2-16 and 27, and an invention referring to the invention in claim 1 in which n=22 without referring to claims 17-26 among the inventions in claims 31-40 are classified as invention 22.

(Invention 23) Invention in claims 1-16, 27, and 31-40 (parts thereof)

An invention in which n=23 among the inventions in claim 1, an invention referring to the invention in claim 1 in which n=23 among the inventions in claims 2-16 and 27, and an invention referring to the invention in claim 1 in which n=23 without referring to claims 17-26 among the inventions in claims 31-40 are classified as invention 23.

Form PCT/ISA/210 (extra sheet) (January 2015)

INTERNATIONAL SEARCH REPORT

International application No. PCT/JP2020/047340

(Invention 24) Invention in claims 1-16, 27, and 31-40 (parts thereof)

An invention in which n=24 among the inventions in claim 1, an invention referring to the invention in claim 1 in which n=24 among the inventions in claims 2-16 and 27, and an invention referring to the invention in claim 1 in which n=24 without referring to claims 17-26 among the inventions in claims 31-40 are classified as invention 24.

(Invention 25) Invention in claims 1-16, 27, and 31-40 (parts thereof)

An invention in which n=25 among the inventions in claim 1, an invention referring to the invention in claim 1 in which n=25 among the inventions in claims 2-16 and 27, and an invention referring to the invention in claim 1 in which n=25 without referring to claims 17-26 among the inventions in claims 31-40 are classified as invention 25.

(Invention 26) Invention in claims 17-26 and 28-40 (parts thereof)

As disclosed in documents 1-4, antisense oligomers against human dystrophin pre-mRNA are known. Meanwhile, in the inventions in claim 17, the targets of skipping suppressed by the antisense oligomer are from the $45^{th}$ exon to the $55^{th}$ exon, and thus the inventions in claim 17 include at least 11 inventions.

Therefore, an invention in which the target of the skipping suppressed by the antisense oligomer is the $45^{th}$ exon among the inventions in claim 17, and an invention referring to the invention in claim 17 in which the target of the skipping suppressed by the antisense oligomer is the $45^{th}$ exon among the inventions in claims 18-26 and 28-40 are classified as invention 26.

(Invention 27) Invention in claims 17-26 and 28-40 (parts thereof)

An invention in which the target of the skipping suppressed by the antisense oligomer is the $46^{th}$ exon among the inventions in claim 17, and an invention referring to the invention in claim 17 in which the target of the skipping suppressed by the antisense oligomer is the $46^{th}$ exon among the inventions in claims 18-26 and 28-40 are classified as invention 27.

(Invention 28) Invention in claims 17-26 and 28-40 (parts thereof)

An invention in which the target of the skipping suppressed by the antisense oligomer is the $47^{th}$ exon among the inventions in claim 17, and an invention referring to the invention in claim 17 in which the target of the skipping suppressed by the antisense oligomer is the $47^{th}$ exon among the inventions in claims 18-26 and 28-40 are classified as invention 28.

(Invention 29) Invention in claims 17-26 and 28-40 (parts thereof)

An invention in which the target of the skipping suppressed by the antisense oligomer is the $48^{th}$ exon among the inventions in claim 17, and an invention referring to the invention in claim 17 in which the target of the skipping suppressed by the antisense oligomer is the $48^{th}$ exon among the inventions in claims 18-26 and 28-40 are classified as invention 29.

(Invention 30) Invention in claims 17-26 and 28-40 (parts thereof)

An invention in which the target of the skipping suppressed by the antisense oligomer is the $49^{th}$ exon among the inventions in claim 17, and an invention referring to the invention in claim 17 in which the target of the skipping suppressed by the antisense oligomer is the $49^{th}$ exon among the inventions in claims 18-26 and 28-40 are classified as invention 30.

Form PCT/ISA/210 (extra sheet) (January 2015)

INTERNATIONAL SEARCH REPORT

International application No. PCT/JP2020/047340

(Invention 31) Invention in claims 17-26 and 28-40 (parts thereof)

An invention in which the target of the skipping suppressed by the antisense oligomer is the 50th exon among the inventions in claim 17, and an invention referring to the invention in claim 17 in which the target of the skipping suppressed by the antisense oligomer is the 50th exon among the inventions in claims 18-26 and 28-40 are classified as invention 31.

(Invention 32) Invention in claims 17-26 and 28-40 (parts thereof)

An invention in which the target of the skipping suppressed by the antisense oligomer is the 51st exon among the inventions in claim 17, and an invention referring to the invention in claim 17 in which the target of the skipping suppressed by the antisense oligomer is the 51st exon among the inventions in claims 18-26 and 28-40 are classified as invention 32.

(Invention 33) Invention in claims 17-26 and 28-40 (parts thereof)

An invention in which the target of the skipping suppressed by the antisense oligomer is the 52nd exon among the inventions in claim 17, and an invention referring to the invention in claim 17 in which the target of the skipping suppressed by the antisense oligomer is the 52nd exon among the inventions in claims 18-26 and 28-40 are classified as invention 33.

(Invention 34) Invention in claims 17-26 and 28-40 (parts thereof)

An invention in which the target of the skipping suppressed by the antisense oligomer is the 53rd exon among the inventions in claim 17, and an invention referring to the invention in claim 17 in which the target of the skipping suppressed by the antisense oligomer is the 53rd exon among the inventions in claims 18-26 and 28-40 are classified as invention 34.

(Invention 35) Invention in claims 17-26 and 28-40 (parts thereof)

An invention in which the target of the skipping suppressed by the antisense oligomer is the 54th exon among the inventions in claim 17, and an invention referring to the invention in claim 17 in which the target of the skipping suppressed by the antisense oligomer is the 54th exon among the inventions in claims 18-26 and 28-40 are classified as invention 35.

(Invention 36) Invention in claims 17-26 and 28-40 (parts thereof)

An invention in which the target of the skipping suppressed by the antisense oligomer is the 55th exon among the inventions in claim 17, and an invention referring to the invention in claim 17 in which the target of the skipping suppressed by the antisense oligomer is the 55th exon among the inventions in claims 18-26 and 28-40 are classified as invention 36.

(Invention 37) Claims 41-46

The invention in claims 41-46 is classified as invention 37.

Document 1: ECHIGOYA, Y. et al. "Multiple Exon Skipping in the Duchenne Muscular Dystrophy Hot Spots: Prospects and Challenges", Journal of Personalized Medicine, 2018, vol. 8, no. 41, pp. 1-28
Document 2: ECHIGOYA, Y. et a., "Quantitative Antisense Screening and Optimization for Exon 51 Skipping in Duchenne Muscular Dystrophy", Molecular Therapy, 2017, vol. 25, no. 11, pp. 2561-2572
Document 3: JP 2016-517279 A (SAREPTA THERAPEUTICS, INC.) 16 June 2016 (2016-06-16) & US 2014/0323544 A1 & WO 2014/153220 A2 & EP 2970963 A2
Document 4: JP 2006-523101 A (ACADEMISCH ZIEKENHUIS LEIDEN) 12 October 2006 (2006-10-12) & US 2006/0099616 A1 & WO 2004/083432 A1 & EP 1606407 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004048570 A
- WO 2009139630 A
- WO 2010048586 A
- US 20100168212 A
- WO 2011057350 A
- WO 2006000057 A
- WO 2007135105 A
- WO 2004083446 A
- JP 2006129594 W
- JP 2006038608 W
- WO 1991009033 A
- WO 2009064471 A
- WO 2013100190 A
- JP 2924179 B
- JP 2008096690 W


### Non-patent literature cited in the description


- **ANNEMIEKE AARTSMA-RUS et al.** *Neuromuscular Disorders,* 2002, vol. 12, S71-S77
- **WILTON S. D.** *Molecular Therapy,* 2007, vol. 15, 1288-96
- **CHRISTOPHE BEROUD et al.** *Human Mutation,* 2007, vol. 28 (2), 196-202
- **YUSUKE ECHIGOYA et al.** *Molecular Therapy-Nucleic Acids,* 2015, vol. 4 (2), e225
- **YOSHITSUGU AOKI et al.** *PNAS,* 2012, vol. 109 (34), 13763-13768
- **LAURA VAN VLIET et al.** *BMC Medical Genetics,* 2008, vol. 9, 105
- **JOSHUA LEE et al.** *PLoS ONE,* 2018, vol. 13 (5), e0197084
- **JOSHUA LEE et al.** *Methods in Molecular Biology,* 2018, vol. 1828, 141-150
- **ANNEMIEKE AARTSMA-RUS et al.** *Am. J. Hum. Genet.,* 2004, vol. 74 (1), 83-92
- **YUSUKE ECHIGOYA et al.** *Molecular Therapy,* 2019, vol. 27 (11), 1-13
- **ROBERTS, RG et al.** *Genomics,* 1993, vol. 16, 536-538
- **SAMBROOK ; RUSSELL.** Molecular Cloning: A Laboratory Manual. Cold Spring Harbor, Laboratory Press, 2001, vol. 3
- **AUSUBEL.** Current Protocols in Molecular Biology. John Wiley & Sons, 1987
- **KARLIN ; ALTSCHUL.** *Proc. Natl. Acad. Sci. USA,* 1990, 872264-2268
- *Proc. Natl. Acad. Sci. USA,* 1993, vol. 90, 5873
- **ALTSCHUL SF et al.** *J. Mol. Biol.,* 1990, vol. 215, 403
- **ENYA et al.** *Bioorganic & Medicinal Chemistry,* 2008, vol. 18, 9154-9160
- **P. E. NIELSEN ; M. EGHOLM ; R. H. BERG ; O. BUCHARDT.** *Science,* 1991, vol. 254, 1497
- **M. EGHOLM ; O. BUCHARDT ; P. E. NIELSEN ; R. H. BERG.** *JACS,* 1992, vol. 114, 1895
- **K. L. DUEHOLM ; M. EGHOLM ; C. BEHRENS ; L. CHRISTENSEN ; H. F. HANSEN ; T. VULPIUS ; K. H. PETERSEN ; R. H. BERG ; P. E. NIELSEN ; O. BUCHARDT.** *J. Org. Chem.,* 1994, vol. 59, 5767
- **L. CHRISTENSEN ; R. FITZPATRICK ; B. GILDEA ; K. H. PETERSEN ; H. F. HANSEN ; T. KOCH ; M. EGHOLM ; O. BUCHARDT ; P. E. NIELSEN ; J. COULL.** *J. Pept. Sci.,* 1995, vol. 1, 175
- **T. KOCH ; H. F. HANSEN ; P. ANDERSEN ; T. LARSEN ; H. G. BATZ ; K. OTTESON ; H. ORUM.** *J. Pept. Res.,* 1997, vol. 49, 80
- **FRANCESCO PIVA et al.** *Bioinformatics,* 2009, vol. 25 (9), 1211-1213
- **FRANCESCO PIVA et al.** *Human Mutation,* 2012, vol. 33 (1), 81-85
- **MATTEO GIULIETTI et al.** *Nucleic Acids Res.,* 2012, vol. 41, D125-D131
- **GIROLAMO GIUDICE et al.** *Database (Oxford),* 2016
- **SCHRAGA SCHWARTZ et al.** *Nucleic Acids Res.,* 2009, vol. 37, W189-W192
- **HSI-YUAN HUANG et al.** *Nucleic Acids Res.,* 2006, vol. 34, W429-W434
- **TZU-HAO CHANG et al.** *BMC bioinformatics,* 2013, vol. 14 (2
- **FRANCOIS-OLIVIER DESMET et al.** *Nucleic Acids Res.,* 2009, vol. 37 (9), e67
- **ANDRE CORVELO et al.** *PLoS Comput Biol.,* 2010, vol. 6 (11), e1001016

SEQUENCE LISTING

<110> NIPPON SHINYAKU CO., LTD.
NATIONAL CENTER OF NEUROLOGY AND PSYCHIATRY

<120> ANTISENSE NUCLEIC ACID CAUSING EXON SKIPPING

<130> G2327WO

<150> JP2019-229763
<151> 2019-12-19

<160> 390

<170> PatentIn version 3.5

<210> 1
<211> 26
<212> DNA
<213> Artificial

<220>
<223> Synthetic Nucleic Acid

<400> 1
tttcgaaaaa acaaatcaaa gactta 26

<210> 2
<211> 40
<212> DNA
<213> Artificial

<220>
<223> Synthetic Nucleic Acid

<400> 2
atgtgctgaa gataaataca atttcgaaaa aacaaatcaa 40

<210> 3
<211> 35
<212> DNA
<213> Artificial

<220>
<223> Synthetic Nucleic Acid

<400> 3
tgctgaagat aaatacaatt tcgaaaaaac aaatc 35

<210> 4
<211> 30
<212> DNA
<213> Artificial

<220>
<223> Synthetic Nucleic Acid

<400> 4
cagatgtgct gaagataaat acaatttcga 30

<210> 5
<211> 28
<212> DNA
<213> Artificial

<220>
<223> Synthetic Nucleic Acid

<400> 5
aagagtccag atgtgctgaa gataaata 28

<210> 6
<211> 28
<212> DNA
<213> Artificial

<220>
<223> Synthetic Nucleic Acid

<400> 6
acccttcaga acctgatctt taagaagt 28

<210> 7
<211> 28
<212> DNA
<213> Artificial

<220>
<223> Synthetic Nucleic Acid

<400> 7
tgacaacaac agtcaaaagt aatttcca 28

<210> 8
<211> 28
<212> DNA
<213> Artificial

<220>
<223> Synthetic Nucleic Acid

<400> 8
atgataattt tctttctagt aatataat 28

<210> 9
<211> 28
<212> DNA
<213> Artificial

<220>
<223> Synthetic Nucleic Acid

<400> 9
tccatagcac cgtgctctaa tattatca 28

<210> 10
<211> 26

<212> DNA
<213> Artificial

<220>
<223> Synthetic Nucleic Acid

<400> 10
ggcaaactct ctcatcctga cacaaa 26

<210> 11
<211> 28
<212> DNA
<213> Artificial

<220>
<223> Synthetic Nucleic Acid

<400> 11
tttatcagat aaaccagctc cgtccagg 28

<210> 12
<211> 28
<212> DNA
<213> Artificial

<220>
<223> Synthetic Nucleic Acid

<400> 12
cttccctctg tcacagattc aattatat 28

<210> 13
<211> 26
<212> DNA
<213> Artificial

<220>
<223> Synthetic Nucleic Acid

<400> 13
aaaacacctt gctgttacga tgcttc 26

<210> 14
<211> 30
<212> DNA
<213> Artificial

<220>
<223> Synthetic Nucleic Acid

<400> 14
tgccccaaag ccacaaaaca ccttgctgtt 30

<210> 15
<211> 26
<212> DNA
<213> Artificial

<220>
<223> Synthetic Nucleic Acid

<400> 15
ggttccaaca taaagccgaa atacac 26

<210> 16
<211> 28
<212> DNA
<213> Artificial

<220>
<223> Synthetic Nucleic Acid

<400> 16
tatgccacaa gttctccttc tggaaagg 28

<210> 17
<211> 28
<212> DNA
<213> Artificial

<220>
<223> Synthetic Nucleic Acid

<400> 17
gatatttcta gcaacttcat tttagcta 28

<210> 18
<211> 30
<212> DNA
<213> Artificial

<220>
<223> Synthetic Nucleic Acid

<400> 18
tgctgaagat aaatacaaat caaagactta 30

<210> 19
<211> 30
<212> DNA
<213> Artificial

<220>
<223> Synthetic Nucleic Acid

<400> 19
aagagtccag atgtgcaaat caaagactta 30

<210> 20
<211> 30
<212> DNA
<213> Artificial

<220>
<223> Synthetic Nucleic Acid

<400> 20
tgctgaagat aaatatttcg aaaaaacaaa 30

<210> 21
<211> 30
<212> DNA
<213> Artificial

<220>
<223> Synthetic Nucleic Acid

<400> 21
aagagtccag atgtgtttcg aaaaaacaaa 30

<210> 22
<211> 28
<212> DNA
<213> Artificial

<220>
<223> Synthetic Nucleic Acid

<400> 22
ttaattgctt tcaggagcat cccatcaa 28

<210> 23
<211> 28
<212> DNA
<213> Artificial

<220>
<223> Synthetic Nucleic Acid

<400> 23
tttgcattag aagccacaaa aaactgag 28

<210> 24
<211> 28
<212> DNA
<213> Artificial

<220>
<223> Synthetic Nucleic Acid

<400> 24
tcatttcaaa ttctgtctgc gtcaatgt 28

<210> 25
<211> 28
<212> DNA
<213> Artificial

<220>
<223> Synthetic Nucleic Acid

<400> 25
ttgctatatt agaagaaaat tcatttca 28

<210>  26
<211>  28
<212>  DNA
<213>  Artificial

<220>
<223>  Synthetic Nucleic Acid

<400>  26
taattgctat attagaagaa aattcatt                                          28

<210>  27
<211>  30
<212>  DNA
<213>  Artificial

<220>
<223>  Synthetic Nucleic Acid

<400>  27
aaataaaatt aattgctata ttagaagaaa                                        30

<210>  28
<211>  30
<212>  DNA
<213>  Artificial

<220>
<223>  Synthetic Nucleic Acid

<400>  28
cagtattaaa aaaaaactct agagatattt                                        30

<210>  29
<211>  28
<212>  DNA
<213>  Artificial

<220>
<223>  Synthetic Nucleic Acid

<400>  29
tagtcacagt attaaaaaaa aactctag                                          28

<210>  30
<211>  28
<212>  DNA
<213>  Artificial

<220>
<223>  Synthetic Nucleic Acid

<400>  30
gtgaaaaaga acaaacatag gttagtca                                          28

<210>  31
<211>  28

<212>  DNA
<213>  Artificial

<220>
<223>  Synthetic Nucleic Acid

<400>  31
atacgagagg tgaaaaagaa caaacata                                        28

<210>  32
<211>  28
<212>  DNA
<213>  Artificial

<220>
<223>  Synthetic Nucleic Acid

<400>  32
tggatacgag aggtgaaaaa gaacaaac                                        28

<210>  33
<211>  28
<212>  DNA
<213>  Artificial

<220>
<223>  Synthetic Nucleic Acid

<400>  33
tttcttagtg atcgtggata cgagaggt                                        28

<210>  34
<211>  25
<212>  DNA
<213>  Artificial

<220>
<223>  Synthetic Nucleic Acid

<400>  34
gtttcttagt gatcgtggat acgag                                           25

<210>  35
<211>  28
<212>  DNA
<213>  Artificial

<220>
<223>  Synthetic Nucleic Acid

<400>  35
tgggtttctt agtgatcgtg gatacgag                                        28

<210>  36
<211>  25
<212>  DNA
<213>  Artificial

<220>
<223> Synthetic Nucleic Acid

<400> 36
tgggtttctt agtgatcgtg gatac 25

<210> 37
<211> 28
<212> DNA
<213> Artificial

<220>
<223> Synthetic Nucleic Acid

<400> 37
gtatttgggt ttcttagtga tcgtggat 28

<210> 38
<211> 28
<212> DNA
<213> Artificial

<220>
<223> Synthetic Nucleic Acid

<400> 38
tgaacaaagt atttgggttt cttagtga 28

<210> 39
<211> 28
<212> DNA
<213> Artificial

<220>
<223> Synthetic Nucleic Acid

<400> 39
ttgtaaaatt taaacatgaa caaagtat 28

<210> 40
<211> 28
<212> DNA
<213> Artificial

<220>
<223> Synthetic Nucleic Acid

<400> 40
tccccacaag gatgttccat gtttaata 28

<210> 41
<211> 28
<212> DNA
<213> Artificial

<220>
<223> Synthetic Nucleic Acid

<400>  41
accttttcaa gagcaaattc gatttctt                                          28

<210>  42
<211>  26
<212>  DNA
<213>  Artificial

<220>
<223>  Synthetic Nucleic Acid

<400>  42
caattagttg gaaacctttt caagag                                            26

<210>  43
<211>  28
<212>  DNA
<213>  Artificial

<220>
<223>  Synthetic Nucleic Acid

<400>  43
tataatgtcc tacaaatcaa ttagttgg                                          28

<210>  44
<211>  26
<212>  DNA
<213>  Artificial

<220>
<223>  Synthetic Nucleic Acid

<400>  44
agctagagga tgttataatg tcctac                                            26

<210>  45
<211>  28
<212>  DNA
<213>  Artificial

<220>
<223>  Synthetic Nucleic Acid

<400>  45
attttgtaa gcttgtcagc tagaggat                                           28

<210>  46
<211>  28
<212>  DNA
<213>  Artificial

<220>
<223>  Synthetic Nucleic Acid

<400>  46
aaagcaccct ctcggttagc tccagttt                                          28

<210> 47
<211> 28
<212> DNA
<213> Artificial

<220>
<223> Synthetic Nucleic Acid

<400> 47
cagaaagaca ccttttatgt gtcaggga 28

<210> 48
<211> 28
<212> DNA
<213> Artificial

<220>
<223> Synthetic Nucleic Acid

<400> 48
ggatacaaga cagaaagaca ccttttat 28

<210> 49
<211> 26
<212> DNA
<213> Artificial

<220>
<223> Synthetic Nucleic Acid

<400> 49
aaaggataca agacagaaag acacct 26

<210> 50
<211> 28
<212> DNA
<213> Artificial

<220>
<223> Synthetic Nucleic Acid

<400> 50
tgacatgccc atatccaaag gatacaag 28

<210> 51
<211> 28
<212> DNA
<213> Artificial

<220>
<223> Synthetic Nucleic Acid

<400> 51
aactgacatg cccatatcca aaggatac 28

<210> 52
<211> 28

<212>  DNA
<213>  Artificial

<220>
<223>  Synthetic Nucleic Acid

<400>  52
agctccatgt gaaaatttcc ctatgaaa    28

<210>  53
<211>  28
<212>  DNA
<213>  Artificial

<220>
<223>  Synthetic Nucleic Acid

<400>  53
tgcagttgta ctggcaaaga aagaaata    28

<210>  54
<211>  28
<212>  DNA
<213>  Artificial

<220>
<223>  Synthetic Nucleic Acid

<400>  54
tgagaaaaga ttaaacagtg tgctacca    28

<210>  55
<211>  28
<212>  DNA
<213>  Artificial

<220>
<223>  Synthetic Nucleic Acid

<400>  55
tttgagaaaa gattaaacag tgtgctac    28

<210>  56
<211>  28
<212>  DNA
<213>  Artificial

<220>
<223>  Synthetic Nucleic Acid

<400>  56
tctttttatt tgagaaaaga ttaaacag    28

<210>  57
<211>  28
<212>  DNA
<213>  Artificial

<220>
<223> Synthetic Nucleic Acid

<400> 57
aagccccatg tctttttatt tgagaaaa 28

<210> 58
<211> 28
<212> DNA
<213> Artificial

<220>
<223> Synthetic Nucleic Acid

<400> 58
aacaaaaatg aagccccatg tcttttta 28

<210> 59
<211> 28
<212> DNA
<213> Artificial

<220>
<223> Synthetic Nucleic Acid

<400> 59
aggcaaaaca aaaatgaagc cccatgtc 28

<210> 60
<211> 28
<212> DNA
<213> Artificial

<220>
<223> Synthetic Nucleic Acid

<400> 60
ttcctgtaag ataccaaaaa ggcaaaac 28

<210> 61
<211> 28
<212> DNA
<213> Artificial

<220>
<223> Synthetic Nucleic Acid

<400> 61
agttcctgta agataccaaa aaggcaaa 28

<210> 62
<211> 25
<212> DNA
<213> Artificial

<220>
<223> Synthetic Nucleic Acid

<400> 62
ttcctgtaag ataccaaaaa ggcaa 25

<210> 63
<211> 28
<212> DNA
<213> Artificial

<220>
<223> Synthetic Nucleic Acid

<400> 63
ggagttcctg taagatacca aaaaggca 28

<210> 64
<211> 25
<212> DNA
<213> Artificial

<220>
<223> Synthetic Nucleic Acid

<400> 64
agttcctgta agataccaaa aaggc 25

<210> 65
<211> 28
<212> DNA
<213> Artificial

<220>
<223> Synthetic Nucleic Acid

<400> 65
tggagttcct gtaagatacc aaaaaggc 28

<210> 66
<211> 28
<212> DNA
<213> Artificial

<220>
<223> Synthetic Nucleic Acid

<400> 66
cctggagttc ctgtaagata ccaaaaag 28

<210> 67
<211> 25
<212> DNA
<213> Artificial

<220>
<223> Synthetic Nucleic Acid

<400> 67
tggagttcct gtaagatacc aaaaa 25

<210> 68
<211> 28
<212> DNA
<213> Artificial

<220>
<223> Synthetic Nucleic Acid

<400> 68
atcctggagt tcctgtaaga taccaaaa 28

<210> 69
<211> 25
<212> DNA
<213> Artificial

<220>
<223> Synthetic Nucleic Acid

<400> 69
cctggagttc ctgtaagata ccaaa 25

<210> 70
<211> 28
<212> DNA
<213> Artificial

<220>
<223> Synthetic Nucleic Acid

<400> 70
ccatcctgga gttcctgtaa gataccaa 28

<210> 71
<211> 26
<212> DNA
<213> Artificial

<220>
<223> Synthetic Nucleic Acid

<400> 71
tgccatcctg gagttcctgt aagata 26

<210> 72
<211> 25
<212> DNA
<213> Artificial

<220>
<223> Synthetic Nucleic Acid

<400> 72
ccaatgccat cctggagttc ctgta 25

<210> 73
<211> 25

<212> DNA
<213> Artificial

<220>
<223> Synthetic Nucleic Acid

<400> 73
gctgcccaat gccatcctgg agttc 25

<210> 74
<211> 25
<212> DNA
<213> Artificial

<220>
<223> Synthetic Nucleic Acid

<400> 74
ttgccgctgc ccaatgccat cctgg 25

<210> 75
<211> 25
<212> DNA
<213> Artificial

<220>
<223> Synthetic Nucleic Acid

<400> 75
acagtttgcc gctgcccaat gccat 25

<210> 76
<211> 30
<212> DNA
<213> Artificial

<220>
<223> Synthetic Nucleic Acid

<400> 76
tgacaacagt ttgccgctgc ccaatgccat 30

<210> 77
<211> 20
<212> DNA
<213> Artificial

<220>
<223> Synthetic Nucleic Acid

<400> 77
acagtttgcc gctgcccaat 20

<210> 78
<211> 25
<212> DNA
<213> Artificial

<220>
<223>  Synthetic Nucleic Acid

<400>  78
tgacaacagt ttgccgctgc ccaat                                              25

<210>  79
<211>  25
<212>  DNA
<213>  Artificial

<220>
<223>  Synthetic Nucleic Acid

<400>  79
tgttctgaca acagtttgcc gctgc                                              25

<210>  80
<211>  30
<212>  DNA
<213>  Artificial

<220>
<223>  Synthetic Nucleic Acid

<400>  80
agtttgccgc tgcccaatat acgagaggtg                                         30

<210>  81
<211>  30
<212>  DNA
<213>  Artificial

<220>
<223>  Synthetic Nucleic Acid

<400>  81
ttgccgctgc ccaatgtgat cgtggatacg                                         30

<210>  82
<211>  30
<212>  DNA
<213>  Artificial

<220>
<223>  Synthetic Nucleic Acid

<400>  82
agtttgccgc tgcccaatta gtgatcgtgg                                         30

<210>  83
<211>  30
<212>  DNA
<213>  Artificial

<220>
<223>  Synthetic Nucleic Acid

<400> 83
ttgccgctgc ccaattgggt ttcttagtga 30

<210> 84
<211> 30
<212> DNA
<213> Artificial

<220>
<223> Synthetic Nucleic Acid

<400> 84
agtttgccgc tgcccaattg ggtttcttag 30

<210> 85
<211> 28
<212> DNA
<213> Artificial

<220>
<223> Synthetic Nucleic Acid

<400> 85
aatttcccta tgaactgaca tgcccata 28

<210> 86
<211> 28
<212> DNA
<213> Artificial

<220>
<223> Synthetic Nucleic Acid

<400> 86
tgaagcccca tgtcaaacag tgtgctac 28

<210> 87
<211> 28
<212> DNA
<213> Artificial

<220>
<223> Synthetic Nucleic Acid

<400> 87
aaaacaaaaa tgaagaacag tgtgctac 28

<210> 88
<211> 28
<212> DNA
<213> Artificial

<220>
<223> Synthetic Nucleic Acid

<400> 88
tgccgctgcc caataaacag tgtgctac 28

<210> 89
<211> 28
<212> DNA
<213> Artificial

<220>
<223> Synthetic Nucleic Acid

<400> 89
cagtttgccg ctgcaaacag tgtgctac 28

<210> 90
<211> 28
<212> DNA
<213> Artificial

<220>
<223> Synthetic Nucleic Acid

<400> 90
tgacaacagt ttgcaaacag tgtgctac 28

<210> 91
<211> 26
<212> DNA
<213> Artificial

<220>
<223> Synthetic Nucleic Acid

<400> 91
aaatgaagcc ccaattaaac agtgtg 26

<210> 92
<211> 28
<212> DNA
<213> Artificial

<220>
<223> Synthetic Nucleic Acid

<400> 92
aaaaatgaag cccaaagatt aaacagtg 28

<210> 93
<211> 28
<212> DNA
<213> Artificial

<220>
<223> Synthetic Nucleic Acid

<400> 93
caaaaatgaa gcccaaagat taaacagt 28

<210> 94
<211> 28

<212>  DNA
<213>  Artificial

<220>
<223>  Synthetic Nucleic Acid

<400>  94
tgccgctgcc caataaagat taaacagt    28

<210>  95
<211>  28
<212>  DNA
<213>  Artificial

<220>
<223>  Synthetic Nucleic Acid

<400>  95
cagtttgccg ctgcaaagat taaacagt    28

<210>  96
<211>  28
<212>  DNA
<213>  Artificial

<220>
<223>  Synthetic Nucleic Acid

<400>  96
tgacaacagt ttgcaaagat taaacagt    28

<210>  97
<211>  28
<212>  DNA
<213>  Artificial

<220>
<223>  Synthetic Nucleic Acid

<400>  97
gccgctgccc aaatgaagca gattaaac    28

<210>  98
<211>  28
<212>  DNA
<213>  Artificial

<220>
<223>  Synthetic Nucleic Acid

<400>  98
tgaagcccca tgtctttgag aaaagatt    28

<210>  99
<211>  28
<212>  DNA
<213>  Artificial

<220>
<223> Synthetic Nucleic Acid

<400> 99
aaacaaaaat gaagtttgag aaaagatt 28

<210> 100
<211> 28
<212> DNA
<213> Artificial

<220>
<223> Synthetic Nucleic Acid

<400> 100
tgccgctgcc caattttgag aaaagatt 28

<210> 101
<211> 28
<212> DNA
<213> Artificial

<220>
<223> Synthetic Nucleic Acid

<400> 101
cagtttgccg ctgctttgag aaaagatt 28

<210> 102
<211> 28
<212> DNA
<213> Artificial

<220>
<223> Synthetic Nucleic Acid

<400> 102
tgacaacagt ttgctttgag aaaagatt 28

<210> 103
<211> 28
<212> DNA
<213> Artificial

<220>
<223> Synthetic Nucleic Acid

<400> 103
tgccgctgcc caattgaagc cccatgtc 28

<210> 104
<211> 28
<212> DNA
<213> Artificial

<220>
<223> Synthetic Nucleic Acid

<400> 104
tgacaacagt ttgctgaagc cccatgtc 28

<210> 105
<211> 28
<212> DNA
<213> Artificial

<220>
<223> Synthetic Nucleic Acid

<400> 105
tagtttgccg ctgctgaagc cccatgtc 28

<210> 106
<211> 28
<212> DNA
<213> Artificial

<220>
<223> Synthetic Nucleic Acid

<400> 106
tgccgctgcc caatcaaaaa tgaagccc 28

<210> 107
<211> 28
<212> DNA
<213> Artificial

<220>
<223> Synthetic Nucleic Acid

<400> 107
cagtttgccg ctgccaaaaa tgaagccc 28

<210> 108
<211> 28
<212> DNA
<213> Artificial

<220>
<223> Synthetic Nucleic Acid

<400> 108
tgacaacagt ttgccaaaaa tgaagccc 28

<210> 109
<211> 28
<212> DNA
<213> Artificial

<220>
<223> Synthetic Nucleic Acid

<400> 109
tgccgctgcc caataaaaca aaaatgaa 28

<210> 110
<211> 28
<212> DNA
<213> Artificial

<220>
<223> Synthetic Nucleic Acid

<400> 110
cagtttgccg ctgcaaaaca aaaatgaa 28

<210> 111
<211> 28
<212> DNA
<213> Artificial

<220>
<223> Synthetic Nucleic Acid

<400> 111
tgacaacagt ttgcaaaaca aaaatgaa 28

<210> 112
<211> 21
<212> DNA
<213> Artificial

<220>
<223> Synthetic Nucleic Acid

<400> 112
cccaatgcca tcctggagtt c 21

<210> 113
<211> 28
<212> DNA
<213> Artificial

<220>
<223> Synthetic Nucleic Acid

<400> 113
aatgcctgac ttacttgcca ttgtttca 28

<210> 114
<211> 28
<212> DNA
<213> Artificial

<220>
<223> Synthetic Nucleic Acid

<400> 114
agtgctaaag cggaaatgcc tgacttac 28

<210> 115
<211> 28

<212> DNA
<213> Artificial

<220>
<223> Synthetic Nucleic Acid

<400> 115
tgctgagaat tgttcaattg gatccaca 28

<210> 116
<211> 28
<212> DNA
<213> Artificial

<220>
<223> Synthetic Nucleic Acid

<400> 116
tgtccctggc ttgtcagtta caagtaca 28

<210> 117
<211> 28
<212> DNA
<213> Artificial

<220>
<223> Synthetic Nucleic Acid

<400> 117
tcaggctgta taaaagcaac tattttgt 28

<210> 118
<211> 28
<212> DNA
<213> Artificial

<220>
<223> Synthetic Nucleic Acid

<400> 118
ctctcctcct tgtccaaata ccgaaata 28

<210> 119
<211> 28
<212> DNA
<213> Artificial

<220>
<223> Synthetic Nucleic Acid

<400> 119
gatgtttcct tctccctctg cctctctc 28

<210> 120
<211> 28
<212> DNA
<213> Artificial

<220>
<223> Synthetic Nucleic Acid

<400> 120
tataaatgat gtttccttct ccctctgc 28

<210> 121
<211> 28
<212> DNA
<213> Artificial

<220>
<223> Synthetic Nucleic Acid

<400> 121
tttttctaag acgagggtgt taagtgga 28

<210> 122
<211> 28
<212> DNA
<213> Artificial

<220>
<223> Synthetic Nucleic Acid

<400> 122
tgcaaatgtt ttcctggtca gagcatgt 28

<210> 123
<211> 28
<212> DNA
<213> Artificial

<220>
<223> Synthetic Nucleic Acid

<400> 123
tttctccttg accgaagctc tggtttta 28

<210> 124
<211> 28
<212> DNA
<213> Artificial

<220>
<223> Synthetic Nucleic Acid

<400> 124
ccaatcccta agttatttct ctgagcaa 28

<210> 125
<211> 28
<212> DNA
<213> Artificial

<220>
<223> Synthetic Nucleic Acid

<400> 125
caaaaatgtc aacttttaaa atttaata 28

<210> 126
<211> 28
<212> DNA
<213> Artificial

<220>
<223> Synthetic Nucleic Acid

<400> 126
gctttccctg taaaatatta aataaaca 28

<210> 127
<211> 28
<212> DNA
<213> Artificial

<220>
<223> Synthetic Nucleic Acid

<400> 127
taaataaaac agacaattca tacagatg 28

<210> 128
<211> 28
<212> DNA
<213> Artificial

<220>
<223> Synthetic Nucleic Acid

<400> 128
aataatttca ttactatatg gatcaagt 28

<210> 129
<211> 26
<212> DNA
<213> Artificial

<220>
<223> Synthetic Nucleic Acid

<400> 129
aaagtgtacc ccagtgccaa taattt 26

<210> 130
<211> 28
<212> DNA
<213> Artificial

<220>
<223> Synthetic Nucleic Acid

<400> 130
tattttatgg aagtgatagg caataaaa 28

<210> 131
<211> 28
<212> DNA
<213> Artificial

<220>
<223> Synthetic Nucleic Acid

<400> 131
tatatcttct agtctatgga caaaatgt 28

<210> 132
<211> 28
<212> DNA
<213> Artificial

<220>
<223> Synthetic Nucleic Acid

<400> 132
tgtatttata actttataaa gttcacaa 28

<210> 133
<211> 28
<212> DNA
<213> Artificial

<220>
<223> Synthetic Nucleic Acid

<400> 133
tattccttgc cattatgagt tggaaagt 28

<210> 134
<211> 28
<212> DNA
<213> Artificial

<220>
<223> Synthetic Nucleic Acid

<400> 134
aaatgggcat cttattagtt gtaataga 28

<210> 135
<211> 28
<212> DNA
<213> Artificial

<220>
<223> Synthetic Nucleic Acid

<400> 135
attgccttct cctgttatta tgtagatt 28

<210> 136
<211> 28

<212>  DNA
<213>  Artificial

<220>
<223>  Synthetic Nucleic Acid

<400>  136
agatacatct caaatccctt ttcttggc    28

<210>  137
<211>  28
<212>  DNA
<213>  Artificial

<220>
<223>  Synthetic Nucleic Acid

<400>  137
agacatttca atcaggctaa actaacaa    28

<210>  138
<211>  28
<212>  DNA
<213>  Artificial

<220>
<223>  Synthetic Nucleic Acid

<400>  138
ttgcaaaata taaataatta ttagttca    28

<210>  139
<211>  26
<212>  DNA
<213>  Artificial

<220>
<223>  Synthetic Nucleic Acid

<400>  139
acaagcgatg aatgtgaatt tggaga    26

<210>  140
<211>  26
<212>  DNA
<213>  Artificial

<220>
<223>  Synthetic Nucleic Acid

<400>  140
attaccaaac aaaagaaaca agcgat    26

<210>  141
<211>  26
<212>  DNA
<213>  Artificial

<220>
<223> Synthetic Nucleic Acid

<400> 141
ggaagaagaa tatgtgcaga attacc 26

<210> 142
<211> 26
<212> DNA
<213> Artificial

<220>
<223> Synthetic Nucleic Acid

<400> 142
ggacagcagg aagaagaata tgtgca 26

<210> 143
<211> 28
<212> DNA
<213> Artificial

<220>
<223> Synthetic Nucleic Acid

<400> 143
tcaccttgga ggtcctacag gacagcag 28

<210> 144
<211> 28
<212> DNA
<213> Artificial

<220>
<223> Synthetic Nucleic Acid

<400> 144
ttagatctgt cgccctacct cttttttc 28

<210> 145
<211> 28
<212> DNA
<213> Artificial

<220>
<223> Synthetic Nucleic Acid

<400> 145
tctcatgaaa tattcttcta aagaaagc 28

<210> 146
<211> 28
<212> DNA
<213> Artificial

<220>
<223> Synthetic Nucleic Acid

<400> 146
atgttagtgc ctttcaccct gcttataa 28

<210> 147
<211> 28
<212> DNA
<213> Artificial

<220>
<223> Synthetic Nucleic Acid

<400> 147
gctgttgatt aatggttgat aggttctt 28

<210> 148
<211> 28
<212> DNA
<213> Artificial

<220>
<223> Synthetic Nucleic Acid

<400> 148
tgaaaaaaag aaataaaaaa tttcttta 28

<210> 149
<211> 28
<212> DNA
<213> Artificial

<220>
<223> Synthetic Nucleic Acid

<400> 149
taactagcca caagtatata ttttagta 28

<210> 150
<211> 28
<212> DNA
<213> Artificial

<220>
<223> Synthetic Nucleic Acid

<400> 150
tcaagaatct ctaaatgata agagatta 28

<210> 151
<211> 28
<212> DNA
<213> Artificial

<220>
<223> Synthetic Nucleic Acid

<400> 151
ttcactttga acaaagtaat ttcaatat 28

<210> 152
<211> 28
<212> DNA
<213> Artificial

<220>
<223> Synthetic Nucleic Acid

<400> 152
accatacata atttaagaaa attcattc 28

<210> 153
<211> 28
<212> DNA
<213> Artificial

<220>
<223> Synthetic Nucleic Acid

<400> 153
tgttaaccat acataattta agaaaatt 28

<210> 154
<211> 28
<212> DNA
<213> Artificial

<220>
<223> Synthetic Nucleic Acid

<400> 154
aaagatgtta accatacata atttaaga 28

<210> 155
<211> 28
<212> DNA
<213> Artificial

<220>
<223> Synthetic Nucleic Acid

<400> 155
aagcaattta aaagatgtta accataca 28

<210> 156
<211> 28
<212> DNA
<213> Artificial

<220>
<223> Synthetic Nucleic Acid

<400> 156
atttaaaaat aagcaattta aaagatgt 28

<210> 157
<211> 28

<212> DNA
<213> Artificial

<220>
<223> Synthetic Nucleic Acid

<400> 157
tggcaattta aaaataagca atttaaaa 28

<210> 158
<211> 28
<212> DNA
<213> Artificial

<220>
<223> Synthetic Nucleic Acid

<400> 158
aaacatggca atttaaaaat aagcaatt 28

<210> 159
<211> 28
<212> DNA
<213> Artificial

<220>
<223> Synthetic Nucleic Acid

<400> 159
tatttgttaa tgcaaactgg gacacaaa 28

<210> 160
<211> 28
<212> DNA
<213> Artificial

<220>
<223> Synthetic Nucleic Acid

<400> 160
ttattttttt ttccaacata gttctcaa 28

<210> 161
<211> 24
<212> DNA
<213> Artificial

<220>
<223> Synthetic Nucleic Acid

<400> 161
tctagcctgg agaaagaaga ataa 24

<210> 162
<211> 24
<212> DNA
<213> Artificial

<220>
<223> Synthetic Nucleic Acid

<400> 162
tcttctagcc tggagaaaga agaa 24

<210> 163
<211> 24
<212> DNA
<213> Artificial

<220>
<223> Synthetic Nucleic Acid

<400> 163
ttttgttctt ctagcctgga gaaa 24

<210> 164
<211> 28
<212> DNA
<213> Artificial

<220>
<223> Synthetic Nucleic Acid

<400> 164
ttgagaaaat aaaattacct tgacttgc 28

<210> 165
<211> 28
<212> DNA
<213> Artificial

<220>
<223> Synthetic Nucleic Acid

<400> 165
acttctttat gcaagcaggc cctggggg 28

<210> 166
<211> 28
<212> DNA
<213> Artificial

<220>
<223> Synthetic Nucleic Acid

<400> 166
caatgattga attaaaaaat agattcat 28

<210> 167
<211> 28
<212> DNA
<213> Artificial

<220>
<223> Synthetic Nucleic Acid

<400> 167
gaactatgaa taacctaatg ggcagaaa 28

<210> 168
<211> 28
<212> DNA
<213> Artificial

<220>
<223> Synthetic Nucleic Acid

<400> 168
ataaagttgt gagaaaaaca ctttagca 28

<210> 169
<211> 28
<212> DNA
<213> Artificial

<220>
<223> Synthetic Nucleic Acid

<400> 169
actggttcag aactgcaggg ttaagaag 28

<210> 170
<211> 28
<212> DNA
<213> Artificial

<220>
<223> Synthetic Nucleic Acid

<400> 170
acacacatat atacatatgt tcttatgt 28

<210> 171
<211> 28
<212> DNA
<213> Artificial

<220>
<223> Synthetic Nucleic Acid

<400> 171
ctcacccccct cagtagataa atctctgt 28

<210> 172
<211> 28
<212> DNA
<213> Artificial

<220>
<223> Synthetic Nucleic Acid

<400> 172
tctgagcaca gagctgattg actgaaac 28

<210> 173
<211> 28
<212> DNA
<213> Artificial

<220>
<223> Synthetic Nucleic Acid

<400> 173
agtggtacct caaataccaa cagttttc 28

<210> 174
<211> 28
<212> DNA
<213> Artificial

<220>
<223> Synthetic Nucleic Acid

<400> 174
atcaaaatga agcgacttga ccgagggc 28

<210> 175
<211> 28
<212> DNA
<213> Artificial

<220>
<223> Synthetic Nucleic Acid

<400> 175
taccttgtct ttgcttctat tgattagt 28

<210> 176
<211> 28
<212> DNA
<213> Artificial

<220>
<223> Synthetic Nucleic Acid

<400> 176
gtttaaaatg aattacagca caattcca 28

<210> 177
<211> 28
<212> DNA
<213> Artificial

<220>
<223> Synthetic Nucleic Acid

<400> 177
ttccaccagt aactgaaaca gacaaatg 28

<210> 178
<211> 29

<212> DNA
<213> Artificial

<220>
<223> Synthetic Nucleic Acid

<400> 178
tggcgcaggg gcaactcttc caccagtaa 29

<210> 179
<211> 28
<212> DNA
<213> Artificial

<220>
<223> Synthetic Nucleic Acid

<400> 179
ttaatgtcta acctttatcc actggaga 28

<210> 180
<211> 28
<212> DNA
<213> Artificial

<220>
<223> Synthetic Nucleic Acid

<400> 180
tggaaacctg aaaggaaaat acatttta 28

<210> 181
<211> 28
<212> DNA
<213> Artificial

<220>
<223> Synthetic Nucleic Acid

<400> 181
cttgtttctc aggtaaagct ctggaaac 28

<210> 182
<211> 28
<212> DNA
<213> Artificial

<220>
<223> Synthetic Nucleic Acid

<400> 182
aagcaaaaag ttccctacct gaacgtca 28

<210> 183
<211> 28
<212> DNA
<213> Artificial

<220>
<223> Synthetic Nucleic Acid

<400> 183
cagtttcctg gggaaaagaa cccatata 28

<210> 184
<211> 26
<212> DNA
<213> Artificial

<220>
<223> Synthetic Nucleic Acid

<400> 184
atctcttcca catccggttg tttagc 26

<210> 185
<211> 28
<212> DNA
<213> Artificial

<220>
<223> Synthetic Nucleic Acid

<400> 185
tagaggttgc ttcattacct tcactggc 28

<210> 186
<211> 28
<212> DNA
<213> Artificial

<220>
<223> Synthetic Nucleic Acid

<400> 186
accctacaaa tatttatcaa ttgctcca 28

<210> 187
<211> 28
<212> DNA
<213> Artificial

<220>
<223> Synthetic Nucleic Acid

<400> 187
taaaggaatt ataattattt tagccaac 28

<210> 188
<211> 28
<212> DNA
<213> Artificial

<220>
<223> Synthetic Nucleic Acid

<400> 188
taaattaact ttagtgggta gaatttct 28

<210> 189
<211> 28
<212> DNA
<213> Artificial

<220>
<223> Synthetic Nucleic Acid

<400> 189
tattattgga tttctattat attttact 28

<210> 190
<211> 28
<212> DNA
<213> Artificial

<220>
<223> Synthetic Nucleic Acid

<400> 190
tcatgaacat cttaatccat ttggtgaa 28

<210> 191
<211> 28
<212> DNA
<213> Artificial

<220>
<223> Synthetic Nucleic Acid

<400> 191
tacttattcg attaacactt tgaagata 28

<210> 192
<211> 28
<212> DNA
<213> Artificial

<220>
<223> Synthetic Nucleic Acid

<400> 192
acttcctctt taacagaaaa gcatacac 28

<210> 193
<211> 28
<212> DNA
<213> Artificial

<220>
<223> Synthetic Nucleic Acid

<400> 193
gggatccagt atacttacag gctccaat 28

<210> 194
<211> 25
<212> DNA
<213> Artificial

<220>
<223> Synthetic Nucleic Acid

<400> 194
cttacaggct ccaatagtgg tcagt 25

<210> 195
<211> 28
<212> DNA
<213> Artificial

<220>
<223> Synthetic Nucleic Acid

<400> 195
tgagtaggag ctaaaatatt ttgggttt 28

<210> 196
<211> 28
<212> DNA
<213> Artificial

<220>
<223> Synthetic Nucleic Acid

<400> 196
tatcattttt tctcatacct tctgcttg 28

<210> 197
<211> 28
<212> DNA
<213> Artificial

<220>
<223> Synthetic Nucleic Acid

<400> 197
ttagtatcag ggttcttcag cgttgtgt 28

<210> 198
<211> 28
<212> DNA
<213> Artificial

<220>
<223> Synthetic Nucleic Acid

<400> 198
gcattgttgc ctgtaagaac aaatatcc 28

<210> 199
<211> 30

<212> DNA
<213> Artificial

<220>
<223> Synthetic Nucleic Acid

<400> 199
aactggggac gcctctgttc caaatcctgc 30

<210> 200
<211> 28
<212> DNA
<213> Artificial

<220>
<223> Synthetic Nucleic Acid

<400> 200
gcttgttaaa aaacttactt cgatccgt 28

<210> 201
<211> 28
<212> DNA
<213> Artificial

<220>
<223> Synthetic Nucleic Acid

<400> 201
aatattagtt tctgttaaat tattttcc 28

<210> 202
<211> 28
<212> DNA
<213> Artificial

<220>
<223> Synthetic Nucleic Acid

<400> 202
acaacaggat tctttgcttt tttgatgg 28

<210> 203
<211> 28
<212> DNA
<213> Artificial

<220>
<223> Synthetic Nucleic Acid

<400> 203
ttattcattg tgttatggct aggatgat 28

<210> 204
<211> 28
<212> DNA
<213> Artificial

<220>
<223> Synthetic Nucleic Acid

<400> 204
atctcacatt tatgttgctt atttaaaa 28

<210> 205
<211> 28
<212> DNA
<213> Artificial

<220>
<223> Synthetic Nucleic Acid

<400> 205
gagacatttt aaatgtaact tccaaacg 28

<210> 206
<211> 28
<212> DNA
<213> Artificial

<220>
<223> Synthetic Nucleic Acid

<400> 206
aaatatatag tagtaaatgc tagtctgg 28

<210> 207
<211> 28
<212> DNA
<213> Artificial

<220>
<223> Synthetic Nucleic Acid

<400> 207
attctttcaa ctagaataaa aggaaaaa 28

<210> 208
<211> 28
<212> DNA
<213> Artificial

<220>
<223> Synthetic Nucleic Acid

<400> 208
ggtatctttg atactaacct tggtttct 28

<210> 209
<211> 28
<212> DNA
<213> Artificial

<220>
<223> Synthetic Nucleic Acid

<400> 209
tggccaactg ctatagattt ttatgaga 28

<210> 210
<211> 28
<212> DNA
<213> Artificial

<220>
<223> Synthetic Nucleic Acid

<400> 210
aaataatgta attcatacct tttatgaa 28

<210> 211
<211> 28
<212> DNA
<213> Artificial

<220>
<223> Synthetic Nucleic Acid

<400> 211
ccccattatt acagccaaca gtagtttt 28

<210> 212
<211> 28
<212> DNA
<213> Artificial

<220>
<223> Synthetic Nucleic Acid

<400> 212
caaatcctca tggtccatcc agtttcac 28

<210> 213
<211> 28
<212> DNA
<213> Artificial

<220>
<223> Synthetic Nucleic Acid

<400> 213
ccaagctcca gtttagctgg attggaaa 28

<210> 214
<211> 28
<212> DNA
<213> Artificial

<220>
<223> Synthetic Nucleic Acid

<400> 214
tttagttggt atttatcgtc ttgaaccc 28

<210> 215
<211> 28
<212> DNA
<213> Artificial

<220>
<223> Synthetic Nucleic Acid

<400> 215
taaaatagaa gtctgagcca agtccgtg 28

<210> 216
<211> 28
<212> DNA
<213> Artificial

<220>
<223> Synthetic Nucleic Acid

<400> 216
gggcaaatga gatcttatgt tcctcgtt 28

<210> 217
<211> 28
<212> DNA
<213> Artificial

<220>
<223> Synthetic Nucleic Acid

<400> 217
tttactttca tttttattta acttaaag 28

<210> 218
<211> 28
<212> DNA
<213> Artificial

<220>
<223> Synthetic Nucleic Acid

<400> 218
taagcaacaa ctataatatt gtgcagtc 28

<210> 219
<211> 28
<212> DNA
<213> Artificial

<220>
<223> Synthetic Nucleic Acid

<400> 219
tggggtgagt tgttgctaca gctcttcc 28

<210> 220
<211> 28

<212> DNA
<213> Artificial

<220>
<223> Synthetic Nucleic Acid

<400> 220
tggaggaact aaattgtaat ataccaac 28

<210> 221
<211> 28
<212> DNA
<213> Artificial

<220>
<223> Synthetic Nucleic Acid

<400> 221
cacctagtga actccataaa aagagaaa 28

<210> 222
<211> 28
<212> DNA
<213> Artificial

<220>
<223> Synthetic Nucleic Acid

<400> 222
cagatgcaat tattaaatat cagaatgg 28

<210> 223
<211> 28
<212> DNA
<213> Artificial

<220>
<223> Synthetic Nucleic Acid

<400> 223
ctcactcacc ctgcaaagga ccaaatgt 28

<210> 224
<211> 23
<212> DNA
<213> Artificial

<220>
<223> Synthetic Nucleic Acid

<400> 224
agtttcttcc aaagcagcct ctc 23

<210> 225
<211> 30
<212> DNA
<213> Artificial

<220>
<223>  Synthetic Nucleic Acid

<400>  225
acatctcaaa tccctgtcca gatgtgctga    30

<210>  226
<211>  30
<212>  DNA
<213>  Artificial

<220>
<223>  Synthetic Nucleic Acid

<400>  226
acatttcaat caggcgtcca gatgtgctga    30

<210>  227
<211>  30
<212>  DNA
<213>  Artificial

<220>
<223>  Synthetic Nucleic Acid

<400>  227
tggagaattg caaaagtcca gatgtgctga    30

<210>  228
<211>  30
<212>  DNA
<213>  Artificial

<220>
<223>  Synthetic Nucleic Acid

<400>  228
gtccagatgt gctgaacatc tcaaatccct    30

<210>  229
<211>  30
<212>  DNA
<213>  Artificial

<220>
<223>  Synthetic Nucleic Acid

<400>  229
gtccagatgt gctgaacatt tcaatcaggc    30

<210>  230
<211>  30
<212>  DNA
<213>  Artificial

<220>
<223>  Synthetic Nucleic Acid

<400> 230
gtccagatgt gctgatggag aattgcaaaa 30

<210> 231
<211> 40
<212> DNA
<213> Artificial

<220>
<223> Synthetic Nucleic Acid

<400> 231
gttaaccata cataatttaa acagtttgcc gctgcccaat 40

<210> 232
<211> 40
<212> DNA
<213> Artificial

<220>
<223> Synthetic Nucleic Acid

<400> 232
tggcaattta aaaataagca acagtttgcc gctgcccaat 40

<210> 233
<211> 420
<212> DNA
<213> Homo Sapiens

<400> 233
aatacaaatg gtatcttaag gtaagtcttt gatttgtttt ttcgaaattg tatttatctt 60

cagcacatct ggactcttta acttcttaaa gatcaggttc tgaagggtga tggaaattac 120

ttttgactgt tgttgtcatc attatattac tagaaagaaa attatcataa tgataatatt 180

agagcacggt gctatggact ttttgtgtca ggatgagaga gtttgcctgg acggagctgg 240

tttatctgat aaactgcaaa atataattga atctgtgaca gagggaagca tcgtaacagc 300

aaggtgtttt gtggctttgg ggcagtgtgt atttcggctt tatgttggaa cctttccaga 360

aggagaactt gtggcatact tagctaaaat gaagttgcta gaaatatcca tcatgataaa 420

<210> 234
<211> 650
<212> DNA
<213> Homo Sapiens

<400> 234
tcttgatggg atgctcctga aagcaattaa ttctcagttt tttgtggctt ctaatgcaaa 60

atacattgac gcagacagaa tttgaaatga attttcttct aatatagcaa ttaattttat 120

ttaaatatct ctagagtttt tttttaatac tgtgactaac ctatgtttgt tctttttcac 180

ctctcgtatc cacgatcact aagaaaccca aatactttgt tcatgtttaa attttacaac 240

atttcataga ctattaaaca tggaacatcc ttgtggggac aagaaatcga atttgctctt 300

gaaaaggttt ccaactaatt gatttgtagg acattataac atcctctagc tgacaagctt 360

acaaaaataa aaactggagc taaccgagag ggtgcttttt tccctgacac ataaaaggtg 420

tctttctgtc ttgtatcctt tggatatggg catgtcagtt tcatagggaa attttcacat 480

ggagcttttg tatttctttc tttgccagta caactgcatg tggtagcaca ctgtttaatc 540

ttttctcaaa taaaaagaca tggggcttca tttttgtttt gccttttttgg tatcttacag 600

gaactccagg atggcattgg gcagcggcaa actgttgtca gaacattgaa 650

<210> 235
<211> 420
<212> DNA
<213> Homo Sapiens

<400> 235
ctgtcagaca gaaaaaagag gtagggcgac agatctaata ggaatgaaaa cattttagca 60

gactttttaa gctttcttta gaagaatatt tcatgagaga ttataagcag ggtgaaaggc 120

actaacatta aagaacctat caaccattaa tcaacagcag taaagaaatt ttttatttct 180

ttttttcata tactaaaata tatacttgtg gctagttagt ggttttctgc tattttaaac 240

ttgaagtttg ctttaaaaat cacccatgat tgcttaaagg tgaatatctt caatatattt 300

taacttcaac aagctgaatc tcagttgttt ttcaagaaga ttttagaaag caattataaa 360

tgattgtttt gtaggaaaga cagatctttg cttagtttta aaaatagcta tgaatatgac 420

<210> 236
<211> 450
<212> DNA
<213> Homo Sapiens

<400> 236
attacaggcg cctgccacca aacctggcaa atttttgtat ttttagtgta gacggggttt 60

caccatattt gccaggctgg tcgcaaactc ctgacctcaa gtgatccgcc cacatcggcc 120

tccctaagcg ctagggttac aggcatgagc cactgcgcct ggccaggaat ttttgaatca 180

gaatttttct tgttcgattt taatctctta tcatttagag attcttgaaa tattgaaatt 240

actttgttca aagtgaatga attttcttaa attatgtatg gttaacatct tttaaattgc 300

ttatttttaa attgccatgt ttgtgtccca gtttgcatta acaaatagtt tgagaactat 360

gttggaaaaa aaaataacaa ttttattctt ctttctccag gctagaagaa caaaagaata 420

tcttgtcaga atttcaaaga gatttaaatg 450

<210> 237
<211> 420
<212> DNA
<213> Homo Sapiens

<400> 237
aaaagcttga gcaagtcaag gtaattttat tttctcaaat cccccagggc ctgcttgcat 60

aaagaagtat atgaatctat tttttaattc aatcattggt tttctgccca ttaggttatt 120

catagttcct tgctaaagtg tttttctcac aactttattt cttcttaacc ctgcagttct 180

gaaccagtgc acataagaac atatgtatat atgtgtgtgt gtgtatttat atatacacac 240

acacatattg catctataca tctacacata tagatgtata gattcaatat gtctaaatgt 300

atataattca cagtttttat ctttgatttg aaattaattt tagattttac ttgagaactt 360

cacaacttca tataatttta aaaactgaag accagattgt ggaatcataa aatctaaatc 420

<210> 238
<211> 450
<212> DNA
<213> Homo Sapiens

<400> 238
ataatgcaat ttctcagcag caagctacgg tatgctatgg catgctatga tacccaagag 60

gctgatgaat ttgttcacat tgttctattt ctgatagaga gataggtttt cagacactaa 120

ctttatttgg agtgttgctt taccatctca catttttctc ttaaaaaatt tatgagggat 180

aatataatcg tttattttct acagagattt atctactgag ggggtgagtg tttcagtcaa 240

tcagctctgt gctcagatag aaaactgttg gtatttgagg taccactggg ccctcggtca 300

agtcgcttca ttttgataga ctaatcaata gaagcaaaga caaggtagtt ggaattgtgc 360

tgtaattcat tttaaacgtt gttgcatttg tctgtttcag ttactggtgg aagagttgcc 420

cctgcgccag ggaattctca aacaattaaa 450

<210> 239
<211> 420
<212> DNA
<213> Homo Sapiens

<400> 239
caaatctcca gtggataaag gttagacatt aaccatctct tccgtcacat gtgttaaatg 60

ttgcaagtat ttgtatgtat tttgtttcct gggtgcttca ttggtcgggg aggaggctgg 120

tatgtggatt gttgttttgt tttgtttttt taacctgacc gtttgctttg gctatatgtt 180

ttgttgtggc tagaaaaaat gatgatggtg aatggcttta cattaatgac caaatgccaa 240

aatttatacc acaatttttt gcataaatta ttctgaagaa tcagactgaa gaaatggcga 300

agtatttaat tcagtggcca ggcatgtact gacagtattt aagctgaaag gacgtggtct 360

ggttctagtt aaacaagtgt cataaatcaa aattaattat tcacacctgt ggtatggact 420

<210> 240
<211> 450

<212> DNA
<213> Homo Sapiens

<400> 240
tattcatttt tataactgca aaggaagcgc gtatggcata taatacacaa cacaccagta 60

tatttagtaa ctgagtgaat aaatgaaaga tgtatttctt tactttatca gttgcagttg 120

gctatgcctt tgtgtaaggt gtgtgttttg aaattccaaa aaggtattag tttctttaaa 180

gcaaagaatt tttgtagcag gttaatgaat aattttgaat acattggtta aatcccaaca 240

tgtaatatat gtaaataatc aatattatgc tgctaaaata acacaaatca gtaagattct 300

gtaatatttc atgataaata acttttgaaa atatattttt aaacattttg gcttatgcct 360

tgagaattat ttaccttttt aaaatgtatt ttcctttcag gtttccagag ctttacctga 420

gaaacaagga gaaattgaag ctcaaataaa 450

<210> 241
<211> 420
<212> DNA
<213> Homo Sapiens

<400> 241
aaggaccatt tgacgttcag gtagggaact ttttgcttta aatatttttg tcttttttaa 60

gaaaaatggc aatatcactg aattttctca tttggtatca ttattaaaga caaaatatta 120

cttgttaaag tgtggtaagg aagactttat tcaggataac cacaataggc acagggacca 180

ctgcaatgga gtattacagg aggttggata gagagagatt gggctcaact ctaaatacag 240

cacagtggaa gtaggaattt atagccaagg agcagtgtag gagtcagtag atggaaaatt 300

attaagagga aacatcaggg gtaagtggga ttctggctaa accaacctca caggattctt 360

gctgaagata ggccagggtt atcttatcag acaacccttg gggaatggtg gagaatactg 420

<210> 242
<211> 450
<212> DNA
<213> Homo Sapiens

<400> 242
tactaaacac agaattttgt aaaacaataa gtgtataaag taaaatgaac attaggatta 60

ttgagattat tgtagctaaa actagtgttt attcatataa attatgttaa taaattgtat 120

tgtcattatt gcattttact tttttgaaaa gtagttaatg cctgtgtttc tatatgagta 180

ttatataatt caagaagata ttggatgaat ttttttaag tttaatgtgt ttcacatctc 240

tgtttctttt ctctgcacca aaagctacat ttttgtgccc ttatgtacca ggcagaaatt 300

gatctgcaat acatgtggag tctccaaggg tatatttaaa tttagtaatt ttattgctaa 360

ctgtgaagtt aatctgcact atatgggttc ttttccccag gaaactgaaa tagcagttca 420

agctaaacaa ccggatgtgg aagagatttt 450

<210> 243
<211> 420
<212> DNA
<213> Homo Sapiens

<400> 243
cagccactca gccagtgaag gtaatgaagc aacctctagc aatatccatt acctcataat 60

gggttatgct tcccctgttg tacatttgcc attgacgtgg actatttata atcagtgaaa 120

taacttgtaa ggaaatactg gccatactgt aatagcagag gcaaagctgt ctttttgatc 180

agcatatcct atttatatat tgtgatctta aggctattaa cgagtcattg ctttaaagga 240

ctcatttctg tcctggtgtg ctgccatcaa tacaaaagta gtcccacctt caaggtagat 300

taaattcttt ggggctttat tgctttgctt gccagccttg atgcttttca tattgtttgg 360

tttaattcaa atcaagctac tgcatcatag tgtctgtctc caacagctgt aaagaatcac 420

<210> 244
<211> 450
<212> DNA
<213> Homo Sapiens

<400> 244
atataattga ctgggggtga gccagtacat taggattttc ctaaagttat ctggataatt 60

ttagtatgca accacaatag atactcttca agaattaagc tagttgctga gagggaactg 120

tttttgttg gtttgttttc actaatgttt gcactctact tcctttaaat aaaattatgc 180

ctggagaaag ggtttttgta tggagcaatt gataaatatt tgtagggtgg ttggctaaaa 240

taattataat tcctttaaaa gaaattctac ccactaaagt taatttagaa gtaaaatata 300

atagaaatcc aataatatat tcaccaaatg gattaagatg ttcatgaatt atcttcaaag 360

tgttaatcga ataagtaatg tgtatgcttt tctgttaaag aggaagttag aagatctgag 420

ctctgagtgg aaggcggtaa accgtttact 450

<210> 245
<211> 420
<212> DNA
<213> Homo Sapiens

<400> 245
actgaccact attggagcct gtaagtatac tggatcccat tctctttggc tctagctatt 60

tgttcaaaag tgcaactatg aagtgatgac tgggtgagag agaaaatttg tttcaattct 120

aaagatagag ataaaccttt gtgttattga ctgtgcaaaa agtcttagag tacattcctt 180

ggaaattgac tctgattcaa agtgttgcat gacaacggga tatggggagt gttctctgga 240

gatacaccca caaggaagag aagagcacaa gggagattgt gggagagtct gaaatgtgat 300

ttgtctgcag cagaggccta agccagtctc gcaggagccc tacatctggg ctggctgtgc 360

agagctgtcc tgaattgcag gcagtgggcc tggcccttgt attcctgatc cagccagcca 420

<210> 246
<211> 450
<212> DNA
<213> Homo Sapiens

<400> 246
tcttgaataa aaaaaaaata agtaaaattt atttccctgg caaggtctga aaacttttgt 60

tttctttacc acttccacaa tgtatatgat tgttactgag aaggcttatt taacttaagt 120

tacttgtcca ggcatgagaa tgagcaaaat cgttttttaa aaaattgtta aatgtatatt 180

aatgaaaagg ttgaatcttt tcattttcta ccatgtattg ctaaacaaag tatccacatt 240

gttagaaaaa gatatataat gtcatgaata agagtttggc tcaaattgtt actcttcaat 300

taaatttgac ttattgttat tgaaattggc tctttagctt gtgtttctaa tttttctttt 360

tcttcttttt tcctttttgc aaaaacccaa aatattttag ctcctactca gactgttact 420

ctggtgacac aacctgtggt tactaaggaa 450

<210> 247
<211> 420
<212> DNA
<213> Homo Sapiens

<400> 247
agatgatcat caagcagaag gtatgagaaa aaatgataaa agttggcaga agtttttctt 60

taaaatgaag attttccacc aatcacttta ctctcctaga ccatttccca ccagttctta 120

ggcaactgtt tctctctcag caaacacatt actctcacta ttcagcctaa gtataatcaa 180

ggatataaat taatgcaaat aacaaaagta gccatacatt aaaaaggaaa tatacaaaaa 240

aaaaaaaaaa aaaaagcaga aaccttacaa gaatagttgt ctcagttaaa tttactaaac 300

aacctggtat tttaaaaatc tattttatac caaataagtc actcaactga gctatttaca 360

tttaaactgt ttgttttggc actacgcagc ccaacatatt gcagaatcaa atataatagt 420

<210> 248
<211> 450
<212> DNA
<213> Homo Sapiens

<400> 248
tttaaaataa atatacctta attttgacgt cacacagaat gatattataa gtataaatag 60

ttatctatct tttaaataca ttgtcgtaat tcagaataac atttcttact caaggcattc 120

agacagtggt ttaagtaatc cgaggtactc cggaatgtct ccatttgagc ctttaaatga 180

agaaaatcta tagtcaagat tttcatttga aatatttttg atatctaaga atgaaacata 240

tttcctgtta aattgttttc tataaaccct tatacagtaa catctttttt atttctaaaa 300

gtgttttggc tggtctcaca attgtacttt actttgtatt atgtaaaagg aatacacaac 360

gctgaagaac cctgatacta agggatattt gttcttacag gcaacaatgc aggatttgga 420

acagaggcgt ccccagttgg aagaactcat 450

<210> 249
<211> 420
<212> DNA
<213> Homo Sapiens

<400> 249
aacaatcatt acggatcgaa gtaagttttt taacaagcat gggacacaca aagcaagatg 60

catgacaagt ttcaataaaa acttaagttc atatatcccc ctcacattta taaaaataat 120

gtgaaataat tgtaaatgat aacaattgtg ctgagatttt cagtccataa tgttaccttt 180

taataaatga atgtaattcc attgaataga agaaatacat ttttaaatca attcagggct 240

tatatagttg caaagcatgc attgatgggt gtggtgacca cagtgtggca gaacatttgt 300

ggcagaacat ttgttcttta gttgtcatct gggctggcat ccatggagat gccagtctct 360

ccctcatatc cttggctgtt ggtccaagca ggcagtggct tcttcctggg ccatctttca 420

<210> 250
<211> 450
<212> DNA
<213> Homo Sapiens

<400> 250
caaactcctg tggcaacaga aagccttcag gcaatgaaat gctggcactg ggaaatcagg 60

ctgatgggtg ctgaagtggc aaggatgagg ggatatggat attctgctgt agtgcttttc 120

taacagatga ttcatatttg gttctaggga tcaagaattg agttaaaatt ttatatatat 180

gttgatgttc tatgtcacct tcaggaaaat aatttaacag aaactaatat ttgccatcaa 240

aaaagcaaag aatcctgttg ttcatcatcc tagccataac acaatgaata atttttttaaa 300

taagcaacat aaatgtgaga taacgtttgg aagttacatt taaaatgtct cctccagact 360

agcatttact actatatatt tatttttcct tttattctag ttgaaagaat tcagaatcag 420

tgggatgaag tacaagaaca ccttcagaac 450

<210> 251
<211> 420
<212> DNA
<213> Homo Sapiens

<400> 251
agaaaatcac agaaaccaag gttagtatca aagatacctt tttaaaataa aatactggtt 60

acatttgata aaattatacc atagattgta atttaatgat gtttaatgta aagttattaa 120

cagaaaatca cgttaaagct gaaatgaaca gtagactttg tatatttatt ttcttagaga 180

cagagtctca ctgtcaccca ggctaaagtg cagtggcaca atcatagctc actgagcctt 240

gaactctggg gctcaagcag tcctcctgcc tcagcctccc tagtagctgg gactactagc 300

caggcgtgta ccaccacgcc tggctaattt tttaaaaatt tttgttttct gtagagatgg 360

gttcttgaac tcttggcctc aagcaattct ccttccttgg cctcccaaag cactaggatt 420

<210> 252
<211> 450
<212> DNA
<213> Homo Sapiens

<400> 252
caacaagtta atgaagaggg aaagaaatgt atgaggtttt tttcgttcaa atgttgttat 60

atgtcacata ttcaacaatt atatatgagc ttatttttgt agttttttttc tcttgtgata 120

aaaacaatta agcccacttt attgccaatt aattgctact aagttgaaat acttgatact 180

ggttattgct caagatgctg catttgaaaa gtttgtcctg aaaggtgggt taccttatac 240

tgtcatgatt gactaaatca tatggtaggt taaaagcaat ctaatatatg tattctgacc 300

tgaggattca gaagctgttt acgaagtatt ttaagacact ccaactagag atttcataaa 360

aaaaactgac attcattctc tttctcataa aaatctatag cagttggcca aagacctccg 420

ccagtggcag acaaatgtag atgtggcaaa 450

<210> 253
<211> 420
<212> DNA
<213> Homo Sapiens

<400> 253
tggagaagca ttcataaaag gtatgaatta cattatttct aaaactactg ttggctgtaa 60

taatggggtg gtgaaactgg atggaccatg aggatttgtt tttccaatcc agctaaactg 120

gagcttggga gggttcaaga cgataaatac caactaaact cacggacttg gctcagactt 180

ctattttaaa aacgaggaac ataagatctc atttgcccgc tgtcacaaaa gtagtgacat 240

aaccaagaga ttaaacaaaa agcaaaatac tgatttatag ctagaagagc catttatcag 300

tctactttga taactctatc caaaggaata tctttctatc tcatcatggc gcacactgcc 360

ttacctgtta tctgataaat aagtcacttt gggattcatg atagagttat agctgtacat 420

<210> 254
<211> 450
<212> DNA
<213> Homo Sapiens

<400> 254
tctcaaattt ggcagtatat taaaaataag ctttcaaaat tgaccaacaa aaactacaaa 60

attgaaaaaa aggtactttg aactttcaca tgttcaaata tatgtatata tatttcacat 120

atatatatga aacctcctct gtggagaggg gtttatagaa atctgtaatt gtcattcttg 180

catgccttcc cccatacaaa cgcctttaag ttaaataaaa atgaaagtaa atagactgca 240

caatattata gttgttgctt aaaggaagag ctgtagcaac aactcacccc attgttggta 300

tattacaatt tagttcctcc atctttctct ttttatggag ttcactaggt gcaccattct 360

gatatttaat aattgcatct gaacatttgg tcctttgcag ggtgagtgag cgagaggctg 420

ctttggaaga aactcataga ttactgcaac 450

<210> 255
<211> 420
<212> DNA
<213> Homo Sapiens

<400> 255
agctgatgaa acaatggcaa gtaagtcagg catttccgct ttagcactct tgtggatcca 60

attgaacaat tctcagcatt tgtacttgta actgacaagc cagggacaaa acaaaatagt 120

tgcttttata cagcctgatg tatttcggta tttggacaag gaggagagag gcagagggag 180

aaggaaacat catttataat tccacttaac accctcgtct tagaaaaagt acatgctctg 240

accaggaaaa catttgcata taaaaccaga gcttcggtca aggagaaact ttgctcagag 300

aaataactta gggattggtt tattaaattt taaaagttga catttttgag tgtttattta 360

atattttaca gggaaagcat ctgtatgaat tgtctgtttt atttagcgtt gctaactgaa 420

<210> 256
<211> 450
<212> DNA
<213> Homo Sapiens

<400> 256
acttgatcca tatagtaatg aaattattgg cactggggta cactttatca tagaatttta 60

ttgcctatca cttccataaa ataatacatt ttgtccatag actagaagat ataacttgtg 120

aactttataa agttataaat acattacttt ccaactcata atggcaagga ataaatctat 180

tacaactaat aagatgccca ttttaaatct acataataac aggagaaggc aatacgccaa 240

gaaaagggat ttgagatgta tcttcttgtt agtttagcct gattgaaatg tcttttgaac 300

taataattat ttatattttg caattctcca aattcacatt catcgcttgt ttcttttgtt 360

tggtaattct gcacatattc ttcttcctgc tgtcctgtag gacctccaag gtgaaattga 420

agctcacaca gatgtttatc acaacctgga 450

<210> 257
<211> 24
<212> DNA
<213> Artificial

<220>
<223> Synthetic Nucleic Acid

<400> 257
gtgtgctacc acatgcagtt gtac 24

<210> 258
<211> 24
<212> DNA
<213> Artificial

<220>
<223> Synthetic Nucleic Acid

<400> 258
tctgtcgccc tacctctttt ttct 24

<210> 259
<211> 24
<212> DNA
<213> Artificial

<220>
<223> Synthetic Nucleic Acid

<400> 259
attcctatta gatctgtcgc ccta 24

<210> 260
<211> 24
<212> DNA
<213> Artificial

<220>
<223> Synthetic Nucleic Acid

<400> 260
ttttcattcc tattagatct gtcg 24

<210> 261
<211> 24
<212> DNA
<213> Artificial

<220>
<223> Synthetic Nucleic Acid

<400> 261
aaatgttttc attcctatta gatc 24

<210> 262
<211> 24
<212> DNA
<213> Artificial

<220>
<223> Synthetic Nucleic Acid

<400> 262
ctaaaatgtt ttcattccta ttag 24

<210> 263
<211> 24
<212> DNA
<213> Artificial

<220>
<223> Synthetic Nucleic Acid

<400> 263
agtctgctaa aatgttttca ttcc 24

<210> 264
<211> 24
<212> DNA
<213> Artificial

<220>
<223> Synthetic Nucleic Acid

<400> 264
agaataaaat tgttattttt tttt 24

<210> 265
<211> 24
<212> DNA
<213> Artificial

<220>
<223> Synthetic Nucleic Acid

<400> 265
gcctggagaa agaagaataa aatt 24

<210> 266
<211> 24
<212> DNA
<213> Artificial

<220>
<223> Synthetic Nucleic Acid

<400> 266
tgttcttcta gcctggagaa agaa 24

<210> 267
<211> 24
<212> DNA
<213> Artificial

<220>
<223> Synthetic Nucleic Acid

<400> 267
gatattcttt tgttcttcta gcct 24

<210> 268
<211> 24
<212> DNA
<213> Artificial

<220>
<223> Synthetic Nucleic Acid

<400> 268
atccatgcat tcttccatgc acag 24

<210> 269
<211> 30
<212> DNA
<213> Artificial

<220>
<223> Synthetic Nucleic Acid

<400> 269
tttcattcct attagttgcc gctgcccaat 30

<210> 270
<211> 30
<212> DNA
<213> Artificial

<220>
<223> Synthetic Nucleic Acid

<400> 270
aaatgttttc attccttgcc gctgcccaat 30

<210> 271
<211> 30
<212> DNA
<213> Artificial

<220>
<223> Synthetic Nucleic Acid

<400> 271
tgttttcatt ccagtttgcc gctgcccaat 30

<210> 272
<211> 30
<212> DNA
<213> Artificial

<220>
<223> Synthetic Nucleic Acid

<400> 272
agatctgtcg acagtttgcc gctgcccaat 30

<210> 273
<211> 30

<212> DNA
<213> Artificial

<220>
<223> Synthetic Nucleic Acid

<400> 273
cattcctatt agacagtttg ccgctgccca 30

<210> 274
<211> 24
<212> DNA
<213> Artificial

<220>
<223> Synthetic Nucleic Acid

<400> 274
tgcattcaat gttctgacaa cagt 24

<210> 275
<211> 24
<212> DNA
<213> Artificial

<220>
<223> Synthetic Nucleic Acid

<400> 275
gtttgcagac ctcctgccac cgca 24

<210> 276
<211> 25
<212> DNA
<213> Artificial

<220>
<223> Synthetic Nucleic Acid

<400> 276
atttgacaga tctgttgaga aatgg 25

<210> 277
<211> 22
<212> DNA
<213> Artificial

<220>
<223> Synthetic Nucleic Acid

<400> 277
ggctgttttc atccaggttg tg 22

<210> 278
<211> 21
<212> DNA
<213> Artificial

<220>
<223> Synthetic Nucleic Acid

<400> 278
agttgctgct cttttccagg t 21

<210> 279
<211> 25
<212> DNA
<213> Artificial

<220>
<223> Synthetic Nucleic Acid

<400> 279
atttgacaga tctgttgaga aatgg 25

<210> 280
<211> 21
<212> DNA
<213> Artificial

<220>
<223> Synthetic Nucleic Acid

<400> 280
ggctccaata gtggtcagtc c 21

<210> 281
<211> 20
<212> DNA
<213> Artificial

<220>
<223> Synthetic Nucleic Acid

<400> 281
cctgagaatt gggaacatgc 20

<210> 282
<211> 20
<212> DNA
<213> Artificial

<220>
<223> Synthetic Nucleic Acid

<400> 282
cctccttcca tgactcaagc 20

<210> 283
<211> 450
<212> DNA
<213> Homo Sapiens

<400> 283
attacaaaaa tatggaaata gagagtagca ggaaaaagta tatggcattt ctattaccta 60

gaaccccact gttgtaggtg tctttccttg acatagctgt gaccatatag ctctgttaaa 120

ttgcatattt gtctaaataa ttcatatttt tttctatccc tatcttgcgt ttggagctca 180

ttttatgatt gttattcttt tatctttcaa taaatactca aaatgttaaa aaaatatgat 240

tcaggaattt ttaattgtct tagccacaaa tttataggat ttccaggatc tgtaacaatg 300

gataaataat tttataaaaa tcctaaattt acacagaatt ataaagatat aatcatttta 360

aacagcacct attttgcaaa ttttataaat ctctttctag gactcatctc tgcttctaca 420

atcaacccag aatgtgactg taaatgcgcg 450

<210> 284
<211> 420
<212> DNA
<213> Homo Sapiens

<400> 284
cacaggcagg ttaaaagtcg gtgagtccag cttcatcatg gtgctttgca tgcatgttgt 60

ccatgaatag tgctaaatga atgcattgtt ttttcttcta aagaaatcaa agctacttat 120

gaacaaaata tgaattttct aaatatcatg ctgtgttgac cacagactag caccacagag 180

tggggtgggg ggtgagggga cagcctgaag tgctttgatt agagttattt ttgttccaag 240

aaatagagga caattaaagc tagtacaaga aacaaaatgt ttattgtggg ttaccaaaga 300

ctcttgcagc aaccaaaggg aaggaaccca agtagctggc ctcaaagagc acagagacca 360

gtggctgaga gccacgaata cattcagttc cctccccgtt ctttcttctc ttttctctcc 420

<210> 285
<211> 450
<212> DNA
<213> Homo Sapiens

<400> 285
aagtgatctg cccgccgcag cctcccaaag ttctgggatt acaggcgtga gccactgctc 60

ccagcaaatg tgtgtttgct gctctgtttc cctgcgtgtt tcttgcctgt cttgtgtgta 120

tctttgtgtc tggggccatc ctgccctgtg ctgcccaacc aagccatctc tgcccacagg 180

tgcccgcctg gctgggtggg tgagcggtgt cagctggagg acccctgtca ctcaggcccc 240

tgtgctggcc gtggtgtctg ccagagttca gtggtggctg gcaccgcccg attctcatgc 300

cggtgccccc gtggcttccg aggtgagagg ggaagagtct ggaggggagg tagtcggggg 360

tgtggtcagt cctaaactca ccctgtcctg gtccctccag gccctgactg ctccctgcca 420

gatccctgcc tcagcagccc ttgtgcccac 450

<210> 286
<211> 420
<212> DNA
<213> Homo Sapiens

<400> 286
cgactgtgcc tgtcttcctg gtgagtgagc cctactcagg agagtcagag gggtgggcgt 60

ggggacagca ggccagcccg gcggtgacca tccttgcccc cttccctgct agggtttgag 120

ggtcagaatt gtgaagtgaa cgtggacgac tgtccaggac accgatgtct caatgggggg 180

acatgcgtgg atggcgtcaa cacctataac tgccagtgcc ctcctgagtg gacaggtggg 240

cactgcggcc agagggagcg gggaggcagg cctcgggtgg acatgcgcca ggtggctgga 300

ctgctgcatc tgtgtgccac aggccagttc tgcacggagg acgtggatga gtgtcagctg 360

cagcccaacg cctgccacaa tgggggtacc tgcttcaaca cgctgggtgg ccacagctgc 420

<210> 287
<211> 30
<212> DNA
<213> Artificial

<220>
<223> Synthetic Nucleic Acid

<400> 287
tgctactctc tatttccata tttttgtaat 30

<210> 288
<211> 30
<212> DNA
<213> Artificial

<220>
<223> Synthetic Nucleic Acid

<400> 288
aaatgccata tactttttcc tgctactctc 30

<210> 289
<211> 30
<212> DNA
<213> Artificial

<220>
<223> Synthetic Nucleic Acid

<400> 289
agtggggttc taggtaatag aaatgccata 30

<210> 290
<211> 30
<212> DNA
<213> Artificial

<220>
<223> Synthetic Nucleic Acid

<400> 290
caaggaaaga cacctacaac agtggggttc 30

<210>  291
<211>  30
<212>  DNA
<213>  Artificial

<220>
<223>  Synthetic Nucleic Acid

<400>  291
ctatatggtc acagctatgt caaggaaaga 30

<210>  292
<211>  30
<212>  DNA
<213>  Artificial

<220>
<223>  Synthetic Nucleic Acid

<400>  292
aaatatgcaa tttaacagag ctatatggtc 30

<210>  293
<211>  30
<212>  DNA
<213>  Artificial

<220>
<223>  Synthetic Nucleic Acid

<400>  293
aaaatatgaa ttatttagac aaatatgcaa 30

<210>  294
<211>  30
<212>  DNA
<213>  Artificial

<220>
<223>  Synthetic Nucleic Acid

<400>  294
acgcaagata gggatagaaa aaaatatgaa 30

<210>  295
<211>  30
<212>  DNA
<213>  Artificial

<220>
<223>  Synthetic Nucleic Acid

<400>  295
aatcataaaa tgagctccaa acgcaagata 30

<210>  296

<211> 30
<212> DNA
<213> Artificial

<220>
<223> Synthetic Nucleic Acid

<400> 296
ttgaaagata aaagaataac aatcataaaa 30

<210> 297
<211> 30
<212> DNA
<213> Artificial

<220>
<223> Synthetic Nucleic Acid

<400> 297
tttaacattt tgagtattta ttgaaagata 30

<210> 298
<211> 30
<212> DNA
<213> Artificial

<220>
<223> Synthetic Nucleic Acid

<400> 298
aaattcctga atcatatttt tttaacattt 30

<210> 299
<211> 30
<212> DNA
<213> Artificial

<220>
<223> Synthetic Nucleic Acid

<400> 299
tttgtggcta agacaattaa aaattcctga 30

<210> 300
<211> 30
<212> DNA
<213> Artificial

<220>
<223> Synthetic Nucleic Acid

<400> 300
gatcctggaa atcctataaa tttgtggcta 30

<210> 301
<211> 30
<212> DNA
<213> Artificial

<220>
<223> Synthetic Nucleic Acid

<400> 301
attatttatc cattgttaca gatcctggaa 30

<210> 302
<211> 30
<212> DNA
<213> Artificial

<220>
<223> Synthetic Nucleic Acid

<400> 302
aaatttagga tttttataaa attatttatc 30

<210> 303
<211> 30
<212> DNA
<213> Artificial

<220>
<223> Synthetic Nucleic Acid

<400> 303
atatctttat aattctgtgt aaatttagga 30

<210> 304
<211> 30
<212> DNA
<213> Artificial

<220>
<223> Synthetic Nucleic Acid

<400> 304
aggtgctgtt taaaatgatt atatctttat 30

<210> 305
<211> 30
<212> DNA
<213> Artificial

<220>
<223> Synthetic Nucleic Acid

<400> 305
atttataaaa tttgcaaaat aggtgctgtt 30

<210> 306
<211> 30
<212> DNA
<213> Artificial

<220>
<223> Synthetic Nucleic Acid

<400> 306
gagatgagtc ctagaaagag atttataaaa 30

<210> 307
<211> 30
<212> DNA
<213> Artificial

<220>
<223> Synthetic Nucleic Acid

<400> 307
ctgggttgat tgtagaagca gagatgagtc 30

<210> 308
<211> 30
<212> DNA
<213> Artificial

<220>
<223> Synthetic Nucleic Acid

<400> 308
cgcgcattta cagtcacatt ctgggttgat 30

<210> 309
<211> 30
<212> DNA
<213> Artificial

<220>
<223> Synthetic Nucleic Acid

<400> 309
ctttgggagg ctgcggcggg cagatcactt 30

<210> 310
<211> 30
<212> DNA
<213> Artificial

<220>
<223> Synthetic Nucleic Acid

<400> 310
tcacgcctgt aatcccagaa ctttgggagg 30

<210> 311
<211> 30
<212> DNA
<213> Artificial

<220>
<223> Synthetic Nucleic Acid

<400> 311
catttgctgg gagcagtggc tcacgcctgt 30

<210> 312
<211> 30
<212> DNA
<213> Artificial

<220>
<223> Synthetic Nucleic Acid

<400> 312
gaaacagagc agcaaacaca catttgctgg 30

<210> 313
<211> 30
<212> DNA
<213> Artificial

<220>
<223> Synthetic Nucleic Acid

<400> 313
acaggcaaga aacacgcagg gaaacagagc 30

<210> 314
<211> 30
<212> DNA
<213> Artificial

<220>
<223> Synthetic Nucleic Acid

<400> 314
gacacaaaga tacacacaag acaggcaaga 30

<210> 315
<211> 30
<212> DNA
<213> Artificial

<220>
<223> Synthetic Nucleic Acid

<400> 315
cacagggcag gatggcccca gacacaaaga 30

<210> 316
<211> 30
<212> DNA
<213> Artificial

<220>
<223> Synthetic Nucleic Acid

<400> 316
gagatggctt ggttgggcag cacagggcag 30

<210> 317

<211> 30
<212> DNA
<213> Artificial

<220>
<223> Synthetic Nucleic Acid

<400> 317
caggcgggca cctgtgggca gagatggctt 30

<210> 318
<211> 30
<212> DNA
<213> Artificial

<220>
<223> Synthetic Nucleic Acid

<400> 318
acaccgctca cccacccagc caggcgggca 30

<210> 319
<211> 30
<212> DNA
<213> Artificial

<220>
<223> Synthetic Nucleic Acid

<400> 319
tgacaggggt cctccagctg acaccgctca 30

<210> 320
<211> 30
<212> DNA
<213> Artificial

<220>
<223> Synthetic Nucleic Acid

<400> 320
ggccagcaca ggggcctgag tgacaggggt 30

<210> 321
<211> 30
<212> DNA
<213> Artificial

<220>
<223> Synthetic Nucleic Acid

<400> 321
tgaactctgg cagacaccac ggccagcaca 30

<210> 322
<211> 30
<212> DNA
<213> Artificial

<220>
<223> Synthetic Nucleic Acid

<400> 322
cgggcggtgc cagccaccac tgaactctgg 30

<210> 323
<211> 30
<212> DNA
<213> Artificial

<220>
<223> Synthetic Nucleic Acid

<400> 323
gggggcaccg gcatgagaat cgggcggtgc 30

<210> 324
<211> 30
<212> DNA
<213> Artificial

<220>
<223> Synthetic Nucleic Acid

<400> 324
cctctcacct cggaagccac gggggcaccg 30

<210> 325
<211> 30
<212> DNA
<213> Artificial

<220>
<223> Synthetic Nucleic Acid

<400> 325
cctcccctcc agactcttcc cctctcacct 30

<210> 326
<211> 30
<212> DNA
<213> Artificial

<220>
<223> Synthetic Nucleic Acid

<400> 326
actgaccaca cccccgacta cctcccctcc 30

<210> 327
<211> 30
<212> DNA
<213> Artificial

<220>
<223> Synthetic Nucleic Acid

<400> 327
caggacaggg tgagtttagg actgaccaca 30

<210> 328
<211> 30
<212> DNA
<213> Artificial

<220>
<223> Synthetic Nucleic Acid

<400> 328
cagtcagggc ctggagggac caggacaggg 30

<210> 329
<211> 30
<212> DNA
<213> Artificial

<220>
<223> Synthetic Nucleic Acid

<400> 329
ggcagggatc tggcagggag cagtcagggc 30

<210> 330
<211> 30
<212> DNA
<213> Artificial

<220>
<223> Synthetic Nucleic Acid

<400> 330
gtgggcacaa gggctgctga ggcagggatc 30

<210> 331
<211> 30
<212> DNA
<213> Artificial

<220>
<223> Synthetic Nucleic Acid

<400> 331
tgtggtgcta gtctgtggtc aacacagcat 30

<210> 332
<211> 30
<212> DNA
<213> Artificial

<220>
<223> Synthetic Nucleic Acid

<400> 332
caccccactc tgtggtgcta gtctgtggtc 30

<210> 333
<211> 30
<212> DNA
<213> Artificial

<220>
<223> Synthetic Nucleic Acid

<400> 333
cctcaccccc caccccactc tgtggtgcta 30

<210> 334
<211> 30
<212> DNA
<213> Artificial

<220>
<223> Synthetic Nucleic Acid

<400> 334
caggctgtcc cctcaccccc caccccactc 30

<210> 335
<211> 30
<212> DNA
<213> Artificial

<220>
<223> Synthetic Nucleic Acid

<400> 335
caaagcactt caggctgtcc cctcaccccc 30

<210> 336
<211> 30
<212> DNA
<213> Artificial

<220>
<223> Synthetic Nucleic Acid

<400> 336
agggaagggg gcaaggatgg tcaccgccgg 30

<210> 337
<211> 30
<212> DNA
<213> Artificial

<220>
<223> Synthetic Nucleic Acid

<400> 337
caaaccctag cagggaaggg ggcaaggatg 30

<210> 338

<211> 30
<212> DNA
<213> Artificial

<220>
<223> Synthetic Nucleic Acid

<400> 338
tctgaccctc aaaccctagc agggaagggg 30

<210> 339
<211> 30
<212> DNA
<213> Artificial

<220>
<223> Synthetic Nucleic Acid

<400> 339
acttcacaat tctgaccctc aaaccctagc 30

<210> 340
<211> 30
<212> DNA
<213> Artificial

<220>
<223> Synthetic Nucleic Acid

<400> 340
gtccacgttc acttcacaat tctgaccctc 30

<210> 341
<211> 619
<212> DNA
<213> Homo Sapiens

<400> 341
tcttgatggg atgctcctga aagcaattaa ttctcagttt tttgtggctt ctaatgcaaa 60

atacattgac gcagacagaa tttgaaatga attttcttct aatatagcaa ttaattttat 120

ttaaatatct ctagagtttt tttttaatac tgtgactaac ctatgtttgt tctttttcac 180

ctctcgtatc cacgatcact aagaaaccca aatactttgt tcatgtttaa attttacaac 240

atttcataga ctattaaaca tggaacatcc ttgtggggac aagaaatcga atttgctctt 300

gaaaaggttt ccaactaatt gatttgtagg acattataac atcctctagc tgacaagctt 360

acaaaaataa aaactggagc taaccgagag ggtgcttttt tccctgacac ataaaaggtg 420

tctttctgtc ttgtatcctt tggatatggg catgtcagtt tcatagggaa attttcacat 480

ggagcttttg tatttctttc tttgccagta caactgcatg tggtagcaca ctgtttaatc 540

ttttctcaaa taaaaagaca tggggcttca tttttgtttt gccttttttgg tatcttacag 600

gaactccagg atggcattg 619

<210> 342
<211> 425
<212> DNA
<213> Homo Sapiens

<400> 342
tcttgaataa aaaaaaaata agtaaaattt atttccctgg caaggtctga aaacttttgt 60

tttctttacc acttccacaa tgtatatgat tgttactgag aaggcttatt taacttaagt 120

tacttgtcca ggcatgagaa tgagcaaaat cgttttttaa aaaattgtta aatgtatatt 180

aatgaaaagg ttgaatcttt tcattttcta ccatgtattg ctaaacaaag tatccacatt 240

gttagaaaaa gatatataat gtcatgaata agagtttggc tcaaattgtt actcttcaat 300

taaatttgac ttattgttat tgaaattggc tctttagctt gtgtttctaa tttttctttt 360

tcttcttttt tccttttttgc aaaaacccaa aatattttag ctcctactca gactgttact 420

ctggt 425

<210> 343
<211> 424
<212> DNA
<213> Homo Sapiens

<400> 343
tttaaaataa atataccta attttgacgt cacacagaat gatattataa gtataaatag 60

ttatctatct tttaaataca ttgtcgtaat tcagaataac atttcttact caaggcattc 120

agacagtggt ttaagtaatc cgaggtactc cggaatgtct ccatttgagc ctttaaatga 180

agaaaatcta tagtcaagat tttcatttga aatatttttg atatctaaga atgaaacata 240

tttcctgtta aattgttttc tataaaccct tatacagtaa catctttttt atttctaaaa 300

gtgttttggc tggtctcaca attgtacttt actttgtatt atgtaaaagg aatacacaac 360

gctgaagaac cctgatacta agggatattt gttcttacag gcaacaatgc aggatttgga 420

acag 424

<210> 344
<211> 434
<212> DNA
<213> Homo Sapiens

<400> 344
caaactcctg tggcaacaga aagccttcag gcaatgaaat gctggcactg ggaaatcagg 60

ctgatgggtg ctgaagtggc aaggatgagg ggatatggat attctgctgt agtgcttttc 120

taacagatga ttcatatttg gttctaggga tcaagaattg agttaaaatt ttatatatat 180

gttgatgttc tatgtcacct tcaggaaaat aatttaacag aaactaatat ttgccatcaa 240

aaaagcaaag aatcctgttg ttcatcatcc tagccataac acaatgaata attttttaaa 300

taagcaacat aaatgtgaga taacgtttgg aagttacatt taaaatgtct cctccagact 360

agcatttact actatatatt tatttttcct tttattctag ttgaaagaat tcagaatcag 420

tgggatgaag taca 434

<210> 345
<211> 425
<212> DNA
<213> Homo Sapiens

<400> 345
tctcaaattt ggcagtatat taaaaataag ctttcaaaat tgaccaacaa aaactacaaa 60

attgaaaaaa aggtactttg aactttcaca tgttcaaata tatgtatata tatttcacat 120

atatatatga aacctcctct gtggagaggg gtttatagaa atctgtaatt gtcattcttg 180

catgccttcc cccatacaaa cgcctttaag ttaaataaaa atgaaagtaa atagactgca 240

caatattata gttgttgctt aaaggaagag ctgtagcaac aactcacccc attgttggta 300

tattacaatt tagttcctcc atctttctct ttttatggag ttcactaggt gcaccattct 360

gatatttaat aattgcatct gaacatttgg tcctttgcag ggtgagtgag cgagaggctg 420

ctttg 425

<210> 346
<211> 400
<212> DNA
<213> Homo Sapiens

<400> 346
gtaagtcttt gatttgtttt ttcgaaattg tatttatctt cagcacatct ggactcttta 60

acttcttaaa gatcaggttc tgaagggtga tggaaattac ttttgactgt tgttgtcatc 120

attatattac tagaaagaaa attatcataa tgataatatt agagcacggt gctatggact 180

ttttgtgtca ggatgagaga gtttgcctgg acggagctgg tttatctgat aaactgcaaa 240

atataattga atctgtgaca gagggaagca tcgtaacagc aaggtgtttt gtggctttgg 300

ggcagtgtgt atttcggctt tatgttggaa cctttccaga aggagaactt gtggcatact 360

tagctaaaat gaagttgcta gaaatatcca tcatgataaa 400

<210> 347
<211> 600
<212> DNA
<213> Homo Sapiens

<400> 347
tcttgatggg atgctcctga aagcaattaa ttctcagttt tttgtggctt ctaatgcaaa 60

atacattgac gcagacagaa tttgaaatga attttcttct aatatagcaa ttaattttat 120

ttaaatatct ctagagtttt tttttaatac tgtgactaac ctatgtttgt tctttttcac 180

ctctcgtatc cacgatcact aagaaaccca aatactttgt tcatgtttaa attttacaac 240

atttcataga ctattaaaca tggaacatcc ttgtggggac aagaaatcga atttgctctt 300

gaaaaggttt ccaactaatt gatttgtagg acattataac atcctctagc tgacaagctt 360

acaaaaataa aaactggagc taaccgagag ggtgcttttt tccctgacac ataaaaggtg 420

tctttctgtc ttgtatcctt tggatatggg catgtcagtt tcatagggaa attttcacat 480

ggagcttttg tatttctttc tttgccagta caactgcatg tggtagcaca ctgtttaatc 540

ttttctcaaa taaaaagaca tggggcttca tttttgtttt gccttttttgg tatcttacag 600

<210> 348
<211> 400
<212> DNA
<213> Homo Sapiens

<400> 348
gtagggcgac agatctaata ggaatgaaaa cattttagca gactttttaa gctttcttta 60

gaagaatatt tcatgagaga ttataagcag ggtgaaaggc actaacatta aagaacctat 120

caaccattaa tcaacagcag taaagaaatt ttttatttct ttttttcata tactaaaata 180

tatacttgtg gctagttagt ggttttctgc tattttaaac ttgaagtttg ctttaaaaat 240

cacccatgat tgcttaaagg tgaatatctt caatatattt taacttcaac aagctgaatc 300

tcagttgttt ttcaagaaga ttttagaaag caattataaa tgattgtttt gtaggaaaga 360

cagatctttg cttagtttta aaaatagcta tgaatatgac 400

<210> 349
<211> 400
<212> DNA
<213> Homo Sapiens

<400> 349
attacaggcg cctgccacca aacctggcaa atttttgtat ttttagtgta gacggggttt 60

caccatattt gccaggctgg tcgcaaactc ctgacctcaa gtgatccgcc cacatcggcc 120

tccctaagcg ctagggttac aggcatgagc cactgcgcct ggccaggaat ttttgaatca 180

gaatttttct tgttcgattt taatctctta tcatttagag attcttgaaa tattgaaatt 240

actttgttca aagtgaatga attttcttaa attatgtatg gttaacatct tttaaattgc 300

ttatttttaa attgccatgt ttgtgtccca gtttgcatta acaaatagtt tgagaactat 360

gttggaaaaa aaaataacaa ttttattctt ctttctccag 400

<210> 350
<211> 400
<212> DNA
<213> Homo Sapiens

<400> 350

gtaattttat tttctcaaat cccccagggc ctgcttgcat aaagaagtat atgaatctat 60

tttttaattc aatcattggt tttctgccca ttaggttatt catagttcct tgctaaagtg 120

tttttctcac aactttattt cttcttaacc ctgcagttct gaaccagtgc acataagaac 180

atatgtatat atgtgtgtgt gtgtatttat atatacacac acacatattg catctataca 240

tctacacata tagatgtata gattcaatat gtctaaatgt atataattca cagtttttat 300

ctttgatttg aaattaattt tagattttac ttgagaactt cacaacttca tataatttta 360

aaaactgaag accagattgt ggaatcataa aatctaaatc 400

<210> 351
<211> 400
<212> DNA
<213> Homo Sapiens

<400> 351
ataatgcaat ttctcagcag caagctacgg tatgctatgg catgctatga tacccaagag 60

gctgatgaat ttgttcacat tgttctattt ctgatagaga gataggtttt cagacactaa 120

ctttatttgg agtgttgctt taccatctca catttttctc ttaaaaaatt tatgagggat 180

aatataatcg tttattttct acagagattt atctactgag ggggtgagtg tttcagtcaa 240

tcagctctgt gctcagatag aaaactgttg gtatttgagg taccactggg ccctcggtca 300

agtcgcttca ttttgataga ctaatcaata gaagcaaaga caaggtagtt ggaattgtgc 360

tgtaattcat tttaaacgtt gttgcatttg tctgtttcag 400

<210> 352
<211> 400
<212> DNA
<213> Homo Sapiens

<400> 352
gttagacatt aaccatctct tccgtcacat gtgttaaatg ttgcaagtat ttgtatgtat 60

tttgtttcct gggtgcttca ttggtcgggg aggaggctgg tatgtggatt gttgttttgt 120

tttgtttttt taacctgacc gtttgctttg gctatatgtt ttgttgtggc tagaaaaaat 180

gatgatggtg aatggcttta cattaatgac caaatgccaa aatttatacc acaatttttt 240

gcataaatta ttctgaagaa tcagactgaa gaaatggcga agtatttaat tcagtggcca 300

ggcatgtact gacagtattt aagctgaaag gacgtggtct ggttctagtt aaacaagtgt 360

cataaatcaa aattaattat tcacacctgt ggtatggact 400

<210> 353
<211> 400
<212> DNA
<213> Homo Sapiens

<400> 353

tattcatttt tataactgca aaggaagcgc gtatggcata taatacacaa cacaccagta 60

tatttagtaa ctgagtgaat aaatgaaaga tgtatttctt tactttatca gttgcagttg 120

gctatgcctt tgtgtaaggt gtgtgttttg aaattccaaa aaggtattag tttctttaaa 180

gcaaagaatt tttgtagcag gttaatgaat aattttgaat acattggtta aatcccaaca 240

tgtaatatat gtaaataatc aatattatgc tgctaaaata acacaaatca gtaagattct 300

gtaatatttc atgataaata acttttgaaa atatattttt aaacattttg gcttatgcct 360

tgagaattat ttaccttttt aaaatgtatt ttcctttcag 400

<210> 354
<211> 400
<212> DNA
<213> Homo Sapiens

<400> 354
gtagggaact ttttgcttta aatatttttg tcttttttaa gaaaaatggc aatatcactg 60

aattttctca tttggtatca ttattaaaga caaaatatta cttgttaaag tgtggtaagg 120

aagactttat tcaggataac cacaataggc acagggacca ctgcaatgga gtattacagg 180

aggttggata gagagagatt gggctcaact ctaaatacag cacagtggaa gtaggaattt 240

atagccaagg agcagtgtag gagtcagtag atggaaaatt attaagagga aacatcaggg 300

gtaagtggga ttctggctaa accaacctca caggattctt gctgaagata ggccagggtt 360

atcttatcag acaacccttg gggaatggtg gagaatactg 400

<210> 355
<211> 400
<212> DNA
<213> Homo Sapiens

<400> 355
tactaaacac agaattttgt aaaacaataa gtgtataaag taaaatgaac attaggatta 60

ttgagattat tgtagctaaa actagtgttt attcatataa attatgttaa taaattgtat 120

tgtcattatt gcattttact tttttgaaaa gtagttaatg cctgtgtttc tatatgagta 180

ttatataatt caagaagata ttggatgaat ttttttaaag tttaatgtgt ttcacatctc 240

tgtttctttt ctctgcacca aaagctacat ttttgtgccc ttatgtacca ggcagaaatt 300

gatctgcaat acatgtggag tctccaaggg tatatttaaa tttagtaatt ttattgctaa 360

ctgtgaagtt aatctgcact atatgggttc ttttccccag 400

<210> 356
<211> 400
<212> DNA
<213> Homo Sapiens

<400> 356

gtaatgaagc aacctctagc aatatccatt acctcataat gggttatgct tcccctgttg 60

tacatttgcc attgacgtgg actatttata atcagtgaaa taacttgtaa ggaaatactg 120

gccatactgt aatagcagag gcaaagctgt ctttttgatc agcatatcct atttatatat 180

tgtgatctta aggctattaa cgagtcattg ctttaaagga ctcatttctg tcctggtgtg 240

ctgccatcaa tacaaaagta gtcccacctt caaggtagat taaattcttt ggggctttat 300

tgctttgctt gccagccttg atgcttttca tattgtttgg tttaattcaa atcaagctac 360

tgcatcatag tgtctgtctc caacagctgt aaagaatcac 400

<210> 357
<211> 400
<212> DNA
<213> Homo Sapiens

<400> 357
atataattga ctgggggtga gccagtacat taggattttc ctaaagttat ctggataatt 60

ttagtatgca accacaatag atactcttca agaattaagc tagttgctga gagggaactg 120

ttttttgttg gtttgttttc actaatgttt gcactctact tcctttaaat aaaattatgc 180

ctggagaaag ggtttttgta tggagcaatt gataaatatt tgtagggtgg ttggctaaaa 240

taattataat tcctttaaaa gaaattctac ccactaaagt taatttagaa gtaaaatata 300

atagaaatcc aataatatat tcaccaaatg gattaagatg ttcatgaatt atcttcaaag 360

tgttaatcga ataagtaatg tgtatgcttt tctgttaaag 400

<210> 358
<211> 400
<212> DNA
<213> Homo Sapiens

<400> 358
gtaagtatac tggatcccat tctctttggc tctagctatt tgttcaaaag tgcaactatg 60

aagtgatgac tgggtgagag agaaaatttg tttcaattct aaagatagag ataaaccttt 120

gtgttattga ctgtgcaaaa agtcttagag tacattcctt ggaaattgac tctgattcaa 180

agtgttgcat gacaacggga tatggggagt gttctctgga gatacaccca caaggaagag 240

aagagcacaa gggagattgt gggagagtct gaaatgtgat ttgtctgcag cagaggccta 300

agccagtctc gcaggagccc tacatctggg ctggctgtgc agagctgtcc tgaattgcag 360

gcagtgggcc tggcccttgt attcctgatc cagccagcca 400

<210> 359
<211> 400
<212> DNA
<213> Homo Sapiens

<400> 359

tcttgaataa aaaaaaaata agtaaaattt atttccctgg caaggtctga aacttttgt 60

tttctttacc acttccacaa tgtatatgat tgttactgag aaggcttatt taacttaagt 120

tacttgtcca ggcatgagaa tgagcaaaat cgttttttaa aaaattgtta aatgtatatt 180

aatgaaaagg ttgaatcttt tcattttcta ccatgtattg ctaaacaaag tatccacatt 240

gttagaaaaa gatatataat gtcatgaata agagtttggc tcaaattgtt actcttcaat 300

taaatttgac ttattgttat tgaaattggc tctttagctt gtgtttctaa tttttctttt 360

tcttcttttt tcctttttgc aaaaacccaa aatattttag 400

<210> 360
<211> 400
<212> DNA
<213> Homo Sapiens

<400> 360
gtatgagaaa aaatgataaa agttggcaga agtttttctt taaaatgaag attttccacc 60

aatcacttta ctctcctaga ccatttccca ccagttctta ggcaactgtt tctctctcag 120

caaacacatt actctcacta ttcagcctaa gtataatcaa ggatataaat taatgcaaat 180

aacaaaagta gccatacatt aaaaaggaaa tatacaaaaa aaaaaaaaaa aaaaagcaga 240

aaccttacaa gaatagttgt ctcagttaaa tttactaaac aacctggtat tttaaaaatc 300

tattttatac caaataagtc actcaactga gctatttaca tttaaactgt ttgttttggc 360

actacgcagc ccaacatatt gcagaatcaa atataatagt 400

<210> 361
<211> 400
<212> DNA
<213> Homo Sapiens

<400> 361
tttaaaataa atataccttta attttgacgt cacacagaat gatattataa gtataaatag 60

ttatctatct tttaaataca ttgtcgtaat tcagaataac atttcttact caaggcattc 120

agacagtggt ttaagtaatc cgaggtactc cggaatgtct ccatttgagc ctttaaatga 180

agaaaatcta tagtcaagat tttcatttga aatattttttg atatctaaga atgaaacata 240

tttcctgtta aattgttttc tataaaccct tatacagtaa catctttttt atttctaaaa 300

gtgttttggc tggtctcaca attgtacttt actttgtatt atgtaaaagg aatacacaac 360

gctgaagaac cctgatacta agggatattt gttcttacag 400

<210> 362
<211> 400
<212> DNA
<213> Homo Sapiens

<400> 362

gtaagttttt taacaagcat gggacacaca aagcaagatg catgacaagt ttcaataaaa 60

acttaagttc atatatcccc ctcacattta taaaaataat gtgaaataat tgtaaatgat 120

aacaattgtg ctgagatttt cagtccataa tgttaccttt taataaatga atgtaattcc 180

attgaataga agaaatacat ttttaaatca attcagggct tatatagttg caaagcatgc 240

attgatgggt gtggtgacca cagtgtggca gaacatttgt ggcagaacat ttgttcttta 300

gttgtcatct gggctggcat ccatggagat gccagtctct ccctcatatc cttggctgtt 360

ggtccaagca ggcagtggct tcttcctggg ccatctttca 400

<210> 363
<211> 400
<212> DNA
<213> Homo Sapiens

<400> 363
caaactcctg tggcaacaga aagccttcag gcaatgaaat gctggcactg ggaaatcagg 60

ctgatgggtg ctgaagtggc aaggatgagg ggatatggat attctgctgt agtgcttttc 120

taacagatga ttcatatttg gttctaggga tcaagaattg agttaaaatt ttatatatat 180

gttgatgttc tatgtcacct tcaggaaaat aatttaacag aaactaatat ttgccatcaa 240

aaaagcaaag aatcctgttg ttcatcatcc tagccataac acaatgaata atttttttaaa 300

taagcaacat aaatgtgaga taacgtttgg aagttacatt taaaatgtct cctccagact 360

agcatttact actatatatt tatttttcct tttattctag 400

<210> 364
<211> 400
<212> DNA
<213> Homo Sapiens

<400> 364
gttagtatca aagatacctt tttaaaataa aatactggtt acatttgata aaattatacc 60

atagattgta atttaatgat gtttaatgta aagttattaa cagaaaatca cgttaaagct 120

gaaatgaaca gtagactttg tatatttatt ttcttagaga cagagtctca ctgtcaccca 180

ggctaaagtg cagtggcaca atcatagctc actgagcctt gaactctggg gctcaagcag 240

tcctcctgcc tcagcctccc tagtagctgg gactactagc caggcgtgta ccaccacgcc 300

tggctaattt tttaaaaatt tttgttttct gtagagatgg gttcttgaac tcttggcctc 360

aagcaattct ccttccttgg cctcccaaag cactaggatt 400

<210> 365
<211> 400
<212> DNA
<213> Homo Sapiens

<400> 365

caacaagtta atgaagaggg aaagaaatgt atgaggtttt tttcgttcaa atgttgttat 60

atgtcacata ttcaacaatt atatatgagc ttatttttgt agttttttc tcttgtgata 120

aaaacaatta agcccacttt attgccaatt aattgctact aagttgaaat acttgatact 180

ggttattgct caagatgctg catttgaaaa gtttgtcctg aaaggtgggt taccttatac 240

tgtcatgatt gactaaatca tatggtaggt taaaagcaat ctaatatatg tattctgacc 300

tgaggattca gaagctgttt acgaagtatt ttaagacact ccaactagag atttcataaa 360

aaaaactgac attcattctc tttctcataa aaatctatag 400

<210> 366
<211> 400
<212> DNA
<213> Homo Sapiens

<400> 366
gtatgaatta cattatttct aaaactactg ttggctgtaa taatggggtg gtgaaactgg 60

atggaccatg aggatttgtt tttccaatcc agctaaactg gagcttggga gggttcaaga 120

cgataaatac caactaaact cacggacttg gctcagactt ctattttaaa aacgaggaac 180

ataagatctc atttgcccgc tgtcacaaaa gtagtgacat aaccaagaga ttaaacaaaa 240

agcaaaatac tgatttatag ctagaagagc catttatcag tctactttga taactctatc 300

caaaggaata tctttctatc tcatcatggc gcacactgcc ttacctgtta tctgataaat 360

aagtcacttt gggattcatg atagagttat agctgtacat 400

<210> 367
<211> 400
<212> DNA
<213> Homo Sapiens

<400> 367
tctcaaattt ggcagtatat taaaaataag ctttcaaaat tgaccaacaa aaactacaaa 60

attgaaaaaa aggtactttg aactttcaca tgttcaaata tatgtatata tatttcacat 120

atatatatga aacctcctct gtggagaggg gtttatagaa atctgtaatt gtcattcttg 180

catgccttcc cccatacaaa cgcctttaag ttaaataaaa atgaaagtaa atagactgca 240

caatattata gttgttgctt aaaggaagag ctgtagcaac aactcacccc attgttggta 300

tattacaatt tagttcctcc atctttctct ttttatggag ttcactaggt gcaccattct 360

gatatttaat aattgcatct gaacatttgg tcctttgcag 400

<210> 368
<211> 400
<212> DNA
<213> Homo Sapiens

<400> 368

gtaagtcagg catttccgct ttagcactct tgtggatcca attgaacaat tctcagcatt 60

tgtacttgta actgacaagc cagggacaaa acaaaatagt tgcttttata cagcctgatg 120

tatttcggta tttggacaag gaggagagag gcagagggag aaggaaacat catttataat 180

tccacttaac accctcgtct tagaaaaagt acatgctctg accaggaaaa catttgcata 240

taaaaccaga gcttcggtca aggagaaact ttgctcagag aaataactta gggattggtt 300

tattaaattt taaaagttga catttttgag tgtttattta atattttaca gggaaagcat 360

ctgtatgaat tgtctgtttt atttagcgtt gctaactgaa 400

<210> 369
<211> 400
<212> DNA
<213> Homo Sapiens

<400> 369
acttgatcca tatagtaatg aaattattgg cactggggta cactttatca tagaatttta 60

ttgcctatca cttccataaa ataatacatt ttgtccatag actagaagat ataacttgtg 120

aactttataa agttataaat acattacttt ccaactcata atggcaagga ataaatctat 180

tacaactaat aagatgccca ttttaaatct acataataac aggagaaggc aatacgccaa 240

gaaaagggat ttgagatgta tcttcttgtt agtttagcct gattgaaatg tcttttgaac 300

taataattat ttatattttg caattctcca aattcacatt catcgcttgt ttcttttgtt 360

tggtaattct gcacatattc ttcttcctgc tgtcctgtag 400

<210> 370
<211> 5
<212> RNA
<213> Homo Sapiens

<400> 370
uagga 5

<210> 371
<211> 6
<212> RNA
<213> Homo Sapiens

<400> 371
uaggau 6

<210> 372
<211> 6
<212> RNA
<213> Homo Sapiens

<400> 372
uaggaa 6

<210> 373
<211> 6
<212> RNA
<213> Homo Sapiens

<400> 373
uaggca 6

<210> 374
<211> 6
<212> RNA
<213> Homo Sapiens

<400> 374
uaggcu 6

<210> 375
<211> 6
<212> RNA
<213> Homo Sapiens

<400> 375
uaggga 6

<210> 376
<211> 6
<212> RNA
<213> Homo Sapiens

<400> 376
uagggc 6

<210> 377
<211> 6
<212> RNA
<213> Homo Sapiens

<400> 377
uagggu 6

<210> 378
<211> 8
<212> RNA
<213> Homo Sapiens

<400> 378
gguagggc 8

<210> 379
<211> 5
<212> RNA
<213> Homo Sapiens

<400> 379
agaac 5

<210> 380
<211> 6
<212> RNA
<213> Homo Sapiens

<400> 380
agcaug 6

<210> 381
<211> 6
<212> RNA
<213> Homo Sapiens

<400> 381
ugacug 6

<210> 382
<211> 6
<212> RNA
<213> Homo Sapiens

<400> 382
ugcaug 6

<210> 383
<211> 5
<212> RNA
<213> Homo Sapiens

<220>
<221> misc_feature
<222> (3)..(3)
<223> n is a, c, g, or u

<400> 383
yunay 5

<210> 384
<211> 2
<212> RNA
<213> Homo Sapiens

<400> 384
gu 2

<210> 385
<211> 437
<212> DNA
<213> Homo Sapiens

<400> 385
ataatgcaat ttctcagcag caagctacgg tatgctatgg catgctatga tacccaagag 60

gctgatgaat ttgttcacat tgttctattt ctgatagaga gataggtttt cagacactaa 120

ctttatttgg agtgttgctt taccatctca catttttctc ttaaaaaatt tatgagggat 180

aatataatcg tttattttct acagagattt atctactgag ggggtgagtg tttcagtcaa 240

tcagctctgt gctcagatag aaaactgttg gtatttgagg taccactggg ccctcggtca 300

agtcgcttca ttttgataga ctaatcaata gaagcaaaga caaggtagtt ggaattgtgc 360

tgtaattcat tttaaacgtt gttgcatttg tctgtttcag ttactggtgg aagagttgcc 420

cctgcgccag ggaattc 437

<210> 386
<211> 419
<212> DNA
<213> Homo Sapiens

<400> 386
tattcatttt tataactgca aaggaagcgc gtatggcata taatacacaa cacaccagta 60

tatttagtaa ctgagtgaat aaatgaaaga tgtatttctt tactttatca gttgcagttg 120

gctatgcctt tgtgtaaggt gtgtgttttg aaattccaaa aaggtattag tttctttaaa 180

gcaaagaatt tttgtagcag gttaatgaat aattttgaat acattggtta aatcccaaca 240

tgtaatatat gtaaataatc aatattatgc tgctaaaata acacaaatca gtaagattct 300

gtaatatttc atgataaata acttttgaaa atatattttt aaacattttg gcttatgcct 360

tgagaattat ttaccttttt aaaatgtatt ttcctttcag gtttccagag ctttacctg 419

<210> 387
<211> 442
<212> DNA
<213> Homo Sapiens

<400> 387
tactaaacac agaattttgt aaaacaataa gtgtataaag taaaatgaac attaggatta 60

ttgagattat tgtagctaaa actagtgttt attcatataa attatgttaa taaattgtat 120

tgtcattatt gcattttact tttttgaaaa gtagttaatg cctgtgtttc tatatgagta 180

ttatataatt caagaagata ttggatgaat ttttttaag tttaatgtgt ttcacatctc 240

tgtttctttt ctctgcacca aaagctacat ttttgtgccc ttatgtacca ggcagaaatt 300

gatctgcaat acatgtggag tctccaaggg tatatttaaa tttagtaatt ttattgctaa 360

ctgtgaagtt aatctgcact atatgggttc ttttccccag gaaactgaaa tagcagttca 420

agctaaacaa ccggatgtgg aa 442

<210> 388
<211> 444
<212> DNA
<213> Homo Sapiens

<400> 388
atataattga ctgggggtga gccagtacat taggattttc ctaaagttat ctggataatt 60

```
ttagtatgca accacaatag atactcttca agaattaagc tagttgctga gagggaactg      120

tttttgttg gtttgttttc actaatgttt gcactctact tcctttaaat aaaattatgc      180

ctggagaaag ggtttttgta tggagcaatt gataaatatt tgtagggtgg ttggctaaaa      240

taattataat tcctttaaaa gaaattctac ccactaaagt taatttagaa gtaaaatata      300

atagaaatcc aataatatat tcaccaaatg gattaagatg ttcatgaatt atcttcaaag      360

tgttaatcga ataagtaatg tgtatgcttt tctgttaaag aggaagttag aagatctgag      420

ctctgagtgg aaggcggtaa accg                                            444
```

```
<210>  389
<211>  443
<212>  DNA
<213>  Homo Sapiens

<400>  389
caacaagtta atgaagaggg aaagaaatgt atgaggtttt tttcgttcaa atgttgttat       60

atgtcacata ttcaacaatt atatatgagc ttatttttgt agtttttttc tcttgtgata      120

aaaacaatta agcccacttt attgccaatt aattgctact aagttgaaat acttgatact      180

ggttattgct caagatgctg catttgaaaa gtttgtcctg aaaggtgggt taccttatac      240

tgtcatgatt gactaaatca tatggtaggt taaaagcaat ctaatatatg tattctgacc      300

tgaggattca gaagctgttt acgaagtatt ttaagacact ccaactagag atttcataaa      360

aaaaactgac attcattctc tttctcataa aaatctatag cagttggcca aagacctccg      420

ccagtggcag acaaatgtag atg                                             443
```

```
<210>  390
<211>  2
<212>  RNA
<213>  Homo Sapiens

<400>  390
ag                                                                      2
```